# EUROPEAN PATENT SPECIFICATION

(11) **EP 0 110 955 B1**
(45) Date of publication and mention of the grant of the patent: **23.08.1995**
(21) Application number: 83902034.4
(22) Date of filing: 12.05.1983
(51) Int. Cl.: A61K 9/22, C07D 213/82, C07D 213/80, C07D 211/90, C07J 43/00

(54) **BRAIN-SPECIFIC DRUG DELIVERY**
GEHIRNSPEZIFISCHE ARZNEIMITTELABGABE
ADMINISTRATION D'UN MEDICAMENT A ACTION SPECIFIQUE SUR LE CERVEAU

(30) Priority: 18.05.1982 US 379316; 27.01.1983 US 461543; 15.03.1983 US 475493
(43) Date of publication of application: 20.06.1984
(60) Divisional application: 86201714.2
(73) Proprietor: UNIVERSITY OF FLORIDA, Gainesville, Florida 32611-2037 (US)
(72) Inventor: BODOR, Nicholas S., Gainesville, FL 32608 (US)
(74) Representative: Pendlebury, Anthony
(86) International application number: US8300725
(87) International publication number: WO8303968

(56) References cited:
- US-A- 3 962 447
- DRUGS OF THE FUTURE, VOL. VI, NO. 3, 1981, PAGES 165-182
- OPTIMIZATION OF DRUG DELIVERY, ED. H. BUNDGAARD, A.B.HANSEN AND H. KOFOD, PUB. MUNKSGAARD, COPENHAGEN, 1982, PAGES 157-177
- DRUG DELIVERY SYSTEMS, ED. R.L.JULIANO, OXFORD UNIVERSITY PRESS, 1980, PAGES 112-176
- CHEMISTRY AND INDUSTRY, 7TH JUNE, 1980, PAGES 441-443
- DESIGN OF BIOPHARMACEUTICAL PROPERTIES THROUGH PRODRUGS AND ANALOGS, ED. E.B.ROCHE, AMERICAN PHARMACEUTICAL ASSOCIATION, 1977, PAGES 98-135
- SCIENCE, vol. 214, no. 4527, 1981, Wahsington D.C., USA; N. BODOR et al.: "Site specific, sustained release of drugs to the brain", pages 1370-1372.
- JOURNAL OF PHARMACEUTICAL SCIENCES, vol. 67, no. 5, May 1978, Washington D.C., USA; N. BODOR et al.: "Elimination of a quaternary pyridinium salt, delivered as its dihydropyridine derivative from brain of mice", pages 685-687.
- CHEMICAL AND ENGINEERING NEWS, 21 December 1981, Washington D.C., USA; R. RAWLS: "New methods let drugs past blood-brain barrier", pages 24,25.
- JOURNAL OF MEDICINAL CHEMISTRY, vol. 26, April 1983, Columbus, Ohio, USA; N. BODOR: "Improved delivery through biological membranes. 13. Brain specific delivery of dopamine with a dihydropyridine-pyridinium salt redox type delivery system", pages 528-534.
- JOURNAL OF MEDICINAL CHEMISTRY, vol. 26, March 1983, Columbus, Ohio, USA; N. BODOR et al.: "Improved delivery through biological membranes. 11. A redox chemical drug-delivery system and its use for brain-specific delivery of phenekylamine", pages 313-317.

## Description

### Field of the Invention:

The present invention relates to a dihydropyridine/pyridinium salt type of redox system for the site-specific or sustained delivery (or both) of a wide variety of drug species to the brain. More especially, this invention relates to the discovery that a biologically active compound coupled to a lipoidal carrier moiety comprising a dihydropyridine nucleus readily and easily penetrates the blood-brain barrier ("BBB") and attains increased levels of concentration in the brain; oxidation of the dihydropyridine carrier moiety in vivo to the ionic pyridinium salts prevents its elimination from the brain, while elimination from the general circulation is accelerated, resulting in significant and prolongedly sustained brain-specific drug activity, whether ascribable to the cleavage of the [D-QC]⁺ entity and sustained release of the drug in the brain and/or to [D-QC]⁺ itself.

### Background Art:

The delivery of drug species to the brain is ofttimes seriously limited by transport and metabolism factors and, more specifically, by the functional barrier of the endothelial brain capillary wall deemed the blood-brain barrier, BBB. Site-specific delivery and sustained delivery of drugs to the brain are even more difficult, and to date no useful simple or generic techniques to achieve such phenomena are known to the art.

Indeed, the barriers separating plasma from the brain and cerebrospinal fluid (CSF) are complex systems involving passive and active transport and subserve a number of important functions The boundary between plasma and the central nervous system (CNS) is much less permeable than that between plasma and other tissue cells to a variety of water soluble substances, such as organic electrolytes, organic acids and bases, as well as to large molecules such as proteins. Such a barrier also provides a path for clearance from the brain of the breakdown products of cellular metabolism. The CNS and its fluids can be considered basically a three-compartment system: the blood or the plasma, CSF and brain tissue. There is a diffusion- controlled exchange between CSF and the extracellular fluid (CF) of the brain. It has also been suggested that the permeabilities of blood-CSF and blood-brain barriers are practically identical with respect to drugs and other foreign substances. Mayer et al, J. Pharmacol. and Exp. Therap., 125, 185 (1959).

The BBB is, moreover, basically the result of the fact that the endothelial cells in the brain capillaries are joined by continuous, tight intercellular junctions, such that material has to pass through the cells rather than between them in order to move from blood to brain It is interesting that there are areas within the brain, such as the sub-fornical body and the postremia in which the capillary cells are not closely linked so that they lack the characteristics of the BBB. They provide the entry of small amounts of compounds which would not ordinarily enter the barriers. Hoffmann and Olszewzki, Neurology (Minneap.), 11, 1081 (1961).

Foreign compounds which enter organs other than the central nervous system with ease, may penetrate the CNS slowly or hardly at all. A number of theories concerning the nature of the barrier have been proposed The widely accepted concept describes the boundary as a fat-like layer interspersed with small pores, although the BBB is not a simple, anatomically well-defined unitary physical entity. Shuttleworth, Prog. Exp. Tumor Res., 17, 279 (1972). Penetration of such a barrier may occur by several processes: lipid soluble substances may passively penetrate into the cells, while small molecules such as water and urea may pass through the pores. In addition to these simple physical processes, carriermediated and active transport processes govern the movement of many molecules through the BBB. Thus, it is generally accepted that lipid solubility, degree of ionic dissociation or protonation and the ability of temporary combination with membrane constituents affect delivery through the BBB. It has been shown, for example, that in the class of barbiturates, a quantitative correlation could be established between their ease to pass into the brain (as reflected by the different times of onset of anesthetic action) and their lipid/water partition coefficient. Mark et al, J. Pharmacol. and Exp. Therap., 123, 79 (1957). The role of lipid solubility in drug penetration through the BBB is also exemplified by the better absorption of the sparingly water-soluble thiamine propyl disulfide (TPD) as compared to the water-soluble thiamine hydrochloride (THCI). Thomson et al, Ann. Int. Med., 74, 529 (1971). Some materials such as glucose and amino acids are transported by active mechanism, characterized by saturation, bidirectional molecular specificity, bidirectional competitive inhibition and bidirectional countertransport. Fishman, Am J. Physiol., 206, 836 (1964).

Changes in permeability of the BBB can be caused by several pathological and toxicological processes. Pardridge, Connor and Crawford, CRC Crit. Rev. Toxicol., 179 (1975). A general increase in the barrier permeability, such as a nonspecific breakdown of the barrier has, however, severe consequences, including cerebral edema.

It too is well documented that the BBB is relatively impermeable to the ionized forms of drugs and other molecules. Drugs which are weak organic electrolytes appear to pass from blood to CSF to reach a steady state ratio characteristic of each molecule according to its pKₐ and the existence of a normal pH gradient between blood and CSF. It is clear that it is the most difficult for quaternary pyridinium or ammonium salts to penetrate the BBB.

And removal of substances from the brain and CSF is obviously a significant factor in regulating drug concentrations in the CNS. There are several efflux processes: bulk flow via the arachnoid villi, diffusion of lipid soluble substances into brain and blood, active transport and metabolism by adjacent meninges. Once a drug or metabolite enters the CSF from blood or brain by simple diffusion, it may rapidly be removed, either by nonselective bulk flow or by active transport mechanism associated with the choroid plexus or other nondefined structures in the CSF compartment. It is generally accepted that highly lipid-soluble drugs leave the CSF more rapidly than poorly lipid-soluble ones, but the barrier to passage of compounds from CSF has only superficial similarity to the blood-CSF barrier.

Drug elimination processes from the brain are significantly directly related to drug accumulation in the brain. It is generally assumed that efflux in the opposite direction involves almost the same processes as for entry, except that the role of the bulk flow and the metabolic processes in the brain are not to be overlooked.

The two elimination processes studied in the earlier literature and which can be said to have a certain bearing on the present invention involve elimination from the brain of ionic species. Thus, it is found that non-metabolized inonic species, such as the acetate ion, have a three times slower elimination rate from the CSF than from the blood. Freundt, Arz., Forsch., 23, 949 (1973). An even more dramatic change in the elimination rate was found in the case of a quaternary piperidinium salt. The quaternary salt, formed in situ after delivery of a haloalkylamine, which undergoes cyclization to the quaternary salt, in the brain, as well, was found to have an at least ten times slower elimination rate from the brain than from the rest of the body. It was concluded by the authors (Ross and Froden, Eur. J. Pharmacol., 13, 46 [1970] that the outflow rate of the quaternary salt corresponded to the inflow rate. Similar results were obtained for the erythrocytes: the efflux of the quaternary salt was very slow. Ross, J. Pharm. Pharmacol., 27, 322 (1975).

And while it too has been suggested to deliver a drug species, specifically N-methylpyridinium-2-carbaldoxime chloride (2-PAM), into the brain, the active nucleus of which in and of itself constituting a quaternary pyridinium salt, by way of the dihydropyridine latentiated prodrug form thereof, such approach is conspicuously delimited to relatively small molecule quaternary pyridinium ring-containing drug species and does not provide the overall ideal result of brain-specific, sustained release of the desired drug, with concomitant rapid elimination from the general circulation, enhanced drug, efficacy and decreased toxicity. Hence, no "trapping" in the brain of the 2-PAM formed in situ results, and obviously no brain-specific, sustained delivery occurs as any consequence thereof: the 2-PAM is eliminated as fast from the brain as it is from the general circulation and other organs. Compare U.S. Patents Nos. 3,929,813 and 3,962,447; Bodor et al, J. Pharm. Sci., 67, No. 5, 685 (1978). It has also been speculated to deliver, e.g., an antitumor agent into the brain by utilizing a dihydropyridine/pyridinium redox carrier moiety therefor, but this particular hypothesis necessarily entails derivatizing the dihydropyridine/pyridinium carrier with a substituent R itself critically designed to control the release rate of the active drug species from the quaternary drivative thereof, as well as being critically functionally coordinated with the particular chemical and therapeutic activity/nature of the antitumor drug species itself; Bodor et al, J. Pharm. Sci., supra.

Accordingly, acutely serious need exists in this art for a truly effective generic but nonetheless flexible method for the site-specific, or sustained delivery, or both, of drug species to the brain, while at the same time avoiding the aforesaid noted and notable disadvantages and drawbacks associated with penetration of the blood-brain barrier, with dihydropyridine latentiated prodrug forms of drug species themselves comprising a pyridinium salt active nucleus, and with the necessity for introducing critically coordinated and designed, release rate-controlling substituents onto any particular drug carrier moiety.

Recently, Bodor et al, Science, Vol. 214, December 18, 1981, pp. 1370-1372, have reported on site-specific sustained release of drugs to the brain. The Science publication outlines a scheme for specific and sustained delivery of drug species to the brain, as depicted in the following Scheme 1:

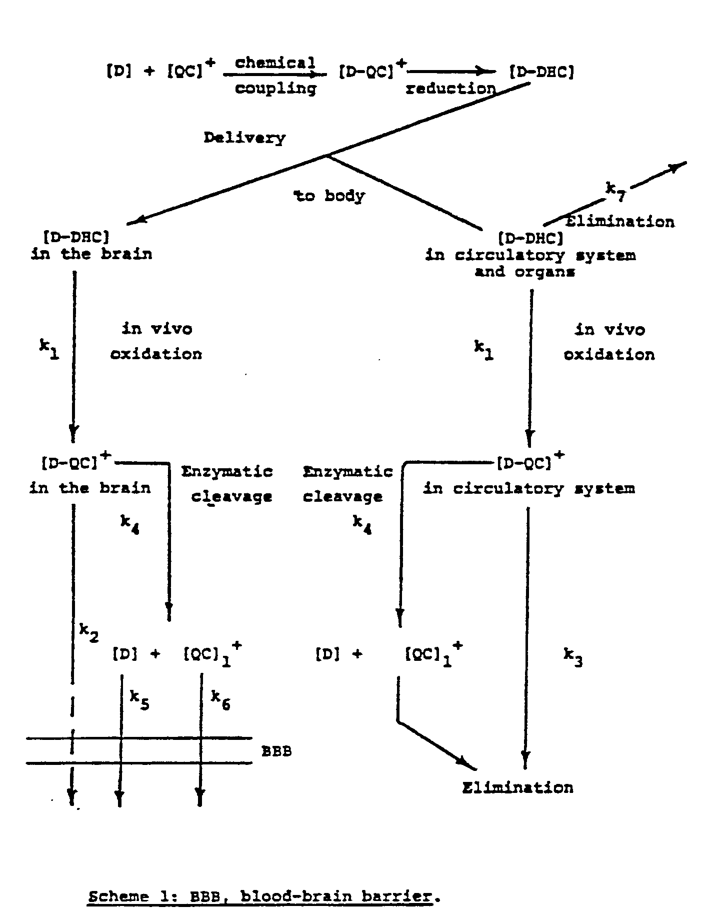

According to the scheme in Science, a drug [D] is coupled to a quaternary carrier [QC]⁺ and the [D-QC]⁺ which results is then reduced chemically to the lipoidal dihydro form [D-DHC]. After administration of [D-DHC] in vivo, it is rapidly distributed throughout the body, including the brain. The dihydro form [D-DHC] is then in situ oxidized (rate constant, kᵢ) (by the NAD 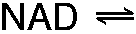 NADH system) to the ideally inactive original [D-QC]⁺ quaternary salt which, because of its ionic, hydrophilic character, should be rapidly eliminated from the general circulation of the body, while the blood-brain barrier should prevent its elimination from the brain (k₃ » k₂; k₃» k₇). Enzymatic cleavage of the [D-QC]⁺ that is "locked" in the brain effects a sustained delivery of the drug species [D], followed by its normal elimination (kₛ), metabolism. A properly selected carrier [QC]⁺ will also be rapidly eliminated from the brain (k₆» k₂). Because of the facile elimination of [D-QC]⁺ from the general circulation, only minor amounts of drug are released in the body (k₃ » k₄); [D] will be released primarily in the brain (k₄ > k₂). The overall result ideally will be a brain-specific sustained release of the target drug species.

Bodor et al have reported, in Science, their work with phenylethylamine as the drug model, which was coupled to nicotinic acid, then quaternized to give compounds of the formula 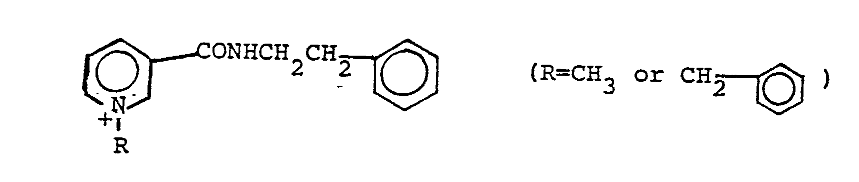 which were subsequently reduced by sodium dithionite to the corresponding compounds of the formula 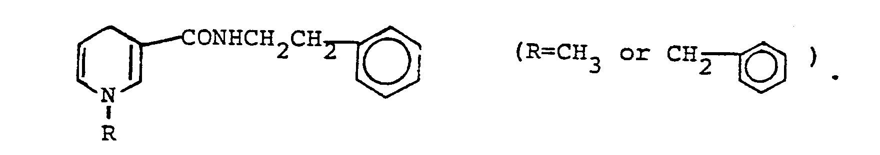 Testing of the N-methyl derivative in vivo supported the criteria set forth in Scheme 1. Bodor et al speculated that various types of drugs might possibly be delivered using the depicted or analogous carrier systems and indicated that use of N-methylnicotinic acid esters and amides and their pyridine ring- substituted derivatives was being studied for delivery of amino- or hydroxyl-containing drugs, including small peptides, to the brain. No other possible specific carriers were disclosed.

Other reports of Bodor et al's work have appeared in The Friday Evening Post, August 14, 1981, Health Center Communications, University of Florida, Gainesville, Florida; Chemical & Engineering News, December 21, 1981, pp. 24-25; and Science News, January 2, 1982, Vol. 121, No. 1, page 7. These publications do not suggest any carrier systems other than the specific N-methyl and N-benzyl nicotinic acid-type carriers disclosed in the Science publication. Other classes of drugs as well as a few specific drugs are mentioned as possible candidates for derivativization; for example, steroid hormones, cancer drugs and memory enhancers are indicated as targets for possible future work, as are enkephalins, and specifically, dopamine and testosterone. The publications do not suggest how to link such drugs to the carrier, except possibly when the drugs are simple structures containing a single NH₂ or, perhaps, simple structures containing a single OH, of the primary or secondary type, as is the case with phenylethylamine or testosterone. There is, for example, no suggestion of how one of ordinary skill in the art would form a drug- carrier combination when the drug has a more complicated chemical structure than phenylethylamine, e.g., dopamine or an enkephalin. Thus, except in a very limited area, where only a combination of certain types of drug structures with certain utility classes and certain carrier structures is taught or suggested, the publications simply do not make the broad concept of the invention as represented by Scheme 1 available to the public. Likewise, the many embodiments of the invention as described hereinafter are unsuggested by the art.

It is also known to this art that Parkinsonism, a striatal dopamine deficiency syndrome [H. Ehringer and O. Hornykiewicz, Klin. Wsch., 38, 1236 (1960)], cannot be treated directly with dopamine, for dopamine and related catecholamines also do not cross the blood-brain barrier [B. E. Roos and G. Steg, Life Sci., 3, 351 (1964)]. L-Dopa, considered as a prodrug for dopamine, was first discovered to be useful in the treatment of Parkinsonism more than twenty years ago [A. Barbeau, Excepta Medica, Int. Congr. Ser., 38, 152 (1961); W. Birkmayer and O. Hornykiewicz, Wien. Klin. Wochnenschr., 73, 787 (1961)]. Indeed, L-Dopa is considered to be the best available treatment for Parkinsonism, but, unfortunately, at the expense of a wide variety of undesirable side effects [A.Barbeau, TIPS, 2, (11), 297 (1981)]. The peripheral side effects of L-Dopa, which range from nausea and vomiting to cardiac arrythmias and hypotension, appear to be due to one or more of the metabolic products thereof, rather than L-Dopa per se. L-Aromatic amino acid decarboxylase enzyme is responsible for the major metabolism of L-Dopa, whether prior, during or after absorption. Concurrent administration of L-Dopa with an inhibitor of aromatic amino acid decarboxylase, which should not be able to penetrate the BBB, reduces the decarboxylation of L-Dopa in peripheral tissues. Such reduction allows higher proportions of L-Dopa to reach the CNS and at the same time diminishes the peripheral side effects considerably, particularly vomiting and cardiac arrythmias, but a number of serious side effects still persist [A.Barbeau, TIPS, supra; A.Barbeau and M.Roy, Neurology, 26, 399 [(1976)]. Attempts have also been made to alleviate the well-known dissolution, absorption and metabolism problems of L-Dopa [H. Ninterber- ger, Biochem. Med., 5, 412 (1971); H. Shindo, T. Komai, K. Tanaka, E. Nakajima and N. Miyakoshi, Chem. Pharm. Bull., 21, 826 (1973); C. O. Rutledge and M. M. Hoehn, Nature (London), 244, 447 (1973); R. L. Bronaugh, R. J. McMurty, M. M. Hoehn and C. O. Rutledge, Biochem. Pharmacol., 24, 1317 (1975)], employing prodrug approaches [N. Bodor, K. B. Sloan, T. Higuchi and K. Sasahara, J. Med. Chem., 20, 1435 (1977); A. M. Felix, D P. Winter, S. S. Wang, I. D. Kulesha, W. R. Pool, D. L. Hane and H. Sheppard, J. Med. Chem., 17, 422 (1974)].

Additionally, dopamine agonists, which are used in the treatment of hyperprolactinemia associated with pituitary adenomas or amenorrhea [R. F. Spark and G. Dickenstein, Ann. Int. Med., 90, 949 (1979)], also induce unwanted side effects.

Thus, especially acutely serious need exists in this art to delivery a dopaminergic agent directly and specifically to the brain, in a sustained manner, and there elicit the desired dopaminergic response, e.g., for the treatment of Parkinsonism or hyperprolactinemia.

The existence of a blood-testis barrier (BTB) by which some substances are prevented from being carried into the seminiferous tubules long has been suspected [P.P.H. Bruyn, R.C. Robertson and R.S Farr, Anat. Rec., 108, 279 (1950); R.J. Goldacre and B. Sylven, Nature (London), 184, 63 (1959); R.J. Goldacre and B. Sylven, J. Cancer, 16, 306 (1962); T.S. Ro and H. Busch, Biochem. Biophys Acta (Amst),108, 317 (1965)]. Some investigators have suggested a similarity between the BBB and the BTB [A.T. Cowie, A.K. Lascelle and J.C. Wallace, J. Physiol. (London), 171, 1976 (1964); R.E. Mancini, O. Vilar, B. Alvarez and A.C. Seiguer, J. Histochem. Cytochem., 13, 376 (1965); M. Kormano, Acta Physiol. Scand., 71, 125 (1967); M. Kormano, Histochemic, g, 327 (1967)]. Don W. Fawcett, Lee V. Leak and Paul M. Heidger, Jr., J. Reprod. Fert. Suppl. 10, 105 (1970) have suggested that the permeability barrier is not in the testis capillary walls because these more closely resemble the capillaries of muscle than those involved in the BBB. M. Dym and Don W. Fawcett, Biology of Reproduction, 3, 308 (1970) concluded that the epithelioid contractile layer around the seminiferous tubules constitutes a significant permeability barrier augmented by an apparently more efficient barrier involving tight cell-to-cell junctions between sertoli cells that inhibits penetration of substances through the germinal epithelium. Despite such histological differences, phar- macokinetic studies [K. Okumura, I.P. Lee and R.L. Dixon, J. Pharmacol. Exp. Therap., 194, 89 (1975); I.P. Lee and R.L. Dixon, Environmental Health Perspectives, 24, 117 (1970)] have demonstrated that the functional BTB resembles the BBB in transport characteristics, both depending on lipid solubility and molecular size. Thus, delivery of drug species to the testes is often seriously limited by the blood-testis barrier. Site-specific delivery and sustained delivery of drugs to the testes are even more difficult. To date, no useful simple or generic techniques to achieve such results are known to the art. It is thus apparent that a serious need exists in this art for a method for the site-specific and/or sustained delivery of drug species to the testes to elicit the desired therapeutic, e.g., hormonal or tumor-inhibiting, response.

### Summary and Objects of the Invention:

Accordingly, a major object of the present invention is the provision of a generic method for the specific and/or target enhanced delivery to the brain of a wide variety of drug species and to achieve brain-specific drug delivery by effecting the bidirectional transport of the drug species into and out of the brain employing dihydropyridine == pyridinium salt carrier type redox systems.

Another object of the invention is to provide for brain specific drug delivery utilizing a dihydropyridine == pyridinium salt carrier type redox system, which drug/carrier system is characterized by enhanced drug efficacy and decreased toxicity. Indeed, consistent herewith systemic toxicity is significantly reduced by accelerating the elimination of the drug/quaternary carrier system, and even central toxicity is reduced by providing a low level, sustained release of the active drug species in the brain.

Yet another object of this invention is the provision of a chemical delivery system for the site-specific and sustained release of drug species to the brain, and one in which a special pro-prodrug reduced form of an active drug species is actually delivered to the body of a patient, not a prodrug as such and not a drug/carrier entity necessarily comprised of critically tailored release rate-controlling substituent(s).

Still another object of this invention is to provide an effective testicular specific chemical delivery system, for the site-specific and/or sustained eliciting of significant drug response in the testes.

Yet another object of this invention is to provide enhanced delivery to the brain and testes of a wide variety of centrally acting agents which are not themselves able to penetrate the blood-brain and blood-testis barriers to any considerable extent.

In accord with the foregoing, the present invention provides, in one aspect, compounds adapted for the site specific/sustained delivery of a centrally acting drug species to the brain, said compounds being compounds of the formula 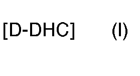 and the non-toxic pharmaceutically acceptable salts thereof, wherein [D] is a centrally acting drug species containing at least one reactive -OH, -COOH, -SH, -NH- or -NH₂ group (including groups such as amides and imides, as well as primary and secondary amino groups), and [DHC] is the reduced, biooxidizable, blood-brain barrier penetrating lipoidal form of a dihydropyridine == pyridinium salt redox carrier, with the proviso that when [DHC] is 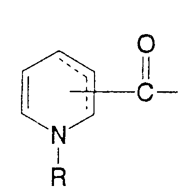 wherein R is lower alkyl or benzyl, the dotted line indicates the presence of a double bond in either the 2 or 3 position of the dihydropyridine ring and the carbonyl group is attached at the 2, 3 or 4 position of the dihydropyridine ring; and [D] is a drug species containing a single amino or OH functional group, the single amino or OH group when present being a primary or secondary amino or primary or secondary OH group, said drug species being linked directly through said amino or OH functional group to the carbonyl function of [DHC], then [D] must be other than a sympathetic stimulant, steroid sex hormone, memory enhancer, long chain alkanol or anticancer or antitumor agent.

In a related aspect, the present invention provides compounds having the formula 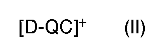 wherein [D] is a centrally acting drug species containing at least one reactive -OH, -COOH, -SH, -NH- or -NH₂ group (including groups such as amides and imides, as well as primary and secondary amino groups), and [QC]⁺ is the hydrophilic, ionic pyridinium salt form of a dihydropyridine == pyridinium salt redox carrier, with the proviso that when [QC]⁺ is 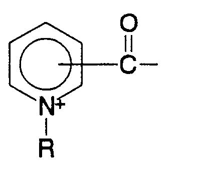 wherein R is lower alkyl or benzyl and the carbonyl group is attached at the 2, 3 or 4 position of the pyridinium ring, and [D] is a drug species containing a single amino or OH functional group, the single amino or OH group when present being a primary or secondary amino or primary or secondary OH group, said drug species being linked directly through said amino or OH functional group to the carbonyl function of [QC]⁺, then [D] must be other than a sympathetic stimulant, steroid sex hormone, memory enhancer, long chain alkanol or anticancer or antitumor agent.

In one preferred aspect of the present invention, there are provided compounds of the formula [D-DHC] and their non-toxic pharmaceutically acceptable salts, wherein [D] is a centrally acting drug species which is glycine, glutamic acid, aspartic acid, tryptophan, an enkephalin, an endorphin or other amino acid or small peptide containing 2 to 20 amino acid units; GABA, y-vinyl GABA, or y-acetylenic GABA; norepinephrine, or epinephrine; serotonin; 5-hydroxy-2-n-propylpentanoic acid, 4-hydroxy-2-n-propylpentanoic acid, or 3-hydroxy-2-n-propylpentanoic acid; methyldopa; tyramine; dopamine, or levodopa; ampicillin; cephalexin; L-alanosine; melphalan; DON, or acivicin; or glucosamine, or 6-amino-6-deoxy-D-glucose; and [DHC] is the reduced, biooxidizable, blood-brain barrier penetrating lipoidal form of a dihydropyridine 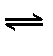 pyridinium salt redox carrier having the formula 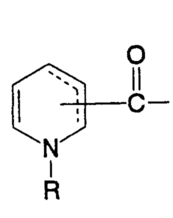 wherein R is methyl or benzyl and the dotted line indicates the presence of a double bond in either the 2 or 3 position of the dihydropyridine ring, the carbonyl function adjacent the dihydropyridine ring being bonded to a reactive amino or hydroxyl functional group in the centrally acting drug species, and at least one of the remaining reactive functional groups in the centrally acting drug species being protected by a hydrolytically or metabolically cleavable hydroxyl or carboxyl protective group; in a related preferred aspect of the invention, there are provided the corresponding ionic pyridinium salt type compounds of the formula [D-QC]^{+.}

In another preferred aspect of the present invention, there are provided compounds of the formula [D-DHC] and their non-toxic pharmaceutically acceptable salts, wherein [D] is a centrally acting drug species which is an antiviral agent, an antiinflammatory steroid, a narcotic or an estrogen and which contains at least two reactive hydroxyl functional groups, and [DHC] is the reduced, biooxidizable, blood-brain barrier penetrating lipoidal form of a dihydropyridine == pyridinium salt redox carrier comprising at least one dihydropyridine ring system of the formula 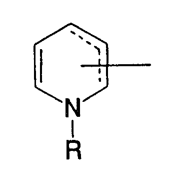 wherein R is lower alkyl or benzyl and the dotted line indicates the presence of a double bond in either the 2 or 3 position of the dihydropyridine ring, a ring carbon atom of each dihydropyridine ring system being connected via a bridging group to a reactive hydroxyl functional group in the centrally acting drug species. In a related preferred aspect of the invention, there are provided the corresponding ionic pyridinium salt type compounds of the formula [D-QC]^{+.}

In yet another preferred aspect of the invention, there are provided compounds of the formula [D-DHC] and their non-toxic pharmaceutically acceptable salts, wherein [D] is a centrally acting drug species which is an anticancer/antitumor and/or antiviral agent, said drug species having a purine or pyrimidine base-type structure bearing a singly or multiply hydroxylated substituent, and [DHC] is the reduced, biooxidizable, blood-brain barrier penetrating, lipoidal form of a dihydropyridine == pyridinium salt redox carrier comprising at least one dihydropyridine ring system of the formula 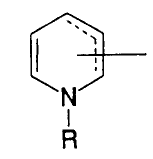 wherein R is lower alkyl or benzyl and the dotted line indicates the presence of a double bond in either the 2 or 3 position of the dihydropyridine ring, a ring carbon atom of each dihydropyridine ring system being connected via a bridging group to a reactive hydroxyl functional group in the centrally acting drug species. In a related preferred aspect of the invention, there are provided the corresponding ionic pyridinium salt type compounds of the formula [D-QC]^{+.}

In still another preferred aspect of the invention, there are provided compounds of the formula [D-DHC] and their non-toxic pharmaceutically acceptable salts, wherein [D] is a centrally acting drug species which is a steroidal progestin or steroidal estrogen, said progestin or estrogen having at least one reactive hydroxyl functional group, one such group being a tertiary hydroxyl functional group in the 17-position; and [DHC] is the reduced, biooxidizable, blood-brain barrier penetrating, lipoidal form of a dihydropyridine == pyridinium salt redox carrier comprising at least one dihydropyridine ring system of the formula 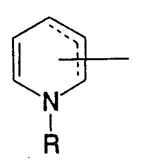 wherein R is lower alkyl or benzyl and the dotted line indicates the presence of a double bond in either the 2 or 3 position of the dihydropyridine ring, a ring carbon atom of one dihydropyridine ring system being connected via a bridging group to the tertiary 17-hydroxyl functional group in the progestin or estrogen, a ring carbon atom of any remaining dihydropyridine ring system being connected via a bridging group to another reactive hydroxyl functional group in said progestin or estrogen. In a related preferred aspect of the invention, there are provided the corresponding ionic pyridinium salt type compounds of the formula [D-QC]^{+.}

In a further preferred aspect of the present invention, there are provided compounds of the formula [D-DHC] and their non-toxic pharmaceutically acceptable salts, wherein [D] is a centrally acting drug species containing at least one reactive -COOH, amide or imide functional group, and [DHC] is the reduced, biooxidizable, blood-brain barrier penetrating lipoidal form of a dihydropyridine == pyridinium salt redox carrier comprising a dihydropyridine ring system of the formula 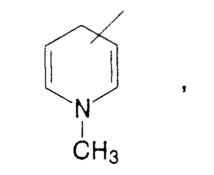 a ring carbon atom of the dihydropyridine ring system being connected via a bridging group to a reactive -COOH, amide or imide functional group in the centrally acting drug species. In a related preferred aspect of the invention, there are provided the corresponding ionic pyridinium salt type compounds of the formula [D-QC]^{+.}

In still a further preferred aspect of the present invention, there are provided compounds of the formula [D-DHC] and their non-toxic pharmaceutically acceptable salts, wherein [D] is a centrally acting drug species containing at least one reactive -OH, -COOH, -SH, -NH- or -NH₂ group (including groups such as amides and imides, as well as primary and secondary amino groups), and [DHC] is the reduced, biooxidizable, blood-brain barrier penetrating lipoidal form of a dihydropyridine pyridinium salt redox carrier comprising a dihydropyridine ring system of the formula. 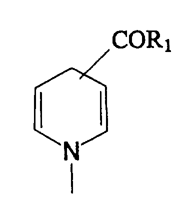 wherein R is -NH₂ or -OR₂ wherein R₂ is alkyl, the dihydropyridine ring optionally being fused to a benzene ring, the nitrogen atom of the dihydropyridine ring system being connected via a bridging group to a reactive -OH, -COOH, -SH, -NH- or -NH₂ group in the centrally acting drug species. In a related preferred aspect of the invention, there are provided the corresponding ionic pyridinium salt type compounds of the formula [D-QC]^{+.}

In yet a further preferred aspect of the present invention, there are provided compounds of the formula [D-DHC] and their non-toxic pharmaceutically acceptable salts, wherein [D] is a centrally acting drug species containing at least one reactive -OH, -COOH, -SH, -NH- or -NH₂ group (including groups such as amides and imides, as well as primary and secondary amino groups), and [DHC] is the reduced, biooxidizable, blood-brain barrier penetrating lipoidal form of a dihydropyridine == pyridinium salt redox carrier comprising a dihydroquinoline or dihydroisoquinoline ring system, a carbon atom of the nitrogen-containing ring portion of said ring system being connected via a bridging group to a reactive -OH, -COOH, -SH, -NH-or -NH₂ group in the centrally acting drug species. In a related preferred aspect of the invention, there are provided the corresponding ionic pyridinium salt type compounds of the formula [D-QC]^{+.} The present invention further provides a generic method for specific and/or target enhanced delivery to the brain of a wide variety of centrally acting drug species, such brain-specific drug delivery being effected via the bidirectional transport of the drug species into and out of the brain by means of dihydropyridine 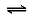 pyridinium salt carrier type redox systems.

In another aspect, the present invention provides, as an effective drug delivery system, compounds having the formula 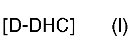 and non-toxic pharmaceutically acceptable salts thereof, wherein [D] is a testicularly acting drug species and [DHC] is the reduced, biooxidizable, blood-testis barrier penetrating, lipoidal form of a dihydropyridine == pyridinium salt redox carrier. In still another aspect, the present invention provides compounds having the formula 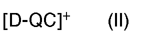 wherein [D] is a testicularly acting drug series and [QC]⁺ is the hydrophilic, ionic pyridinium salt form of a dihydropyridine pyridinium salt redox carrier. In both of these aspects of the invention, compounds excluded by the provisos set forth with the first definitions of formulas (I) and (II) hereinabove are likewise excluded here. Also according to this invention, there is hereby provided a generic method for specific and/or target enhanced delivery to the testes of a wide variety of testicularly acting drug species, such testicular-specific drug delivery being effected via the bidirectional transport of the drug species into and out of the testes by means of dihydropyridine pyridinium salt carrier type redox systems.

In yet another aspect, the present invention provides, as an effective dopaminergic chemical delivery system, compounds having the formula 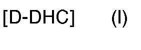 and non-toxic pharmaceutically acceptable salts thereof, wherein [D] is a dopamine having the structural formula 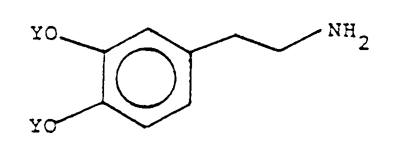 in which each Y is independently hydrogen or a hydrolytically or metabolically cleavable hydroxyl protective group, and [DHC] is defined as above. In still another aspect, the present invention provides compounds having the formula 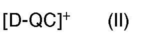 wherein [D] is a dopamine having the structural formula 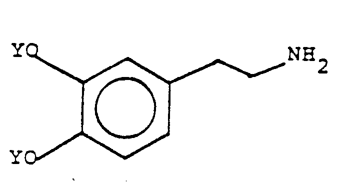 in which each Y is independently hydrogen or a hydrolytically or metabolically cleavable hydroxyl protective group, and [QC]⁺ is defined as above.

Briefly, one presently preferred chemical delivery system for dopamine according to this invention has the structure (2) in the following Scheme 2, and wherein the amino function of dopamine is appropriately linked to the dihydropyridine-type carrier system, while the catechol function is advantageously protected, for example, as a corresponding ester function, e.g., the dipivalyl ester illustrated. The brain-specific delivery of dopamine, or the otherwise eliciting of a dopaminergic response, requires a succession of processes, including oxidation of the dihydropyridine ring to the corresponding pyridinium salt (for example, structure 3), which provides the basis for "locking-in" the brain the molecule, hydrolysis of the, e.g., pivalyl esters (see structure 4) likely via the 3- and/or 4-monopivalyl esters and, finally, the release of dopamine (1) from 4, which can be either a hydrolysis or a reductive process [a possible reductive release of dopamine was very recently suggested by a model for a presynaptic terminal, L. L. Miller, A. N. K. Lau and E. K. Miller, J. Am. Chem. Soc., 104, 5242 (1982)].

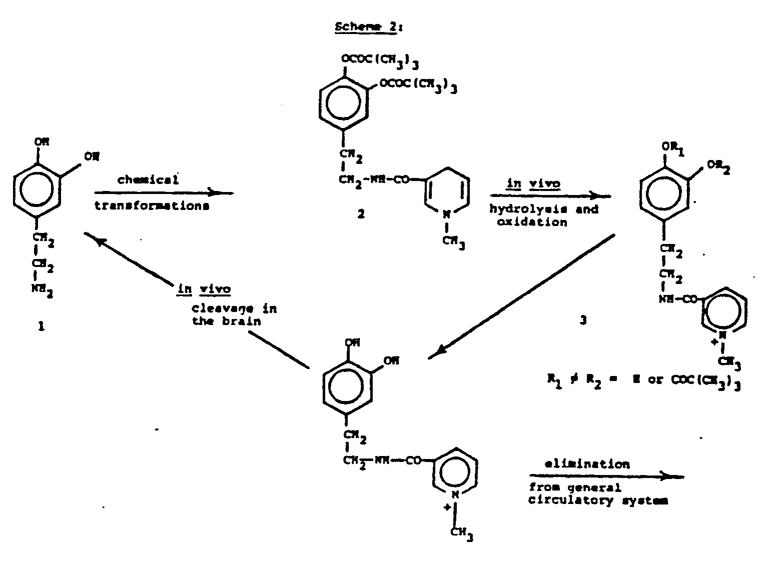

As per the above Scheme 2, for the brain specific delivery of dopamine (1), structure 2 is one chemical delivery system consistent herewith, and is one precursor locked in the brain and eliminated rapidly from the rest of the body. Structures depict intermediates formed during the stepwise hydrolysis and oxidation processes.

### Brief Description of the Drawings:

FIGURE 1 is a graph plotting the time course of 1-methyl-3-{N-[β-(3,4-dipivalyloxyphenyl)-ethyl]-}carbamoyl-1,4-dihydropyridine 5c (O) and its products, monopivalyl-dihydro derivative 11 (V), the dihydrodopamine derivative 5a (A) and the quaternary dopamine precursor 6a (•) in plasma;
FIGURE 2 is a graph plotting the time course of 1-methyl-3-{N-[β-(3,4-dipivayloxyphenyl)-ethyl]-}carbamoyl-1,4-dihydropyridine 5c (O) and its products, monopivalyl-dihydro drivative 11 (V) and the quaternary dopamine precursor 6a (•) in whole blood;
FIGURE 3 is a graph plotting the time course of 1-methyl-3-{N-[β-(3,4-dipivalyloxyphenyl)-ethyl]-}carbamoyl-1,4-dihydropyridine 5c (O) and its products, monopivalyl-dihydro derivative 11 (V), the dihydrodopamine derivative 5a (A) and the quaternary dopamine precursor 6a (•) in 20% brain homogenate;
FIGURE 4 is a graph plotting the time course of 1-methy-3-{N-[β-(3,4-dipivalyloxyphenyl)-ethyl]-carbamoyl-1,4-dihydroxypyridine 5c (O) and its product, the quaternary dopamine precursor 6a (•) in 20% liver homogenate;
FIGURE 5 is a semilog plot of peak heights of 1-methyl-3-{N-[,8-(3,4-dipivalyloxyphenyl)-ethyl]-}carbamoyl-1,4-dhydropyridine 5c against time in plasma (0), brain homogenate (A), whole blood (O) and liver homogenate (_{D});
FIGURE 6 is a graph plotting concentrations against time of 1-methyl-3-{N-[β-(3,4-dihydroxyphenyl)-ethyl]}carbamoyl pyridinium cation (6a) in brain (•) and in blood (O) following administration of 1-methyl-3-{N-[β-(3,4-dipivayloxyphenyl]}-ethyl]} carbamoyl-1, 4-dihydropyridine (5c), with the error bars indicating SEM; and
FIGURE 7 is a graph plotting the effects of compounds 5c (▲) and 6a (•) administered I.V. at 1 mg/kg dose level, on the serum prolactin levels in rats.

### Detailed Description of the Invention:

More particularly in accord with the present invention, the following definitions are applicable:
The term "lipoidal" as used herein is intended to designate a carrier moiety which is lipid-soluble or lipophilic.

The expression "hydroxyl protective group" is intended to designate a group which prevents premature metabolism of an OH group or groups prior to the compound's reaching the desired site in the body. Typical hydroxyl protective groups contemplated by the present invention (e.g., for Y in the case of the dopamine derivatives) are acyl groups and carbonates.

When the hydroxyl protective group is acyl (i.e., when it is an organic radical derived from a carboxylic acid by removal of the hydroxyl group), it preferably represents an acyl radical selected from the group consisting of alkanoyl having 2 to 8 carbon atoms; alkenoyl having one or two double bonds and 3 to 8 carbon atoms; 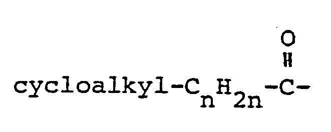 wherein the cycloalkyl portion contains 3 to 7 ring atoms and n is zero, one, two or three; phenoxyacetyl; pyridinecarbonyl; and 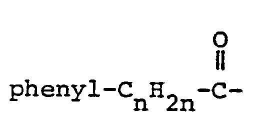 wherein n is zero, one, two or three and phenyl is unsubstituted or is substituted by 1 to 3 alkyl each having 1 to 4 carbon atoms, alkoxy having 1 to 4 carbon atoms, halo, trifluoromethyl, dialkylamino having 2 to 8 carbon atoms or alkanoylamino having 2 to 6 carbon atoms.

When the acyl group is alkanoyl, there are included both unbranched and branched alkanoyl, for example, acetyl, propionyl, butyryl, isobutyryl, valeryl, isovaleryl, 2-methylbutanoyl, pivalyl (pivaloyl), 3-methylpentanoyl, 3,3-dimethylbutanoyl, 2,2-dimethylpentanoyl and the like. Pivalyl and isobutyryl are especially preferred.

When the acyl group is alkenoyl, there are included, for example, crotonyl, 2,5-hexadienoyl and 3,6-octadienoyl. When the acyl group is 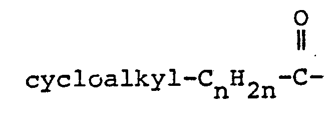 there are included cycloalkanecarbonyl and cycloalkanealkanoyl groups wherein the cycloalkane portion can optionally bear 1 or 2 alkyl groups as substituents, e.g. cyclopropanecarbonyl, 1-methylcyclopropanecar- bonyl, cyclopropaneacetyl, a-methylcyclopropaneacetyl, 1-methylcyclopropaneacetyl, cyclopropanepropionyl, a-methylcyclopropanepropionyl, 2-isobutylcyclopropanepropionyl, cyclobutanecarbonyl, 3,3-dimethylcyclobutanecarbonyl, cyclobutaneacetyl, 2,2-dimethyl-3-ethylcyclobutaneacetyl, cyclopentanecarbonyl, cyclohexaneacetyl, cycloheptanecarbonyl and cycloheptanepropionyl.

When the acyl group is pyridinecarbonyl, there are included picolinoyl (2-pyridinecarbonyl), nicotinoyl (3-pyridinecarbonyl) and isonicotinoyl (4-pyridinecarbonyl).

When the acyl group is 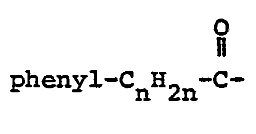 there are included, for example, benzoyl, phenylacetyl, a-phenylpropionyl, ,8-phenylpropionyl, p-toluyl, m-toluyl, o-toluyl, o-ethylbenzoyl, p-tert-butylbenzoyl, 3,4-dimethylbenzoyl, 2-methyl-4-ethylbenzoyl, 2,4,6-trimethylbenzoyl, m-methylphenylacetyl, p-isobutylphenylacetyl, ,8-(p-ethylphenyl)-propionyl, p-anisoyl, m-anisoyl, o-anisoyl, m-isopropoxybenzoyl, p-methoxyphenylacetyl, m-isobutoxyphenylacetyl, m-diethylaminobenzoyl, 3-methoxy-4-ethoxybenzoyl, 3,4,5-trimethoxybenzoyl, p-dibutylaminobenzoyl, p-n-butoxybenzoyl, 2,4,6-triethoxybenzoyl, 3,4-diethoxyphenylacetyl, β-(3,4,5-trimethoxyphenyl)propionyl, o-iodobenzoyl, m-bromobenzoyl, p-chlorobenzoyl, p-fluorobenzoyl, 2-bromo-4-chlorobenzoyl, 2,4,6-trich- lorobenzoyl, p-chlorophenylacetyl, a-(m-bromophenyl)propionyl, p-trifluoromethylbenzoyl, 2,4-di-(trifluoromethyl)benzoyl, m-trifluoromethylphenylacetyl, β-(p-trifluoromethylphenyl)propionyl, 2-methyl-4-methoxybenzoyl, 3-chloro-4-ethoxybenzoyl, β-(3-methyl-4-chlorophenyl)propionyl, p-dimethylaminobenzoyl,p-(N-methyl-N-ethylamino)benzoyl, o-acetamidobenzoyl, m-propionamidobenzoyl, 3-chloro-4-acetamidophenylacetyl and p-acetamidophenylpropionyl.

When the hydroxyl protective group is a carbonate grouping, it has the structural formula 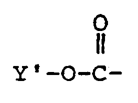 i.e., it is an organic radical which can be considered to be derived from a carbonic acid by removal of the hydroxyl group from the COOH portion. Y' preferably represents alkyl having 1 to 7 carbon atoms; alkenyl having one or two double bonds and 2 to 7 carbon atoms; cycloalkyl-CₙH₂n-wherein the cycloalkyl portion contains 3 to 7 ring atoms and n is zero, one, two or three; phenoxy; 2-, 3- or 4-pyridyl; or phenyl-CₙH₂n-wherein n is zero, one, two or three and phenyl is unsubstituted or is substituted by 1 to 3 alkyl each having 1 to 4 carbon atoms, alkoxy having 1 to 4 carbon atoms, halo, trifluoromethyl, dialkylamino having 2 to 8 carbon atoms or alkanoylamino having 2 to 6 carbon atoms. Most preferably, Y' is C1-C7 alkyl, particularly ethyl or isopropyl.

Similarly, the expression "carboxyl protective group" is intended to designate a group which prevents premature metabolism of a COOH group or groups prior to the compound's reaching the desired site in the body. Typical carboxyl protecting groups are the groups encompassed by Y' above, especially C1-C7 alkyl, particularly ethyl or isopropyl.

By "centrally acting" drug species, active agent or compound as utilized herein, there is of course intended any drug species or the like, the principal pharmacological activity of which is CNS and a result of direct action in the brain.

Exemplary such centrally acting drug species are the CNS-amines and other nervous system agents, whether sympathetic or parasympathetic, e.g., phenylethylamine, dopamine, tyramine, L-DOPA, muscle relaxants, tranquilizers and antidepressants, e.g., benzodiazepine tranquilizers such as diazepam and oxazepam, phenothiazine tranquilizers such as carphenazine, fluphenazine and the like, mild and strong analgesics and narcotics, sedatives and hypnotics, narcotic antagonists, vascular agents, stimulants, anesthetics, small peptides, such as the di-, tri, tetra- and pentapeptides, and other small 6-20 aminoacid unit containing peptides, e.g., the enkephalins (for example, Tyr-Gly-Gly-Phe-Leu), which, besides being analgesics, initiate epileptic activity in the brain at doses that are about tenfold lower than for effecting analgesic activity, larger peptides, such as pituitary hormones and related agents, antiepileptic and anticonvulsant drugs generally, including hydantoins such as phenytoin and ethotoin, barbituates such as phenobarbital, hormones, such as the steroid hormones, e.g., estradiol, testosterone, 17 a-ethynyl testosterone, and the like (recent studies on histological mapping of hormone-sensitive and specific steroid binding cells in the brain have underscored the importance of the steroid action in the brain on sexual behavior), amphetamine-like drugs, anticancer and anti-Parkinsonism agents, antihypertensives, agents to enhance learning capacity and the memory processes, including treatment of dementias, such as Alzheimer's disease, antibacterials, centrally active hypotensive agents, diagnostic agents, such as radio-pharmaceuticals, monoamine oxidase (MAO) inhibitor drugs, CNS or brain important/essential amino acids, such as tryptophan, and any like centrally acting compounds.

Other illustrative ultimate species of centrally active drug entities are: amphetamine, dextroamphetamine, levamphetamine, methamphetamine, phenmetrazine and phentermine, which are stimulants and appetite suppressants; codeine, oxycodone, pentazocine, anileridine, hydromorphone, morphine and oxymorphone, which are narcotic analgesics; desipramine, nortriptyline, opipramol and protriptyline, which are stimulates used, e.g., in endogenous depressions; clonidine and methyldopa, which are sympatholytic agents used, e.g., in hypertension; biperiden, cycrimine and procyclidine, which are centrally acting anticholinergics; tranylcypromine, a sympathomimetic stimulant and MAO inhibitor; acetophenazine, carphenazine, fluphenazine, perphenazine and piperacetazine, which are phenothiazine-type tranquilizers; chlordiazepoxide, clorazepate, nitrazepam and temazepam, which are benzodiazepine-type tranquilizers; haloperidol and clopenthixol, which are transquilizers; norepinephrine, a sympathetic stimulant/adrenergic agent; nalorphine and naloxone, narcotic antagonists; hydralazine, a hypotensive; ethotoin, phenobarbital and aminoglutethimide, anticonvulsants; ethamivan, a medullary stimulant; bemegride, a barbiturate antagonist; amiphenazole, a stimulant; iopydol, iodopyracet, iodouppurate, iodamide and iopanoic acid, which are radiodiagnostics; ephedrine, pseudoephedrine, oxymetazoline and phenylephrine, which are sympathomimetic amines and decongestants; estradiol, estrone and estriol, the natural estrogens; amoxicillin, oxacillin, carbenicillin and ampicillin, pencillin-type antibiotics; amobarbital, a sedative; trihexyphenidyl, a centrally acting anticholinergic; hydroxyzine, a tranquilizer; chlortetracycline, tetracycline and methacycline, which are tetracycline-type antibiotics; clindamycin, lincomycin, nalidixic acid, oxolinic acid and phenazopyridine, antibacterials/antibiotics; flurazepam, bromazepam and lorazepam, tranquilizers; phenytoin, an anticonvulsant; glutethimide, a mild hypnotic/sedative; bethanidine and guanethidine, hypoten- sives/sympatholytics; methyprylon; propranolol, a ,8-blocker antihypertensive; dicloxacillin, an antibacterial; butalbital, a barbiturate sedative; GABA, y-vinyl GABA, y-acetylenic GABA, neurotransmitters for possible use in epilepsy; valproic acid and its metabolites such as 5-hydroxy-2-n-propylpentanoic acid, 4-hydroxy-2-n-propylpentanoic acid, 3-hydroxy-2-n-propylpentanoic acid, for use as anticonvulsants; apomorphine, a narcotic depressant; methotrexate, podophyllotoxin derivatives (etoposide, teniposide), doxorubicin, daunomycin and cyclophosphamide, anticancer/antitumor agents; methylphenidate, a stimulant; thiopental, an anesthetic; naloxone, a narcotic antagonist; ethinyl estradiol and mestranol, estrogens; norgestrel and norethindrone, progestins; cephalothin, cephalexin and cefoxitin, cephalosporin antibiotics; atenolol and metoprolol, ,8-blockers/hypotensives; ACTH corticotropin); LH-RH, a neurotransmitter; sulfadiazine and other sulfonamide antibiotics; ribavirin and acyclovir, antiviral agents; chlorambucil and melphalan, nitrogen mustard-type anticancer/antitumor agents; methotrexate and aminopterin, which are folic acid antagonist- type anticancer/antitumor agents; cisplatin-analogue type anticancer/antitumor agents; doxorubicin, daunamycin, dactinomycin and mitomycin C, used in cancer chemotherapy; thioguanine, a purine/pyrimidine antagonist used in cancer treatment; vincristine and vinblastine, anticancer alkaloids; hydroxyurea and DON, anticancer urea derivatives; N,N'-bis(dichloracetyl)-1,8-octamethylene diamine, an agent for male fertility inhibition; levorphanol, a narcotic analgesic; and benzestrol and diethylstilbestrol, synthetic estrogens.

Preferred classes of centrally acting drugs for use herein are the central neurotransmitters, steroids, anticancer and antitumor agents, antiviral agents, tranqilizers, memory enhancers and hypotensives. Among the neurotransmitters, there can be mentioned amino acids, such as GABA, glycine, glutamic acid and aspartic acid; catecholamines, such as dopamine, norepinephrine and epinephrine; serotonin and tryptamine; and peptides such as neurotensin, luteinizing hormone-releasing hormone (LHRH), somatostatin, enkephalins such as met^{s-}enkephalin and leu^{s-}enkephalin, endorphins such as -y-,a- and ,8-endorphins, oxytocin M and vasopressin. Among the steroids, there can be mentioned antiinflammatory adrenal cortical steroids such as hydrocortisone, betamethasone, cortisone, dexamethasone, flumethasone, fluprednisolone, meprednisone, methyl prednisolone, prednisolone, prednisone, triamcinolone, cortodoxone, fludrocortisone, flurandrenolone acetonide paramethasone and the like; male sex hormones, such as testosterone and methyl testosterone; and female sex hormones, both estrogens and progestins, e.g., ethinyl estradiol, norgestrel, mestranol, norethindrone, ethisterone, estradiol, estriol, estrone, dimethisterone, allylestrenol, cingestol, ethynerone, lynestrenol, norgesterone, norvinisterone, ethynodiol, oxogestone, tigestol, quinestrol and the like. Among the anticancer and antitumor agents, there can be mentioned Ara-AC, pentostatin(2'-deoxycoformycin), Ara-C, 3-deazaguanine, dihydro-5-azacytidine, tiazofurin, sangivamycin, Ara-A, 6-MMPR, PCNU, spiromustine, bisbenzimidazole, L-alanosine, DON, L-ICRF, trimethyl TMM, 5-methyl tetrahydrohomofolic acid, glyoxylic acid sulfonylhydrazone, DACH, SR-2555, SR-2508, desmethylmisonidazole, mitoxantrone, menogarol, aclacinomycin A, phyllanthoside, bactobolin, aphidocolin, homoharringtonine, levonantradol, acivicin, streptozotocin, hydroxyurea, chlorambucil, cyclophosphamide, uracil mustard, melphalan, 5-FUDR, vincristine, vinblastine, cytosine arabinoside, 6-mercaptopurine, thioguanine, 5-azacytidine, methotrexate, adriamycin (doxorubicin), daunomycin, largomycine polypeptide, aminopterin, dactinomycin, mitomycin C, and podophyllotoxin derivatives, such as etoposide (VP-16) and teniposide. Among the antiviral agents, there can be mentioned ribavirin; acyclovir, amantadine, diarylamidines such as 5-amidino-2-(5-amidino-2-benzofuranyl)indole and 4',6-diimidazolino-2-phenylbenzo(b)thiophene; 2-aminox- azoles such as 2-guanidino-4,5-di-n-propyloxazole and 2-guanidino-4,5-diphenyloxazole; benzimidazole analogues such as the syn and anti isomers of 6[[(hydroxyimino)phenyl] methyl]-1-[(1-methylethyl)sulfonyl]-1H-benzimidazol-2-amine; bridgehead C-nucleosides such as 5,7-dimethyl 2-β-D-ribofuranosyl-s-triazole(1,5-a)-pyrimidine; glycosides such as 2-deoxy-D-glucose, glucosamine, 2-deoxy-2-f!uoro-D-mannose and 6-amino-6-deoxy-D-glucose; phenyl glucoside derivatives such as phenyl-6-chloro-6-deoxy-β-D-glucopyranoside; (S)-9-(2,3-dihydroxypropyl)adenine; 6-azauridine; and 5,6-dichloro-1-β-D-ribofuranosylbenzimidazole. Among the tranquilizers, there can be mentioned benzodiazepine tranquilizers such as diazepam, oxazepam, lorazepam, chlordiazepoxide, flurazepam, bromazepam, clorazepate, nitrazepam and temazepam; hydan- toin-type tranquilizers such as phenytoin, ethotoin, mephenytoin; phenothiazine-type tranquilizers such as acetophenazine, carphenazine, fluphenazine, perphenazine and piperacetazine; and others. Among the hypotensives, there can be mentioned clonidine, methyldopa, bethanidine, debrisoquin, hydralazine and guanethidine.

By "testicularly acting drug species" as used herein, there is intended any drug which is capable of exerting a useful pharmacological effect when delivered to the testes. Generally speaking, those centrally acting drug species disclosed hereinabove for delivery to the brain can likewise be delivered to and concentrated in the testes utilizing the instant dihydropyridine == pyridinium salt carrier type redox system; any such centrally acting drugs which exert a therapeutic or other useful biological effect when delivered to the testes are considered to be within the ambit of the expression "testicularly acting drug species".

Exemplary of the drugs contemplated for delivery to the testes in accord with the present invention are antibiotics/antibacterials, antiviral agents, anticancer agents, hormonal agents and analgesics. The antibiot- ics/antibacterrials can be, for example, sulfonamides, penicillins, cephalosporins, tetracyclines, and other antibacterials, such as nalidixic acid and phenazopyridine. Among the antiviral agents, there can be mentioned especially ribavirin and acyclovir (ACV). The drugs used in cancer chemotherapy contemplated for delivery to the testes include nitrogen mustards, folic acid antagonists, platinum coordination complexes, antibiotic cancer agents, podophyllotoxins, purine and pyrimidine antagonists, alkaloids, urea derivatives, and hormonal anticancer agents. Among the hormonal agents contemplated for delivery to the testes are pituitary gonadotropins, such as follicle stimulating hormone (FSH), which maintains spermatogenesis and may be used to treat male infertility and cryptorchism; nonpituitary gonadotropins, such as human chorionic gonadotropin (HCG), which may be used for treatment of cryptorchism or hypogonadism in the male; adrenal cortical hormones, particularly glucocorticoids, which may be used in the treatment of inflammation (and possibly also in neoplasm), such as, for example, hydrocortisone, betamethasone, dexamethasone, flumethasone, fluprednisolone, mepredisone, methyl prednisolone, prednisolone, prednisone and triamcinolone; agents for male fertility inhibition, e.g., agents which impair gametogenesis or otherwise hamper sperm production, such as N,N'-bis(dichloracetyl)-1,8-octamethylene diamine; androgens, e.g., testosterone and methyltestosterone, agents of this type being useful for treating male hypogonadism, cryptorchism and the male climateric, as well as for maintaining or improving normal sexual function in the male and preventing decline in testicular function; and antiandrogens, which may be of use in male contraception and depression of male sexual behavior, especially in veterinary practice. Analgesics contemplated for use herein include narcotic analgesics such as morphine, hydromorphone, levorphanol and oxymorphone, agents of this kind being of use in the treatment of testicular pain, such as that associated with testicular cancer. Other testicularly acting drug species will be apparent to those skilled in the art. Presently preferred drug species contemplated for administration to the testes utilizing the present dihydropyridine == pyridinium salt carrier type redox system are the hormonal agents, principally the androgens, i.e., testosterone and its close analogues such as methyltestosterone; and the anticancer agents, especially those known to be useful in the treatment of testicular cancer, i.e., chlorambucil, melphalan, methotrexate and cisplatin-type compounds.

It will be appreciated that by "dihydropyridine carrier" or "[DHC]", there is intended any nontoxic carrier moiety as hereinbefore defined comprising, containing or including the dihydropyridine nucleus, whether or not a part of any larger basic nucleus, and whether substituted or unsubstituted, the only criterion therefor being capacity for BBB (or BTB) penetration and in vivo oxidation thereof to the corresponding. quaternary pyridiniun salt carrier [QC]^{+.} As aforesaid, the ionic pyridinium salt drug/carrier prodrug entity [D-QC]⁺ which results from such in vivo oxidation is prevented from efflux from the brain (or testes), while elimination from the general circulation is accelerated. Subsequently, the covalent or equivalent bond coupling the drug species [D] to the quaternary carrier [QC]⁺ is metabolically cleaved which results in sustained delivery of the drug [D] in the brain (or testes) and facile elimination of the carrier moiety [QC]^{+.} Such "covalent or equivalent bond" between the drug and the quaternary carrier can be a simple direct chemical bond, e.g., an amide, an ester, or any other like bond, or same can even be comprised of a linking group or function, e.g., a thiazolidine bridge or a peptide linkage, typically necessitated when the drug species is not susceptible to direct chemical coupling to either the dihydropyridine carrier or the quaternary carrier. Nonetheless, the bond in the formulae [D-QC]⁺ and [D-DHC] is intended to be, and is hereby defined as inclusive of all such alternatives. And the cleavage of the [D-QC]⁺ prodrug to sustainedly delivery the drug species [D] in the brain (or testes) with concomitant facile elimination of the carrier moiety [QC]⁺ is characteristically enzymatic cleavage, e.g., by esterase, amidase, cholinesterase, hydrolytic enzyme, or peptidase, albeit any type of in brain (or in testis) cleavage which might result, whether enzymatic, metabolic or otherwise, of course remains within the ambit of this invention. Thus, the drug release rate controlling parameter of the subject pro-prodrugs is imparted simply via the cleavable bonding between drug and carrier, and not by any release rate controlling substituent(s).

The expression "non-toxic pharmaceutically acceptable salts" as used herein generally includes the nontoxic salts of compounds of formula (I), wherein [D] is a centrally acting (or testicularly acting) drug species and [DHC] is the reduced, biooxidizable, blood-brain barrier penetrating form of a dihydropyridine 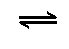 pyridinium salt redox carrier as hereinbefore defined formed with nontoxic, pharmaceutically acceptable inorganic or organic acids HX. For example, the salts include those derived from inorganic acids such as hydrochloric, hydrobromic, sulfuric, sulfamic, phosphoric, nitric and the like; and the salts prepared from organic acids such as acetic, propionic, succinic, glycollic, stearic, lactic, malic, tartaric, citric, ascorbic, pamoic, maleic, hydroxymaleic, phenylacetic, glutamic, benzoic, salicylic, sulfanilic, fumaric, methanesulfonic, toluenesulfonic and the like.

In one embodiment according to this invention, simple nontoxic carrier systems [D-QC]⁺ 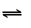 [D-DHC] are envisaged, utilizing a wide variety of models for D, such as those above outlined. Representative such carrier systems and models are: 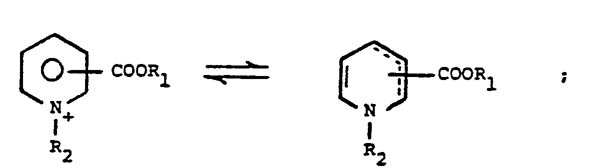 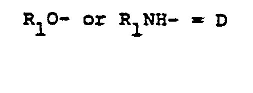 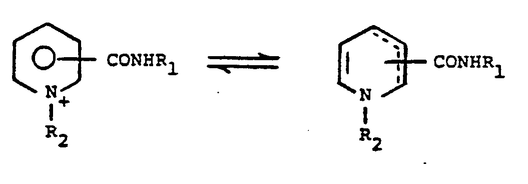 wherein R₂ is simply alkyl, e.g., CH₃, or benzyl, albeit virtually any other effective substituent is intended. Exemplary of such simple carrier systems are N-alkyl nicotinamide and nicotinate ester derivatives, tethered to such drug species as dopamine and melphalan. The trigonelline (N-methylnicotinic acid) system is quite effective as a carrier; it also is readily eliminated from the circulation and is virtually non-toxic.

Indeed, the present invention provides a flexible arsenal of dihydropyridine == pyridinium salt redox carriers for the site-specific/sustained delivery of virtually any centrally/testicularly acting drug species as hereinbefore defined to the brain/testes. Moreover, any dihydropyridine/pyridinium salt redox carrier entity as defined herein is contemplated and intended hereby generically, and any such carrier moiety need not be, and is not derivatized with a drug release rate controlling substituent critically tailored to meet, or be coordinated with, the chemical nature and delivery requirements of the particular drug species sought to be preferentially administered to the brain or testes. As utilized herein, the term "carrier" is to be understood as connoting just such a non-derivatized, non-drug/carrier coordinated entity, for consistent herewith it is the "carrier" entity itself and not the nature of any activity or release rate controlling/modifying substituent which is responsible for providing the desired brain-specific or testicular-specific result.

Additional examples of such redox carriers include the quaternary pyridinium alcohols (1), the analog isoquinoline acid and alcohol systems (2), and multicharged delivery forms, exemplified by structure 3 (D represents drug, Z a covalent link) and obviously the corresponding dihydro forms.

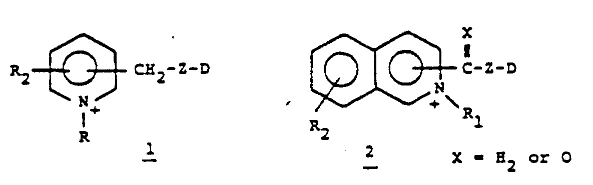

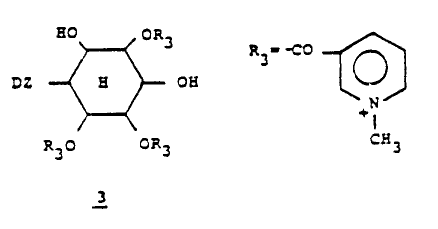

Yet other redox carriers include those comprising an acidic chain directly linked to the heterocyclic nitrogen, in quaternary or tertiary amine form. Also the hydroxide type carriers, e.g., the glucosamine analog indicated below. Representative are: 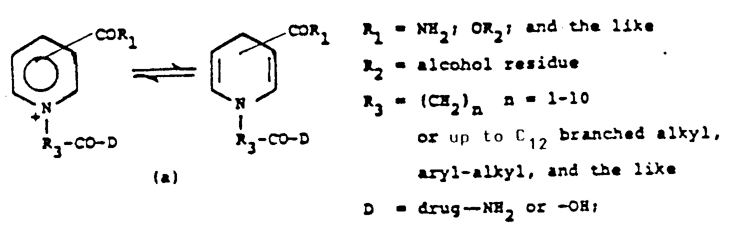 Preparation:

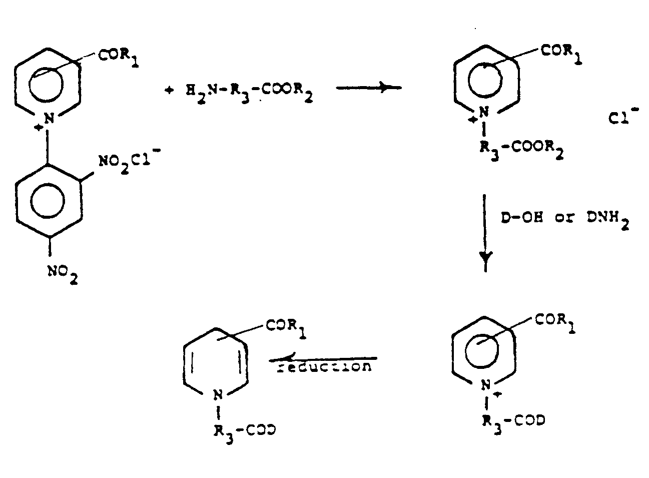

Method of: H. Lattre et al., Annalen, 579, 123 (1953).

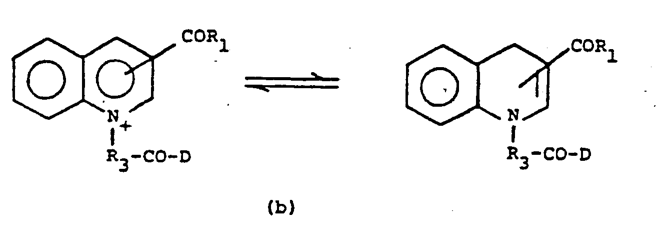

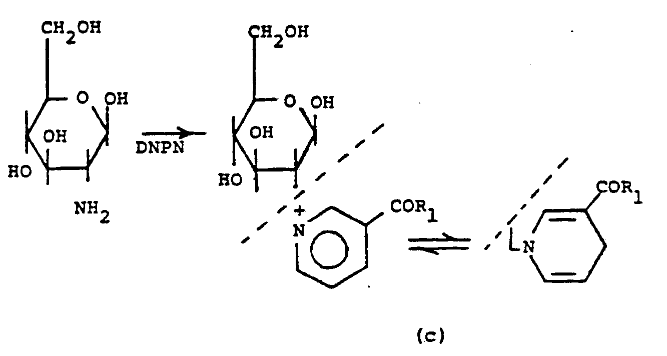

Generally preferred dihydropyridine 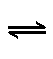 pyridinium salt redox carriers for use in the present invention include the following (where D represents the drug), and obviously the corresponding dihydro forms:
(a) the pyridinium systems 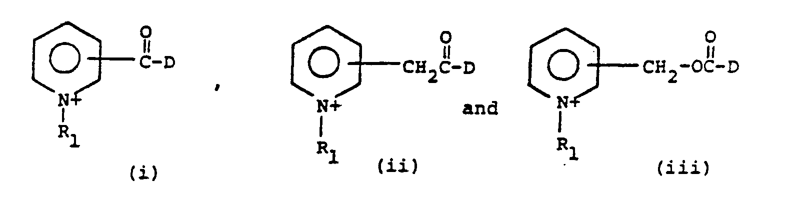 in which the depicted substituent is in the 2-, 3- or 4- position, and R₁, is C₁-C₇ alkyl or C₇-C₁₀ aralkyl, preferably methyl or benzyl;
(b) the pyridinium system 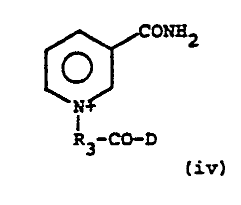 in which R₃ is C, to C₃ alkylene, i.e., (CH₂)ₙ where n=1-3;
(c) the isoquinolinium and quinolinium systems 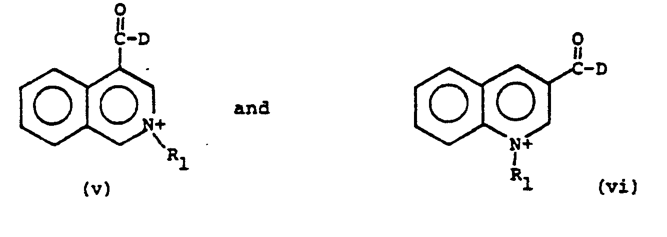 in which R₁ is defined as above; and
(d) the quinolinium and isoquinolinium systems 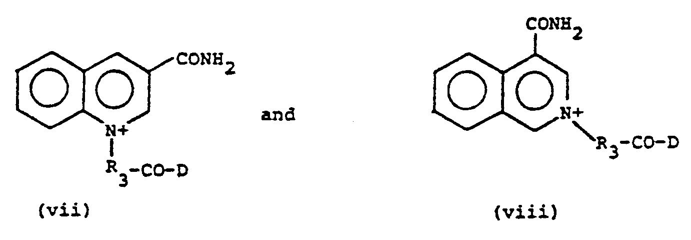 in which R₃ is defined as above. The corresponding dihydro forms of the foregoing preferred pyridinium salts are depicted below, wherein the position and identity of the structural variables are as indicated above:

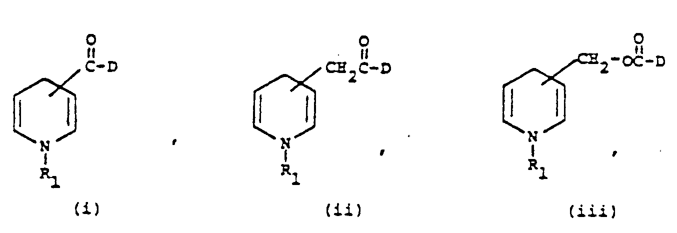

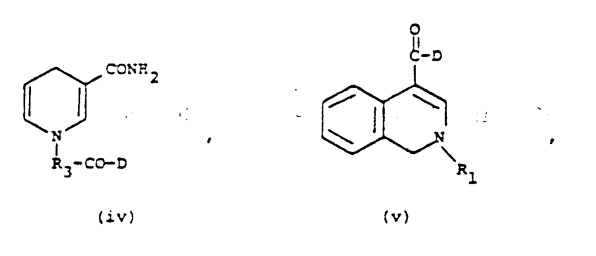

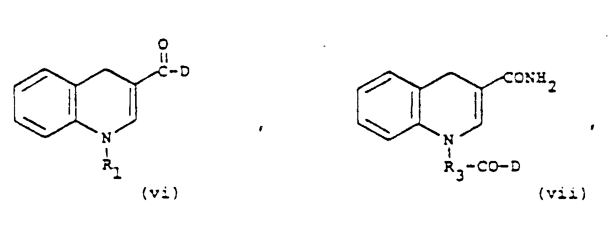

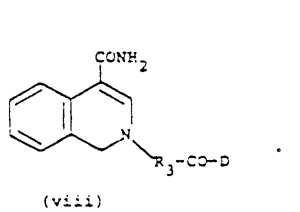

In a preferred embodiment of the present invention, sustained delivery of drug to the brain in pharmacologically effective concentrations has now been demonstrated, paralleled with much lower concentrations in the peripheral circulation and other tissues, utilizing dopamine as the target drug species and a trigonelline-type carrier system, with the catechol moiety thereof in certain instances being acylated, e.g., acetylated or pivalylated. According to Scheme 3 which follows, one specific delivery system for dopamine, compound 5, on administration (e.g., by injection) is distributed throughout the body and by reason of its lipophilic character facilely penetrates the blood-brain barrier and enters the CNS. Following oxidation both in the brain and in the other tissues, the corresponding hydrophilic quaternary salt (6) is formed. The quaternary salt 6 is essentially "locked in" the brain and its concentration is considered to increase with time until reaching a maximum, which depends primarily on the relative rates of entrance of the dihydro compound (5) to the brain (Kᵢ) as compared to K₂ to the other tissues, the rate of oxidation of the dihydro form to the quaternary (K₃ and K₇) and the rates of its disappearance from the brain (K₄ + Kₛ). At the same time, the very water soluble quaternary form(s) 6 is/are excreted readily via the kidney and the liver (K₈ » K₄). Derivatives 6 are considered to be essentially inactive forms (Kₐ » Kg), and thus systemic activity/toxicity is minimized. Hence, the concentration of 5 and in the blood rapidly increases. The ratio of the quaternary salt in the brain relative to the blood increases to the point where 6, or metabolites thereof, can only be found in the brain. The quaternary 6, whether in the brain, blood or other tissues, is deemed to release dopamine and the non-toxic compound, trigonelline, depending upon the rates of site-specific conversion of the precursor to the drug at each of these sites. The concentration of any released dopamine at any time is much higher in the brain than in the blood or other tissues. Also, as the enzymatic transformation of the quaternary percursor to the drug (dopamine) is relatively slow, same permits a sustained release of dopamine. Too, the simultaneous protection/lipophilic derivatization of the catechol system in dopamine has also now been demonstrated.

It will be appreciated that a compound of formula (I), such as compound 5, may be administered as depicted in Scheme 3, or in the form of a non-toxic pharmaceutically acceptable salt thereof, i.e., a salt which can be represented by the formula 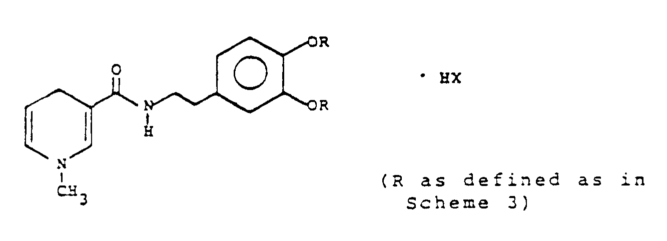 wherein HX is as defined before; and that, regardless of the actual form in which the compound is administered, it will be converted in vivo to a quaternary salt of the compound 6 type, the anion X- being an anion present in vivo. It is not necessary that the compound anion be introduced as part of the compound 5. And even when the compound is used in its salt form, the anion of is not necessarily the same as that present in compound 5. In any event, the exact identity of the anionic portion of the compound 6 is immaterial to the depicted enzymatic transformation.

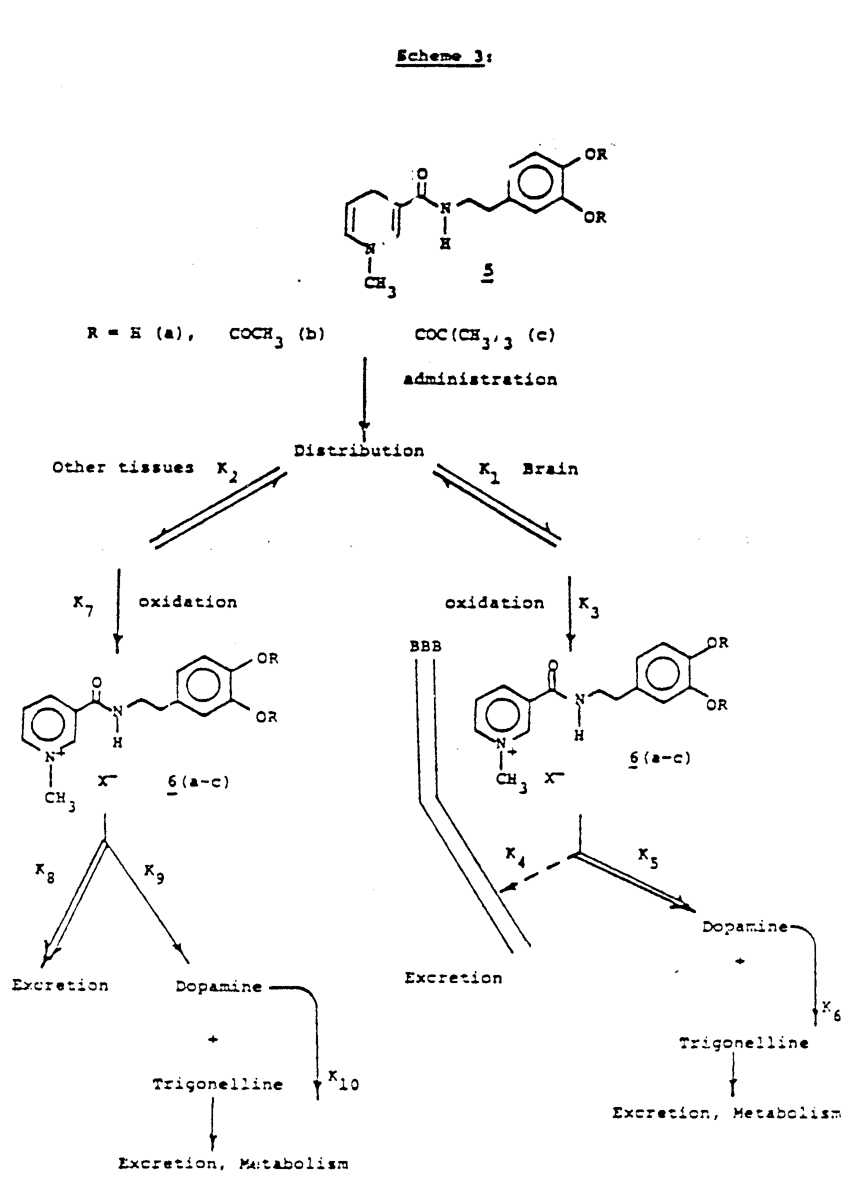

With specific reference to the immediately above, the 1,4-dihydropyridine derivatives (5) were prepared as in the following Scheme 4: 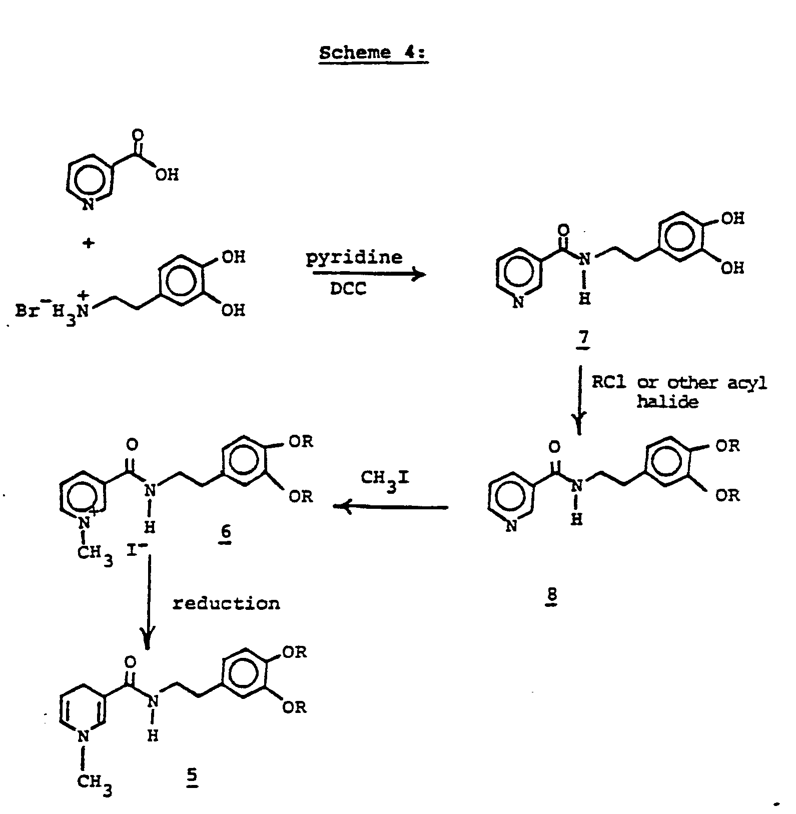
R = H (a), COCH₃ (b), COC(CH₃)₃ (c)
DCC = dicyclohexylcarbodiimide

Similar schemes can be shown for the preparation of the other dopamine derivatives of the invention. The step which introduces the protecting groups is of course only required when it is desired to protect the catechol hydroxyl groups. Moreover, when carbonate rather than acyl protecting groups are desired, the step of introducing the protecting groups will involve reacting thee catechol with a halocarbonate of the type Y'OCOCI or Y'OCOBr (formed by reaction of Y'OH with COCI₂ or COBr₂), rather than with an acyl halide YCI or YBr, Y and Y' being as generically defined hereinabove. Also, the order of steps shown in Scheme 4 may be altered; quaternization, followed by reduction, need not be in the final two steps but may be carried out earlier in the reaction sequence. Yet other reaction schemes and reactants (e.g., using an anhydride rather than an acyl halide to convert 7 to 8) will be readily apparent to those skilled in thee art, Scheme 4 being simply a preferred approach for the specific compounds there depicted. Variations of this approach are likewise applicable to preparing derivatives of other hydroxy-containing amines.

In an attempt to ascertain whether any biotransformation of the free catechol is taking place by COMT (catechol-O-methyltransferase) either before or after oxidation, the possible O-methyl metabolites (9 and 10) were synthesized separately following Scheme 4 with 3-methoxytyramine hydrochloride as the starting material.

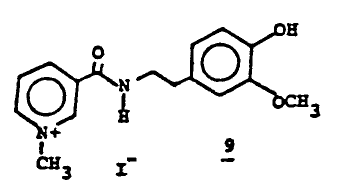

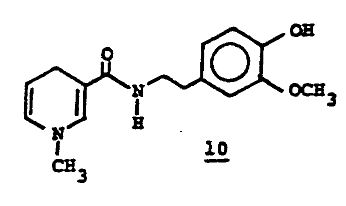

The stability of the 1,4-dihydropyridine derivatives (5) was determined in the presence of the oxidizing agents, alcoholic AgN0₃ and hydrogen peroxide. The in vitro rates of oxidation of the 1,4-dihydropyridine derivative (5c) in 80% plasma, 20% brain homogenate, 20% liver homogenate and in whole blood were determined.

The dihydropyridine derivative (5c) was then selected for the in vivo study. A solution in DMSO (dimethylsulfoxide) was injected through the jugular vein to a group of male Sprague-Dawley rats which were then sacrificed at various time intervals; their blood and brains were analyzed for the quaternary percursor of dopamine (6a). The in vivo dopaminergic activities of the selected compounds 5c vs. 6a were then determined.

Consistent with the above, it was found that N-nicotinoyldopamine (7) could be obtained in good yields by coupling dopamine hydrobromide with nicotinic acid in pyridine as a solvent and with dicyclohexylcarbodiimide as the coupling agent. Attempts to prepare 7 by using dopamine free base were largely unsuccessful. As for the catechol protecting groups, the acetyl and pivalyl moieties were selected due to their rather different steric and partitioning parameters. Acylation could be accomplished with the acyl chlorides by using conventional methods. Reduction of the quaternaries (6a-c and 9) was accomplished by using sodium dithionite in mildly basic aqueous solution, (NaHC0₃). It was observed that the dihydro compound obtained in the case of the quaternary 6b gave a faint green color with ferric ions, indicating partial hydrolysis of at least one of the acetyl moieties during reduction, even in the cold, weakly basic solution used as a medium. The dihydropyridine derivatives isolated (5a-c and 10) were determined to have the expected 1,4-dihydropyridine structure, based on their NMR and UV spectra. Attempts to prepare the ;8- protonated enamine salts of the isolated dihydro derivatives were also largely unsuccessful, due to acid catalyzed addition reactions The 1,4-dihydropyridine derivatives (5a-c) were found to be relatively stable towards oxidation. Compound 5c was quantitatively oxidized to the corresponding quaternary salt 6c by H₂0₂ or alcoholic AgNO₃ solution.

The diacetyl derivatives (5b and 6b) appeared to be labile to hydrolysis and therefore were not pursued in vitro. The dipivalyldihydro derivative (5c) was thoroughly investigated for its in vitro rates of disappearance and metabolic degradation in various biological fluids. It is evident that 5c represents a rather complex case, as besides oxidation, a two-step hydrolysis will also take place. Scheme 5 illustrates the interconversion of the possible components.

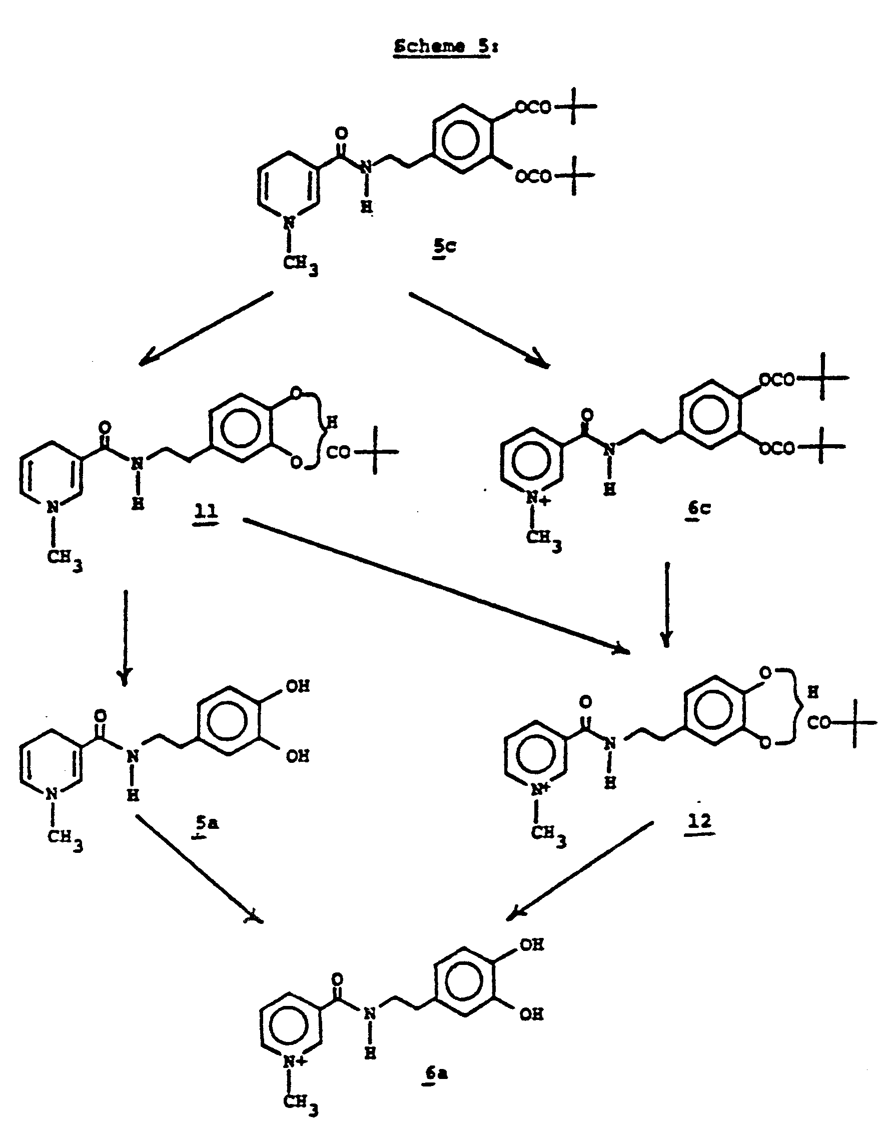

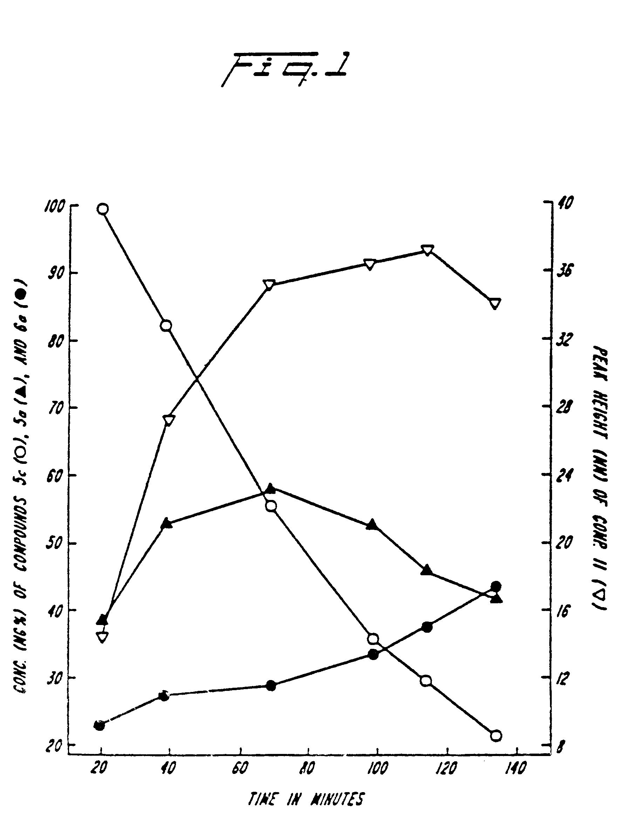

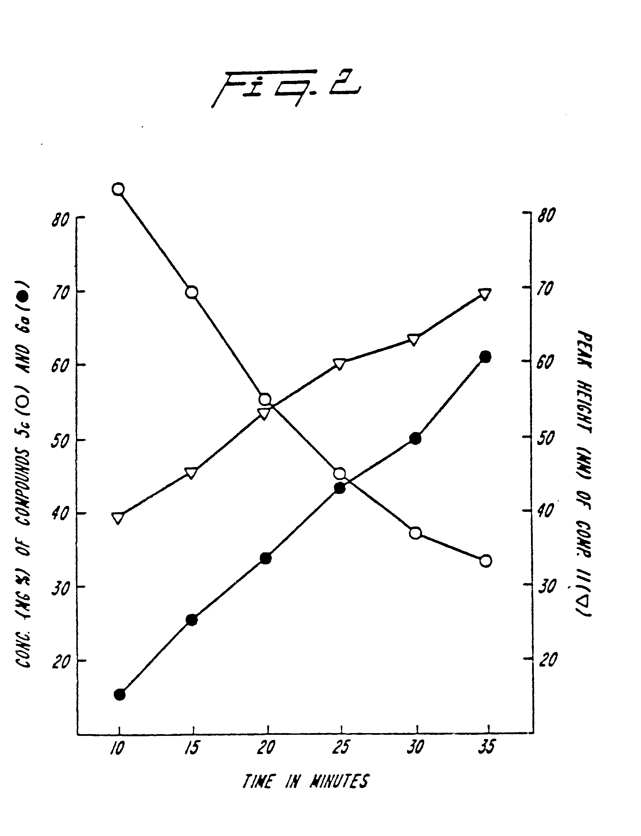

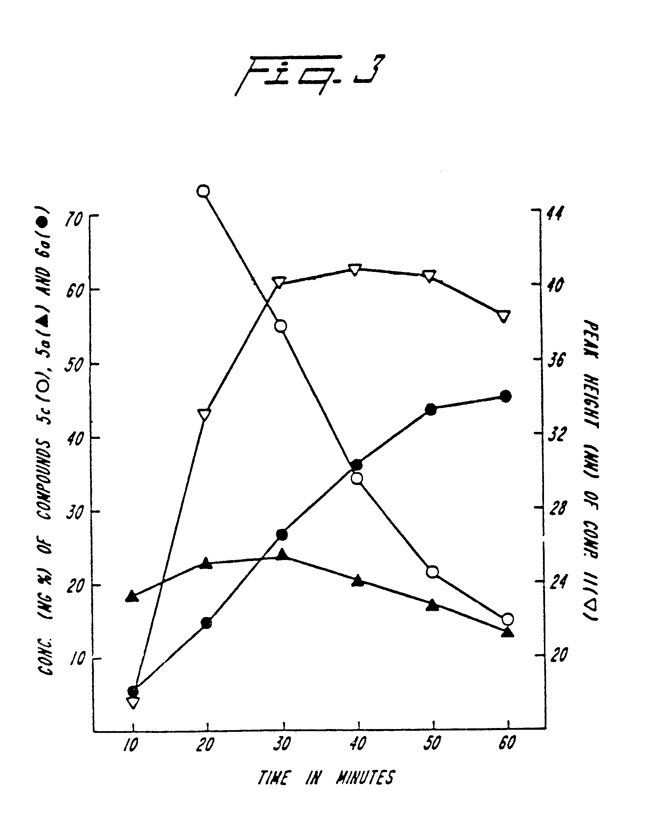

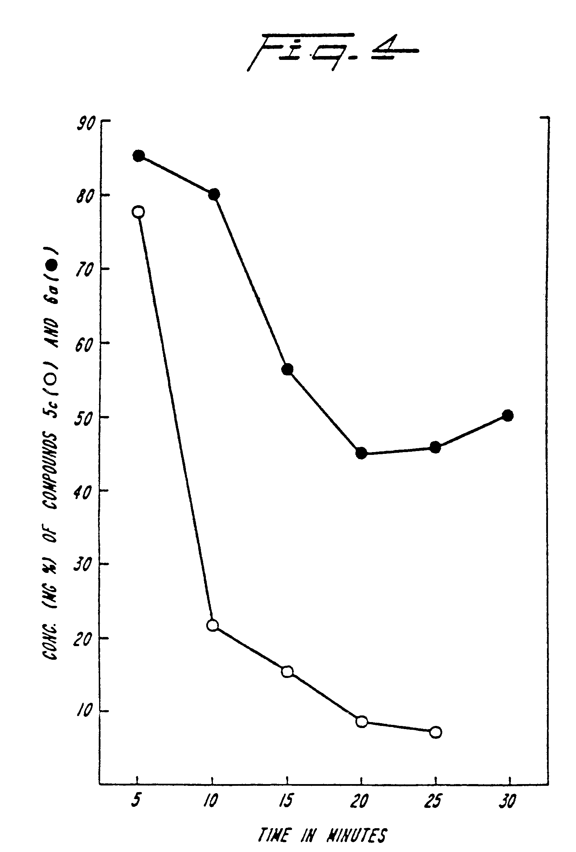

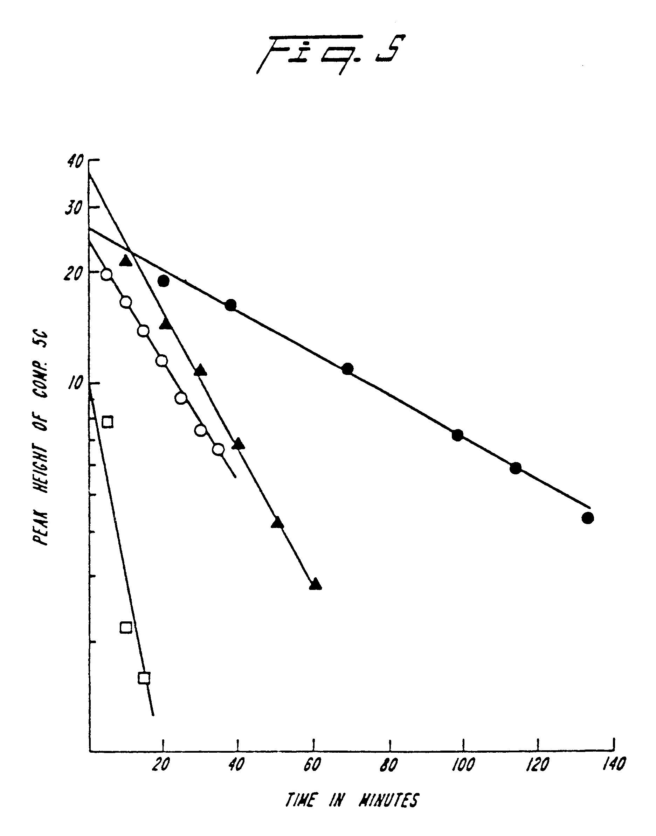

Figures 1-4 illustrate the results of such an investigation. The apparent half-lives for the disappearance of 5c in biological fluids at 37°C were calculated. Although the process does not truly follow first order kinetics, the data fit very closely a pseudo first order process (Figure 5). The obtained values, 51 min (80% plasma), 17 min (20% brain homogenate), 18 min (whole citrated blood) and 6 min (20% liver homogenate), reflect an acceptable stability of the dihydro derivative 5c. The disappearance of 5c is accompanied by formation of some monoester (11) and dihydroxy dihydro form (5a) in all the media except the liver homogenate. The rate of hydrolysis of the first ester moiety is faster than the second and a reasonable amount of monoester 11 builds up with time. The monohydroxy quaternary 12 could not be detected except in the blood as a very small peak which does not change significantly with time. A steady increase in the concentration of the dihydroxy quaternary 6a was observed in all media except liver homogenate. Thus, it is established that this derivative, 6a, is forming as the main product of the various interconversion routes and it is the direct precursor thus concluded to be locked in the brain in the in vivo experiment. No formation of the methoxy derivatives and 1 could be detected in ay of the biological fluids studied; 5a and 6a do not appear to be good substrates for COMT.

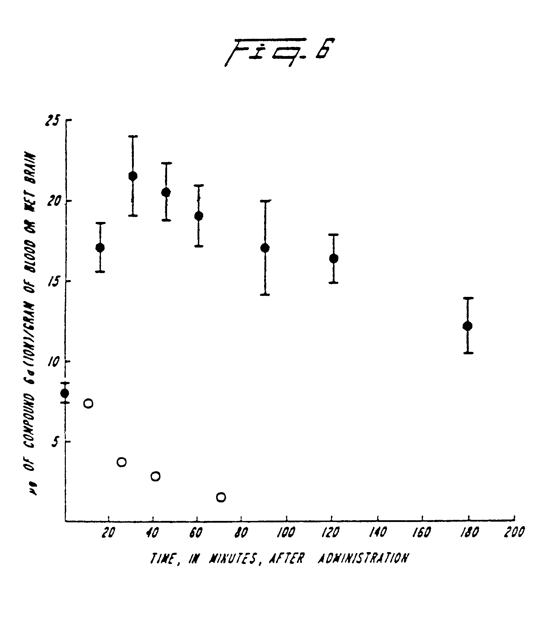

The first objective of the in vivo studies was to trace the appearance and disappearance of 6a in blood and brain following administration of 5c. Figure 6 summarizes such results, and is consistent with the mechanism shown in Scheme 3. After one single injection of the 1,4-dihydropyridine derivative 5c to the rat, the dihydroxy quaternary 6a (ion), which is the only detectable derivative, could be seen to appear and then to disappear quickly from the blood, with a half-life of 27 min. On the contrary, the concentration of 6a (ion) is increasing in the brain steadily, reaching a maximum at about 30 min following administration. The descending portion indicates a half-life of disappearance from the brain of about 3.2 h. No formation of O-methyl metabolites (9, 10) could be detected in the brain. This confirms the in vitro results that 6a (or 5a) is not a good substrate for COMT.

To determine whether dopamine itself was finally released in the brain upon completion of the aforesaid complex delivery process, 5c was administered intrajugularly and changes in brain-dopamine concentrations following that administration were studied. Some of the rats showed up to threefold increase in the dopamine concentrations, others practically none. Since it is possible (and even desired) that the intrinsic brain metabolism of the dopamine does not permit significant build-up of its concentration, specific pharmacologic activity was investigated, using changes in the in vivo prolactin secretion. It is known that dopamine and its agonists decrease prolactin secretion following their binding to stereospecific receptors located on lactophors in the anterior pituitary (AP) gland [G.P. Mueller, J.W. Simpkins, J. Meites and K.E. Moore, Neuroendocrinology, 20, 121 (1976); W. Wuttke, E. Cassell and J Meites, Endocrinology, 88, 737 (1971); J.A. Clemens, E.B. Smalstig and C.J. Shaar, Acta Endocrinol., 79, 230 (1975)]. This effect is dose- dependent and it can also be observed in vitro, incubating anterior pituitaries with dopamine or its agonists [R.M. MacLeod Ed. L. Martini and W.F. Ganong, Raven Press].

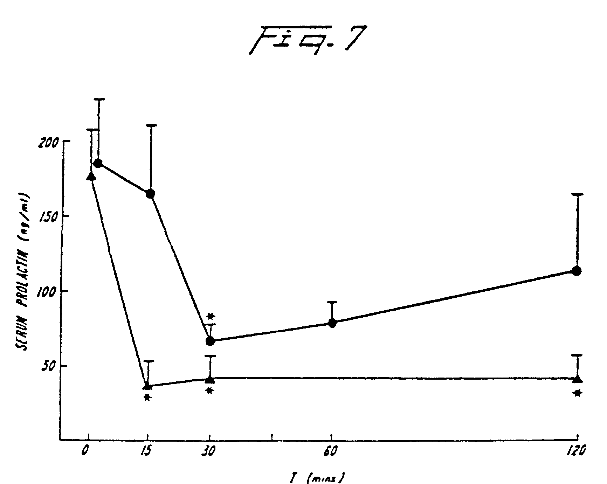

It was then determined that exposure of male rats to 17-,8-estradiol for two days elevated serum prolactin levels to greater than 150 ng/ml. Intravenous administration of 5c caused a 79% decrease in serum prolactin concentrations and this dramatic reduction was maintained through 120 min after treatment. In contrast, 6a had no significant effect on the serum prolactin concentrations by 15 min, and caused a 67% reduction by 30 min. Thereafter, serum prolactin levels increased progressively to levels which are not significantly different from vehicle injected controls, by 60 and 120 min. These results are summarized in Figure 7. The rapid onset and prolonged inhibitory effects of 5c on prolactin secretion is consistent with the time course of the appearance of 6a in the brain following administration of 5c. The "trapping" of 6a in the brain subsequent to I.V. injection of 5c provides a constant source of a potent dopaminergic agent, either dopamine or 6a itself. The significantly lower effect of 6a when administered I.V. does not unequivocally clarify which alternative is the more responsible. This was resolved by in vitro comparison of the relative activities of dopamine versus 6a.

Fresh anterior pituitaries obtained from female rats were incubated with various concentrations of dopamine (DA) and 6a, respectively, and their effects on the rate of release of prolactin were measured. It was found that at 2x10⁻⁸M concentrations, neither DA nor 6a had any effect, but at 2x10-⁷ M, DA caused a 57% reduction of the prolactin rate secretion, while 6a had no effect. These results are summarized in the following Table I.

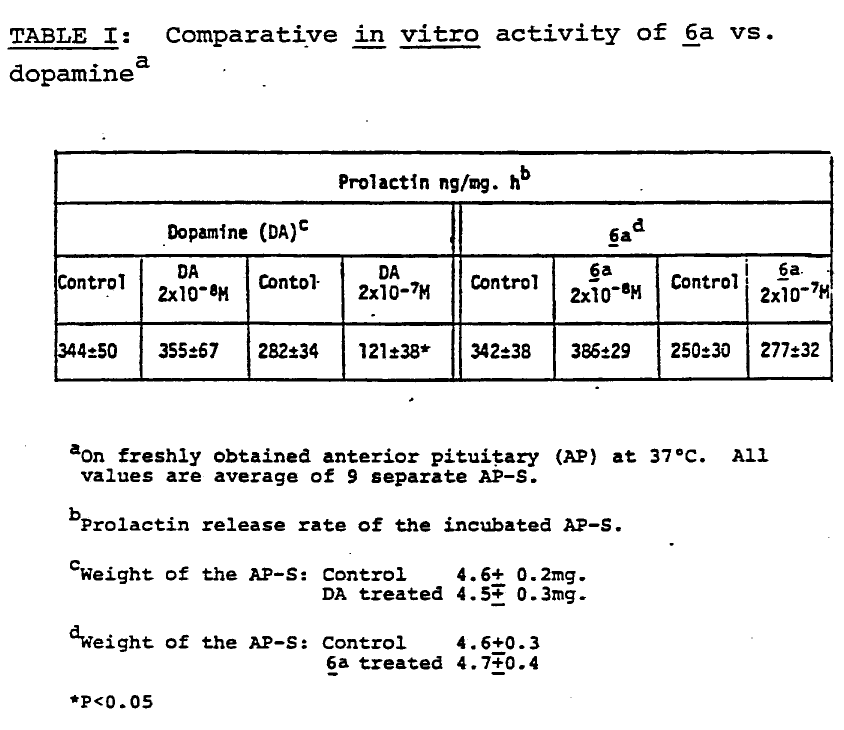

These results indicate that if 6a has any activity, it must be significantly less than that of DA. Based on the delayed onset of the activity when 6a was administered I.V. and considering the in vitro results, it logically follows that the high and prolonged activity of the 6a locked in the brain following administration of 5c is due to the fact that 6a is slowly releasing the active DA in the brain.

Accordingly, provided hereby is a potent, brain-specific dopaminergic agents comprising a lipophilic dihydropyridine carrier-type chemical delivery system of dopamine ["pro-prodrug" or "pro-pro-prodrug" in the case of the catechol protective group(s)], which penetrates the BBB by passive transport. The rapid oxidation in the brain of the carrier moiety to the corresponding quaternary pyridinium salt results in an activated amide of dopamine. The oxidation process is much faster than amide cleavage of the beginning compound or of 6. Moreover, the ionic nature of the activated quaternary salt results in a significant slowdown of the efflux of this specific form through the BBB, resulting in a selective concentration enhancement of the precursor 6a in the brain. Too, brain-specific dopaminergic activity is assured, logically as dopamine is released from this activated form upon hydrolytic, enzymatic or metabolic cleavage, as is facile excretion of the carrier moiety from the brain.

In yet another embodiment of the invention, like synthesis of the analogous tyramine system has been carried out, and the corresponding determinations made. Such tyramine system is represented as follows:

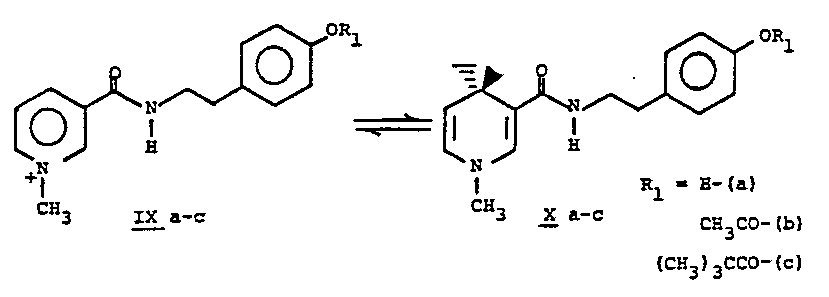

Naturally, selection of the particular dihydropyridine == pyridinium salt redox carrier to be used will depend on the chemical structure of the specific drug involved. And not only should the nature of the functional group which is to be linked to the carrier system be considered in selecting the carrier, but the manner in which the ultimate compound is prepared should be tailored to the presence of any other reactive groups in the molecule. The following examples of specific drug/carrier combinations and their manner of synthesis are set forth for the purpose of illustration only and are not to be considered limitative in any way whatsoever.

Thus, in one specific illustration, the selected drug is 17a-ethynyltestosterone, estradiol or the like and the selected carrier system is trigonelline 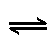 dihydrotrigonelline; according to this embodiment, the drug is reacted with nicotinoyl chloride, the resultant ester is then quaternized with methyl iodide, and the quaternary iodide is then reduced with Na₂S₂0₄ to afford the drug-CDS (chemical delivery system).

Another specific illustration involves selecting melphalan and the same type of carrier system as above, but forming an amide rather than an ester linkage. Thus, melphalan is converted to its hydrobromide, which is reacted with nicotinic acid to afford the amide having the formula 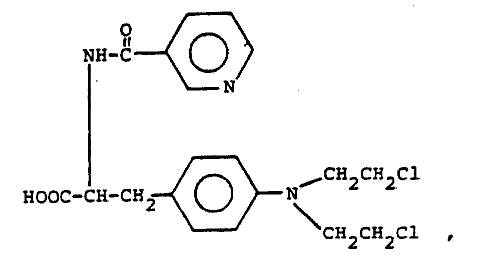 which can be esterified, if desired (to increase lipoidal characteristics), followed by, when the ethyl ester is prepared, quaternizing same with methyl iodide to form 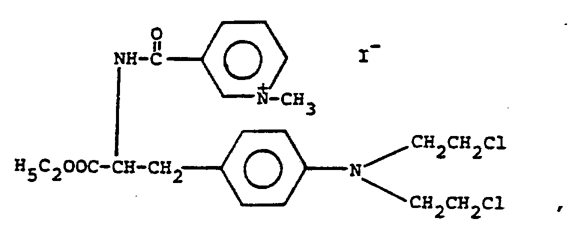 which can then be reduced to afford the melphalan-CDS 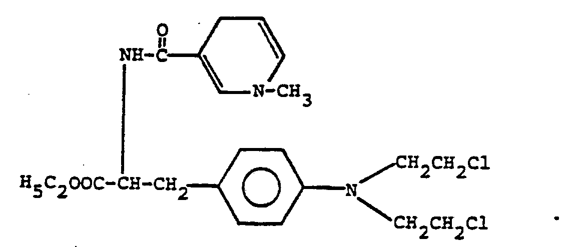 As one of several alternative schemes, melphalan can be derivatized by first esterifying it, e.g., to convert the carboxy function to the ethyl ester, then reacting the resultant melphalan ethyl ester with nicotinoyl chloride to form the amide of the formula 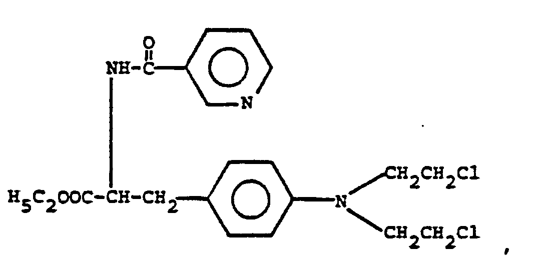 which can then be quaternized and the quaternary salt subsequently reduced as indicated above to afford to same melphalan-CDS as depicted above.

Yet another specific illustration utilizes chlorambucil as the target drug, in which case the desired nicotinic acid carrier system is linked to the drug via a bridging group. Thus, nicotinic acid can be reacted with an appropriate di- or polyhydroxy compound such as ethylene glycol, propylene glycol or inositol and the resultant intermediate is linked via its free hydroxy group(s) to the carboxylic acid function of chlorambucil. That intermediate is then quaternized and the quaternary salt is reduced to afford the chlorambucil-CDS. In the case of nicotinic acid and ethylene glycol starting materials, the chlorambucil-CDS has the formula

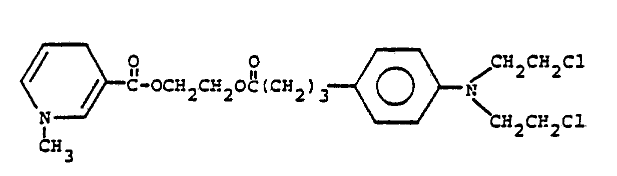

On the other hand, when a polyhydroxy compound is reacted with nicotinic acid in the first step, a variety of products are possible. Thus, for example, when inositol is used, the final product may contain anywhere from 1 carrier/5 drug residues to 5 carrier/1 drug residue. In the case of the inositol trinicotinate intermediate 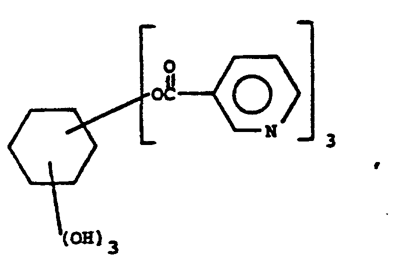 conditions for reacting same with chlorambucil can be selected so that one, two or three of the hydroxy functions react with the acid. When all three hydroxys react, the ultimate chlorambucil-CDS has the formula 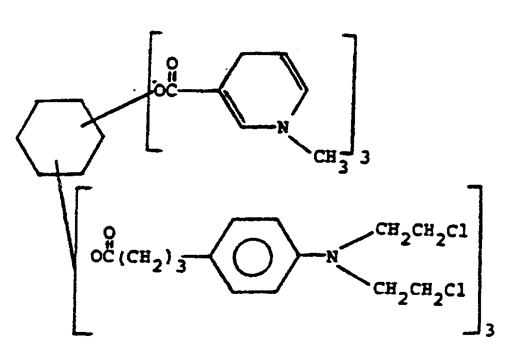 and contains 3 drug residues and 3 carrier groupings.

As another example, methotrexate, which has the structural formula 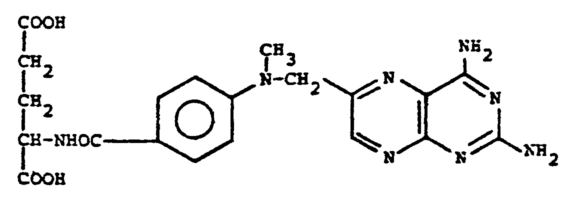 can be derivatized similarly to chlorambucil via its carboxy function(s), e.g., utilizing the inositol trigonel- linates or a glucosamine analogue.

As a further example, podophyllotoxin and its derivatives can be linked to a carrier system of this invention. These drugs can be represented by the structual formula 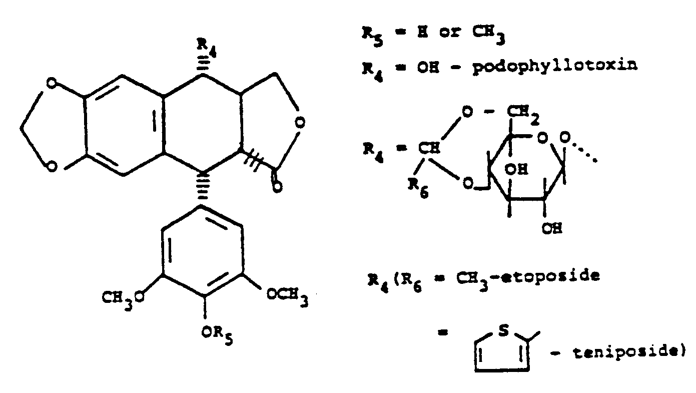 and can be derivatized by reacting the hydroxy group in podophyllotoxin (R₄ = OH) or the hydroxy groups in the glycosidic portions in R₄ with acidic type redox carriers, e.g., in a inner analogous to the testosterone-CDS depicted above. Known cisplatin analogues, in which typically the amino groups have been replaced with organic radicals, can be similarly derivatized according to the invention, the method of choice depending on the nature of the functional groups in the organic radicals.

Similarly, syntheses and like determinations as regards the redox carrier-linked enkephalins can be carried out. First synthesized is the known leucine enkephalin XI. The quaternary pyridinium analog XII, the corresponding O-benzyl ether XIII and the amide XIV are next synthesized.

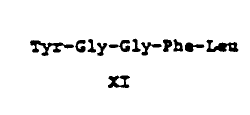

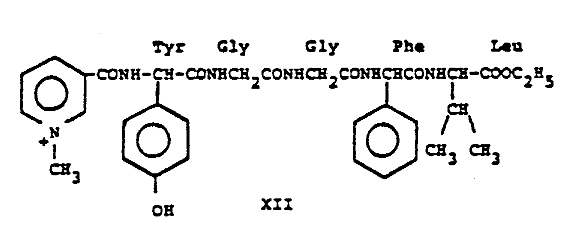

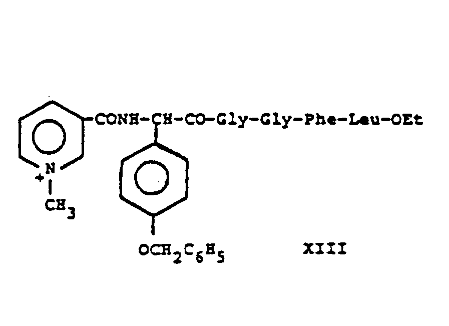

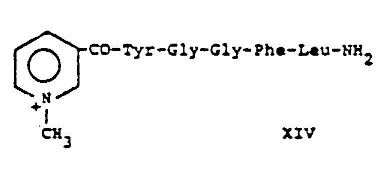

The O-benzyl pentapeptide ethyl ester derivative of XI is synthesized, sequentially and then coupled with nicotinic acid, followed by methylation. Alternate methods involve introduction of carrier at earlier stage in the synthesis. The reduction of XII and XIII results in a mixture of products due to the base sensitivity of the ester. Likewise prepared are the corresponding leucinol trigonelline ester XV and its dihydro derivative XVI.

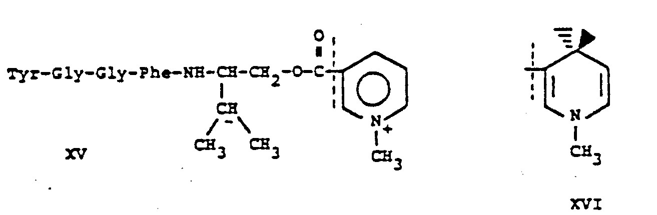

Thus, the site-specific brain delivery of the enkephalins for the treatment of epilepsy is established consistent with the Scheme 1, as is their analgesic activity.

Similarly, as regards the benzodiazepine tranquilizers, e.g.:
1. Oxazepam: 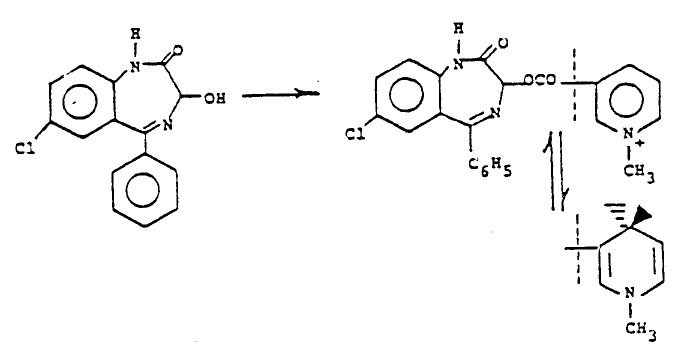
2. Diazepam: 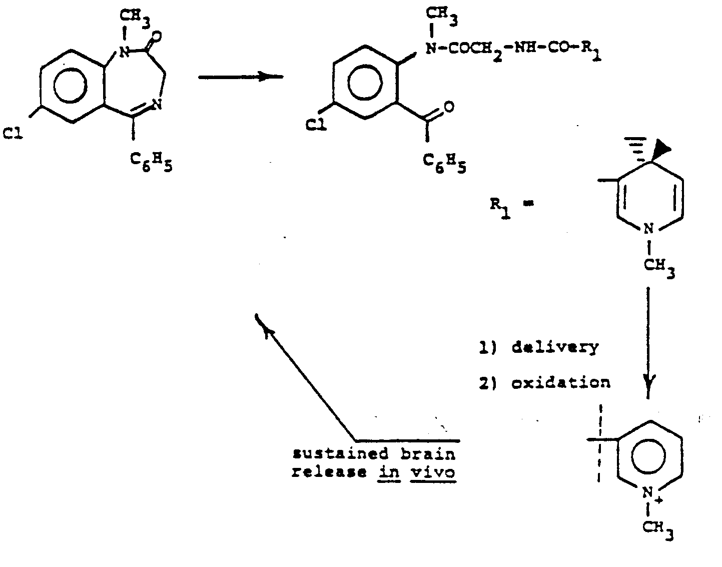
This reaction scheme utilizes conventional opening of the 7-member ring, accompanied by coupling of the drug to the carrier. The following drugs can be similarly derivatized to the corresponding dihydro derivatives:

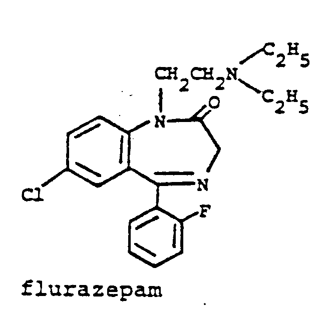

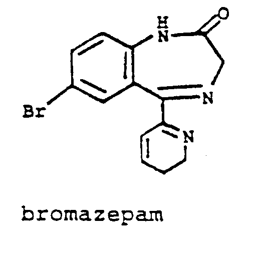

And in another preferred embodiment of the invention, there is provided the effective, selective and nontoxic treatment of epilepsy, based upon the mechanism illustrated in Scheme 1. Indeed, commencing from the "GABA-hypothesis" of epilepsy, the brain-specific, enhanced and sustained release of GABA (y-aminobutyric acid) itself, and various other compounds either directly or indirectly affecting the concentrations of GABA in the brain, is circumscribed consistent herewith. Model compounds include carboxylic acids, most specifically valproic acid, as well as some of the GABA analogs which inhibit irreversibly the GABA-T, such as y-vinyl and/or y-acetylenic GABA. Using the aforesaid trigonelline (N-methylnicotinic acid) 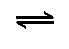 dihydrotrigonelline system, for example, the selected compounds can be effectively delivered per Scheme 1. Thus, representative target compounds are the dihydropyridine carrier-drug combinations 1 and the corresponding pyridinium carrier-drug species, for example, GABA and its esters: 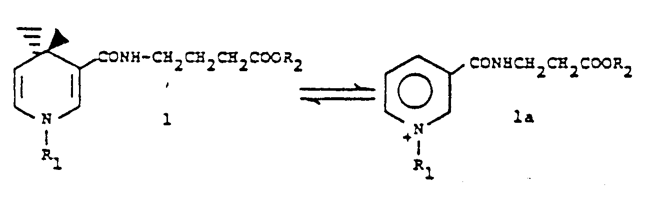
R = CH₃, C₃H₇ or CH₂C₆H₅
R₂ = H, C₂Hs, CH(CH₃)₂, etc.

Related derivatives for y-vinyl and y-acetylenic GABA are:

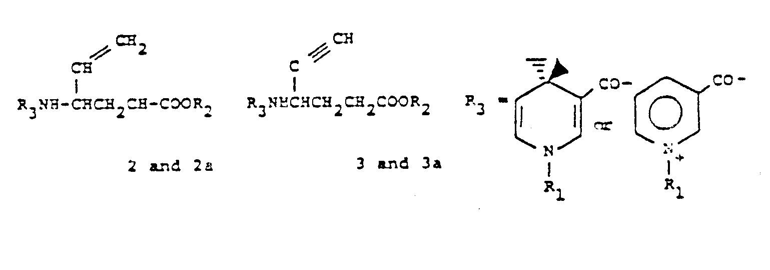

In the case of valproic acid, other alternatives are:

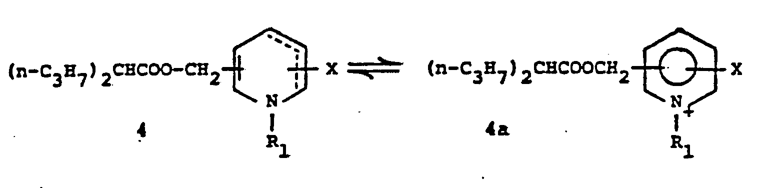

X = H, CONH₂, CHNOR₂, etc.

In another embodiment of like delivery system, applicable for both the GABA and related compounds and for the carboxylic acids, or for any other drug species to be linked to such a carrier, either directly or indirectly, i.e., mediated by a carboxylic acid, e.g., succinic acid, or other linkage, provided is a mono-or poly-substituted nontoxic polyol (such as inositol or sugars) having the trigonelline gonelline system and the compounds to be delivered linked to the same molecule as exemplified by the GABA case (5 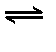 5a) and valproic acid (6 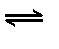 6a): 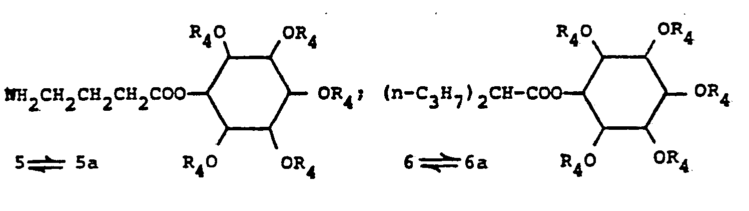 R₄ = H, GABA or valproic acid, but at least one of R₄ is: 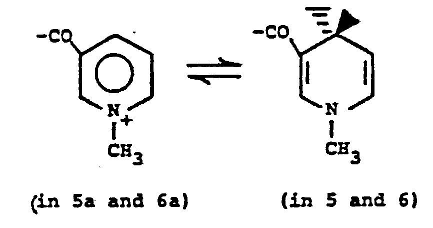 R₄ can be partially replaced by additional GABA or valproic acid, changing the carrier/drug ratio as necessary. Some of the valproic acid metabolites can be coupled with carriers of the redox type, via the various hydroxy groups formed during the oxidative degradation: 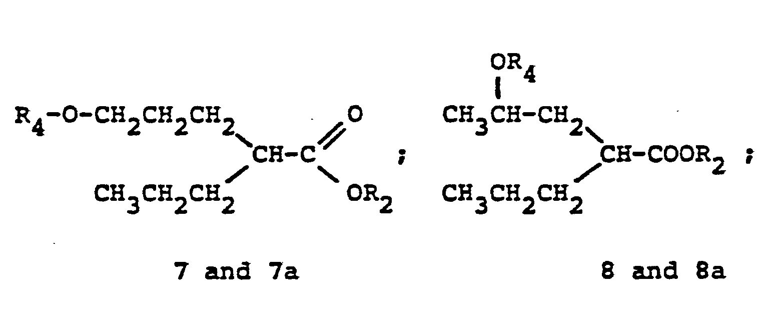 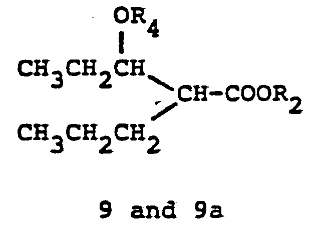 Illustrative examples are the corresponding derivatives of the 5-, 4-, and 3-hydroxy-2-n-propyl pentanoic acid derivatives. Additional carrier systems, such as the isoquinoline == dihydroisoquinoline system can also be developed consistent herewith.

Moreover, based upon the observation that NADH content is significantly reduced in epileptic and like seizures, the use of the subject redox system (in reduced form) will bias the NAD = NADH balance towards NADH during the dihydro carrier - quaternary transformation. Also, the brain-specific delivery of small peptides consistent herewith, e.g., the enkephalins which have been found to initiate epileptic seizures, has led to the design of a variety of long lasting potent antagonists.

And the subject chemical delivery system is also useful for the delivery of other anticonvulsants in a sustained, brain-specific fashion, e.g., the benzodiazepines and hydantoins, and those compounds, like apomorphine, which are useful in the treatment of photosensitive epilepsy.

It will of course be appreciated in the immediately above regard that the drug treatment of epilepsy has always posed formidable problems. There are many different anticonvulsants available, some more specific for different types of seizures. Indeed, there exist a wide variety of opinions as to which is the most suitable drug for any particular type of seizure, and drug mixtures are typically employed. An inevitable result of the traditional therapy is the development of chronic toxicity, but such result is conspicuously avoided according to the present invention.

It too will be appreciated that the desired therapeutic effects of all antiepileptic agents investigated, as well as their undesired toxic effects, reflect a statistically significant correlation with the drug levels in plasma. This correlation is based upon a close relationship between the drug concentrations in plasma and brain tissue. In contrast, a primary attribute of this invention is to enable attainment of high and sustained brain levels of the selected active agents, essentially against the plasma-brain concentration gradient and independent of the drug concentration in the blood.

GABA and related compounds are logical candidates. It has been shown that GABA neuron function is impaired in at least certain types of human epilepsy. Animal studies also showed that seizures are induced by reduction of GABA neuron function to a critical degree by (1) inhibition of GABA synthesis, (2) blockade of GABA receptors or (3) inhibition of GABA-receptor mediated ionic events. In addition, enhancement of GABA synaptic activity (by direct receptor stimulation or by increasing GABA levels in the synapse) has a potent and wide spectrum anticonvulsant effect. These findings foreshadowed that an enhanced and sustained GABA brain delivery or a brain-specific delivery in a sustained manner of a good GABA-agonist would be efficacious in different forms of epilepsy It is well known that GABA itself, when administered systematically, does not penetrate the normal blood-brain barrier to any significant extent. Among the potential sites at which drugs may act to influence GABA-mediated synaptic function, the first target is to affect the BBB transfer of GABA via a redox delivery system. The second main target is to effect the catabolism of GABA. This invention, accordingly, specifically provides for the efficacious delivery of the GABA-T inhibitors, y-vinyl and y-acetylene-GABA, but the delivery of valproic acid, specifically to the brain and without requiring high circulating blood levels, is also envisaged. In order to achieve the required activity, sodium valproate must have a relatively high, 50-100 ug/ml, level in the blood. The value of valproic acid is well established in most types of epilepsy. It is evident that valproic acid produces significant increases in both brain and synaptosomal GABA concentrations. Valproic acid itself undergoes extensive metabolism.

In capsule summary, the present invention provides for the significantly improved treatment of epilepsy, and concomitant reduction in toxicity of a number of antiepileptic drug species currently in use. And made available to the brain is a variety of important compounds, such as GABA and a wealth of GABA-ergic agents.

It will be apparent from the foregoing discussion of specific drug-carrier combinations that a wide variety of synthetic approaches can be utilized, depending on the chemical nature of the selected drug. Various illustrative synthetic schemes as applied to specific centrally acting drugs in accord with this invention are set forth below in the section entitled "Illustrative Synthetic Methods". While the sequence of reaction steps can be varied in many cases, in general, the final step (except in the case of optional salt formation) will be reduction of a pyridinium compound of formula (II) to the corresponding dihydropyridine compound of formula (I). The reduction is usually conducted at a temperature from about -10°C to room temperature, for a period of time from about 10 mins to 2 hrs, conveniently at atmospheric pressure. Typically, a large excess of reducing agent is employed, e.g., a 1:5 molar ratio of reducing agent to starting [D-QC]⁺ compound. The process is conducted in the presence of a suitable reducing agent, preferably an alkali metal dithionite such as sodium dithionite or an alkali metal borohydride such as sodium borohydride or lithium aluminum borohydride, in a suitable solvent. Sodium dithionite reduction is conveniently carried out in an aqueous solution; the dihydro product [D-DHC] is usually insoluble in water and thus can be readily separated from the reaction medium. In the case of sodium borohydride reduction, an organic reaction medium is employed, e.g., a lower alkanol such as methanol, an aqueous alkanol or other protic solvent.

Suitable nontoxic pharmaceutically acceptable carriers (i.e diluents or vehicles) for use with the topic compounds [D-DHC], e.g., those less toxic than the target drug species themselves, will be apparent to those skilled in this art. Compare, for example, Remington's Pharmaceutical Sciences, 4th Edition (1970). Obviously, the choice of suitable carriers (i.e diluents or vehicles) will depend upon the exact nature of the particular dosage form selected, as well as upon the identity of the active drug species [D]. The therapeutic dosage ranges for administration of the compounds according to this invention will generally be the same as, or less than, those characteristically used in this art for administration of the known drug species [D], per se. Naturally, such therapeutic dosage ranges will vary with the size of the patient, the condition for which the [D-DHC] compound is administered, the particular dosage form employed, and the like. The quantity of given dosage form needed to deliver the desired dose of [D] will of course depend upon the concentration of [D-DHC] in any given pharmaceutical composition/dosage form thereof.

The ability of the topic compounds to cross the BBB, as well as the BTB in the male, and to be "locked into" the brain and testes allows administration of the drug in a site-specific manner. A combination of the present dihydropyridine == pyridinium salt redox system with a sustained release system will further enhance this site-specificity. Thus, a preferred embodiment of the invention comprises formulating the [D-DHC] compound or the salt of a [D-DHC] compound utilizing a sustained release carrier (ie diluent or vehicle) system and/or route of administration capable of slowly releasing the chemical, e.g., sustained release tablets and capsules for oral administration; subcutaneous injection, or implantation of drugs in solid pellet form (for example, distributed in a biodegradable polymer); intramuscular injection of the compound in solution in oil or suspended in a repository vehicle; a transdermal delivery device or form such as an ointment to be applied locally to the desired site (when the drug is susceptible of delivery through the skin) and the like. The rate of release of compound from the sustained release system should be comparable to the rate of in vivo oxidation of the dihydro form of the redox system in order to achieve the greatest degree of enhancement of specificity.

### Illustrative Synthetic Methods

### I. Methods for Derivatizing -NH₂ or -NH- Functions in Drugs

### Method A

The drug is reacted with nicotinoyl chloride, with nicotinic anhydride, or with nicotinic acid in the presence of a suitable dehydrating agent such as dicyclohexylcarbodiimide, in an appropriate organic solvent, to afford the corresponding nicotinamide. The nicotinamide is then quaternized, typically by treatment with methyl iodide in a suitable organic solvent, to afford the quaternary derivative [D-QC]⁺, which is then reduced by treatment with sodium dithionite or sodium borohydride as generally described hereinabove to afford the desired compound [D-DHC].

The representative drugs listed below may be derivatized in this manner to the corresponding [D-QC]⁺ and [D-DHC] compounds.

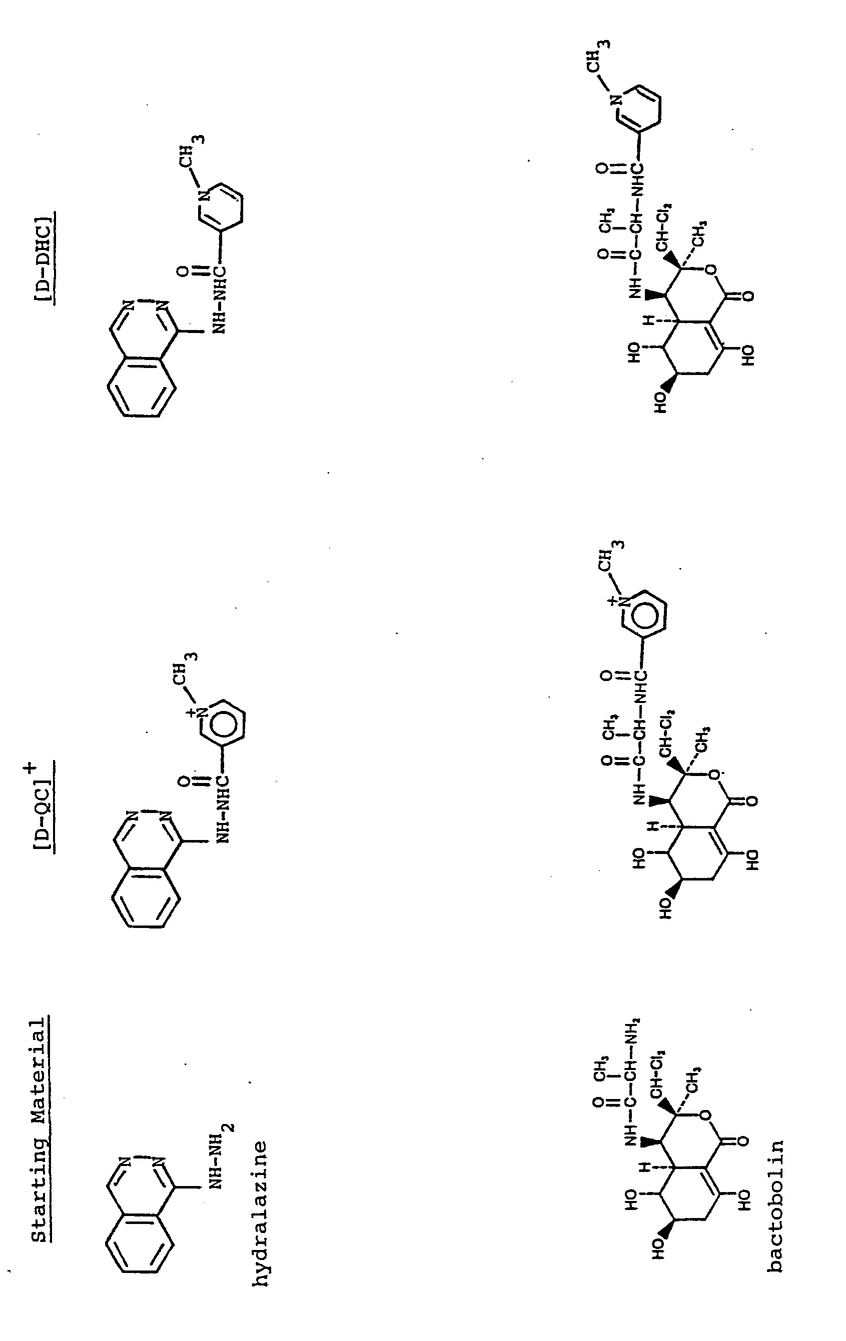

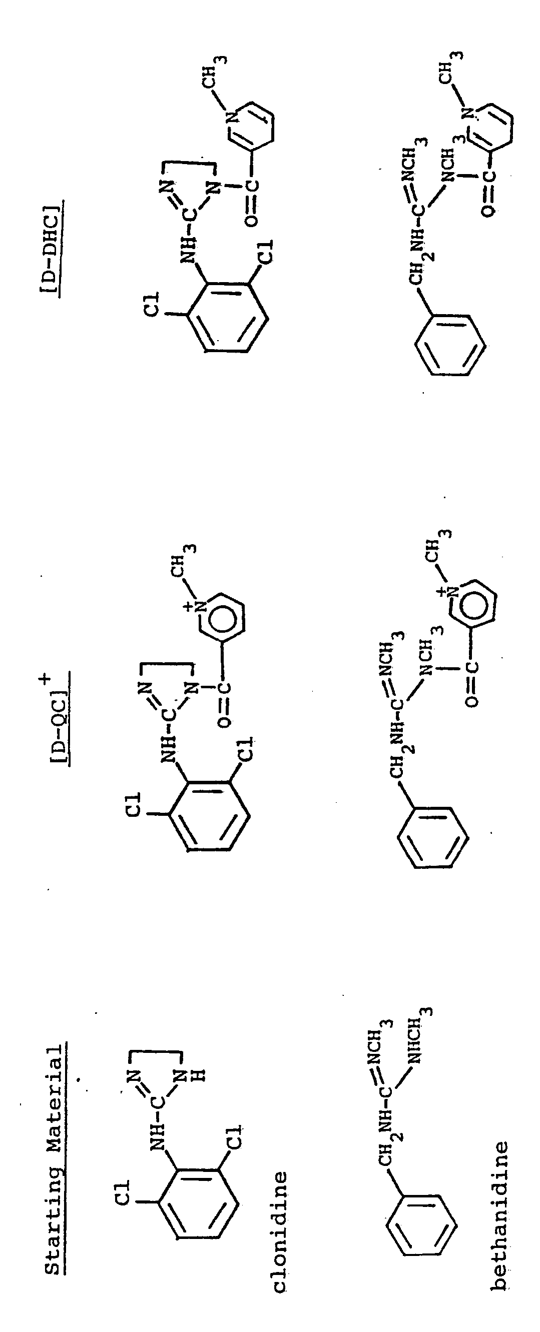

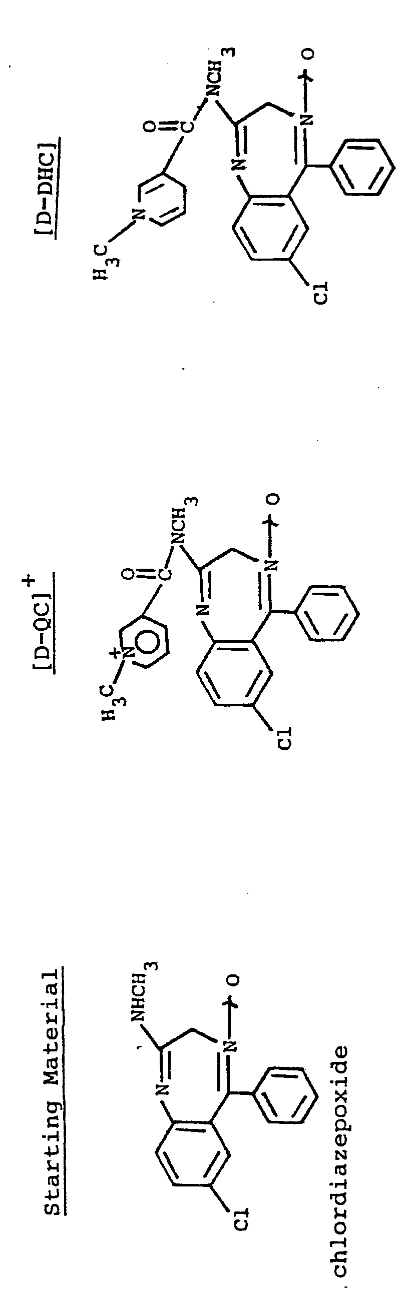

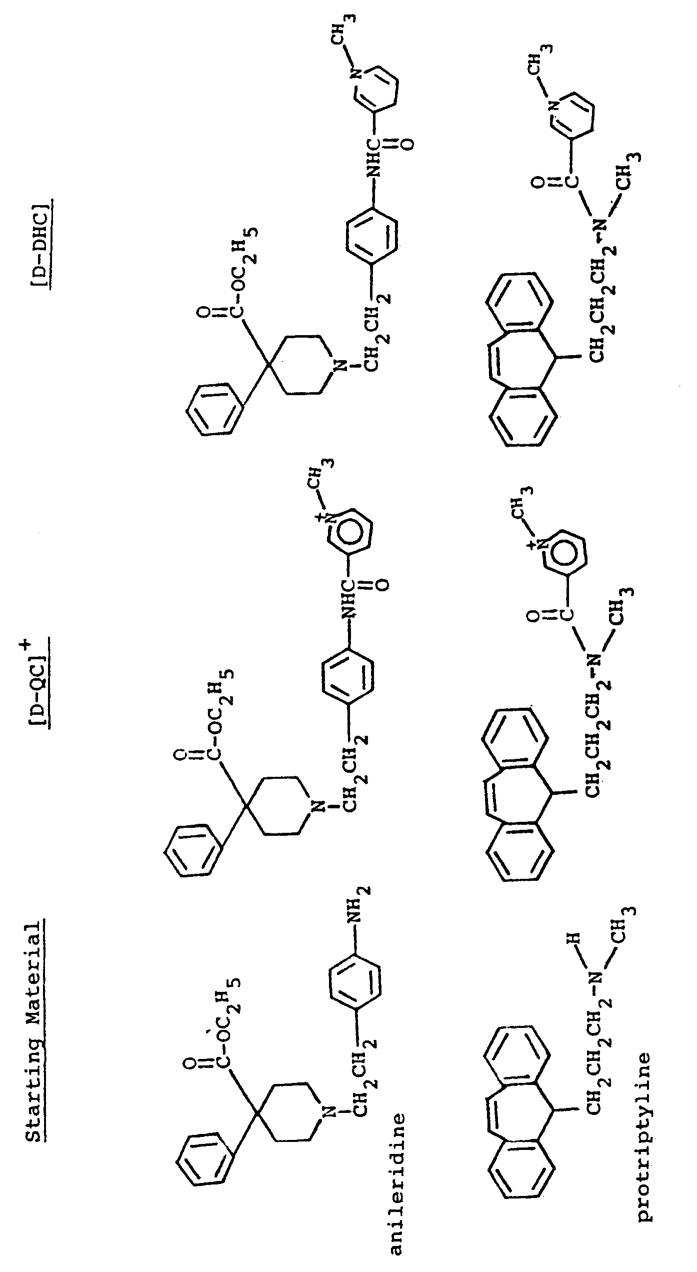

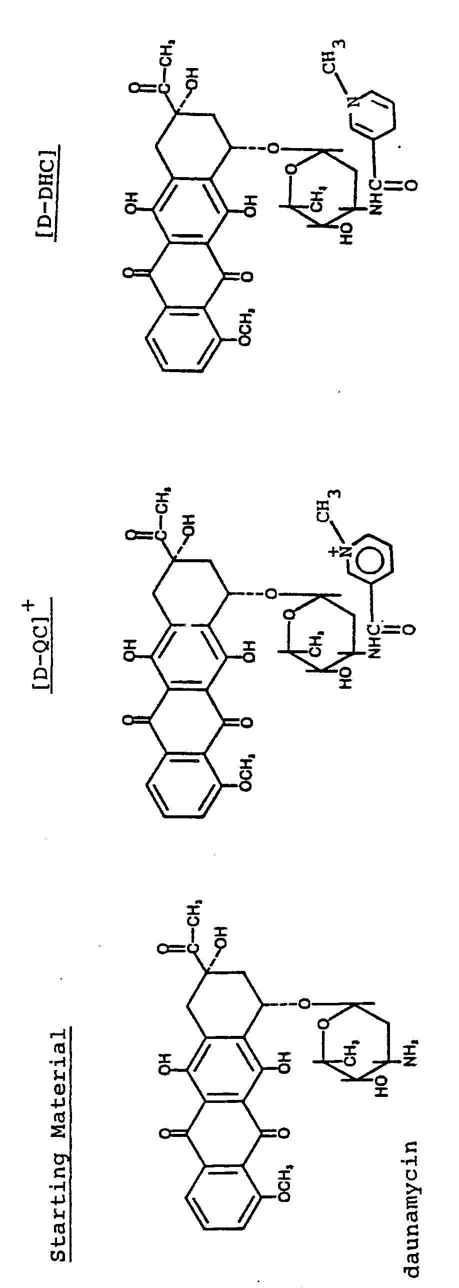

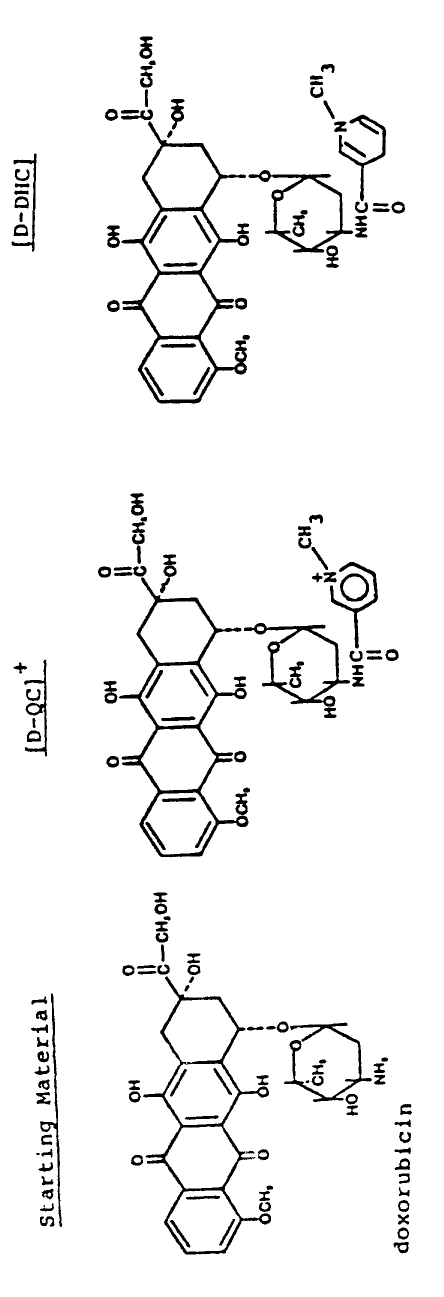

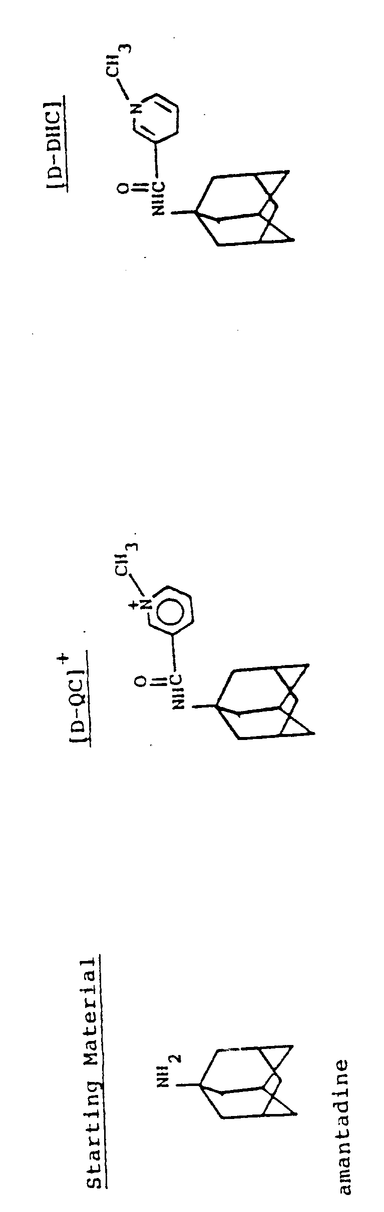

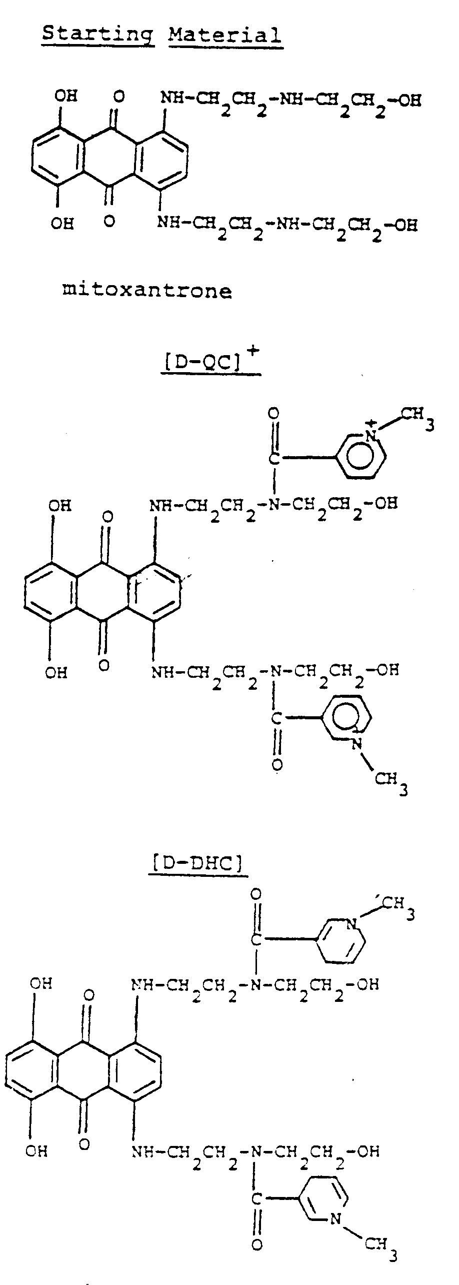

### Method B

This is a variation of Method A used when the drug contains a -COOH function which is to be protected.

The drug is first converted to the corresponding ethyl ester by conventional esterification techniques. That ester is then used as the starting material and Method A is repeated.

Obviously, other esters may be similarly prepared in the first step by use of other esterifying agents.

The representative drugs listed below may be derivatized in this manner to the corresponding [D-QC]⁺ and [D-DHC] compounds.

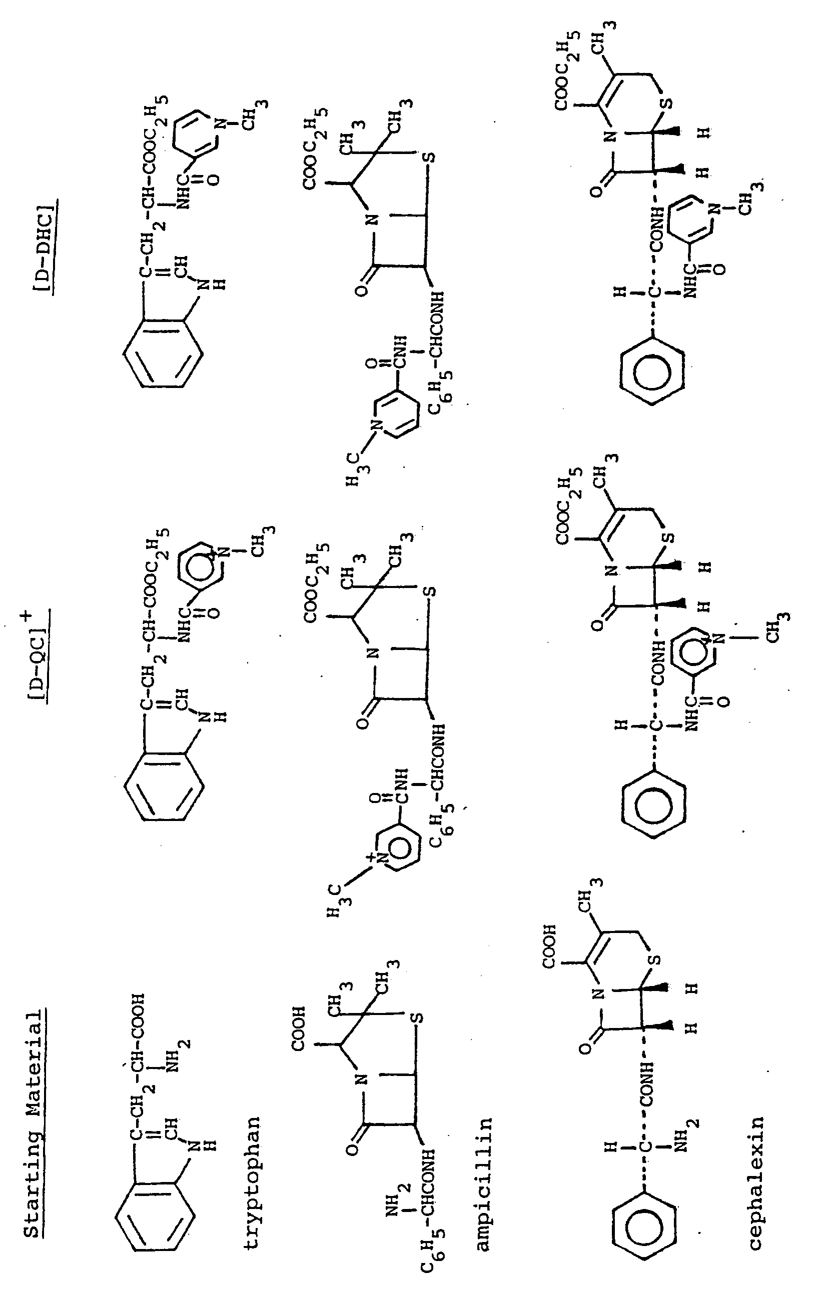

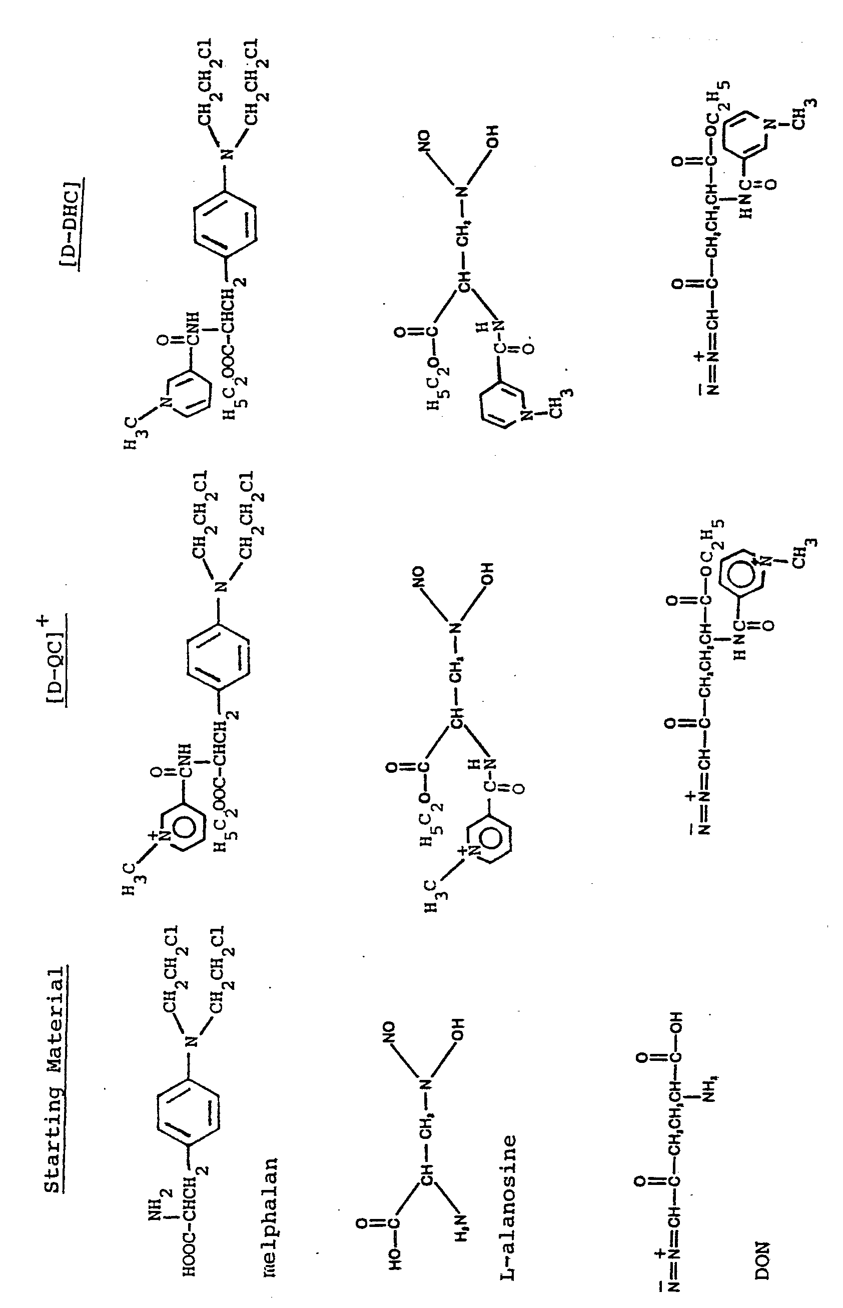

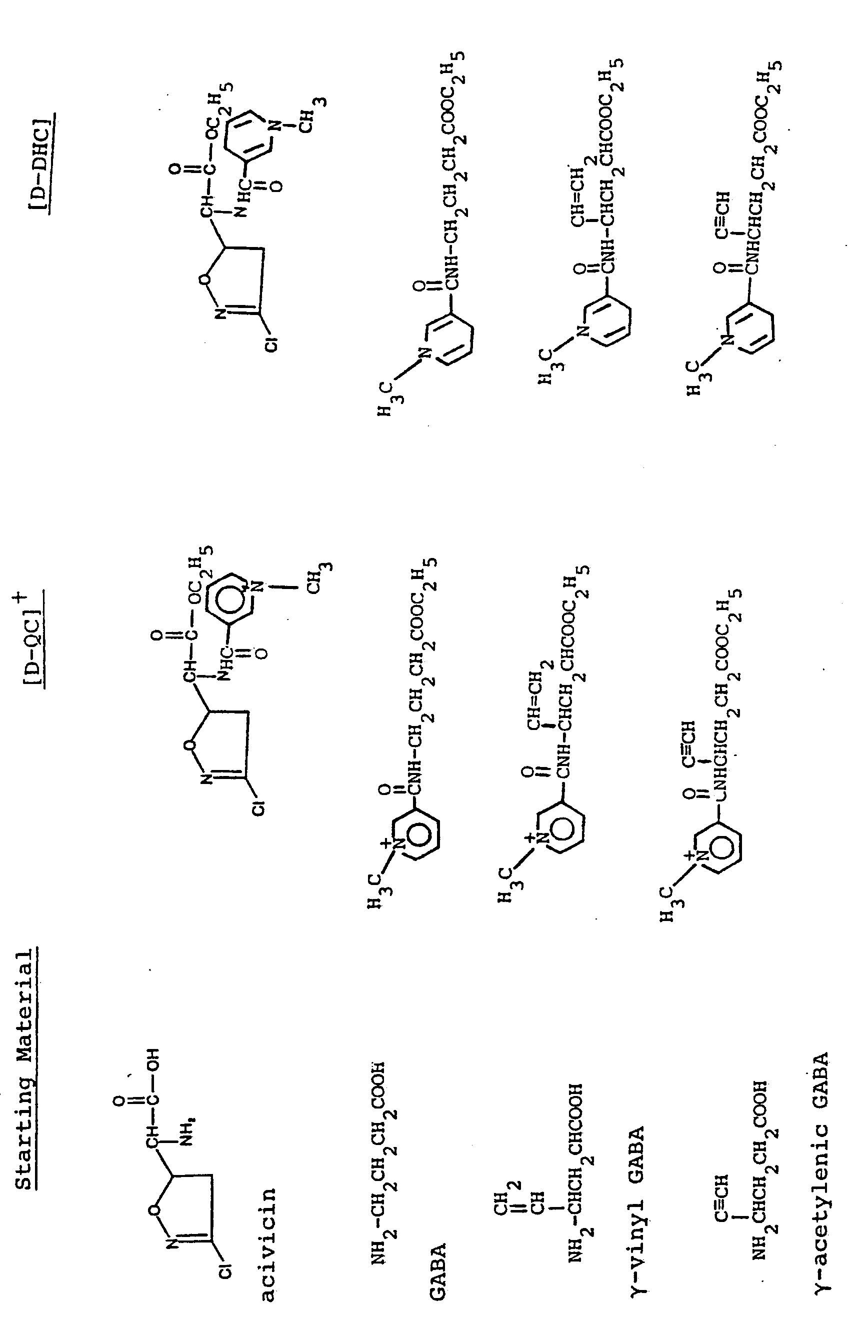

### Method C

This is a variation of Method A used when the drug contains one or more OH functions which are to be protected.

The drug is first reacted with excess trimethylacetyl chloride to convert the hydroxy group(s) to pivalyloxy group(s). (This process is generally conducted in the presence of a base; however, strongly acid conditions are used if an amine function is present.) That protected derivative is then used as the starting material and subjected to Method A. Alternatively, the first two steps may be reversed, i.e. the drug may be first converted to the nicotinamide, which may then be reacted with trimethylacetyl chloride to form the protected nicotinamide.

Various other hydroxy protecting groups may be introduced in similar fashion.

The representative drugs listed below may be derivatized in this manner to the corresponding [D-QC]⁺ and [D-DHC] compounds.

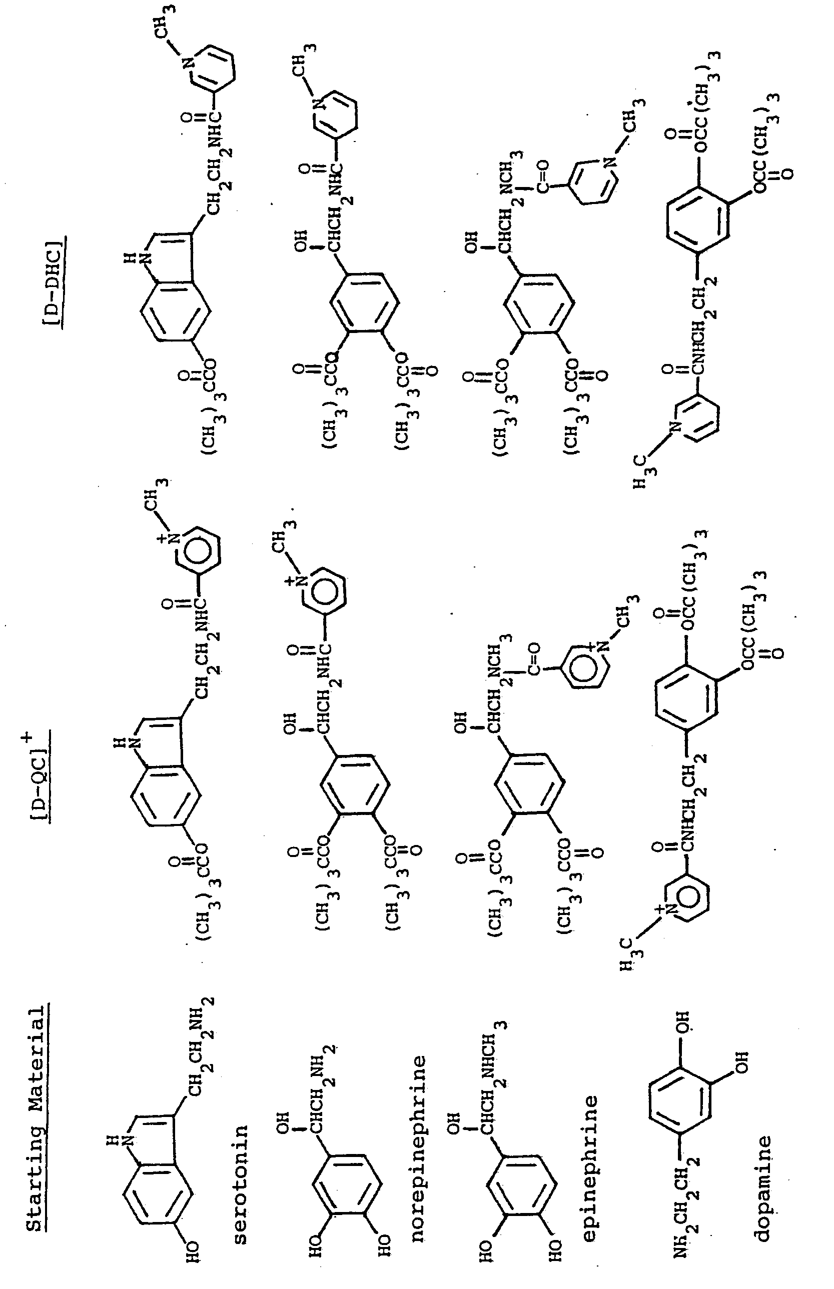

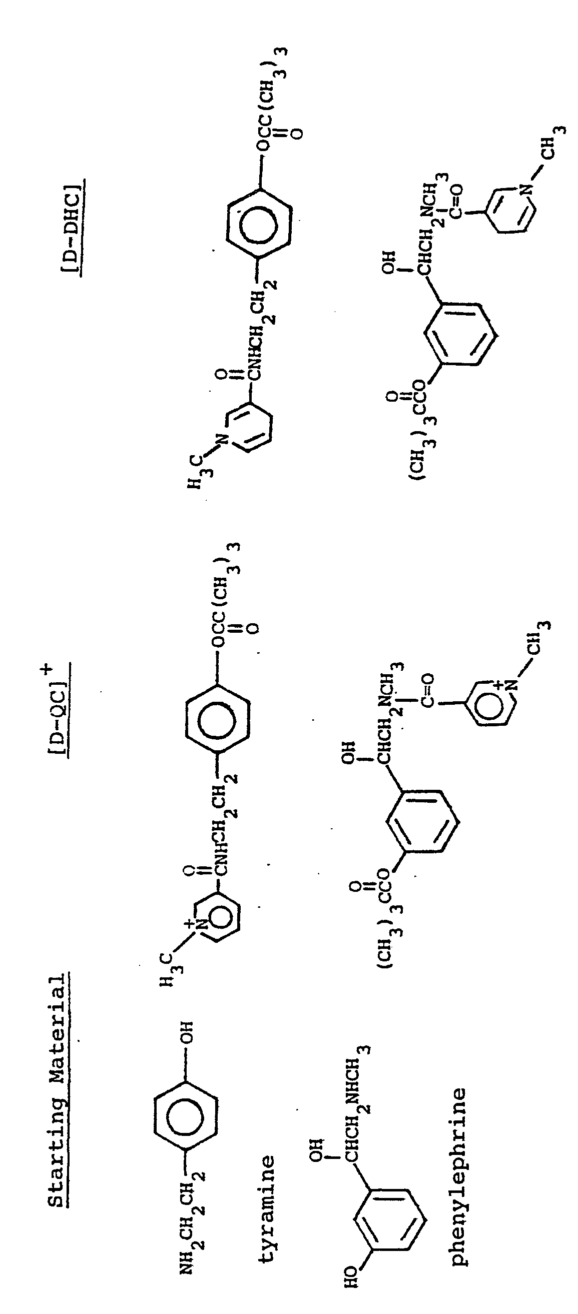

### Method D

This variation of Method A can be used when the drug contains one or more OH and COOH functions which are to be protected. The protecting groups, typically the ethyl ester and pivalyloxy groups, are introduced as described in Methods B and C, in the sequence considered most convenient. The amine function is derivatized according to Method A.

The representative drugs listed below may be derivatized in this manner to the corresponding [D-QC]⁺ and [D-DHC] compounds.

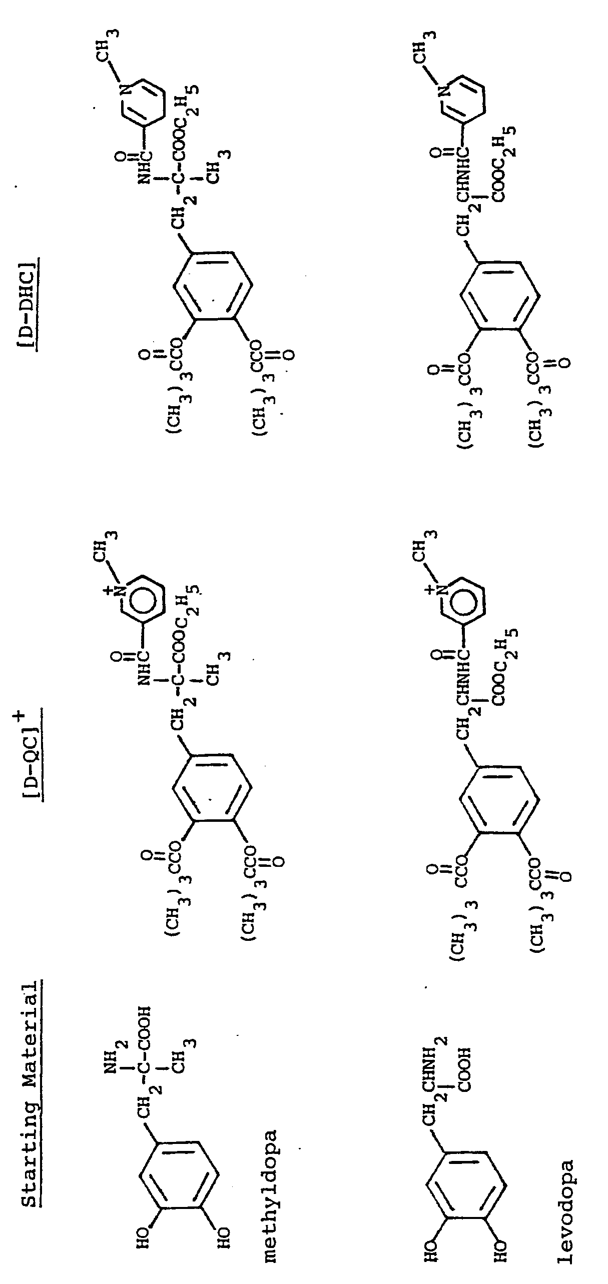

### Method E

This method is of particular use when the -NH-function is part of an amide or imide or a very low pKa primary or secondary amine.

The drug is first reacted with an aldehyde [e.g. formaldehyde, benzaldehyde, acetaldehyde or chloral (Cl₃CCHO)]; for example, in the case of chloral, one converts the -NH- function to a 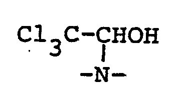 function and thus forms a suitable bridging group. The resultant compound is then reacted with nicotinic acid in the presence of a suitable dehydrating agent, or with nicotinoyl chloride or nicotinic anhydride, to form the corresponding nicotinic acid ester of the partial formula 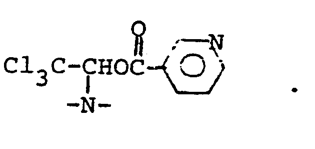 The resultant intermediate is then quaternized and reduced as in Method A.

The representative starting drugs listed below may be derivatized in this manner to the corresponding [D-QC]⁺ and [D-DHC] compounds.

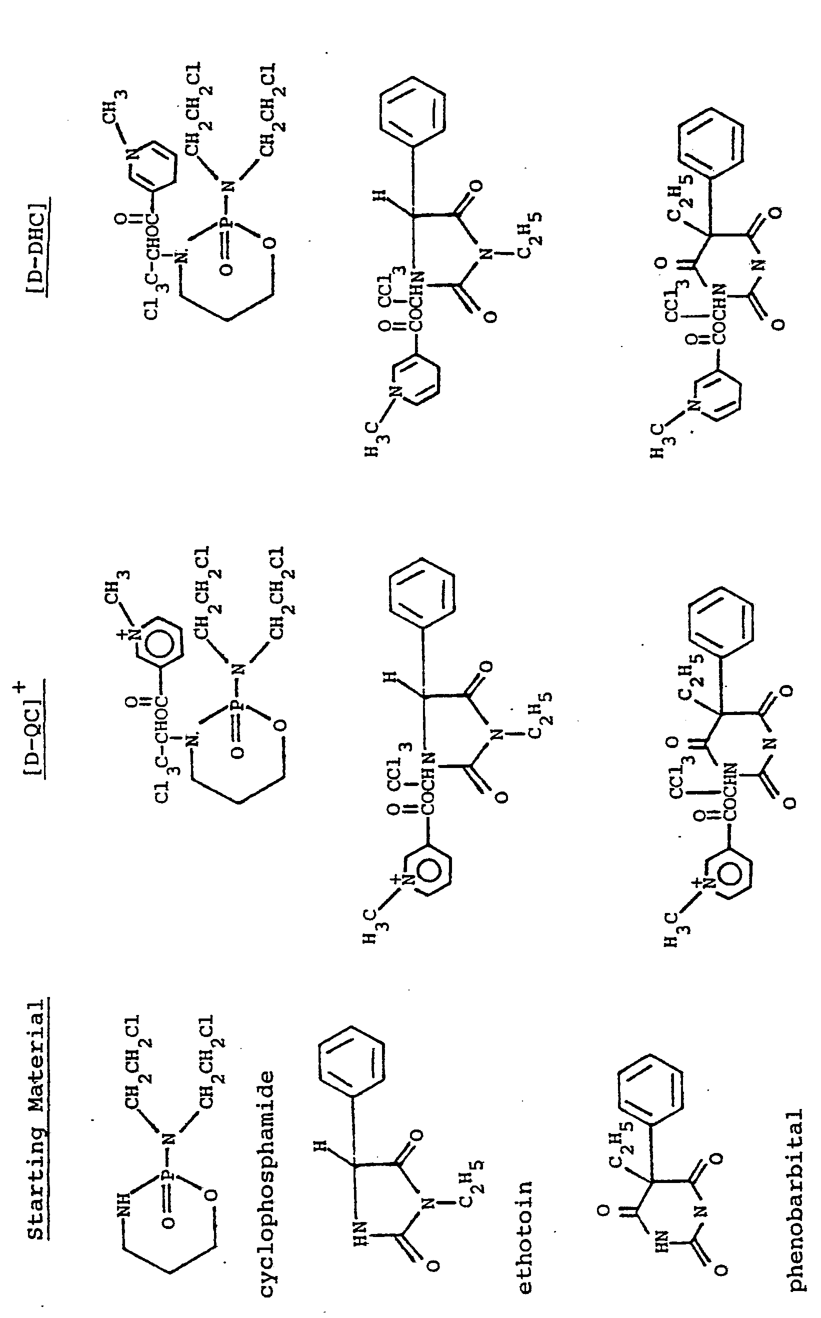

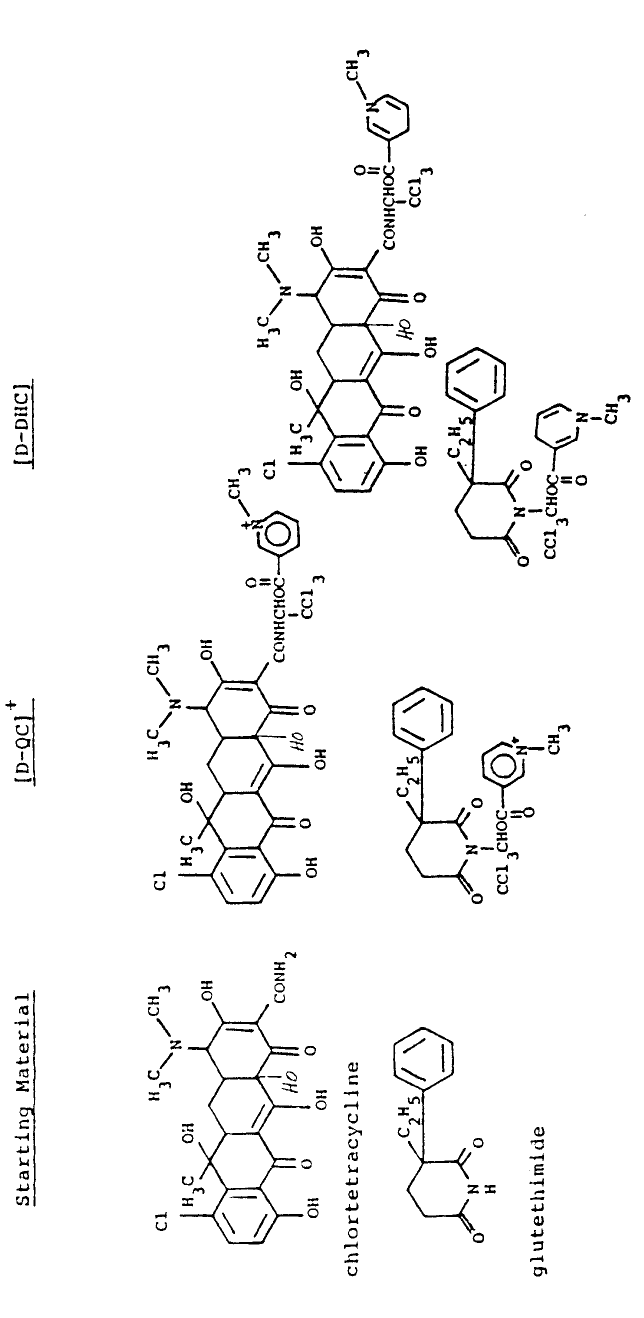

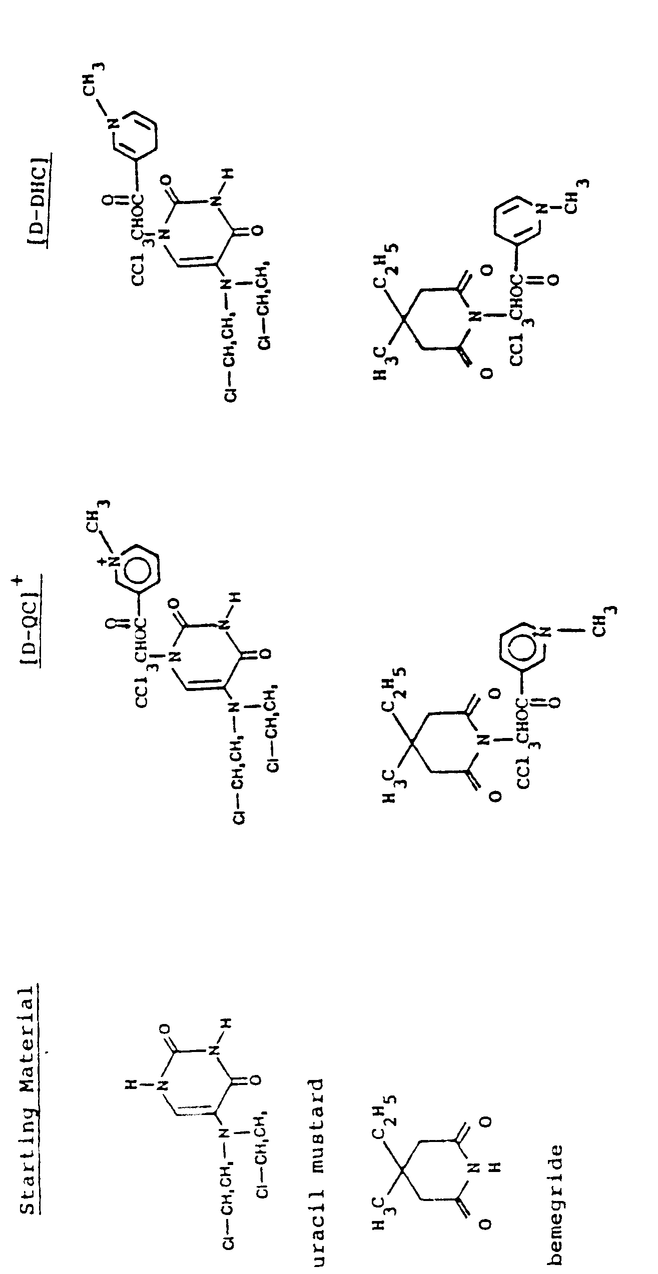

### Method F

Method A is followed, except that in the first step, the drug is reacted with 3-quinolinecarboxylic acid or its acid chloride or anhydride instead of nicotinic acid or its acid chloride.

The representative drugs listed below may be derivatized in this manner to the corresponding [D-QC] and [D-DHC] compounds, as may the remaining drugs listed with Method A. Methamphetamine and phenmetrazine may be similarly derivatized to the corresponding [D-QC]⁺ and [D-DHC] compounds.

Similarly, Method F may be combined with Methods B, C or D to afford the corresponding N-methyl-3-quinolinecarboxamide derivatives, e.g. of the drugs listed with those methods.

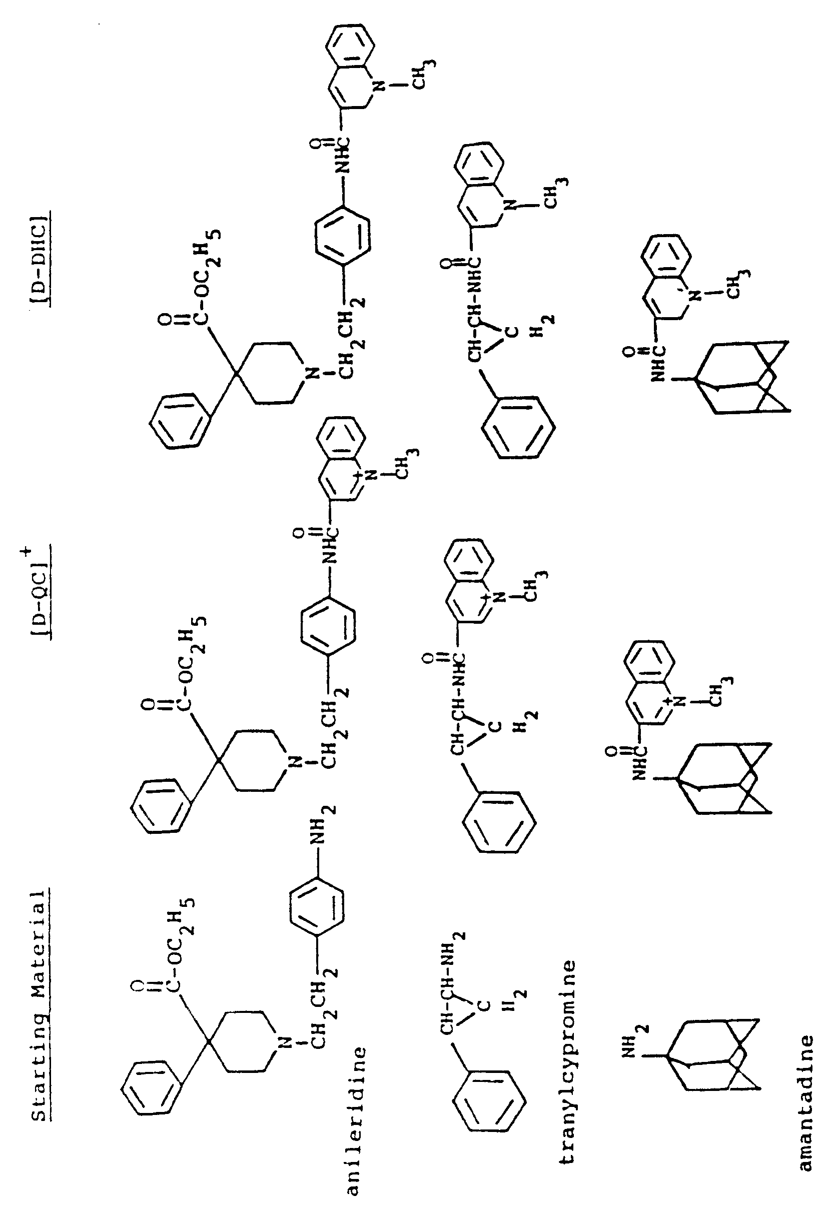

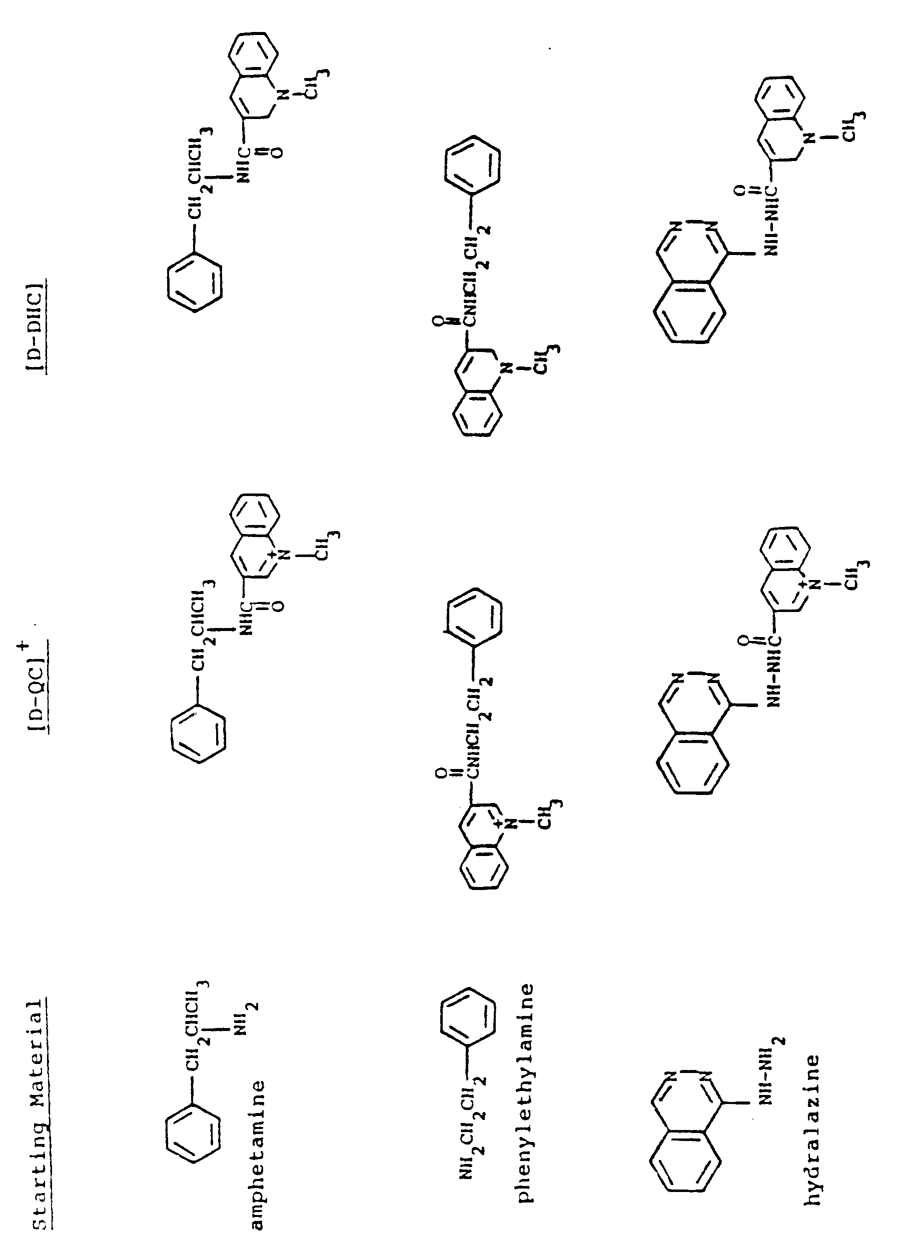

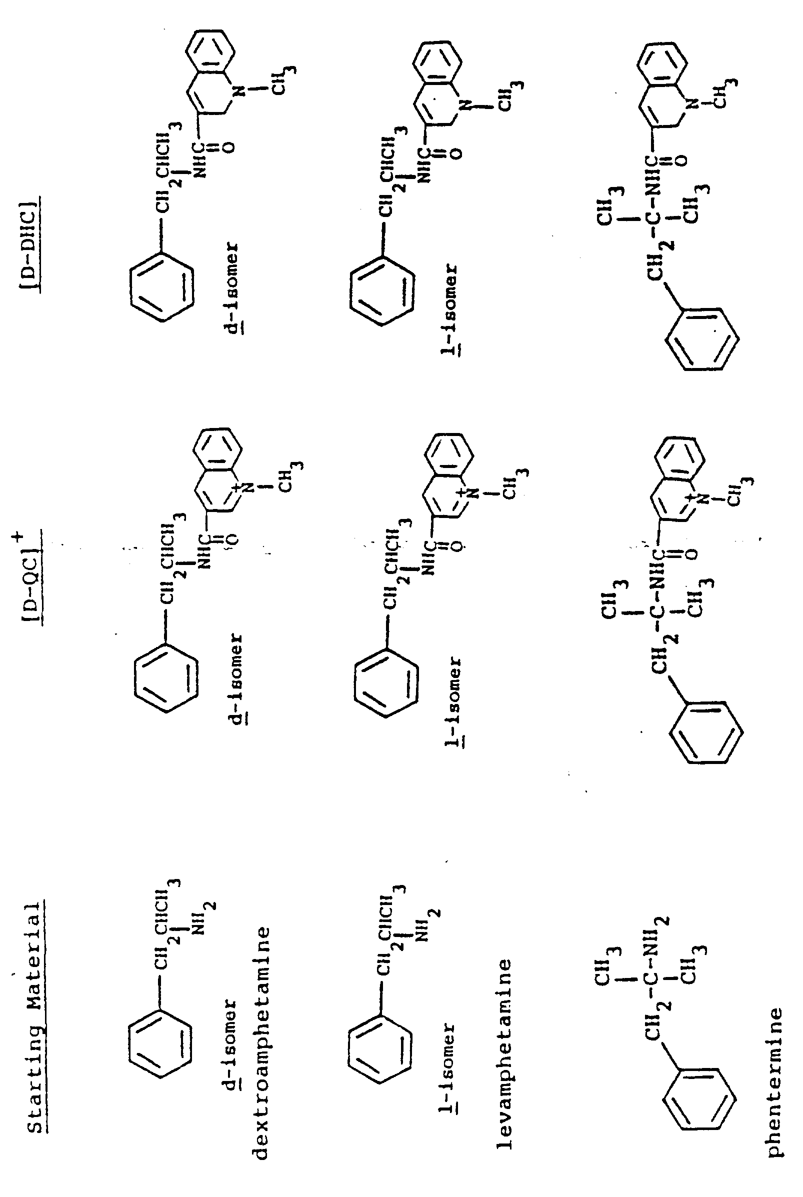

### Method G

Method A is followed, except that in the first step, a starting material of the formula 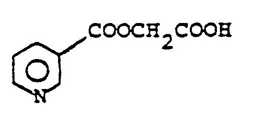 is used in place of nicotinic acid. (That starting material may be prepared by reacting nicotinic anhydride, nicotinoyl chloride or nicotinic acid with glycolic acid.)

The representative drugs listed below may be derivatized in this manner to the corresponding [D-QC]⁺ and [D-DHC] compounds, as may the remaining drugs listed with Method A. Tranylcypromine, methamphetamine, phentermine, phenmetrazine, detroamphetamine, levamphetamine and phenylethylamine may be similarly derivatized to the corresponding [D-QC]⁺ and [D-DHC] compounds.

Similarly, Method G may be combined with Methods B, C or D to afford the corresponding derivatives, e.g. of the drugs listed with those methods.

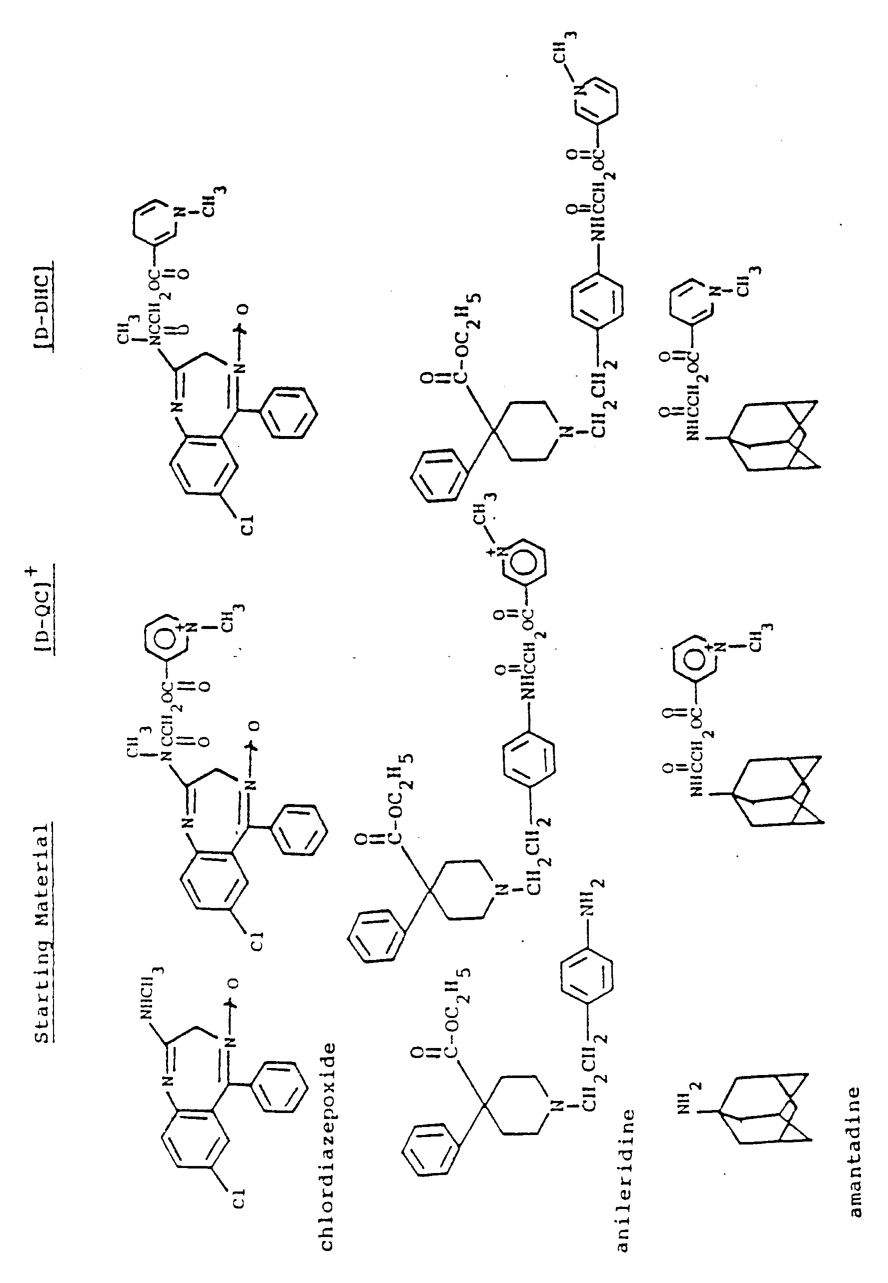

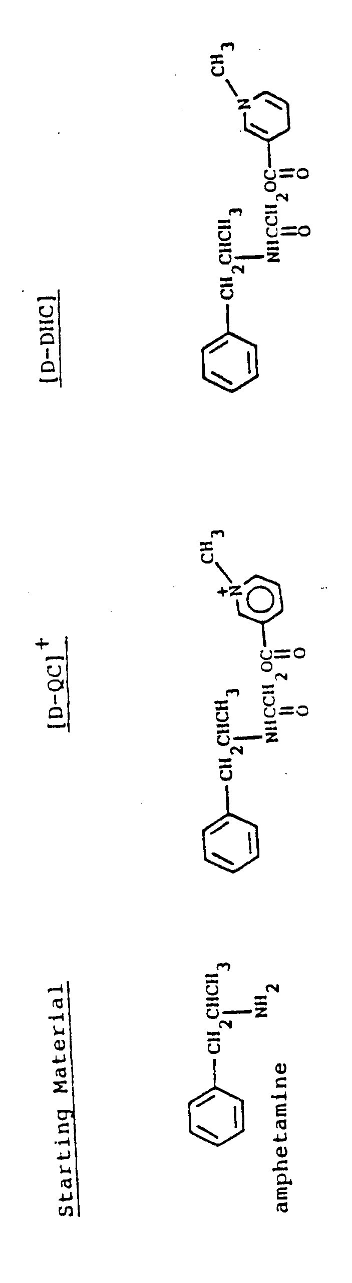

### Method H

Method A is followed, except that in the first step, a starting material of the formula 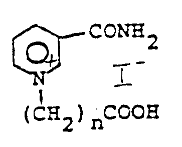 wherein n = 1-3, preferably 2, is used in place of nicotinic acid. (That starting material may be prepared from nicotinamide, e.g. when n = 2, by reacting 3-iodopropionic acid with nicotinamide.)

The drugs listed below may be derivatized in this manner to the corresponding [D-QC]⁺ and [D-DHC] compounds, as may the remaining drugs listed with Method A. Tranylcypromine, methamphetamine, phenmetrazine, dextroamphetamine, levamphetamine and phenylethylamine may be similarly derivatized to the corresponding [D-QC]⁺ and [D-DHC] compounds.

Similarly, Method H may be combined with Methods B, C or D to afford the corresponding derivatives, e.g. of the drugs listed with those methods.

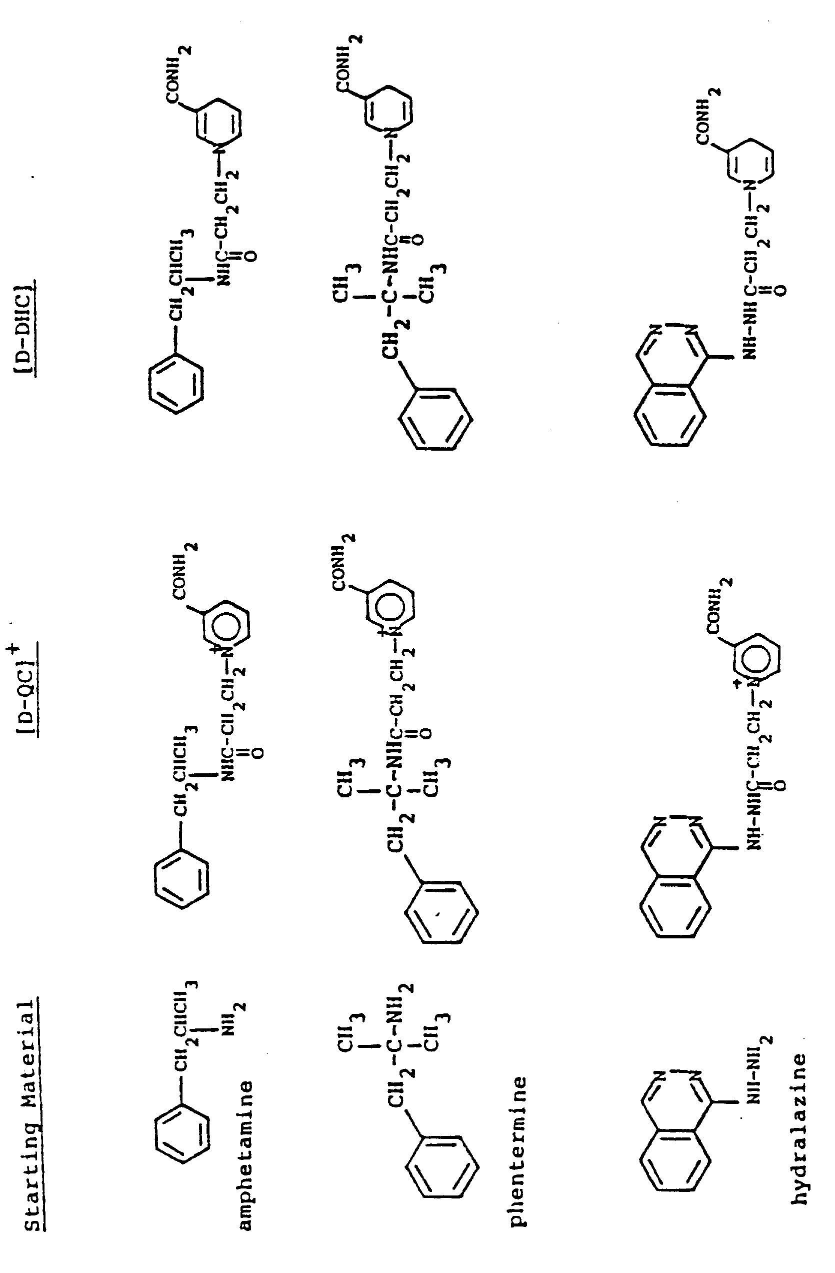

### II. Methods for Derivatizing -OH and -SH Functions in Drugs

### Method I

The drug is reacted with nicotinoyl chloride, with nicotinic anhydride, or with nicotinic acid in the presence of a suitable dehydrating agent such as dicyclohexylcarbodiimide, in an appropriate organic solvent, to afford the corresponding nicotinate. The nicotinate is then quaternized and subsequently reduced as described above in Method A. When the drug contains more than one reactive hydroxyl or thiol functions, reaction conditions may be varied so that more than one hydroxyl or thiol function will be converted to nicotinate groupings.

The representative drugs listed below may be derivatized in this manner to the corresponding [D-QC]⁺ and [D-DHC] compounds.

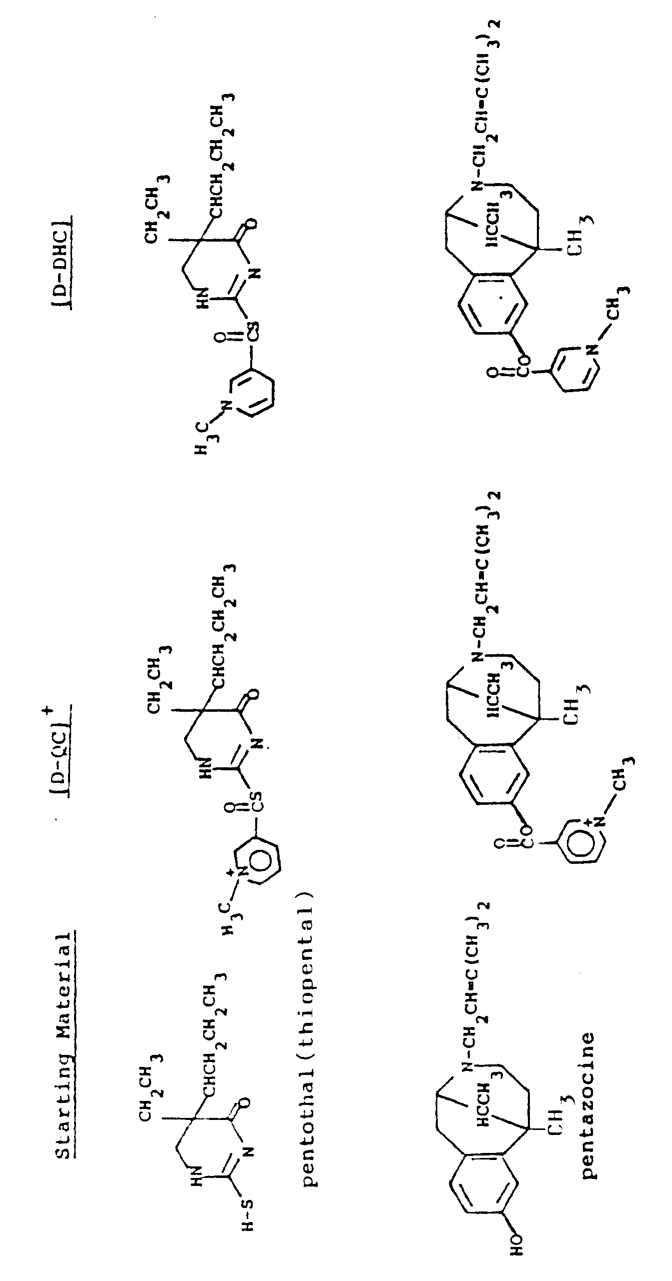

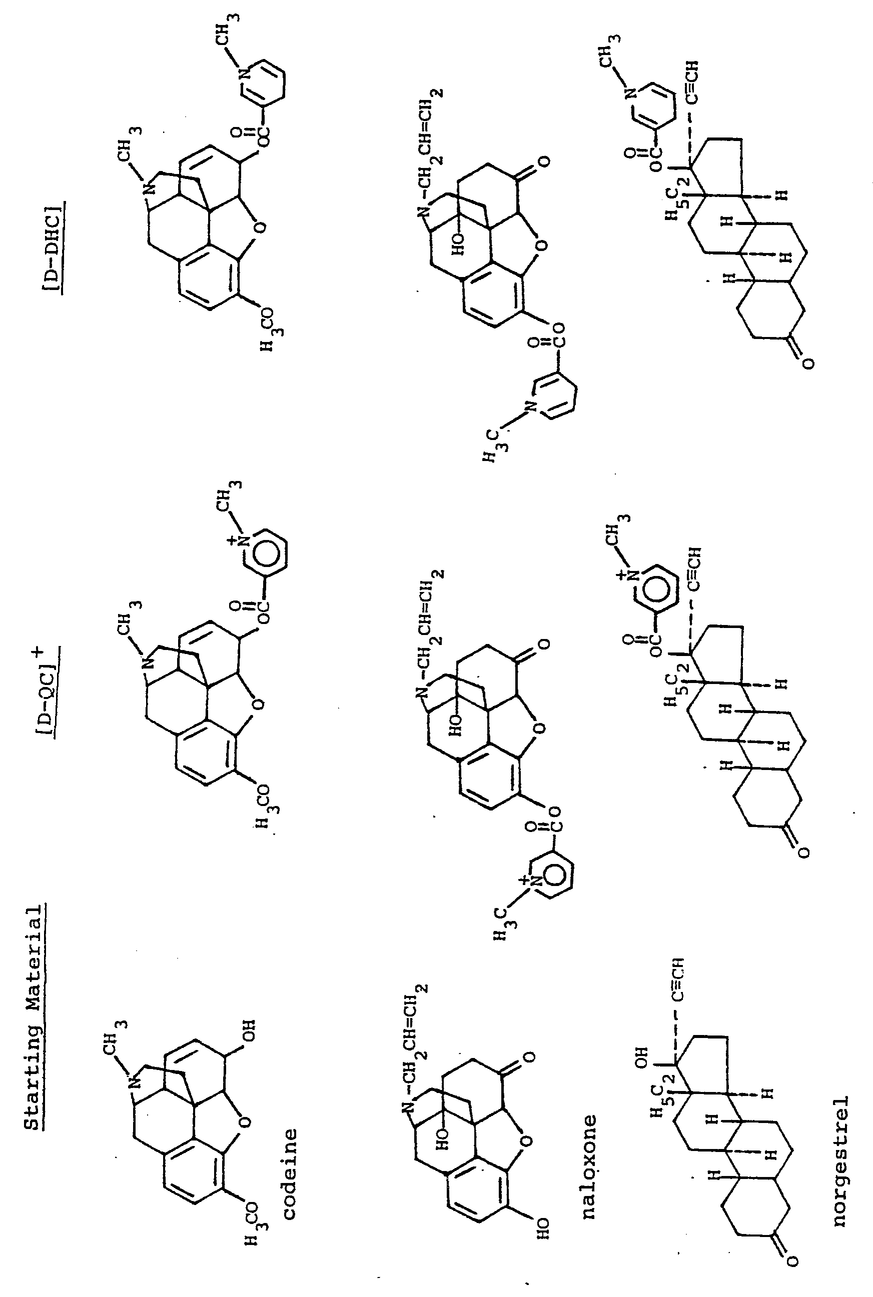

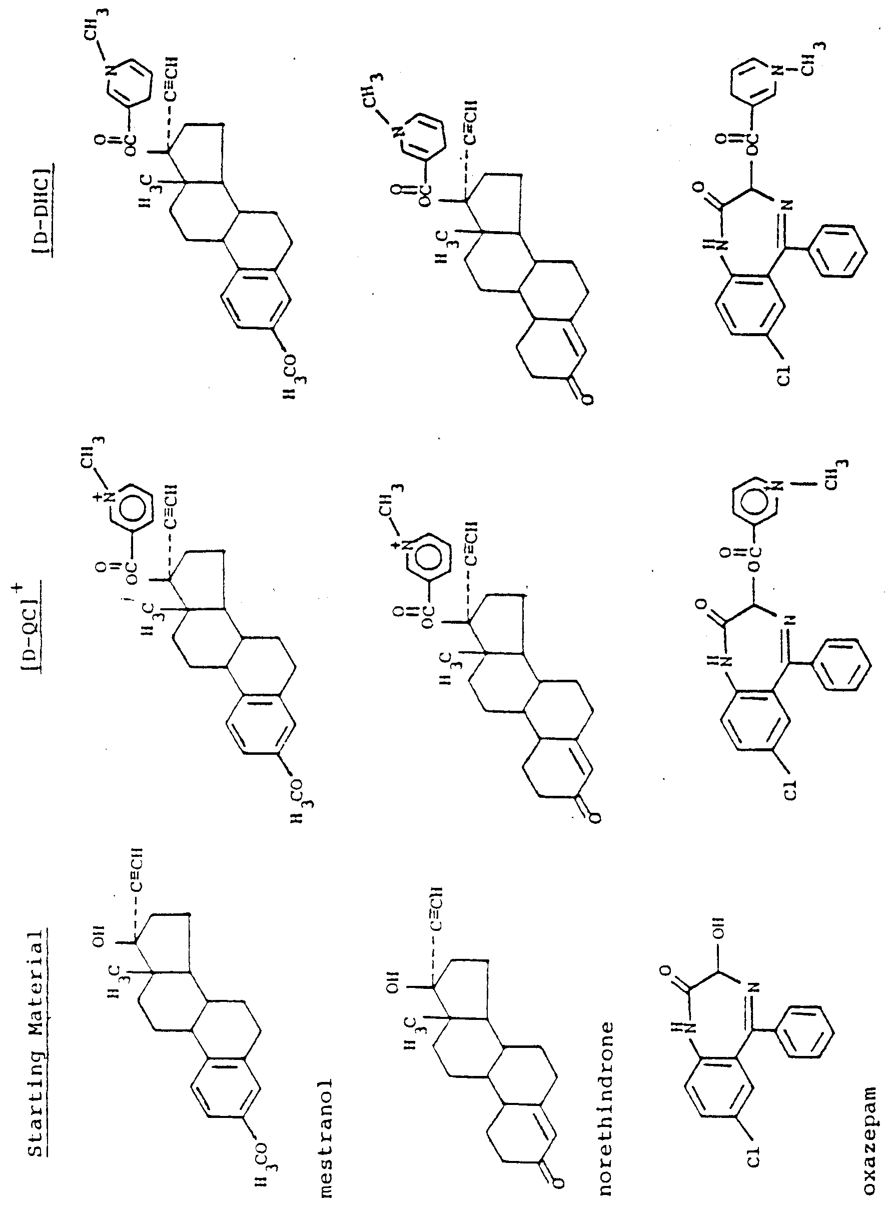

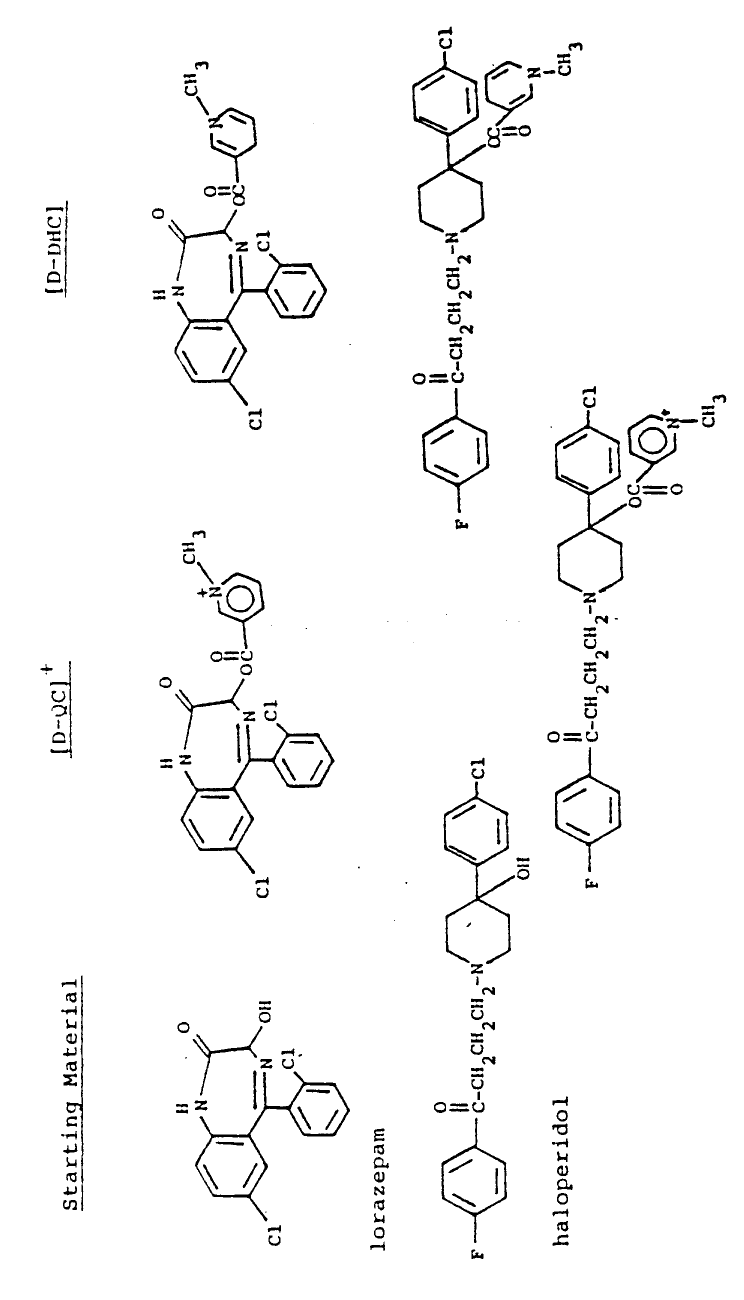

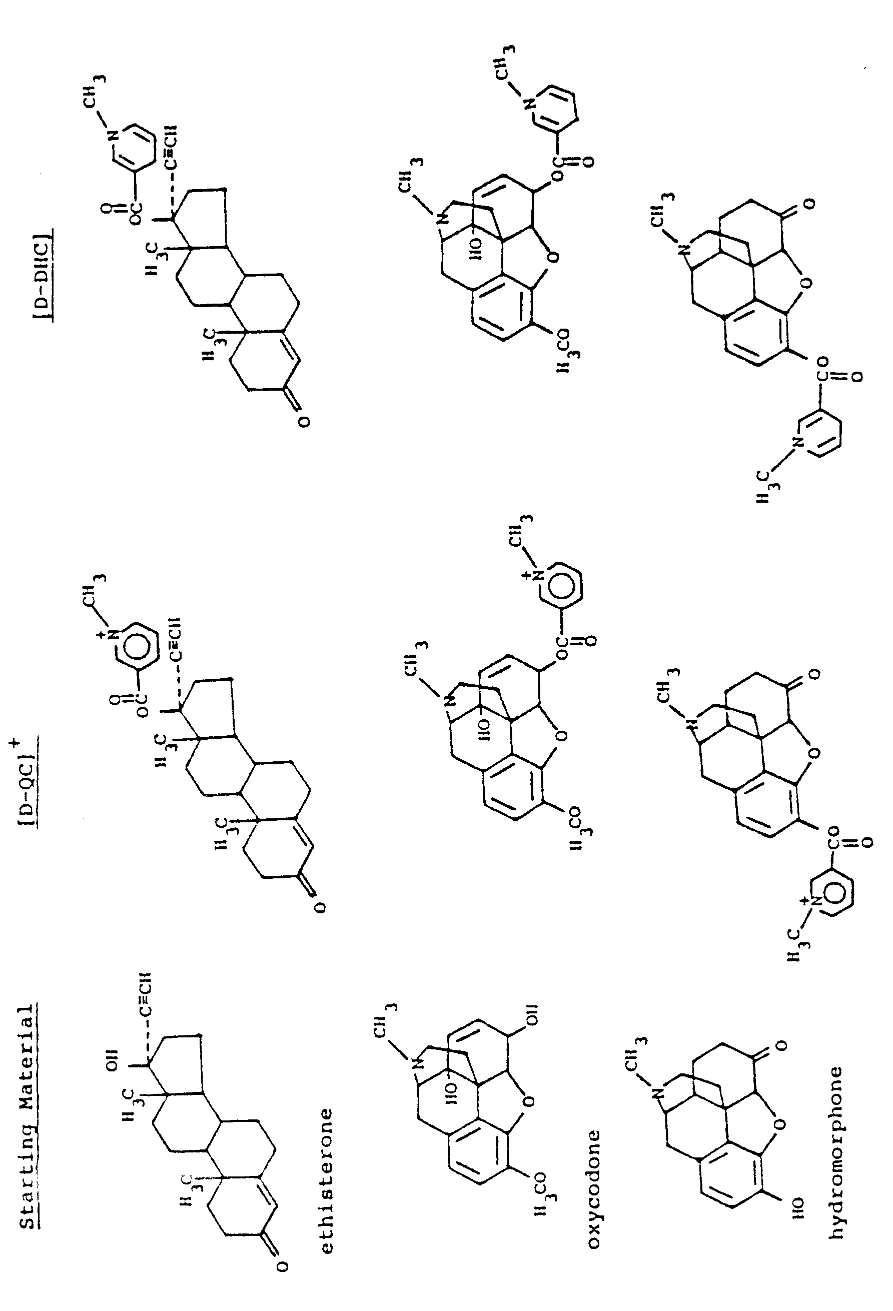

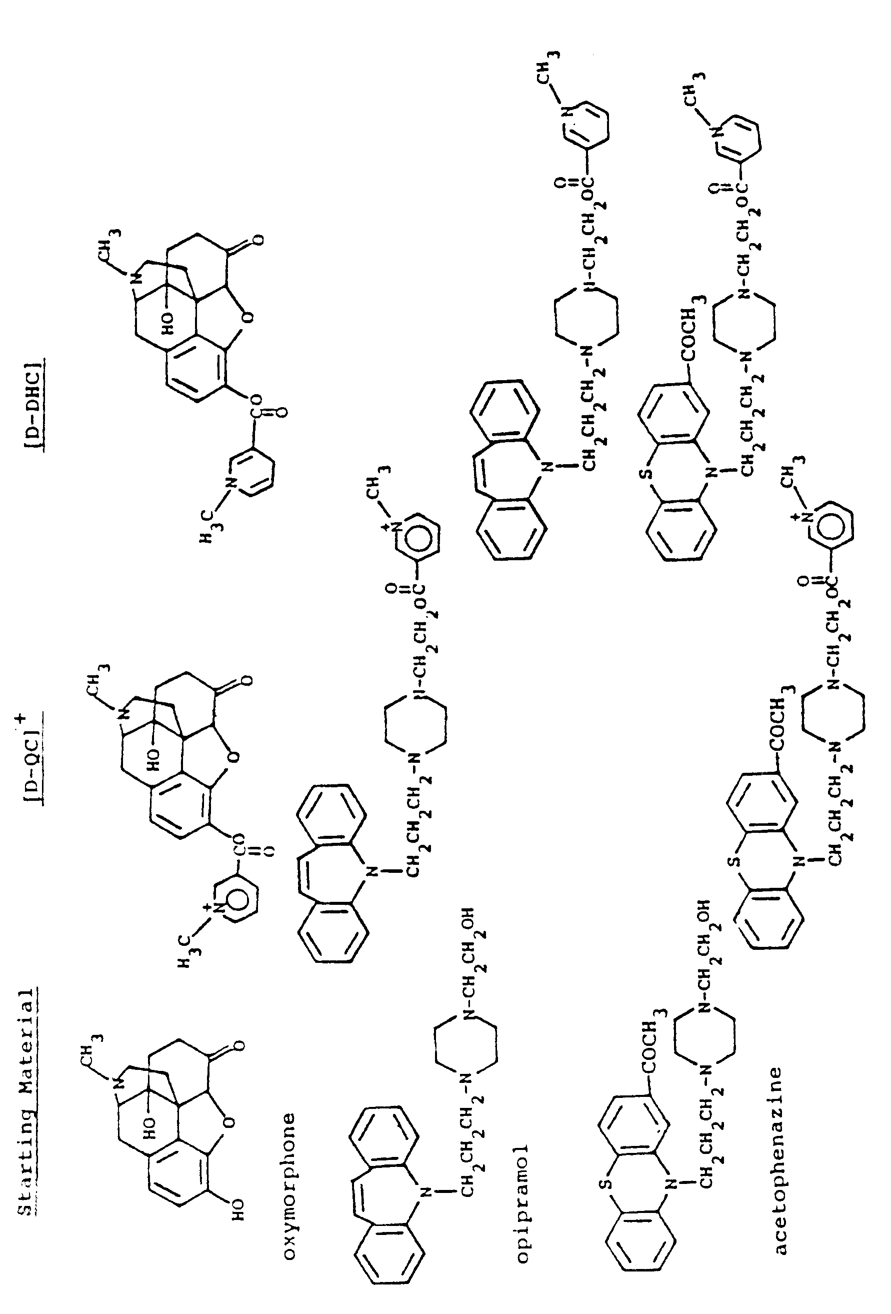

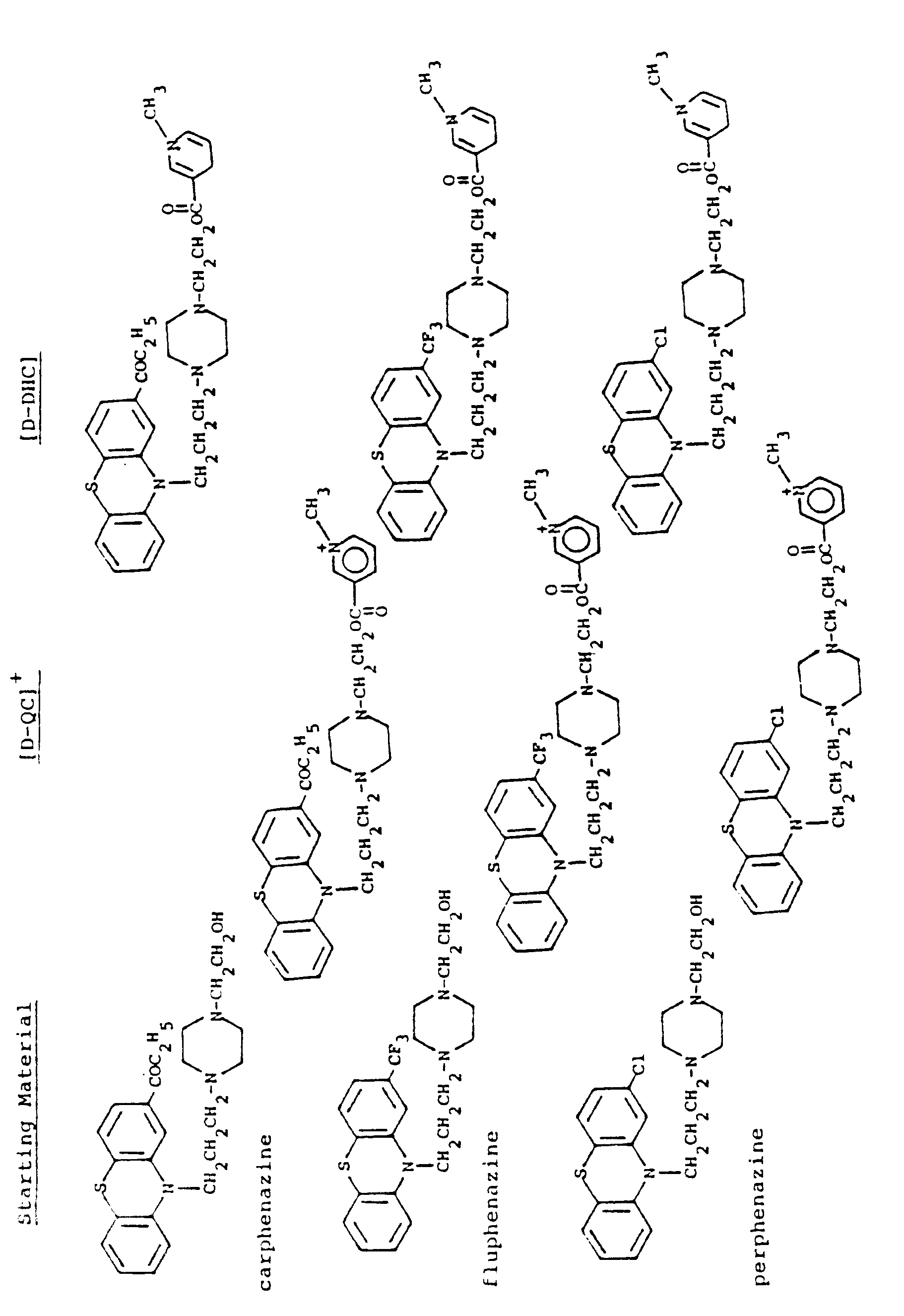

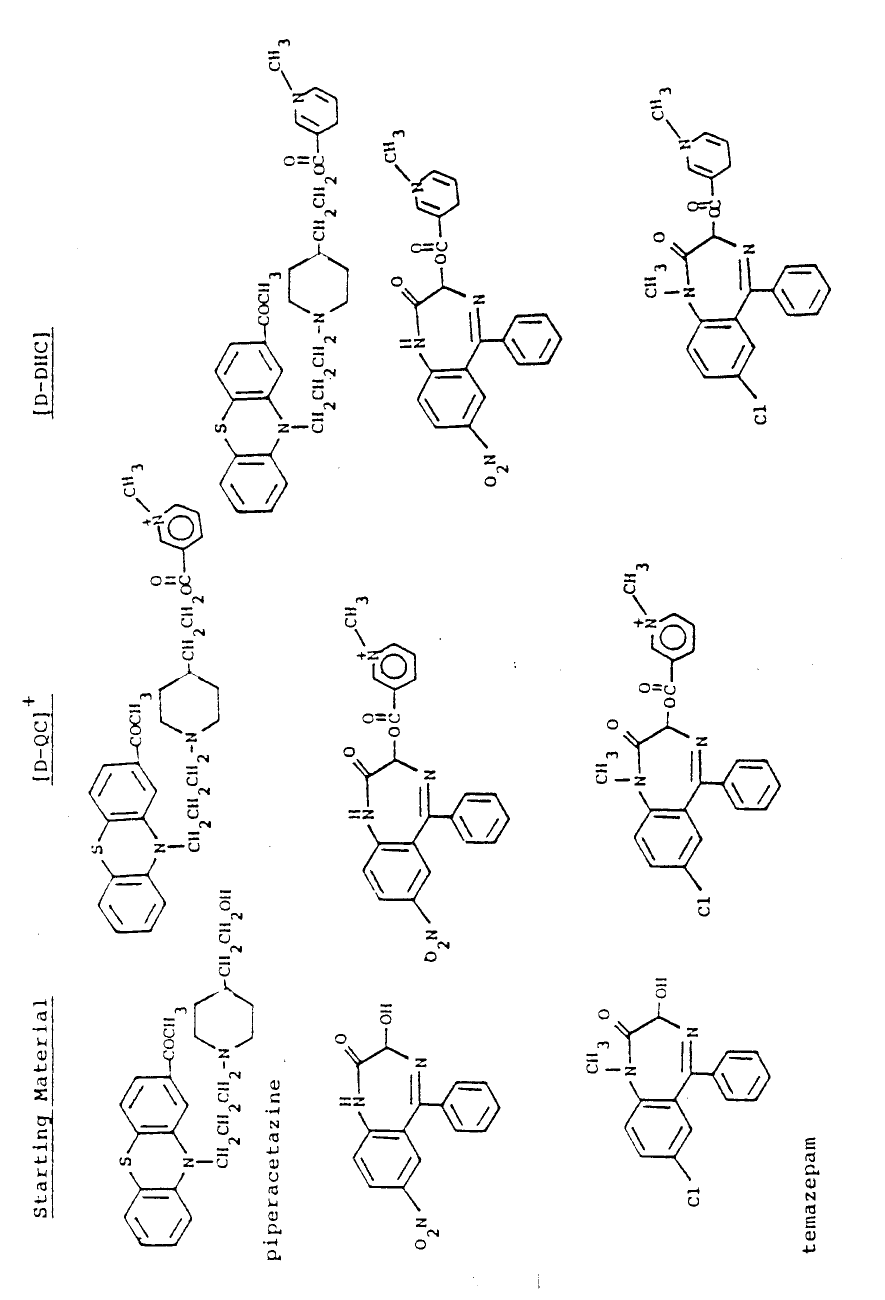

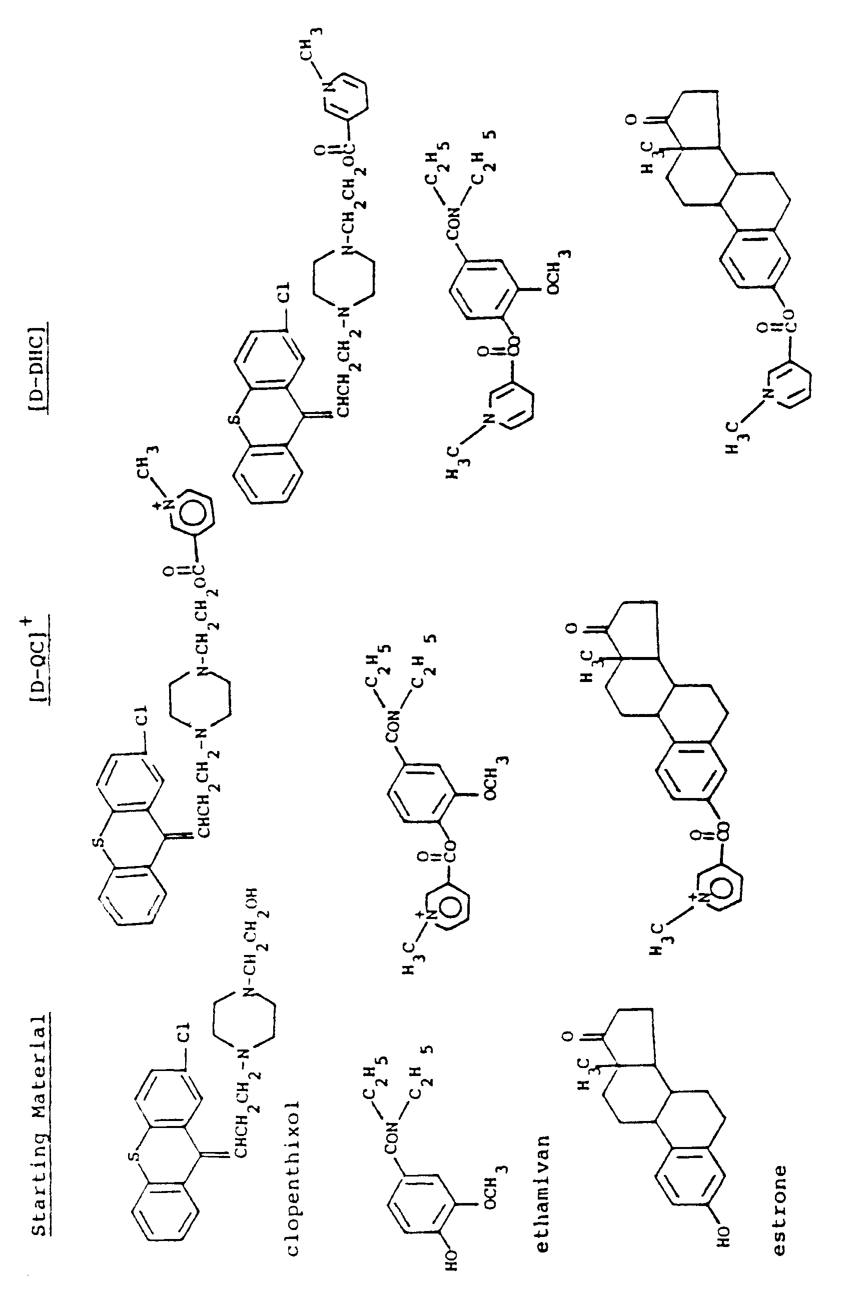

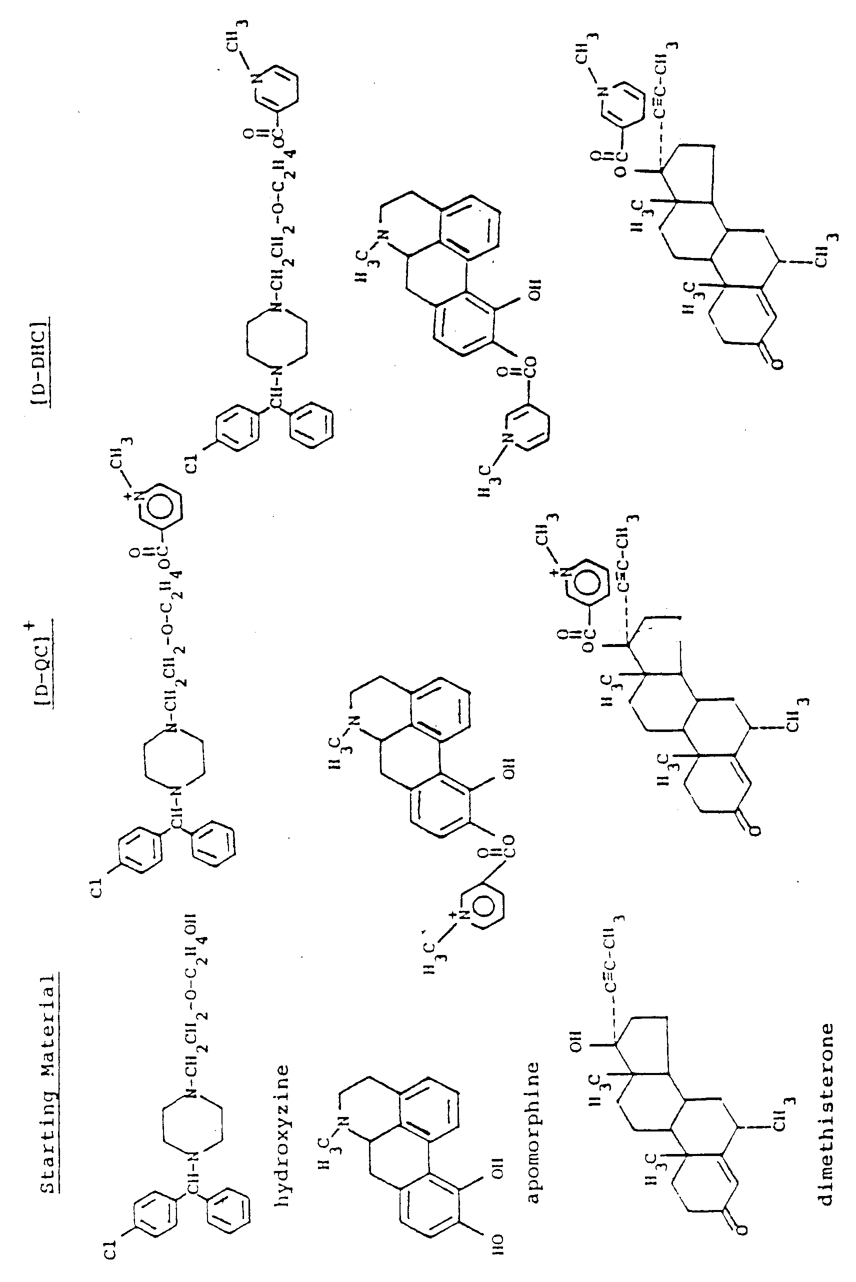

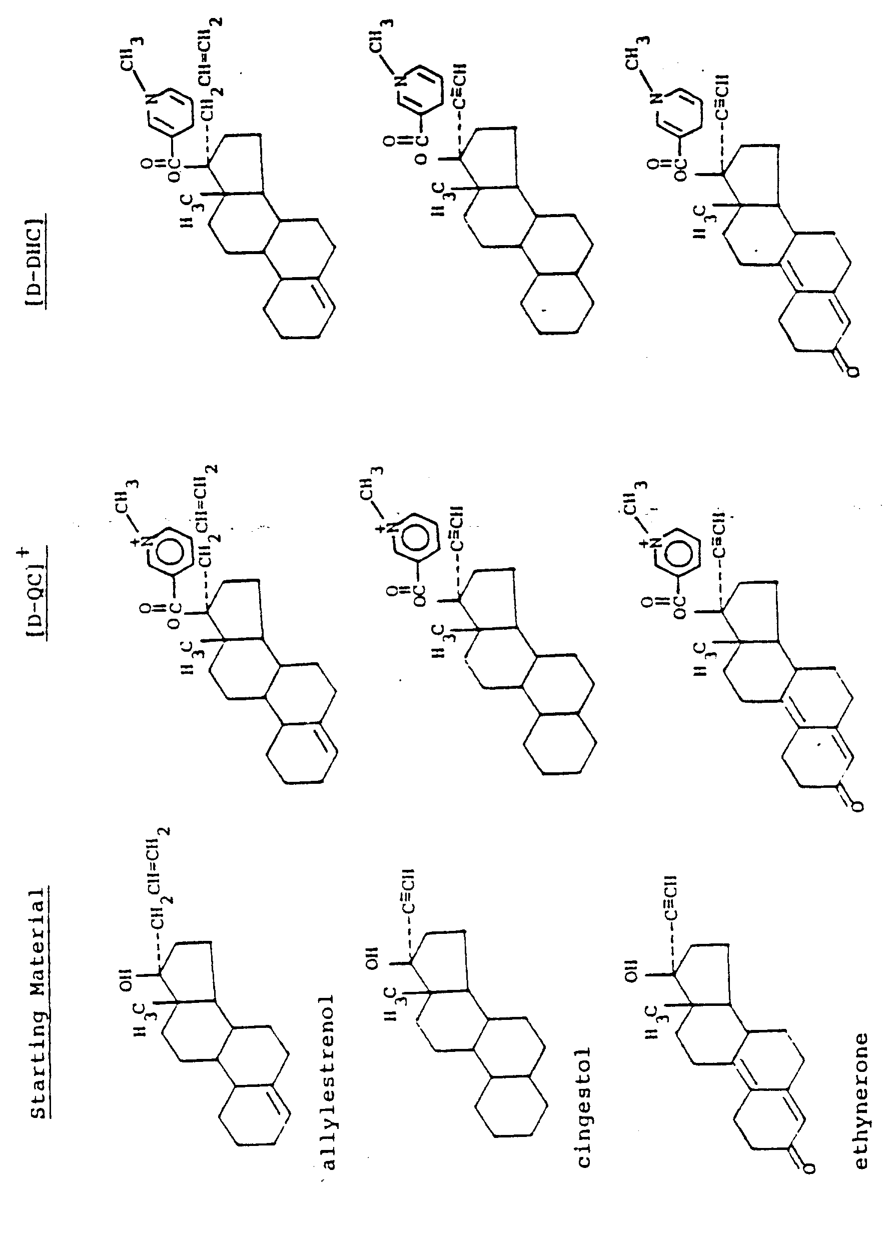

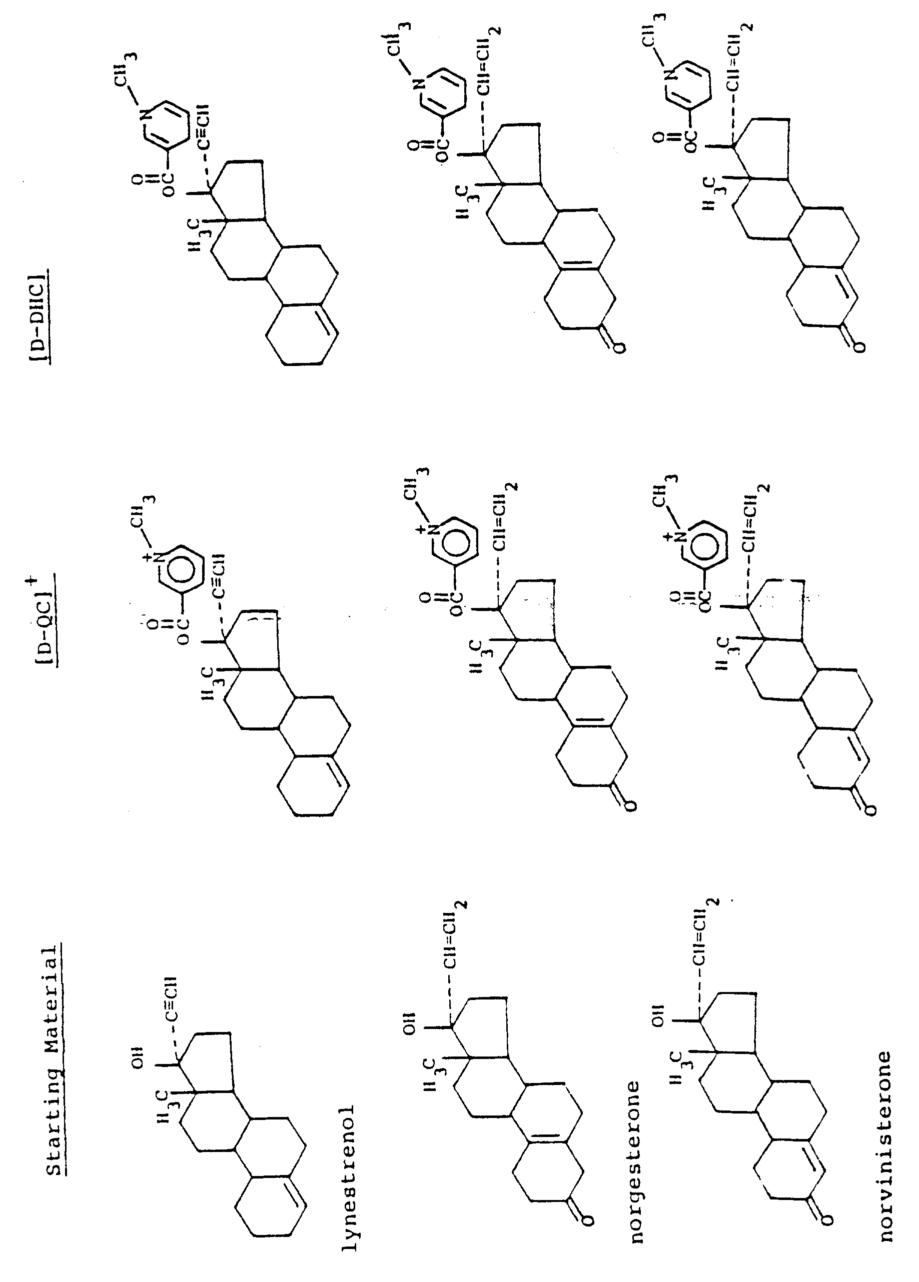

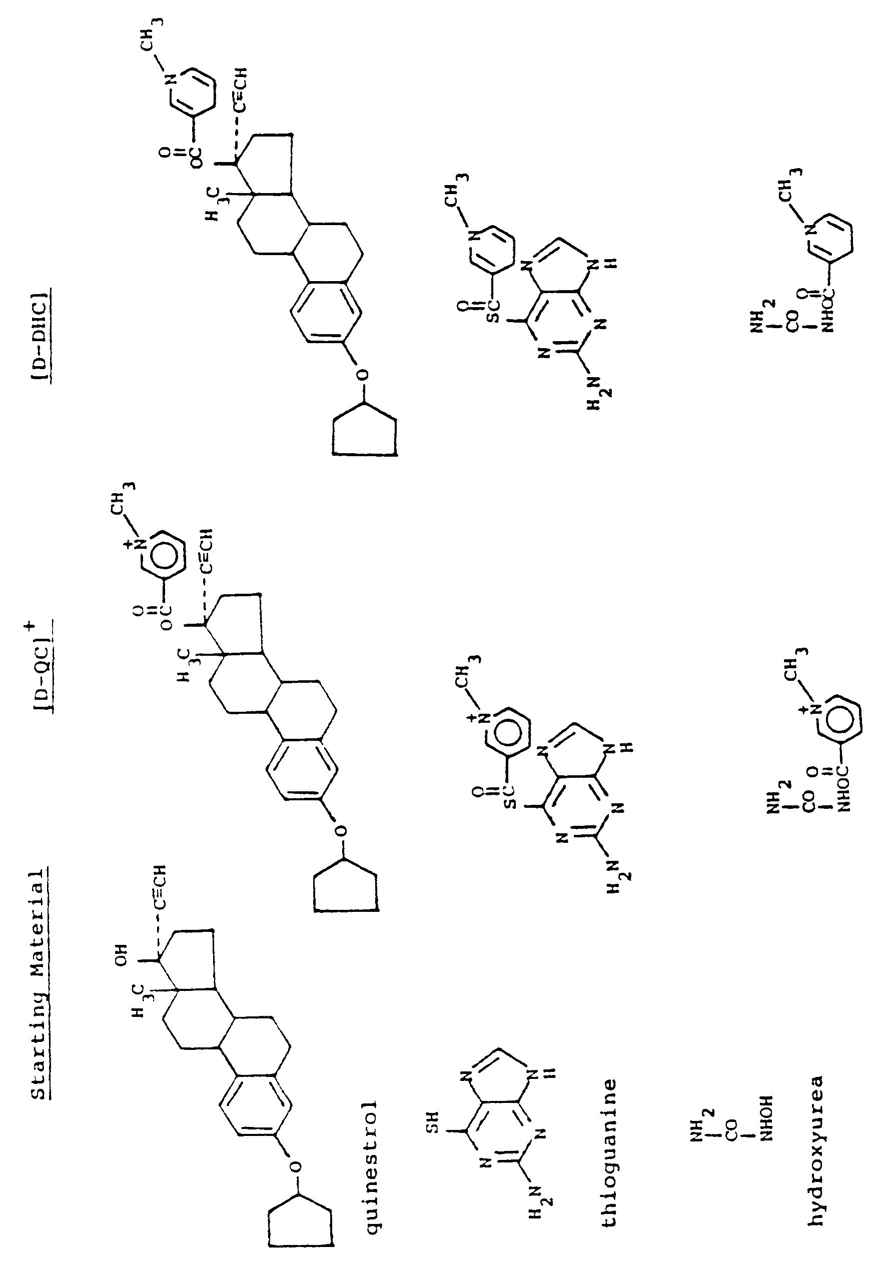

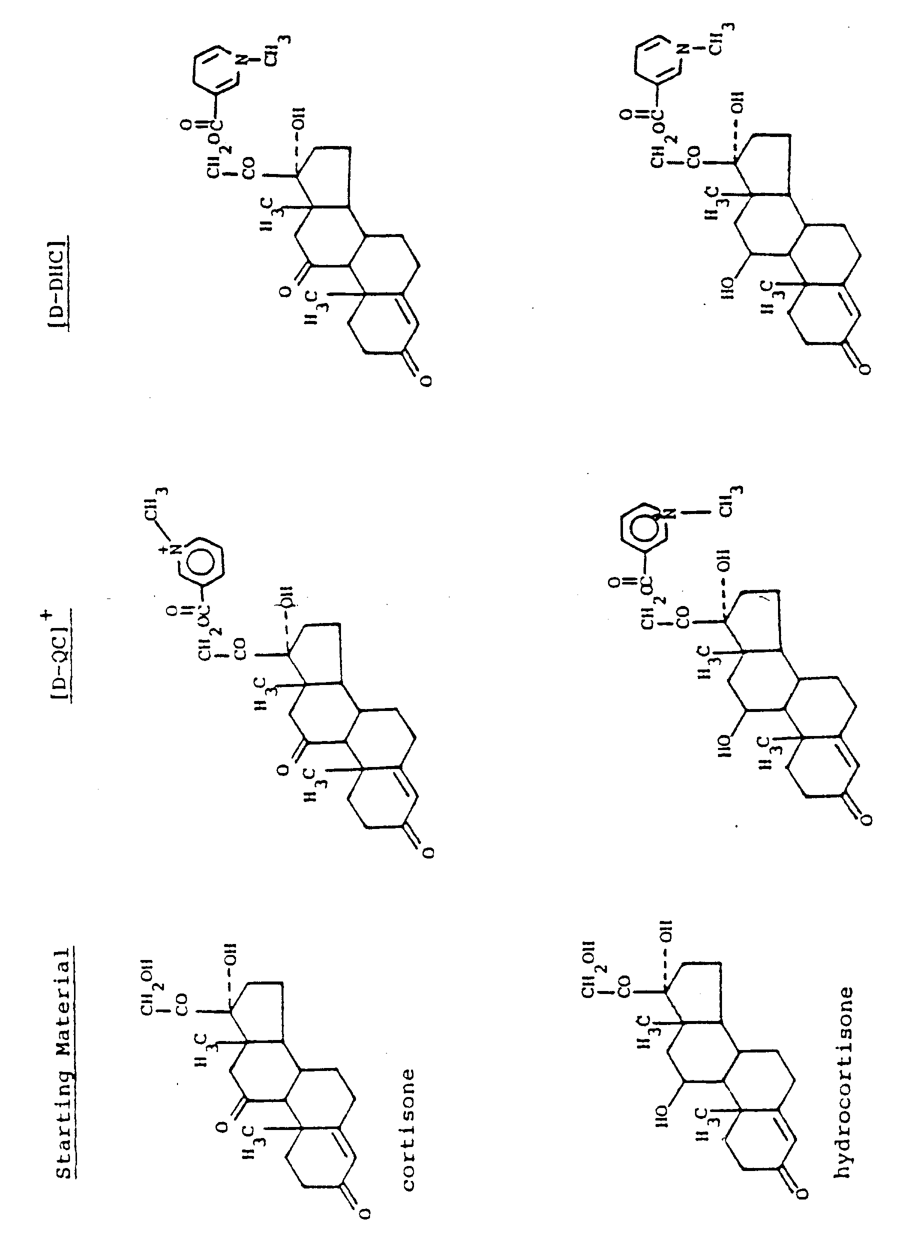

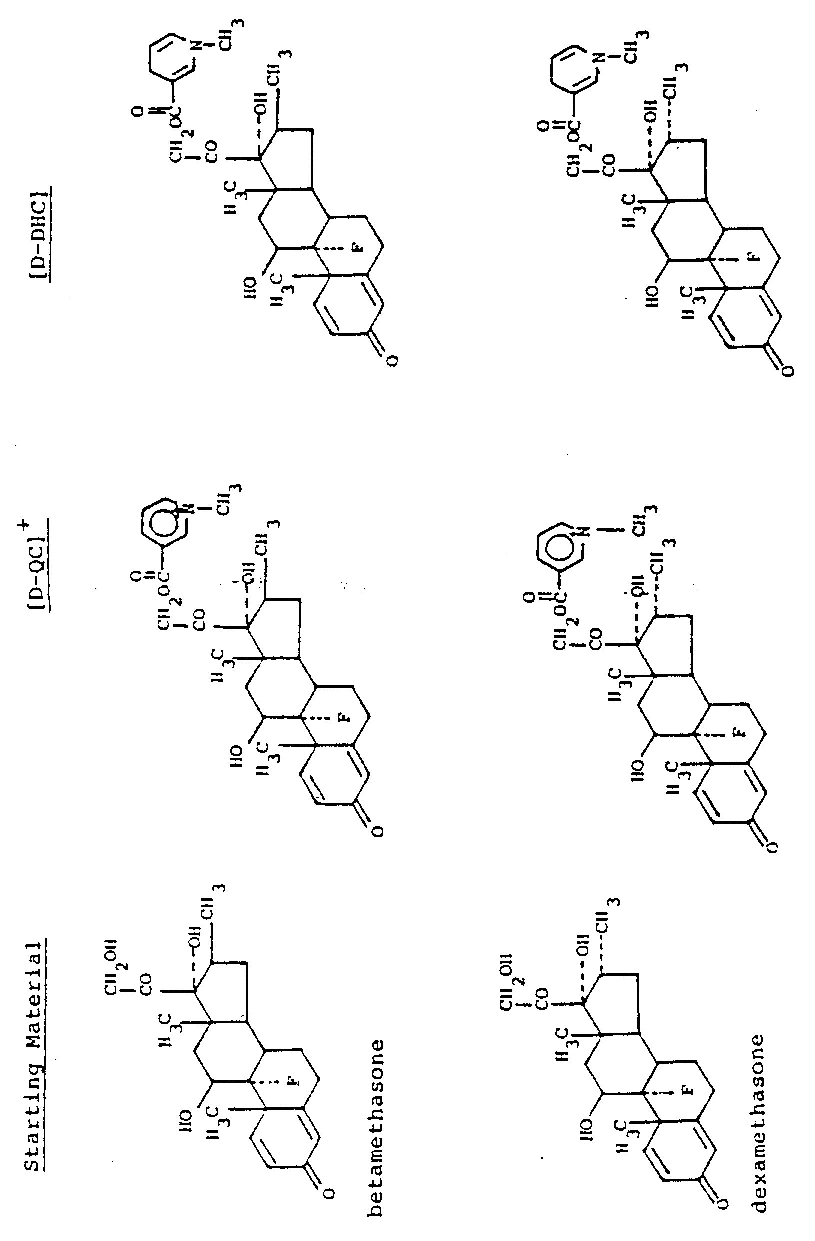

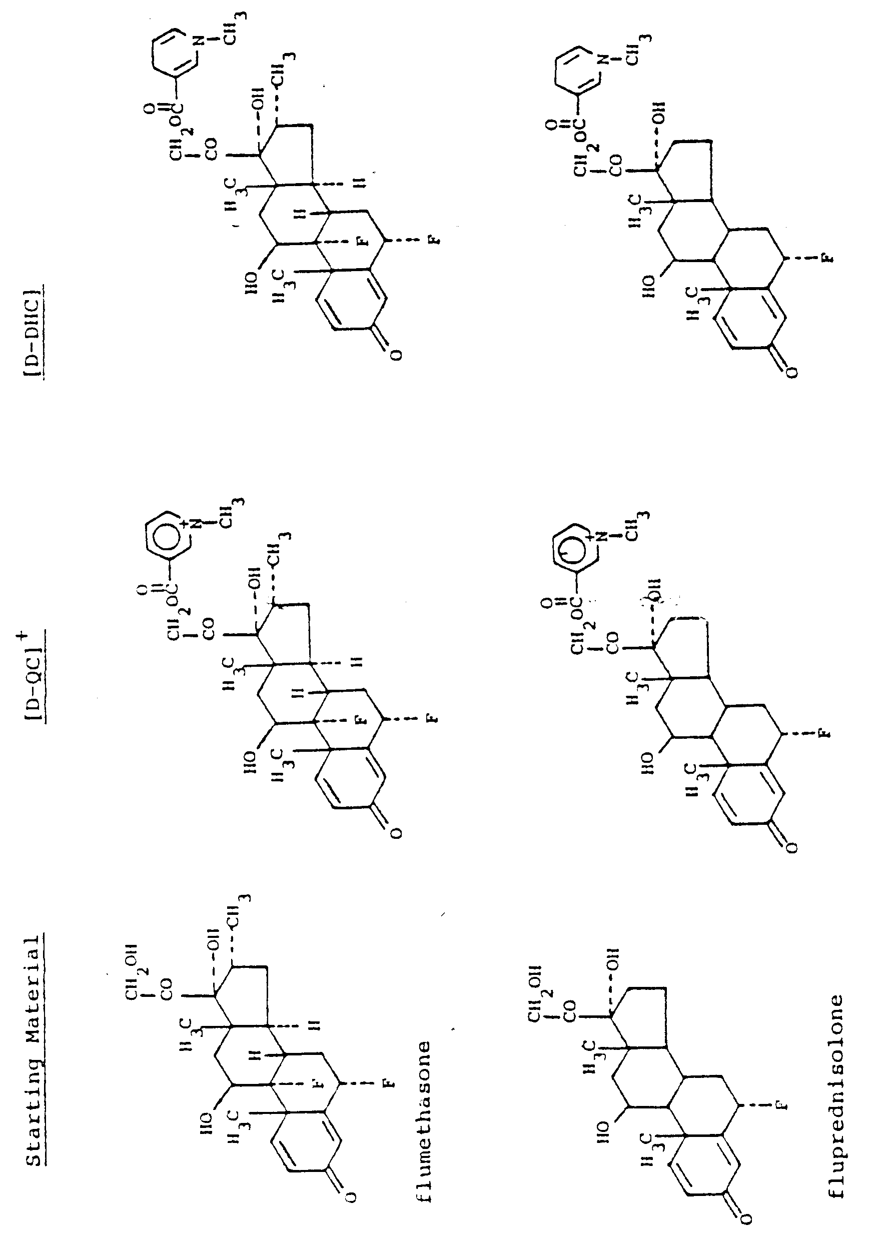

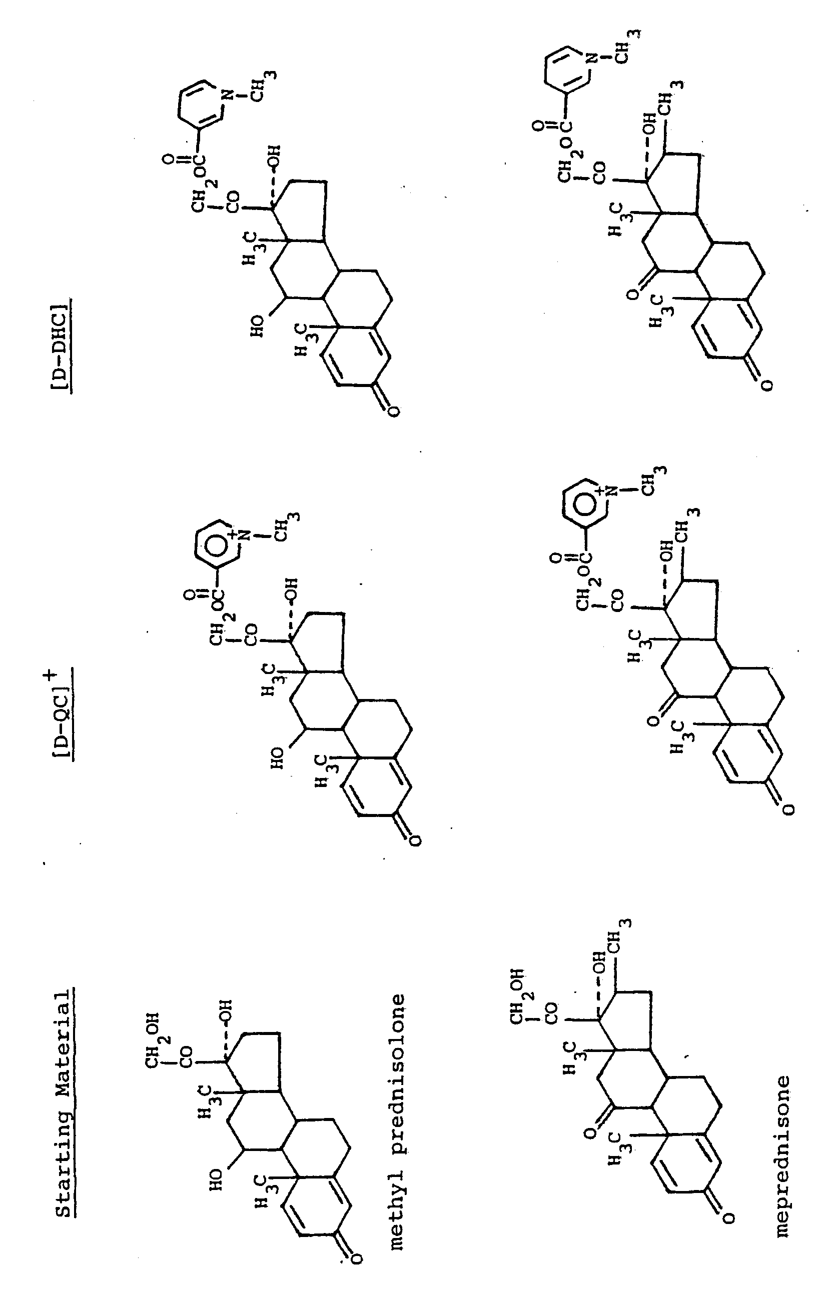

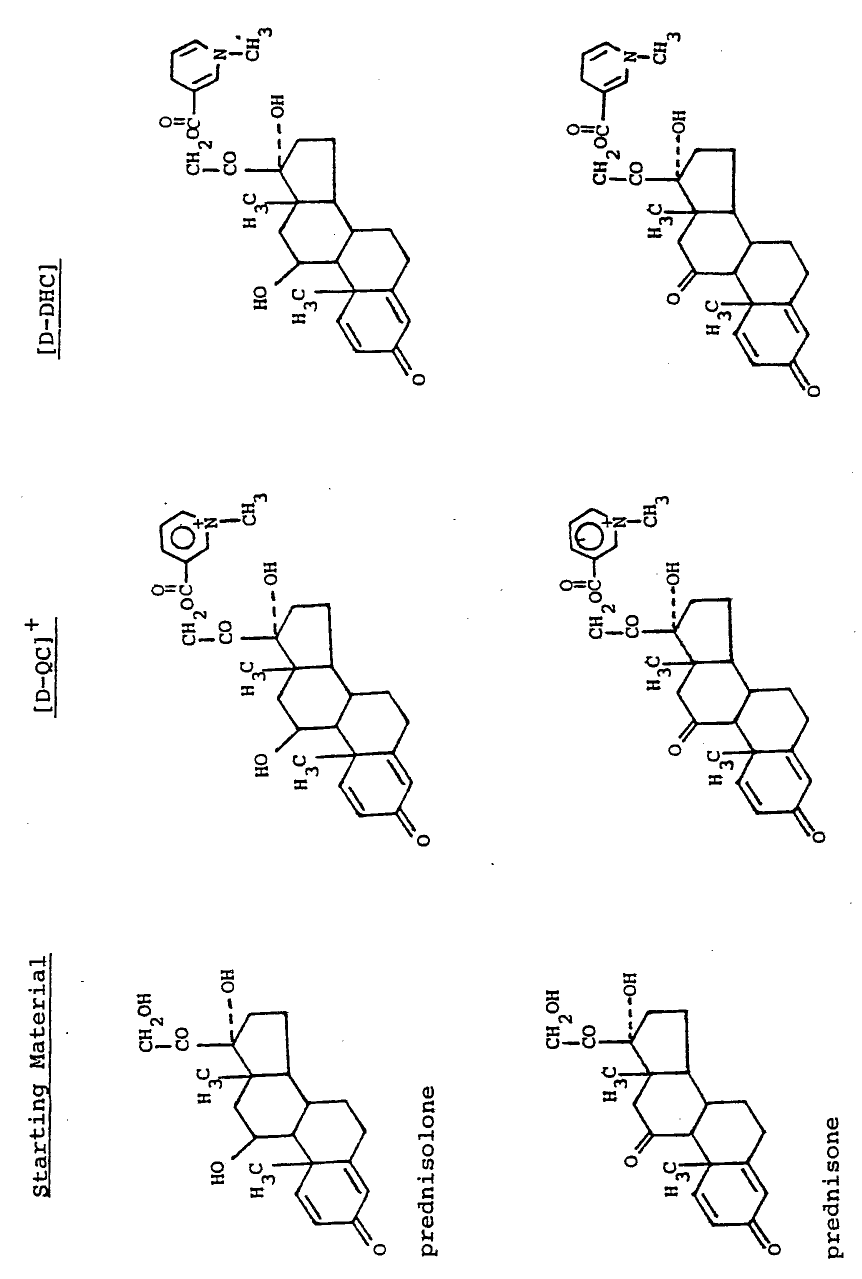

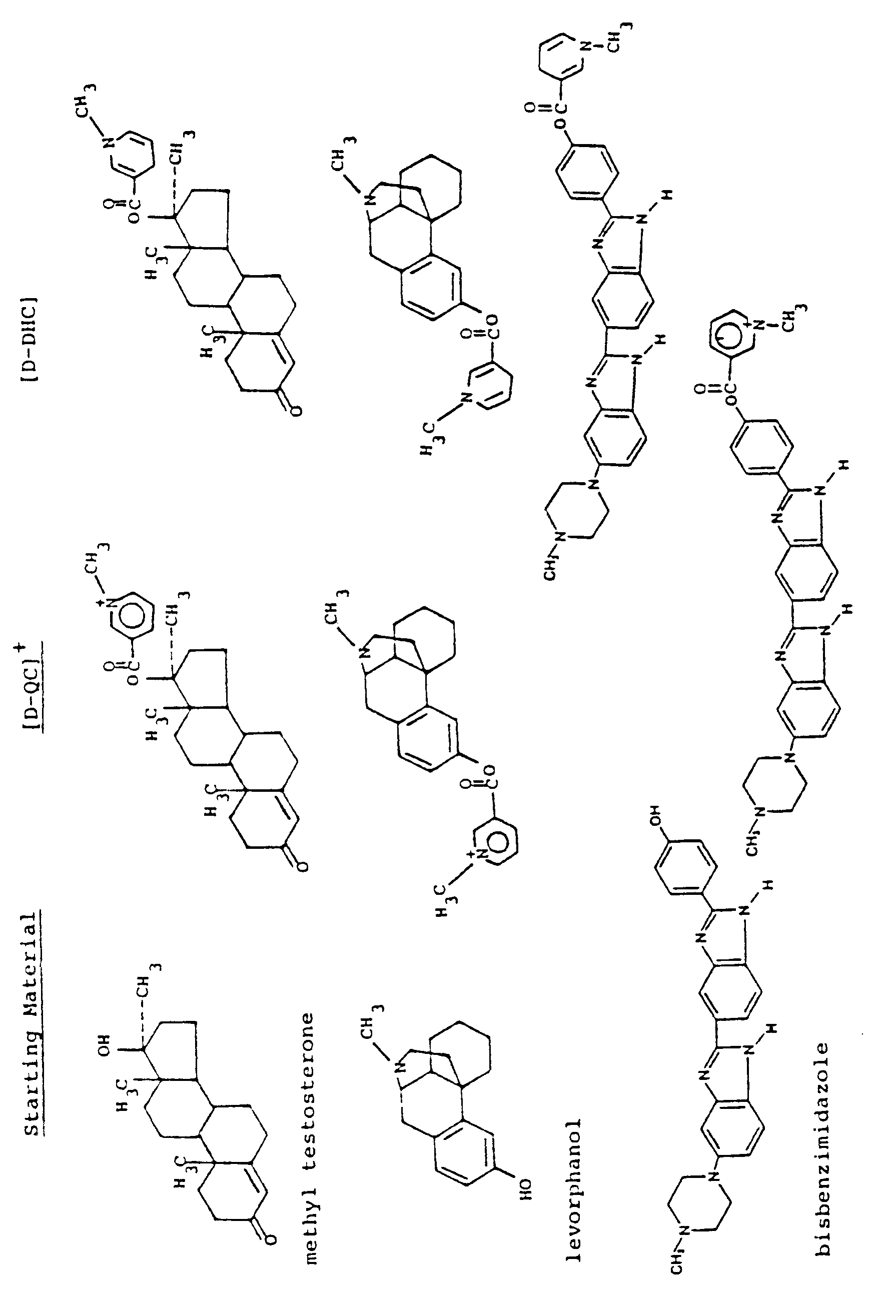

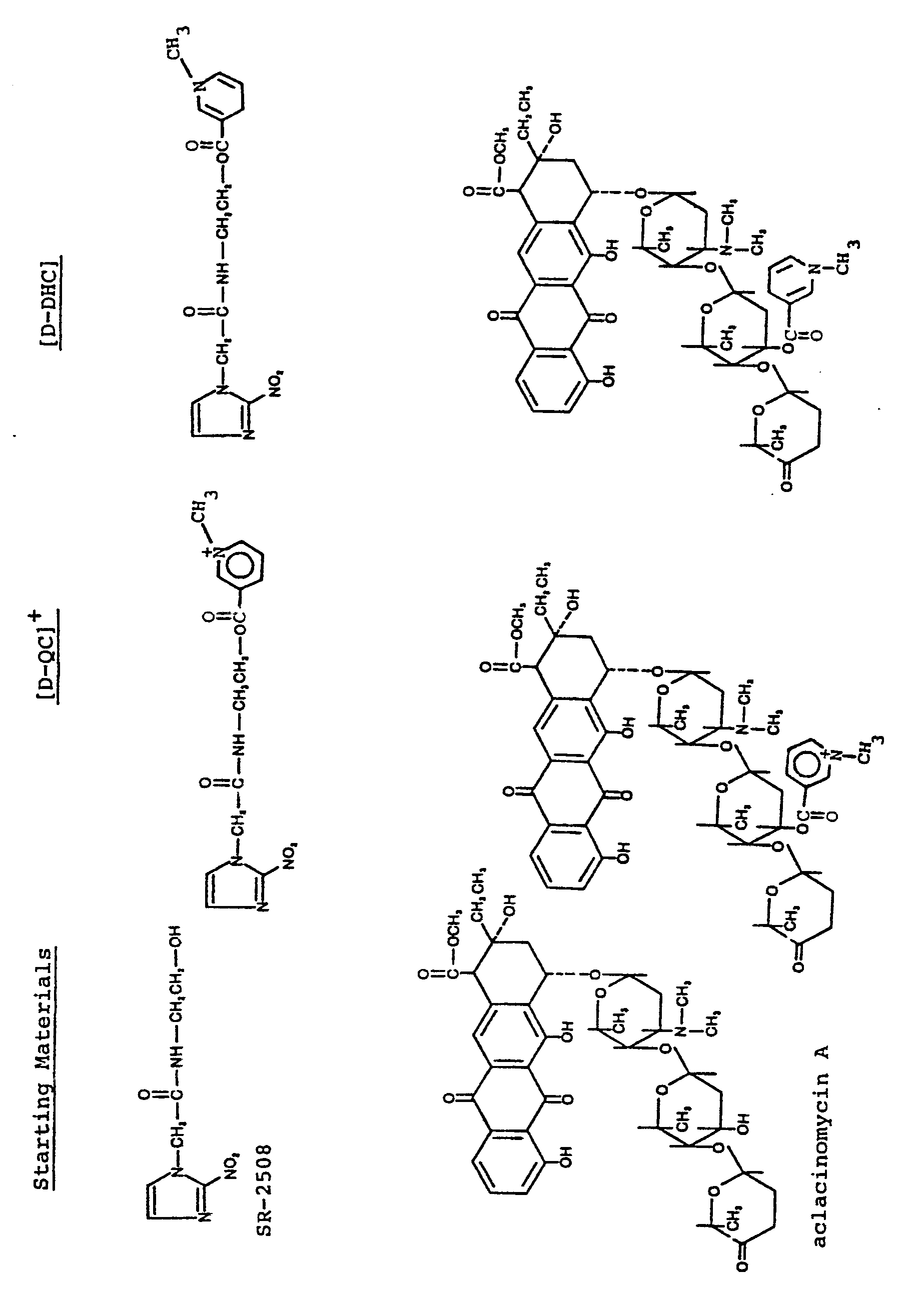

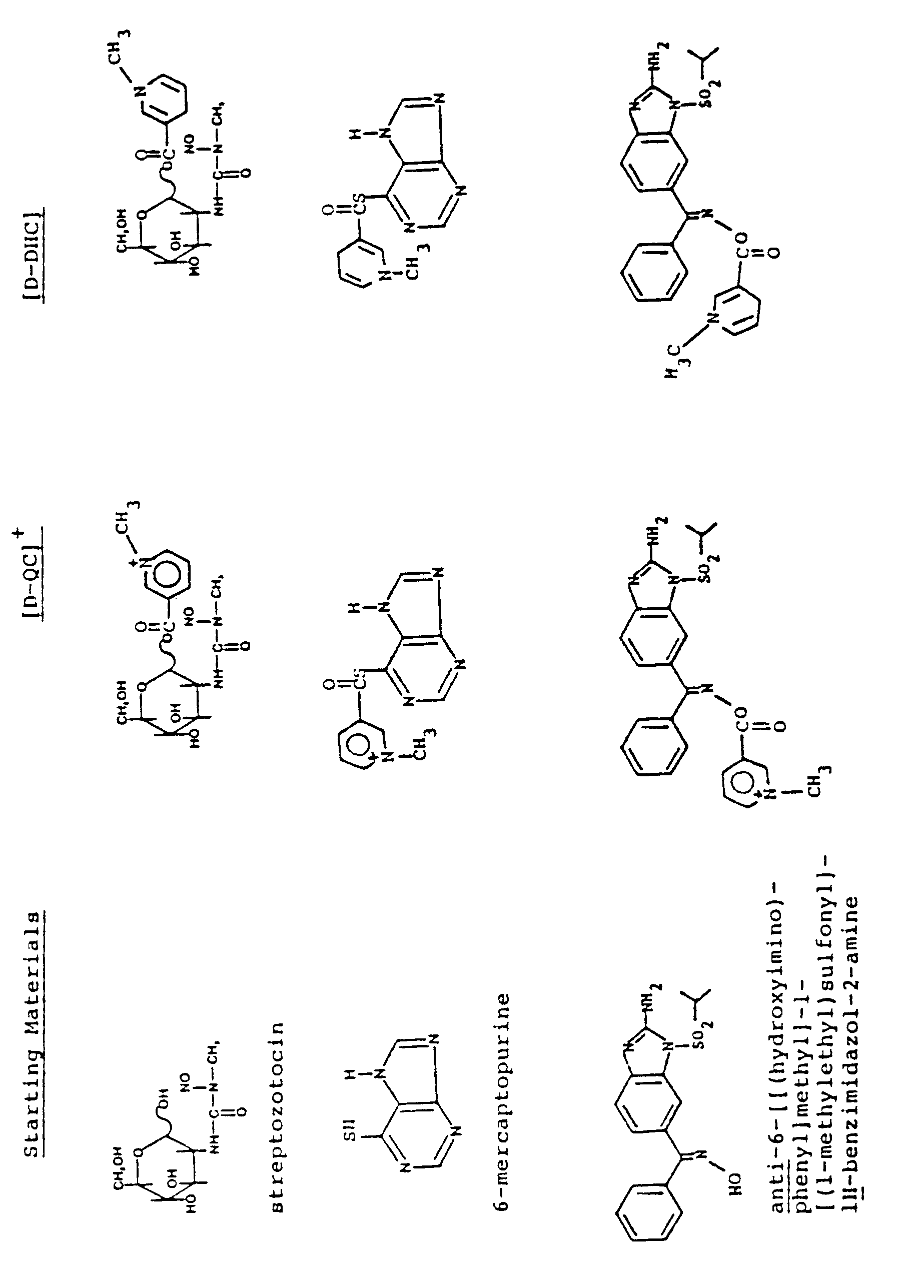

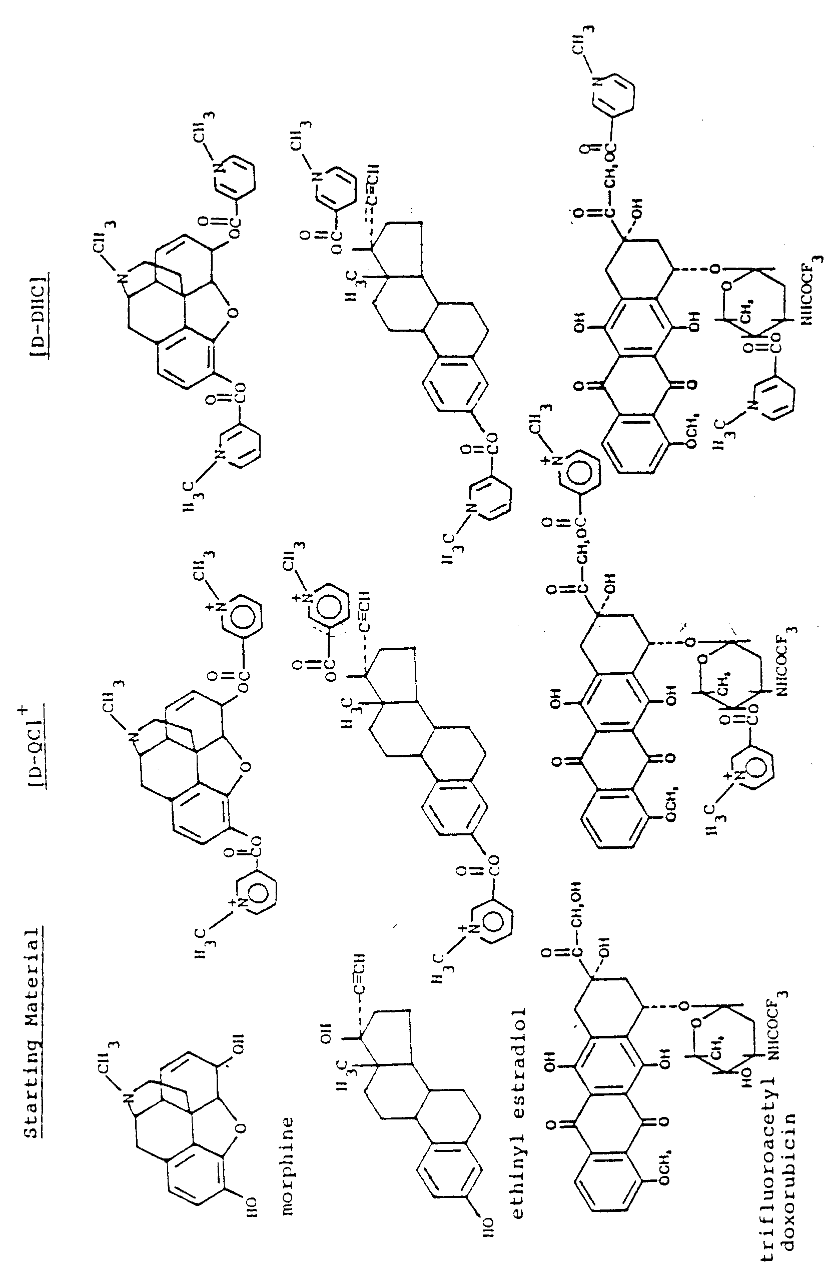

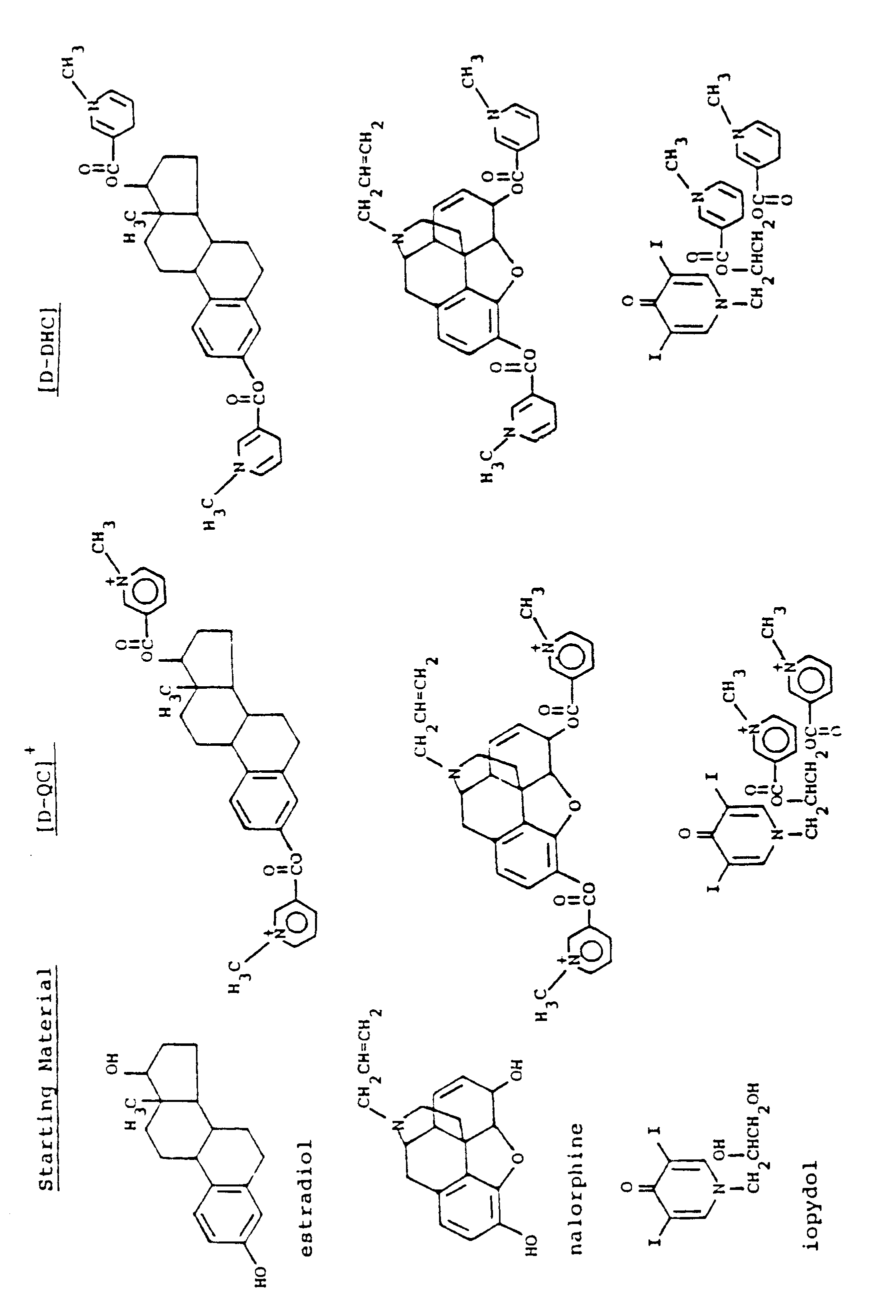

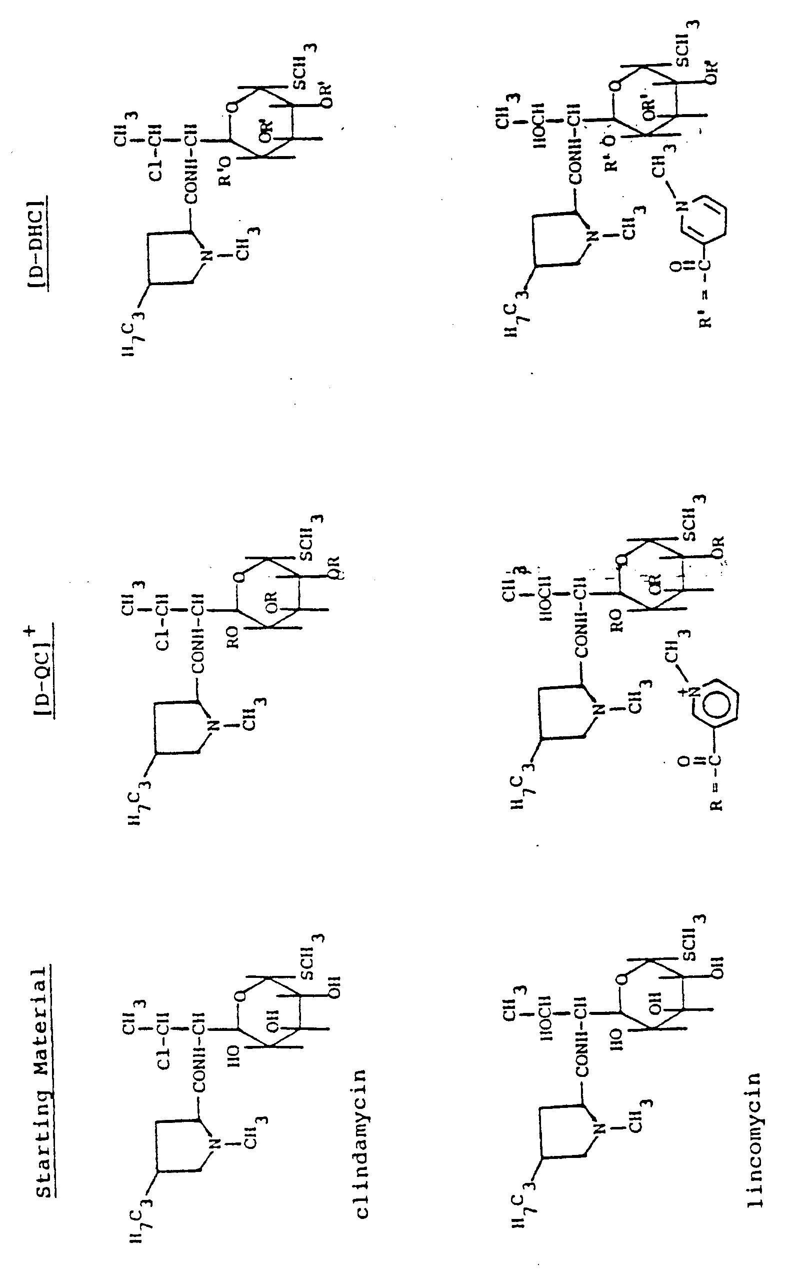

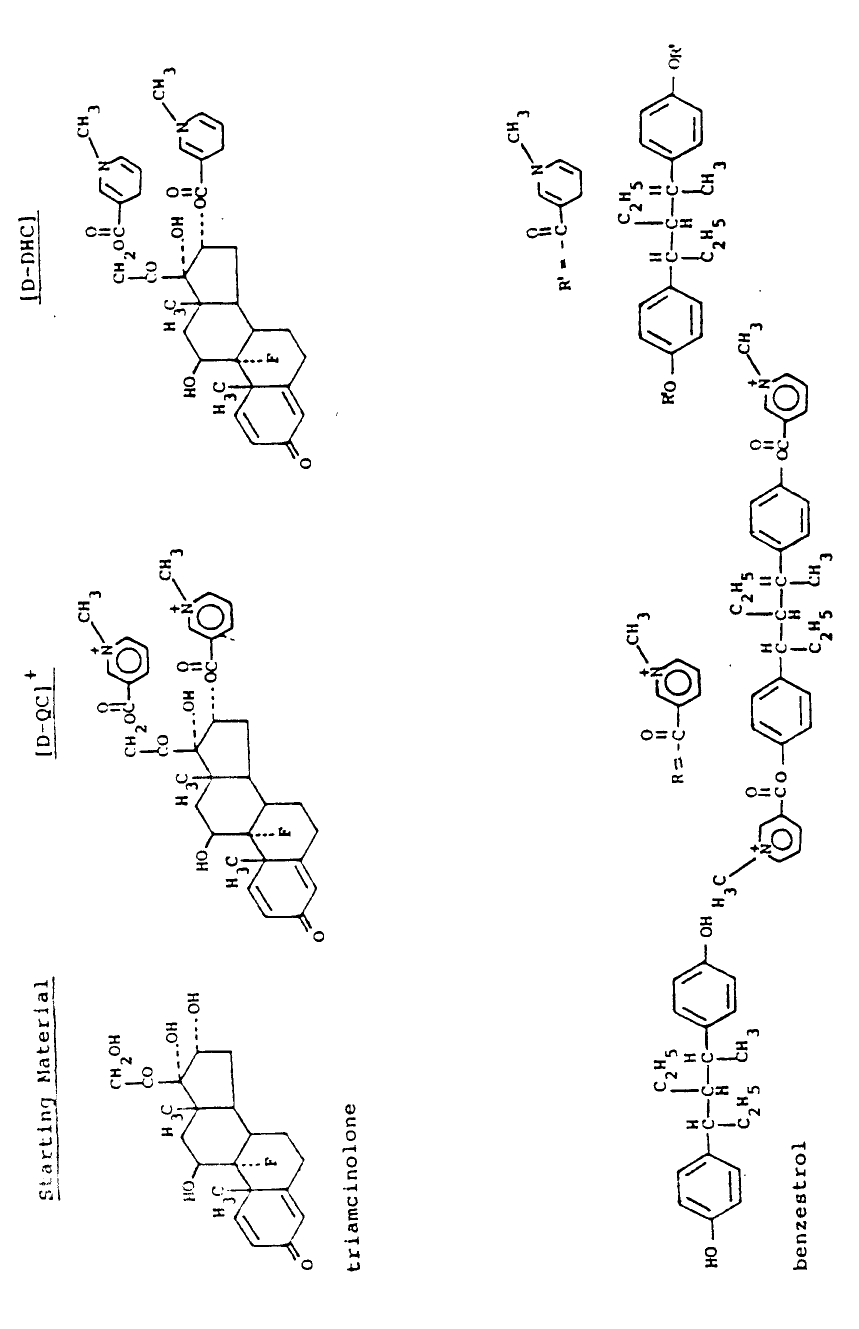

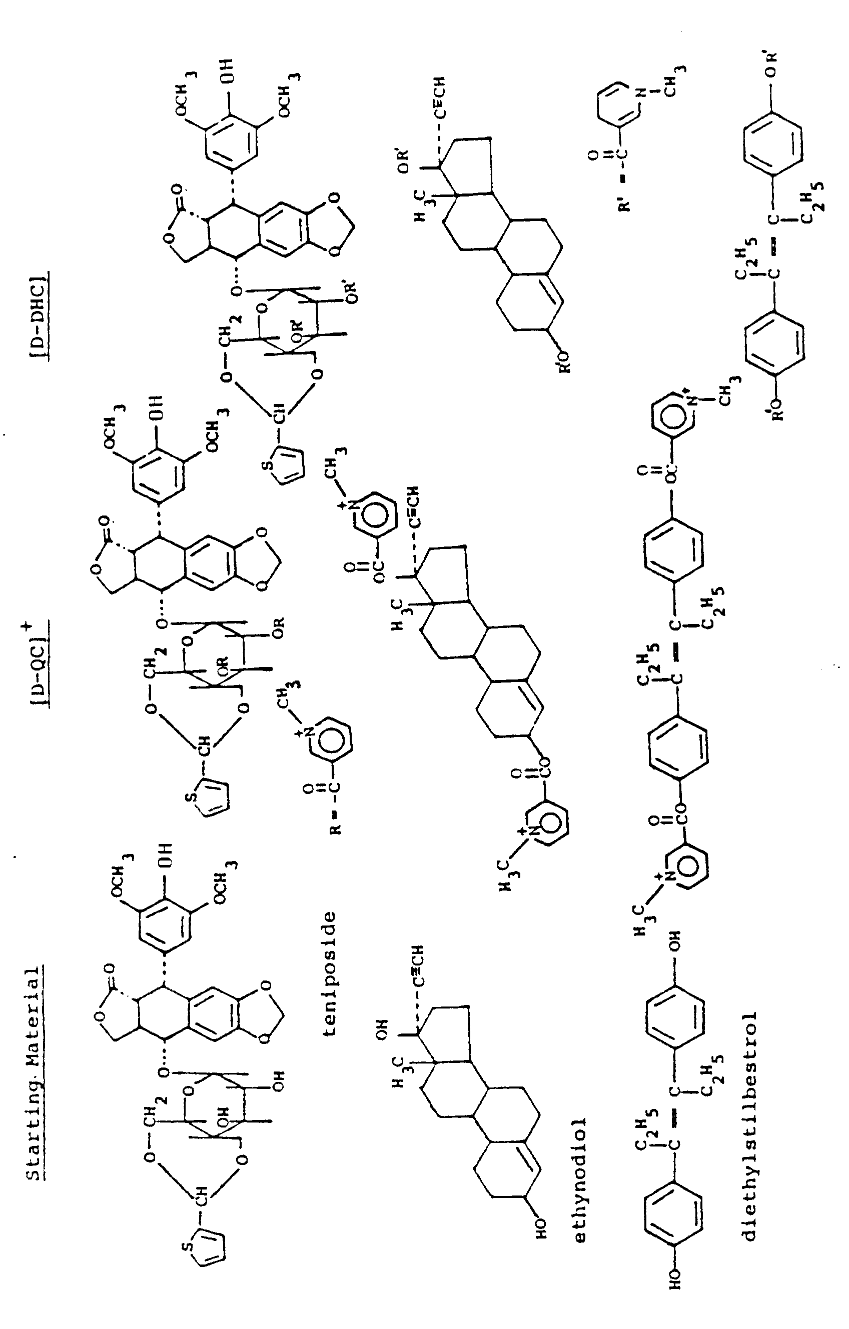

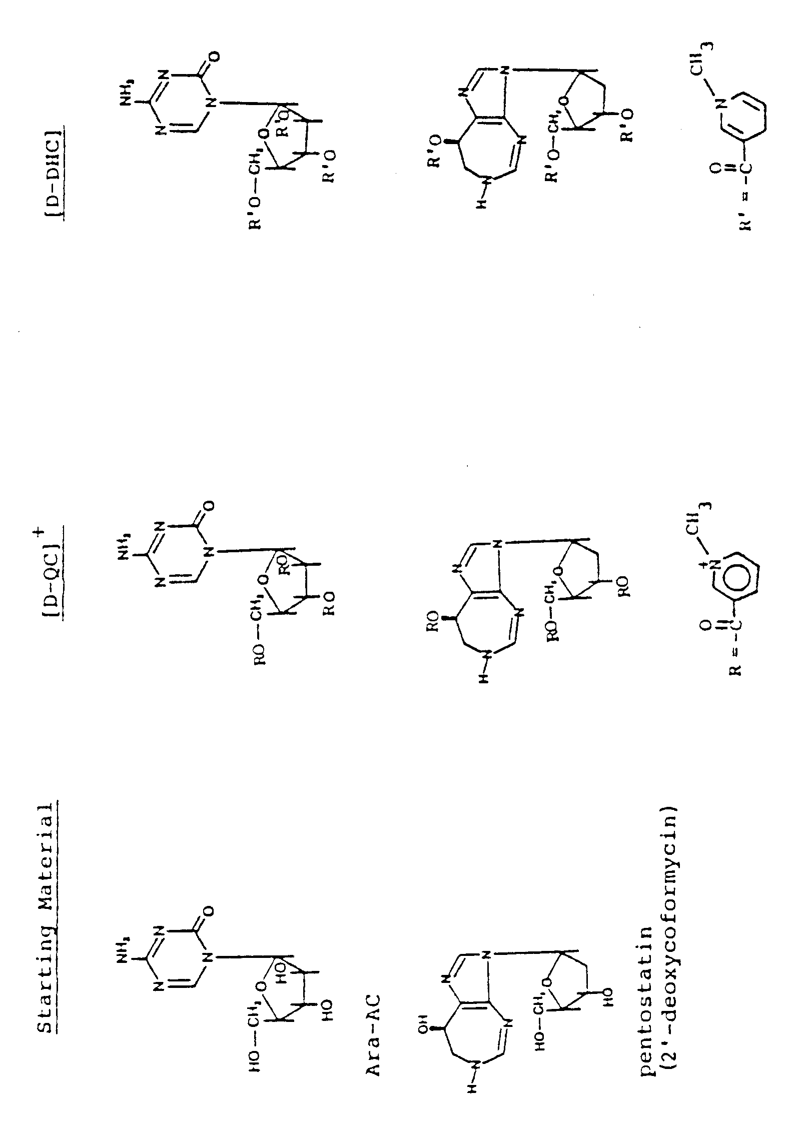

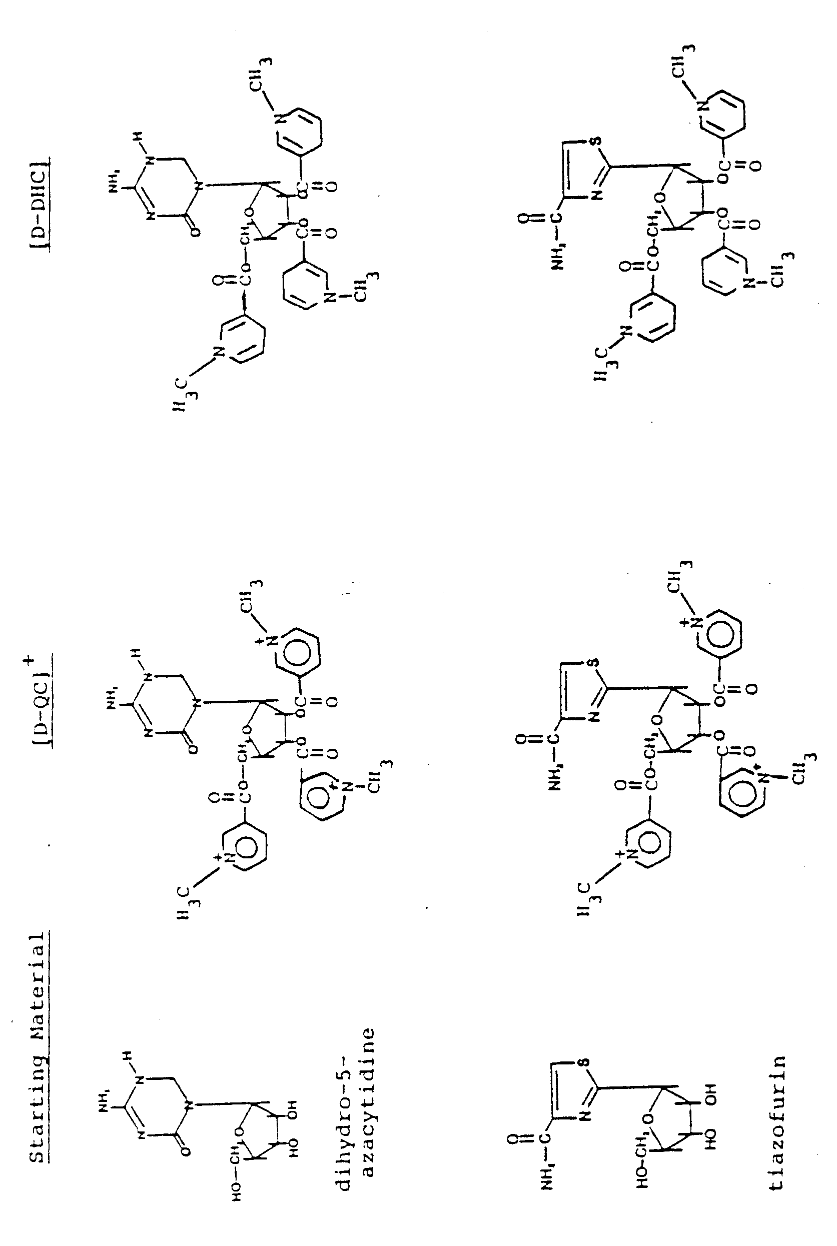

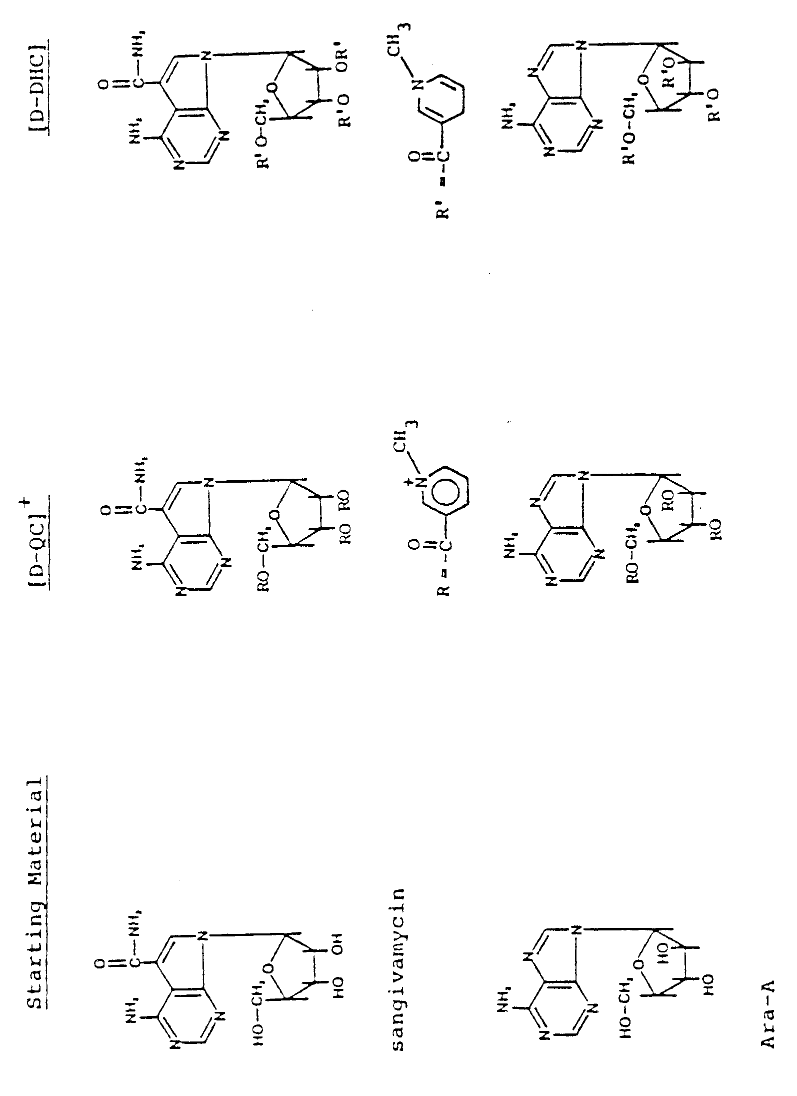

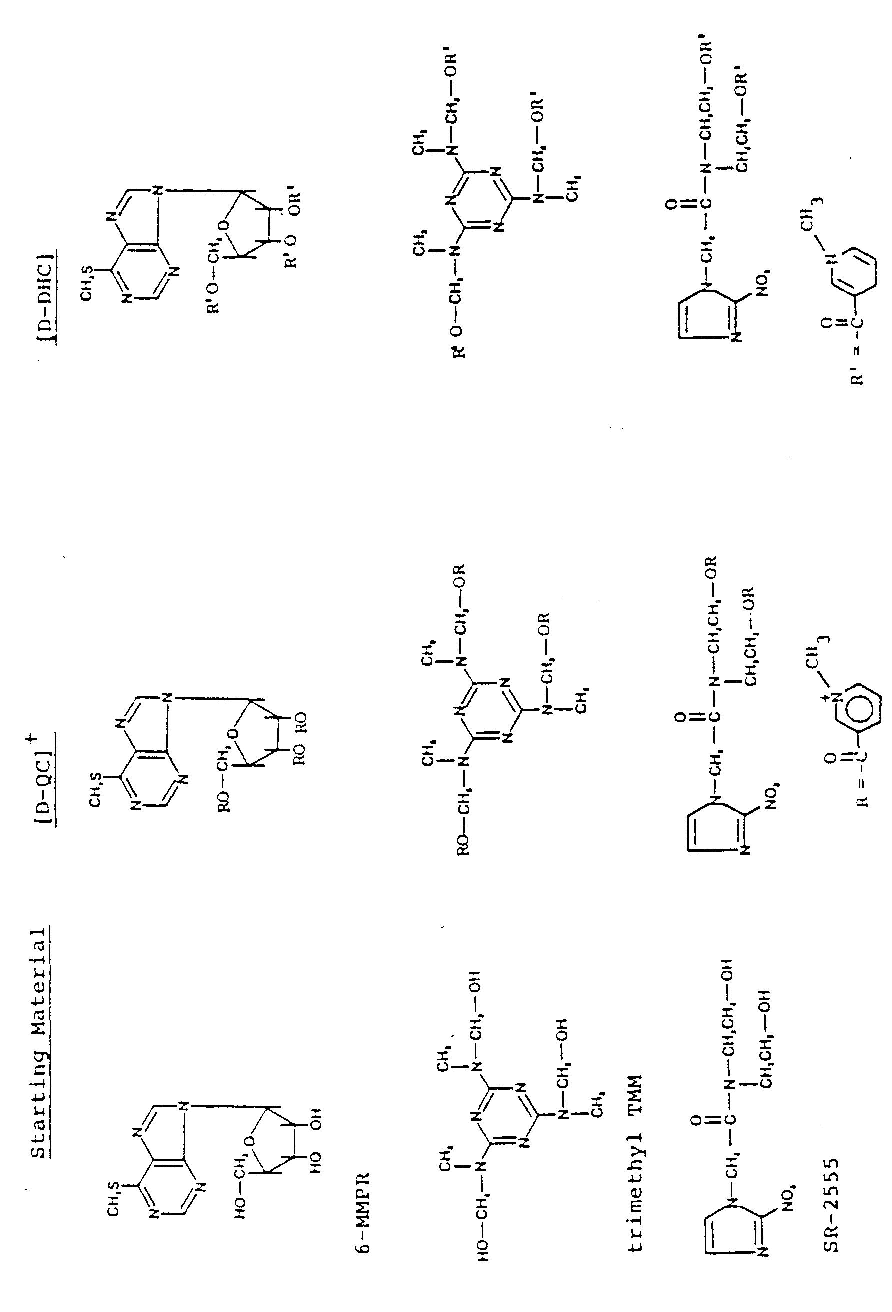

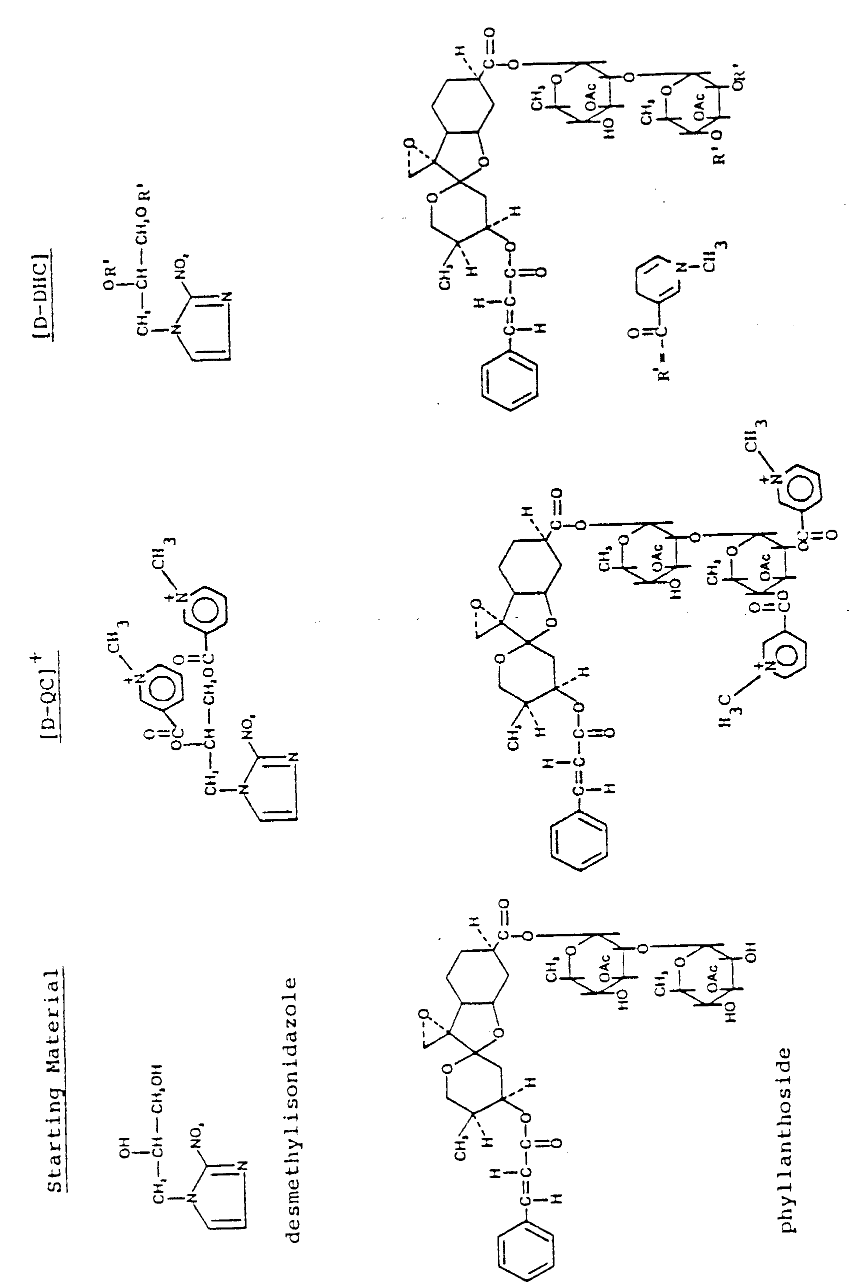

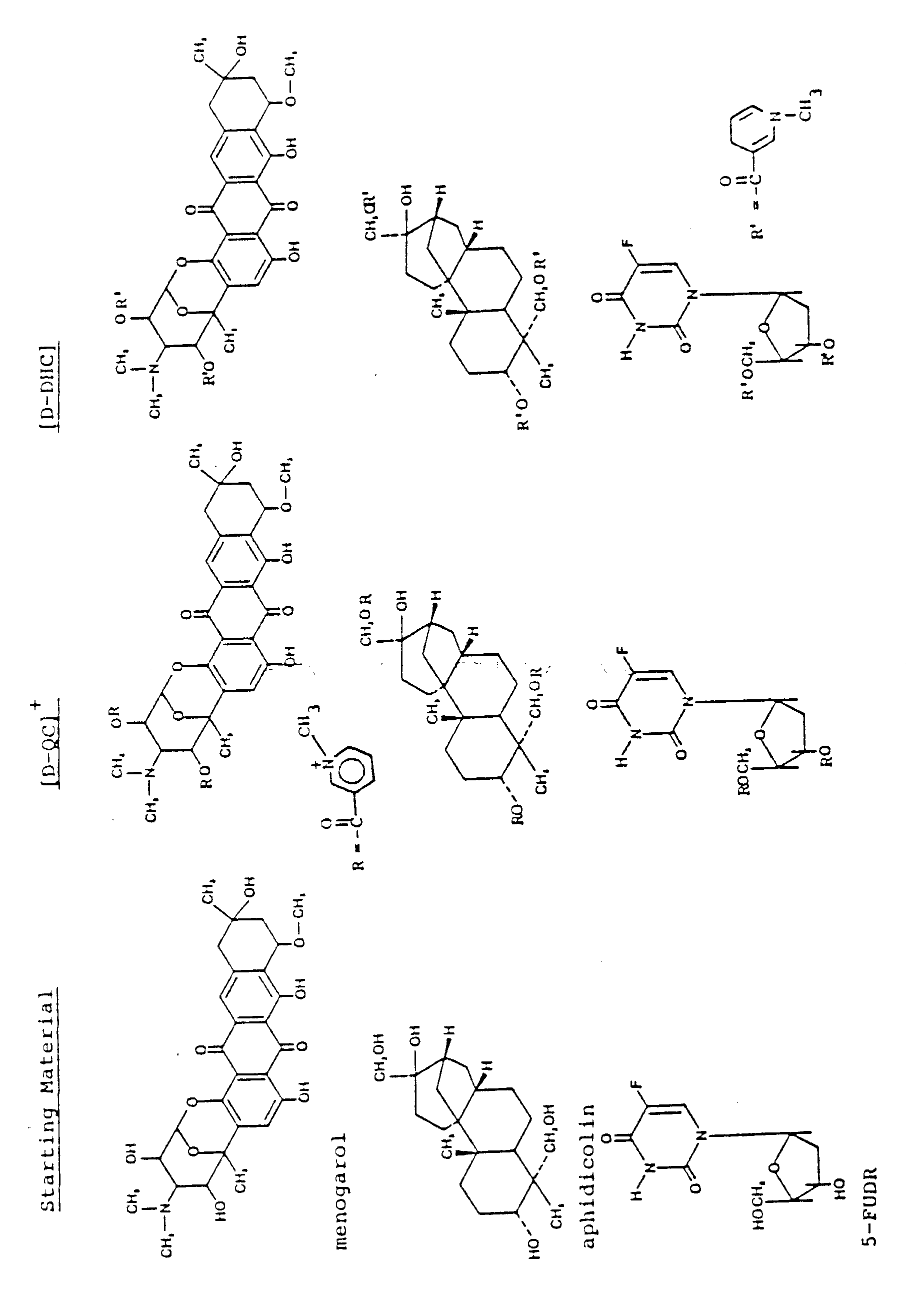

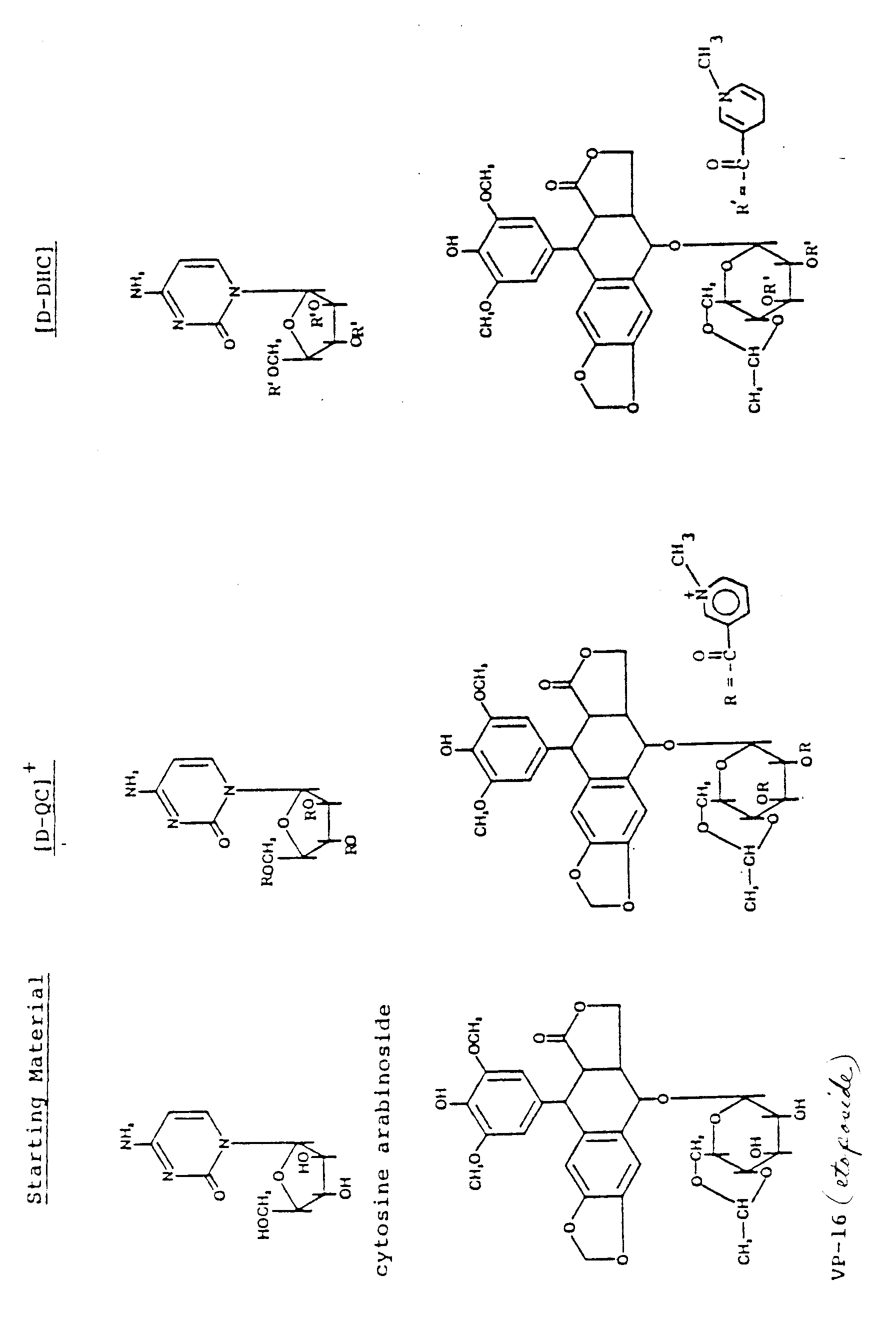

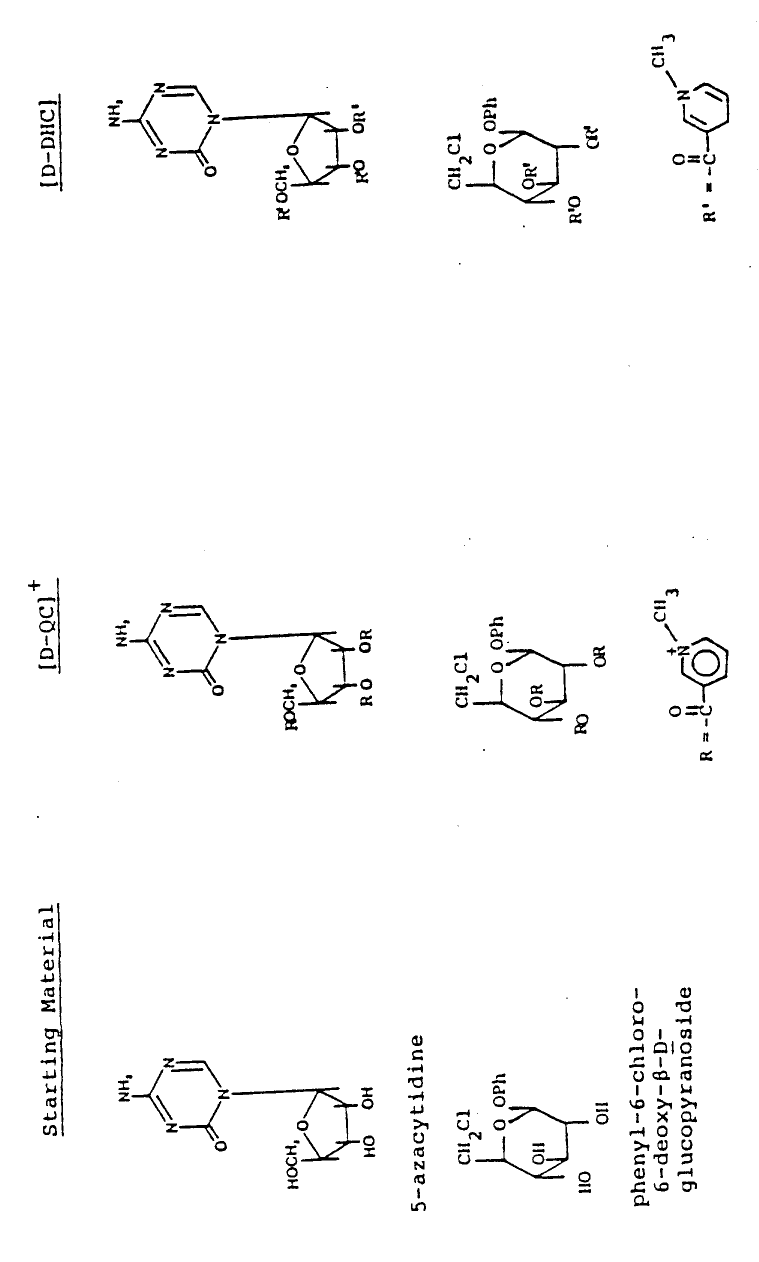

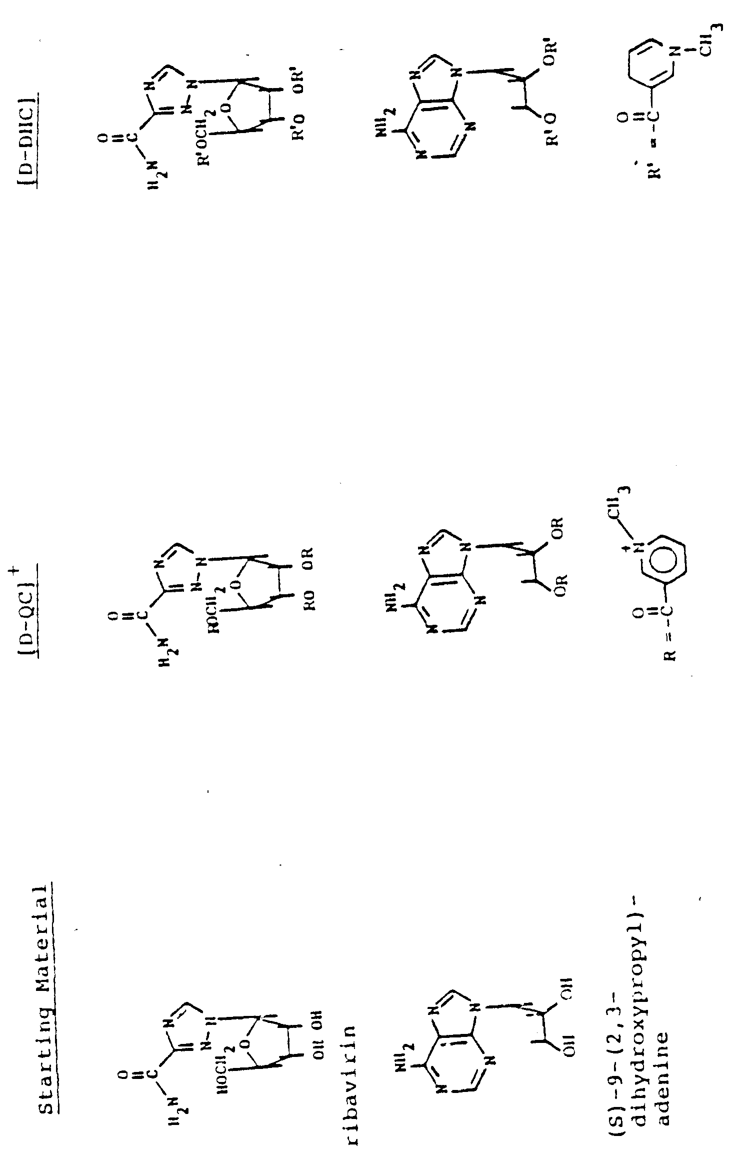

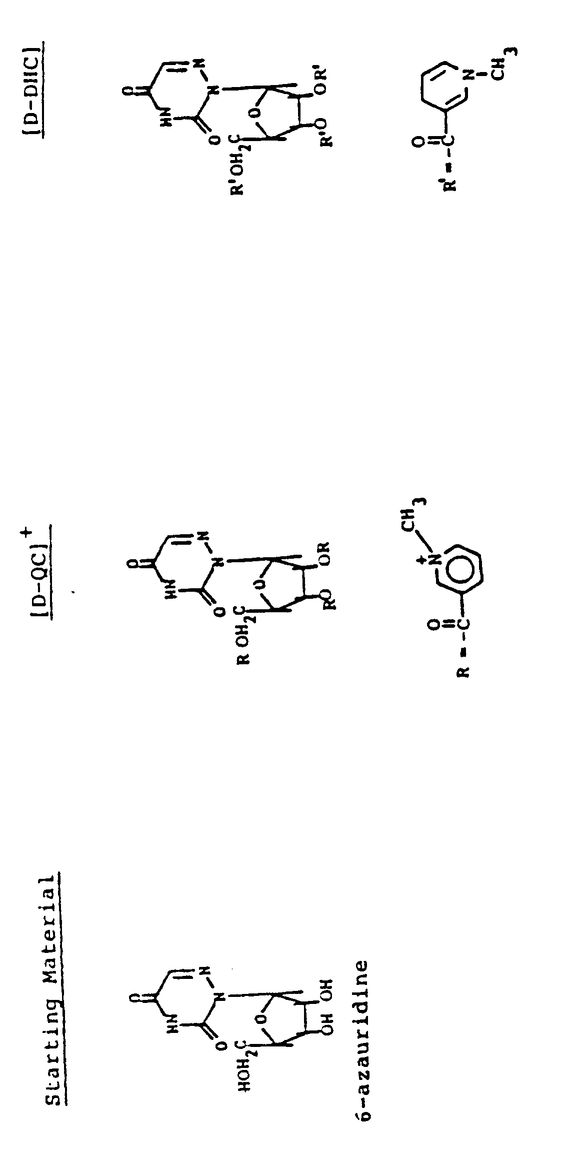

### Method J

This is a variation of Method I used when the drug contains a -COOH function which is to be protected.

The drug is first converted to the corresponding ethyl ester by conventional esterification techniques. That ester is then used as the starting material and Method I is repeated. The -COOH group may be similarly converted to other ester groups.

The representative drugs listed below may be derivatized in this manner to the corresponding [D-QC]⁺ and [D-DHC] compounds.

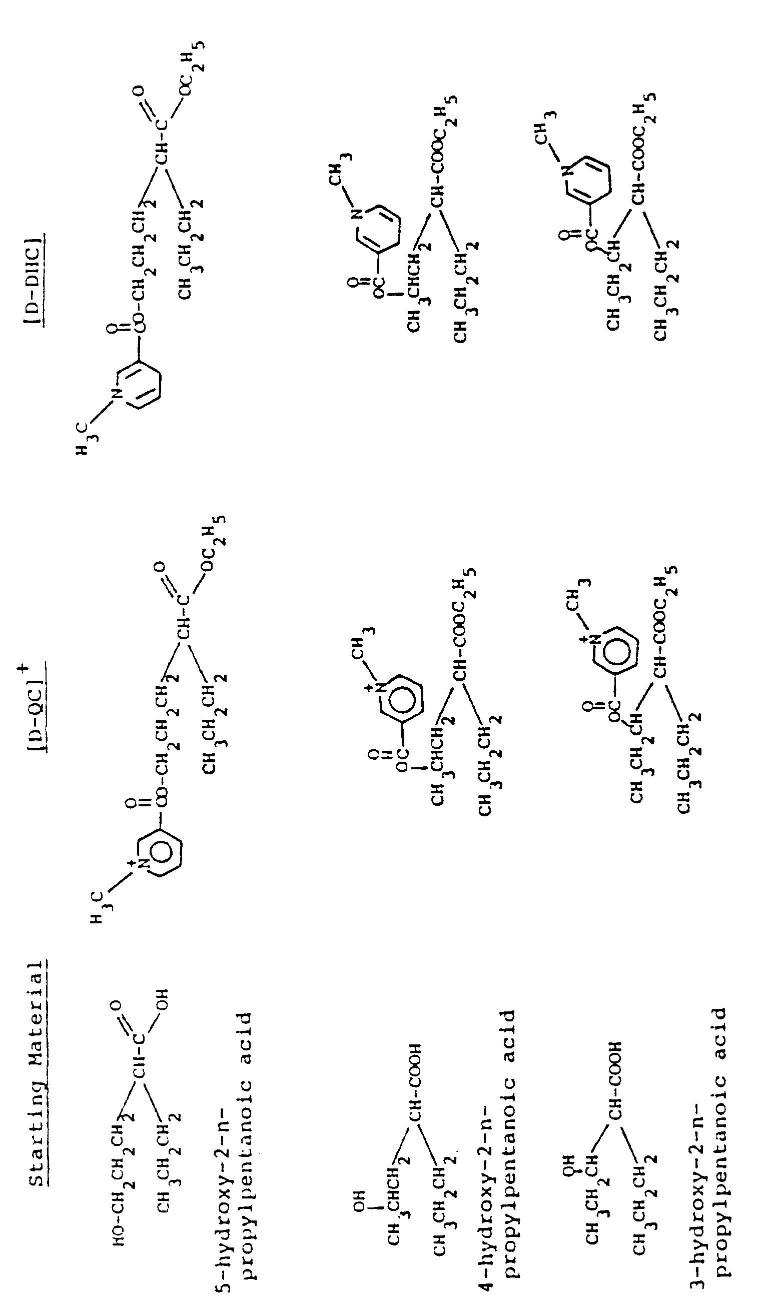

### Method K

Method I is followed, except that in the first step, a starting material of the formula 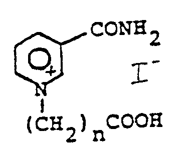 wherein n = 1-3, preferably 2, prepared as in Method H, is used in place of nicotinic acid.

The representative drugs listed below may be derivatized in this manner to the corresponding [D-QC]⁺ and [D-DHC] compounds, as may the remaining drugs listed with Method I.

Similarly, Method K may be combined with Method J to afford the corresponding derivatives, e.g. of the drugs listed with that method.

A starting material of the formula set forth immediately above can also be substituted for nicotinic acid in Method E to afford the corresponding derivatives, e.g. of the drugs listed with that method.

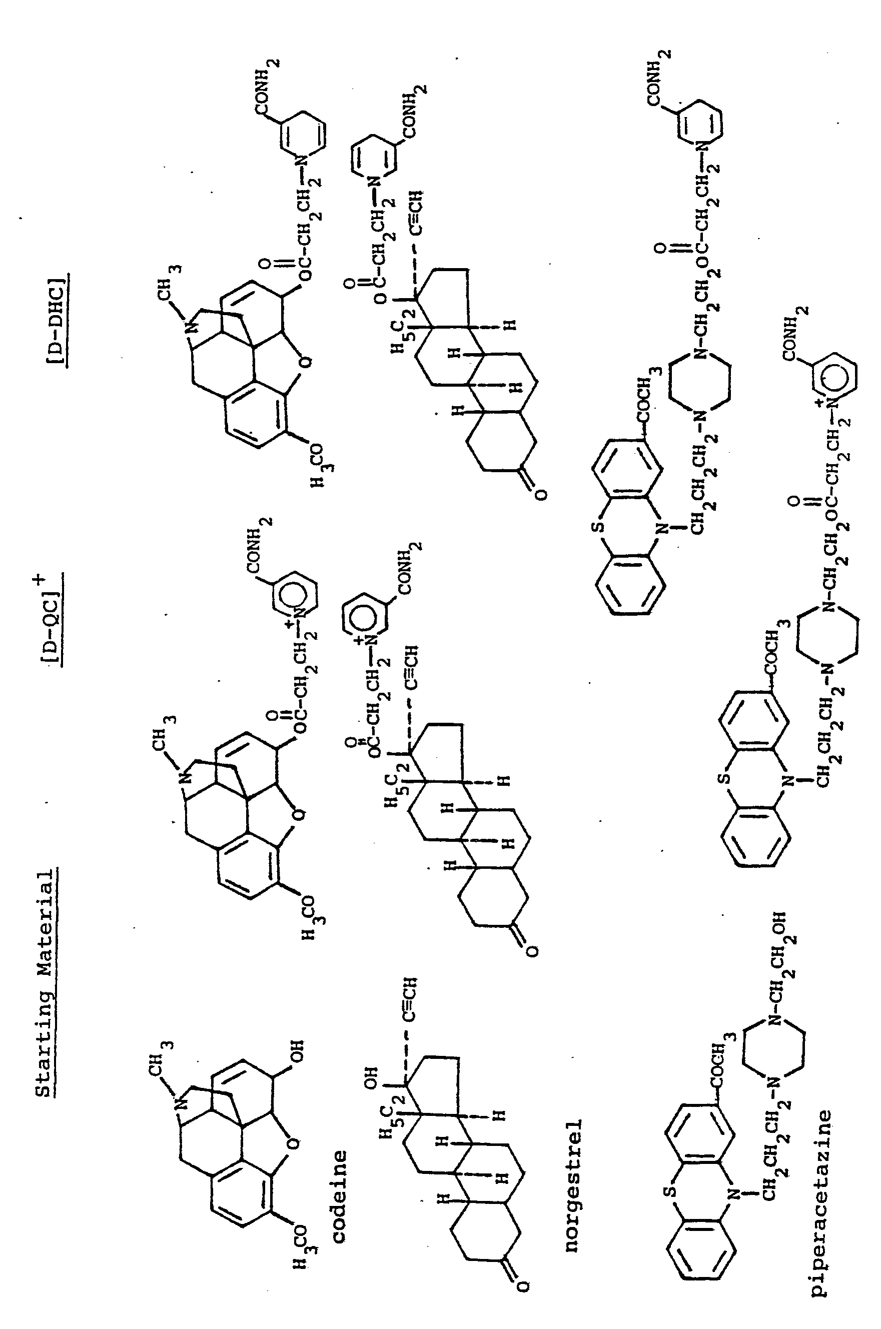

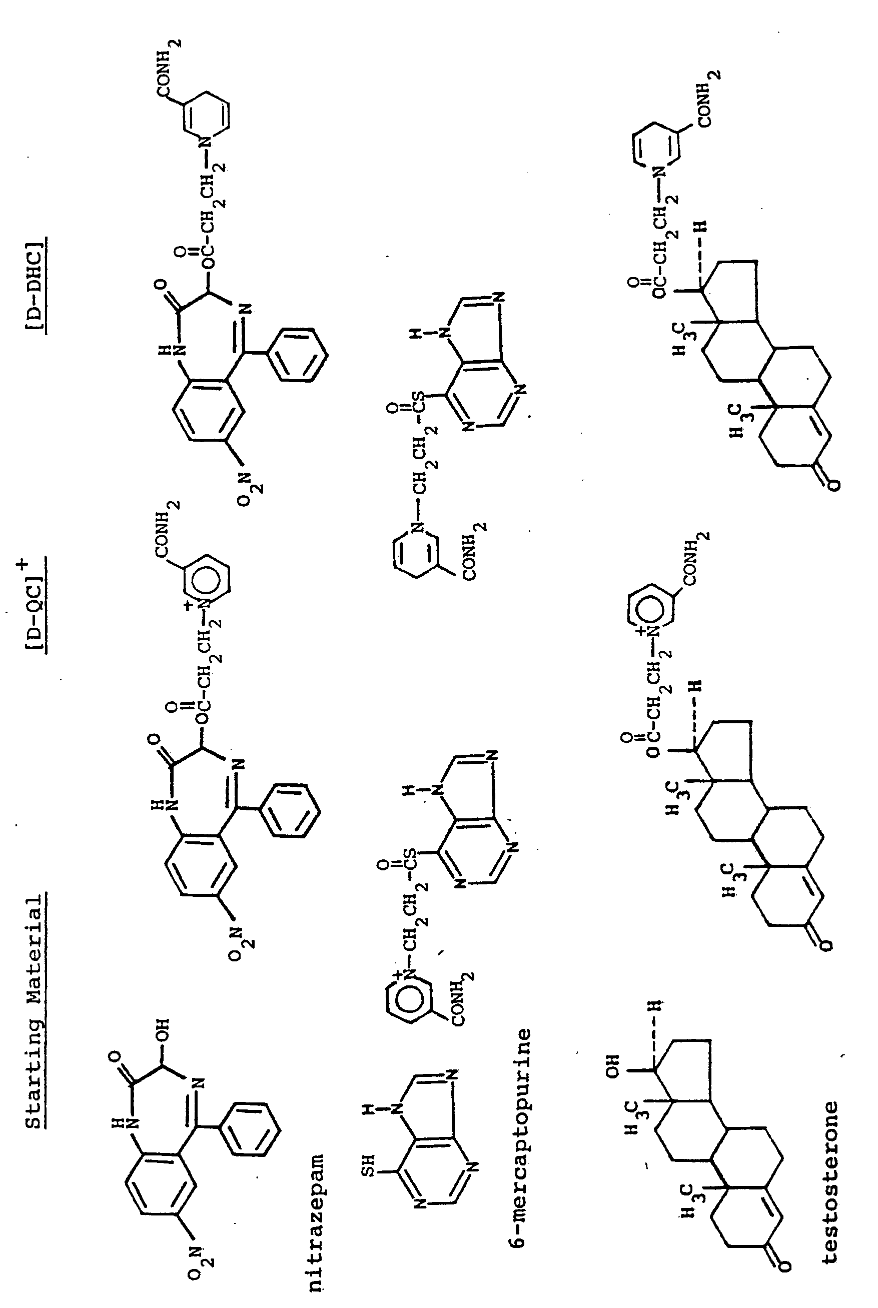

### Method L

Method I is followed, except that in the first step, the drug is reacted with 3-quinolinecarboxylic acid or its acid chloride or anhydride instead of nicotinic acid or its acid chloride.

The representative drugs listed below may be derivatized in this manner to the corresponding [D-QC]⁺ and [D-DHC] compounds, as may the remaining drugs listed with Method I.

Similarly, Method L may be combined with Method J to afford the corresponding derivatives, e.g. of the drugs listed with that method.

3-Quinolinecarboxylic acid or its acid chloride or anhydride can also be substituted for nicotinic acid or its acid chloride in Method E to afford the corresponding derivatives, e.g. of the drugs listed with that method.

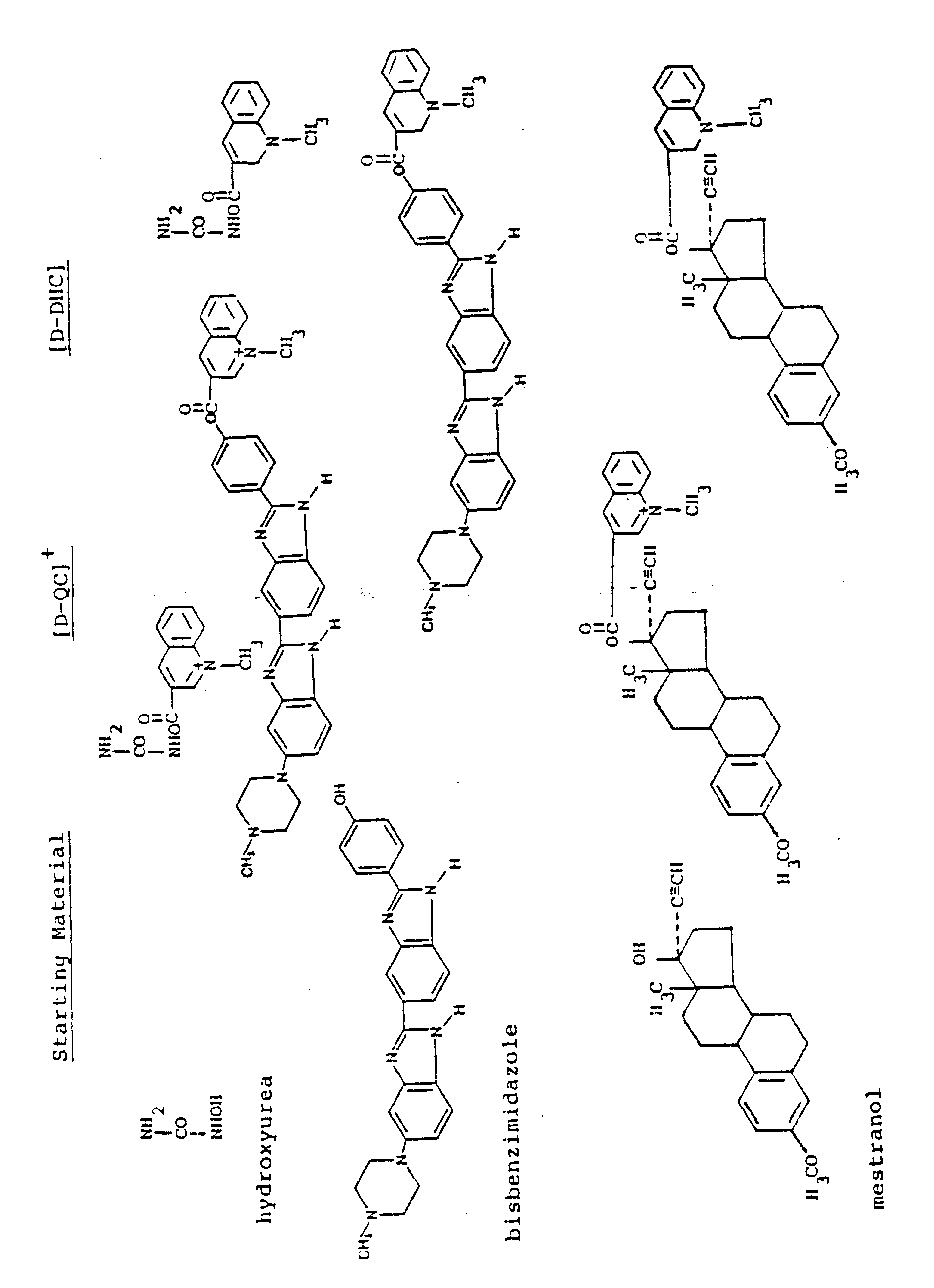

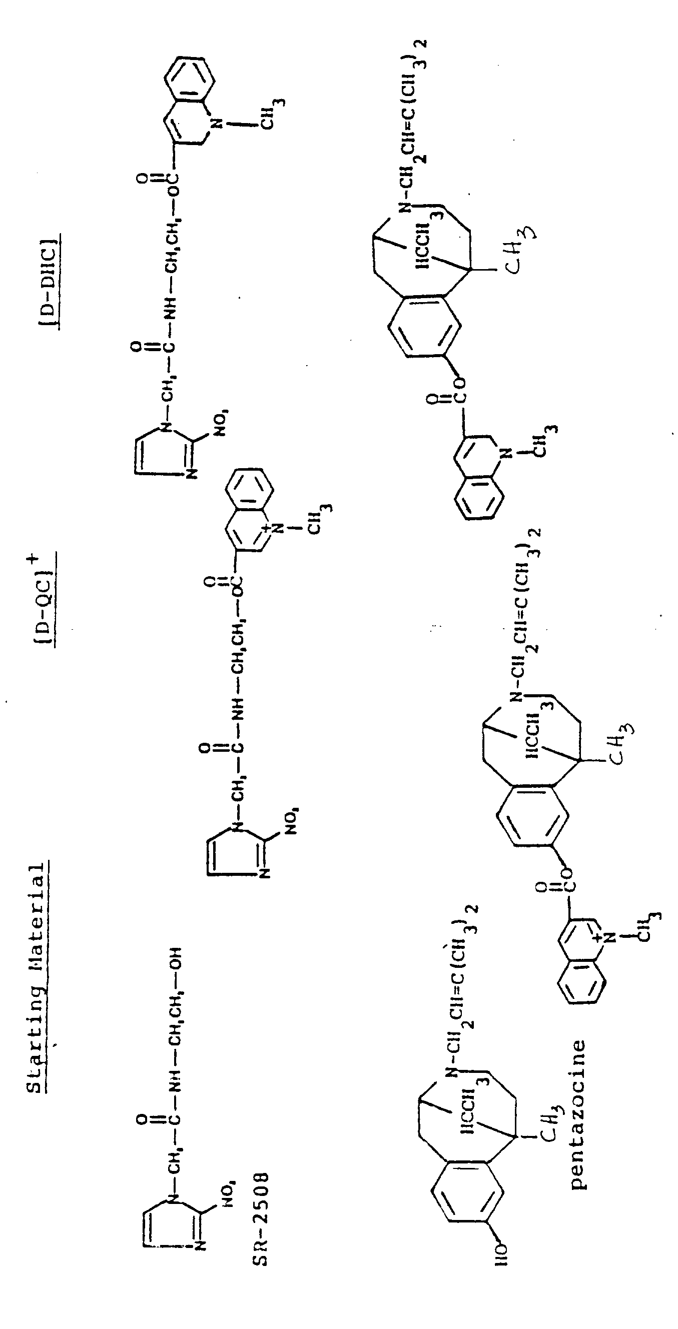

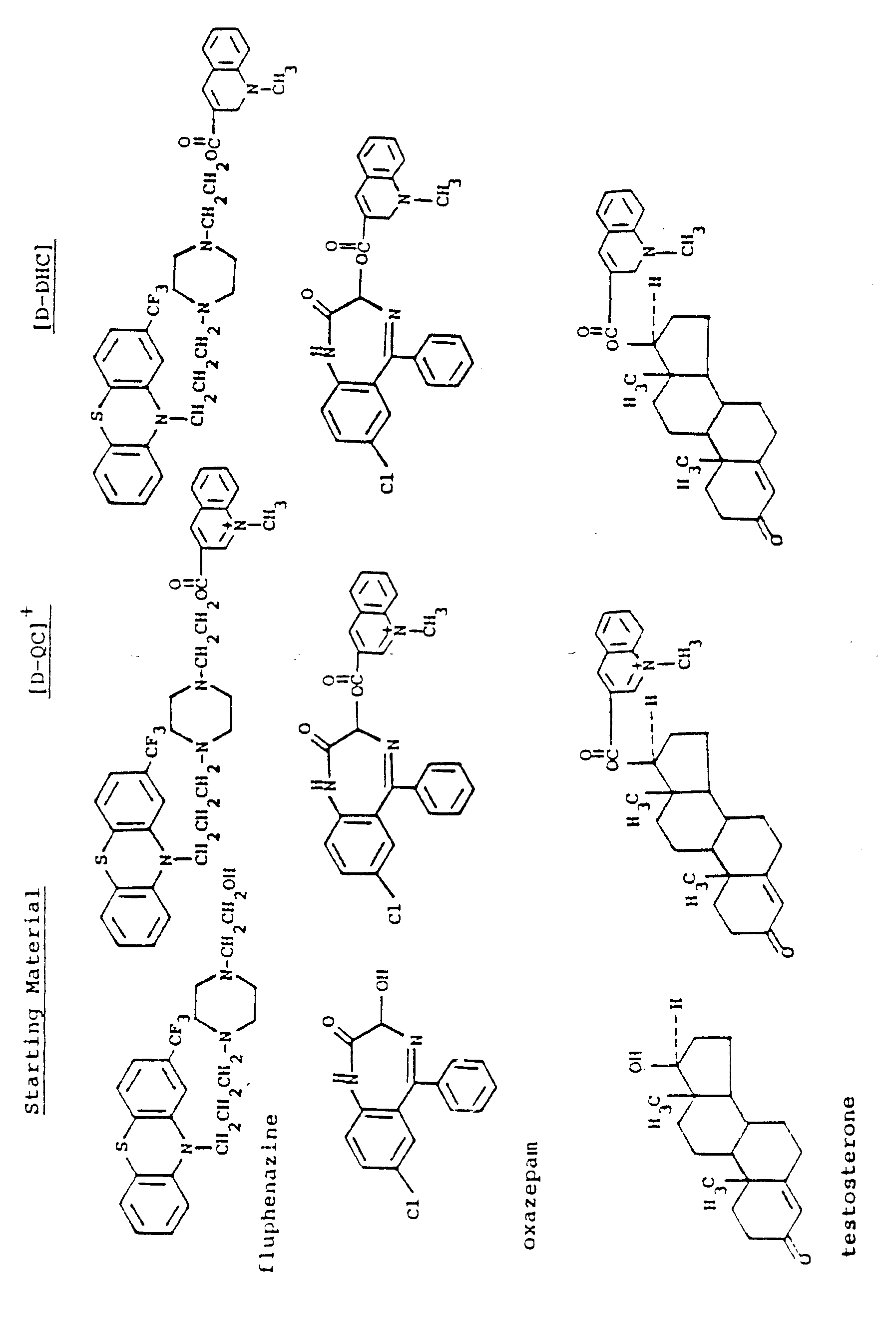

### Method M

Method I is followed, except that in the first step, a starting material of the formula 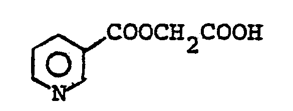 is used in place of nicotinic acid.

The representative drugs listed below may be derivatized in this manner to the corresponding [D-QC]⁺ and [D-DHC] compounds, as may the remaining drugs listed with Method I.

Similarly, Method M may be combined with Method J to afford the corresponding derivatives, e.g. of the drugs listed with that method.

A starting material of the formula set forth immediately above can also be substituted for nicotinic acid in Method E to afford the corresponding derivatives, e.g. of the drugs listed with that method.

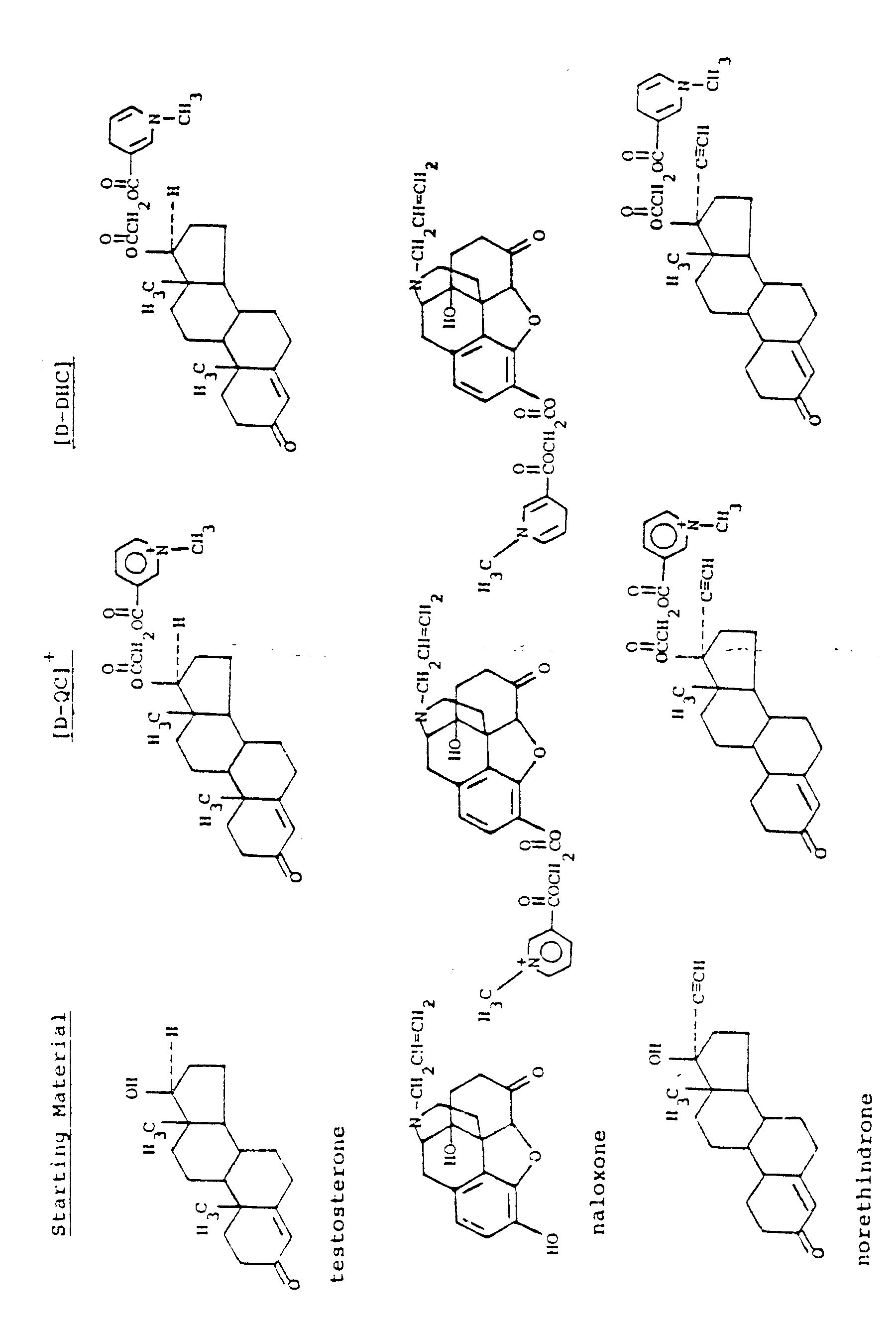

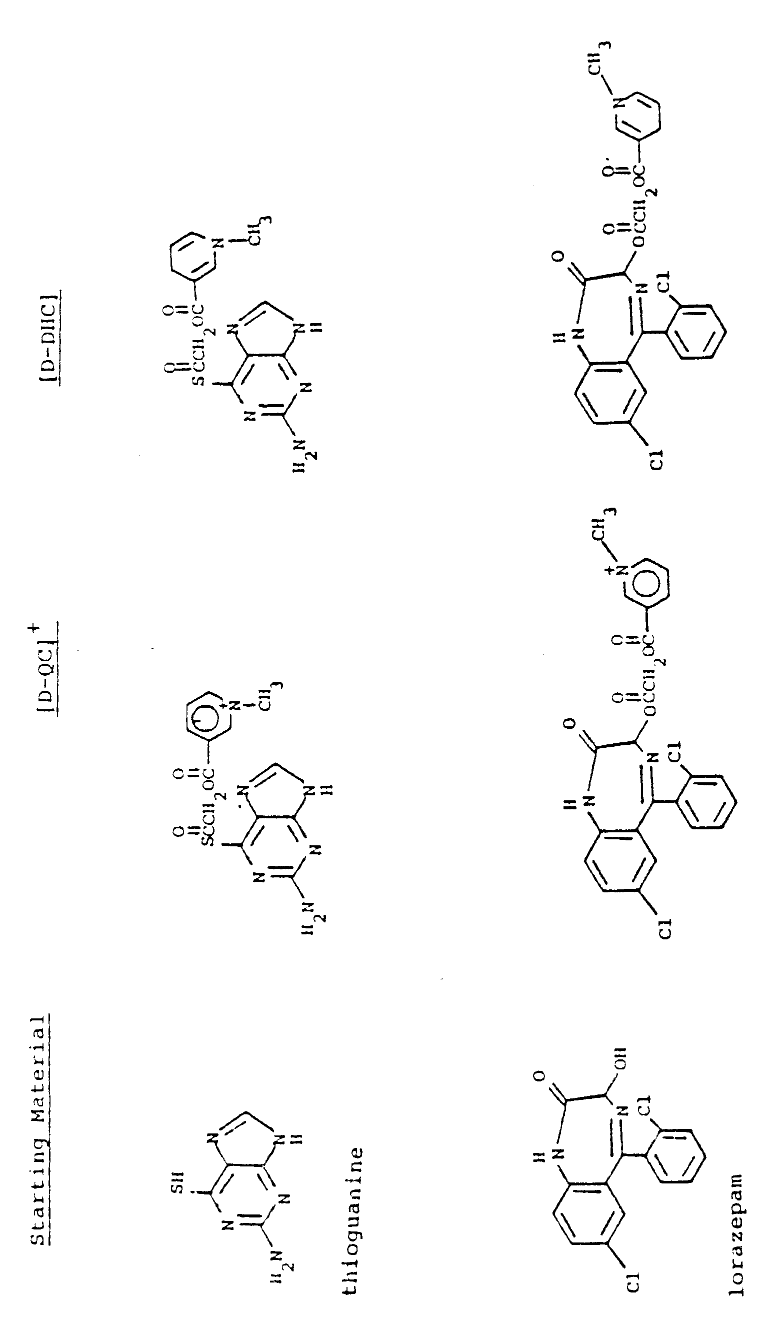

III. Methods for Derivatizing -COOH Functions in Drugs

### Method N

The drug is reacted with excess alcohol of the formula 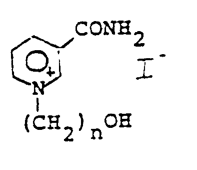 wherein n = 1-3, preferably 2, to convert the -COOH function to the corresponding 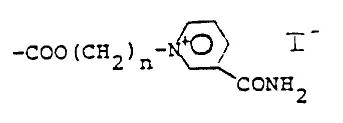 ester grouping. That ester is thus quaternized and subsequently reduced as described above in Method A. When the drug contains more than one reactive carboxyl function, reaction conditions may be varied so that more than one carboxyl function will be converted to ester groupings. (The starting alcohol may be prepared from nicotinamide, e.g. when n = 2, by reacting 2-iodoethanol with nicotinamide.)

The representative drugs listed below may be derivatized in this manner to the corresponding [D-QC]⁺ and [D-DHC] compounds.

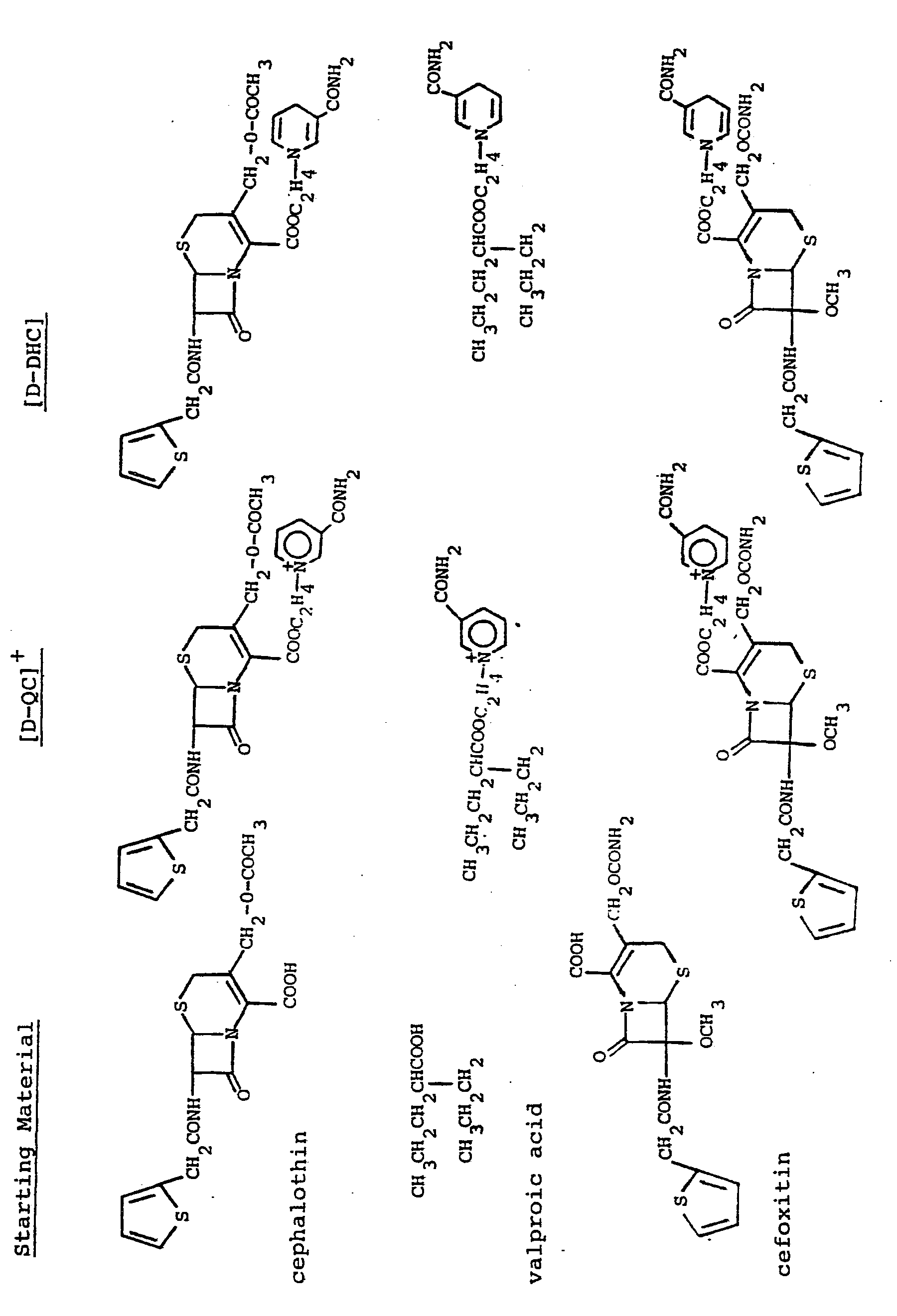

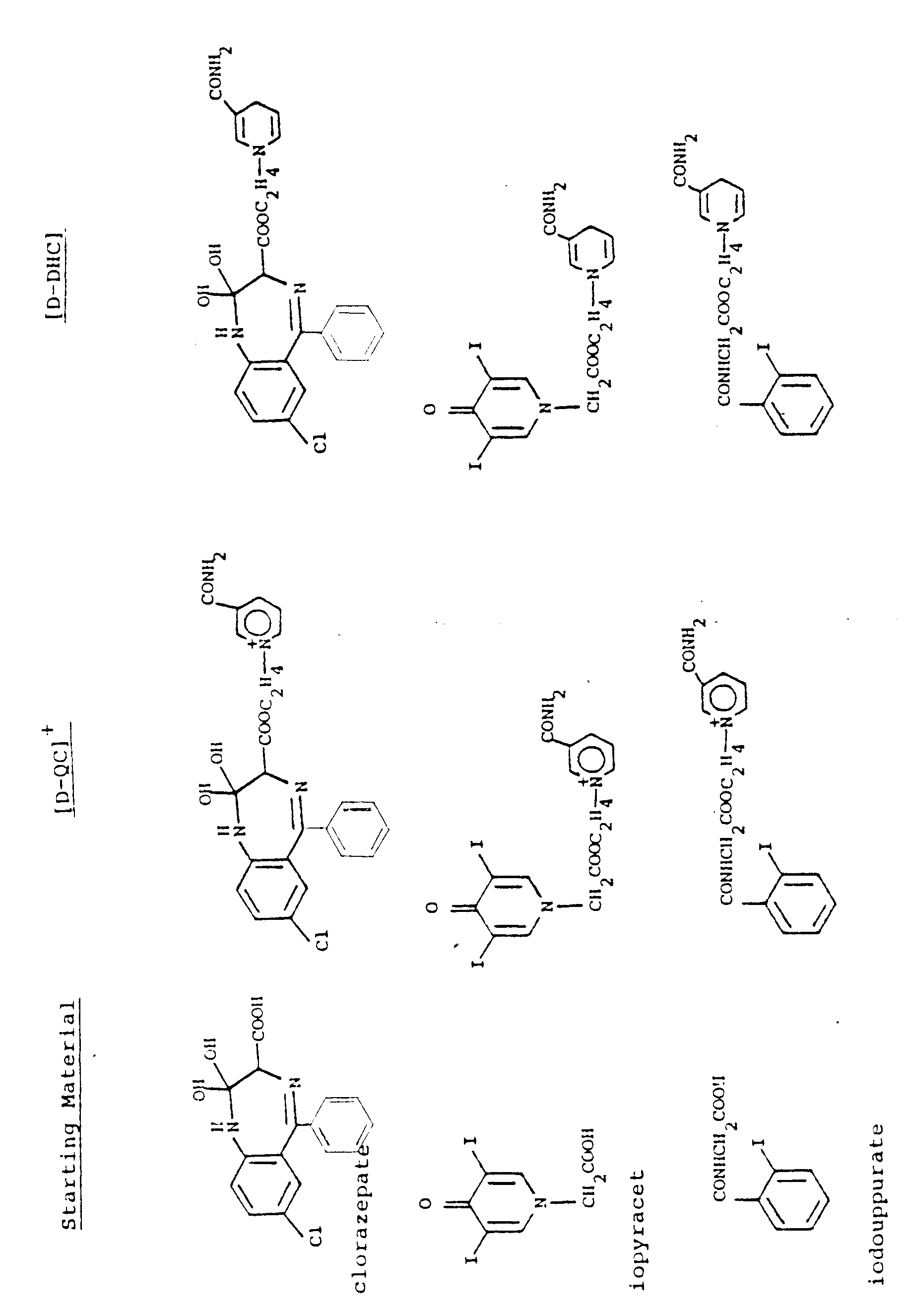

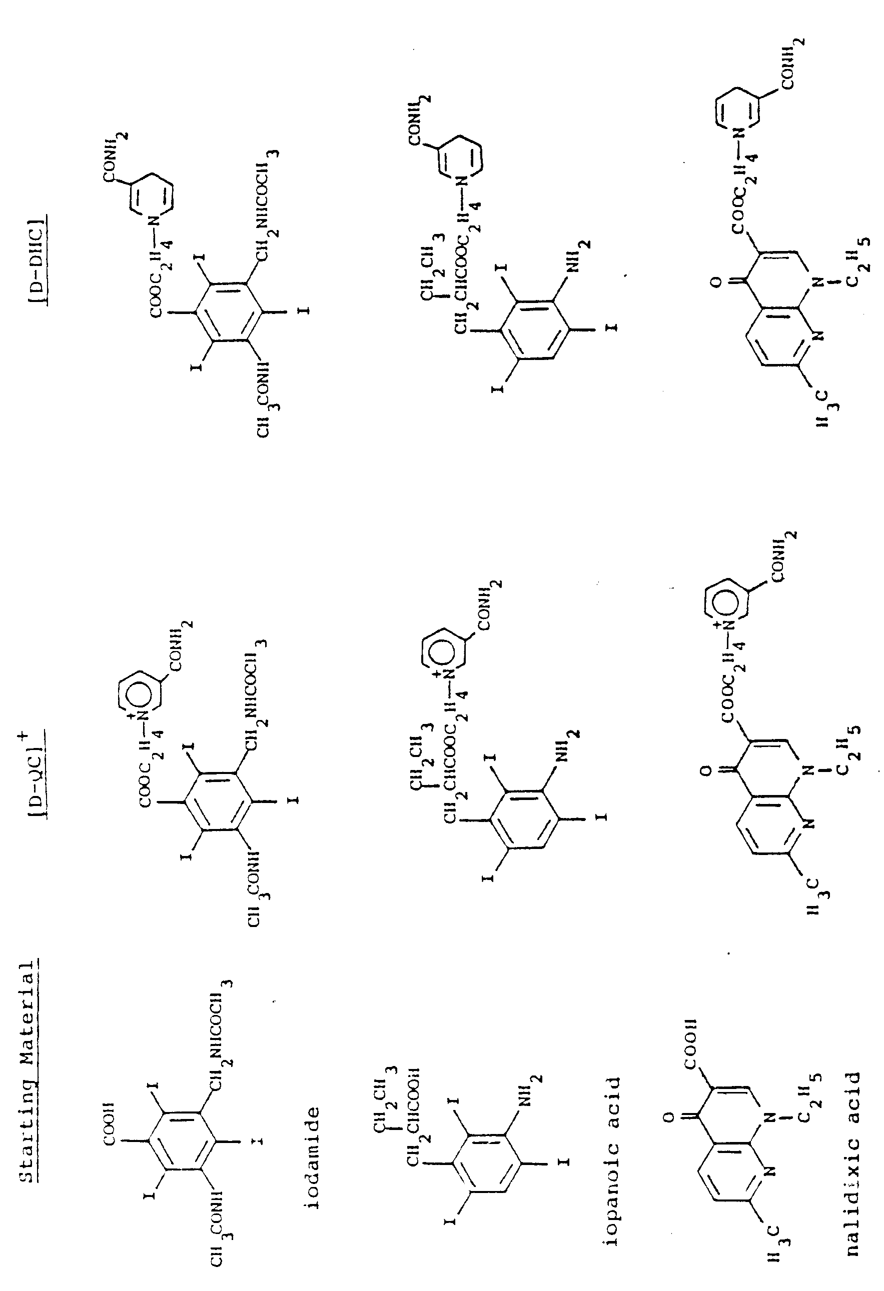

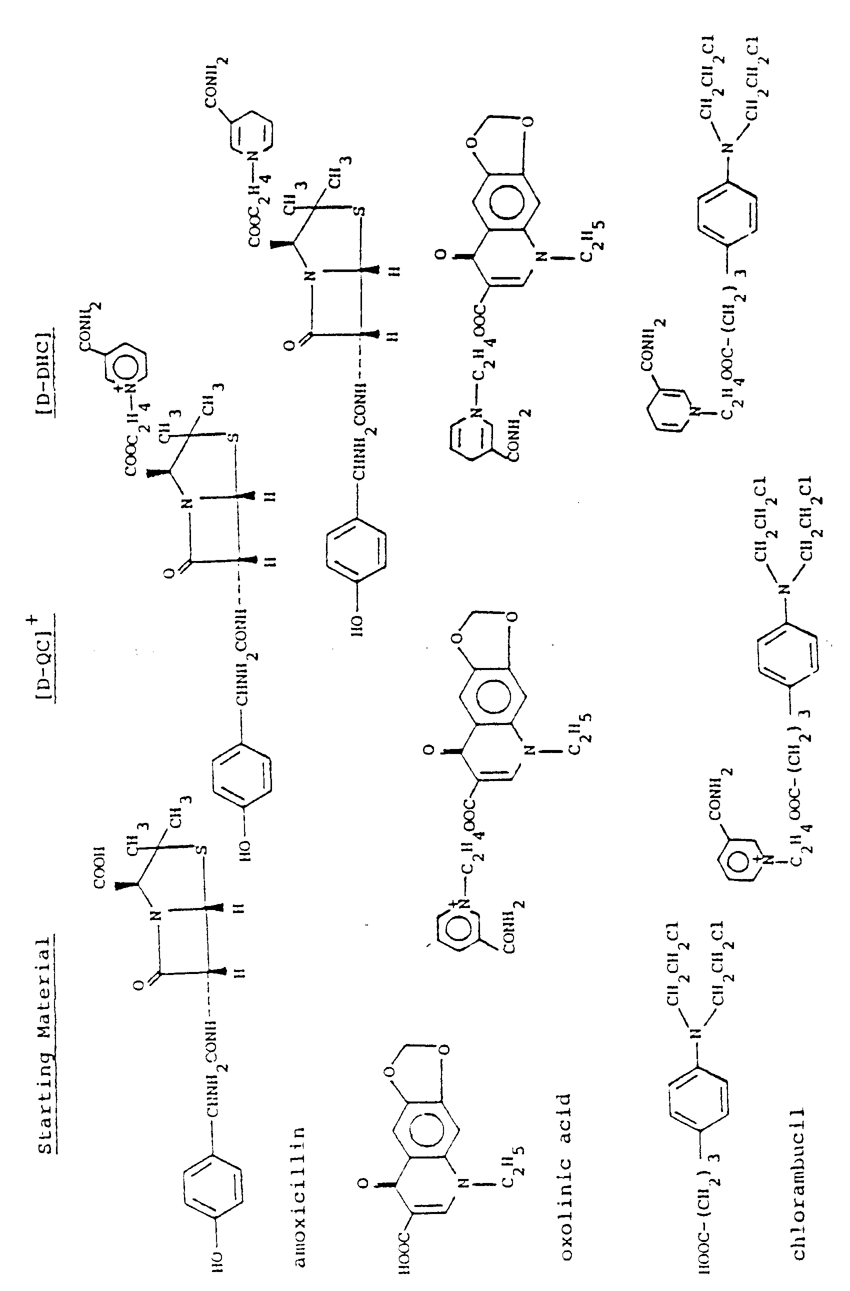

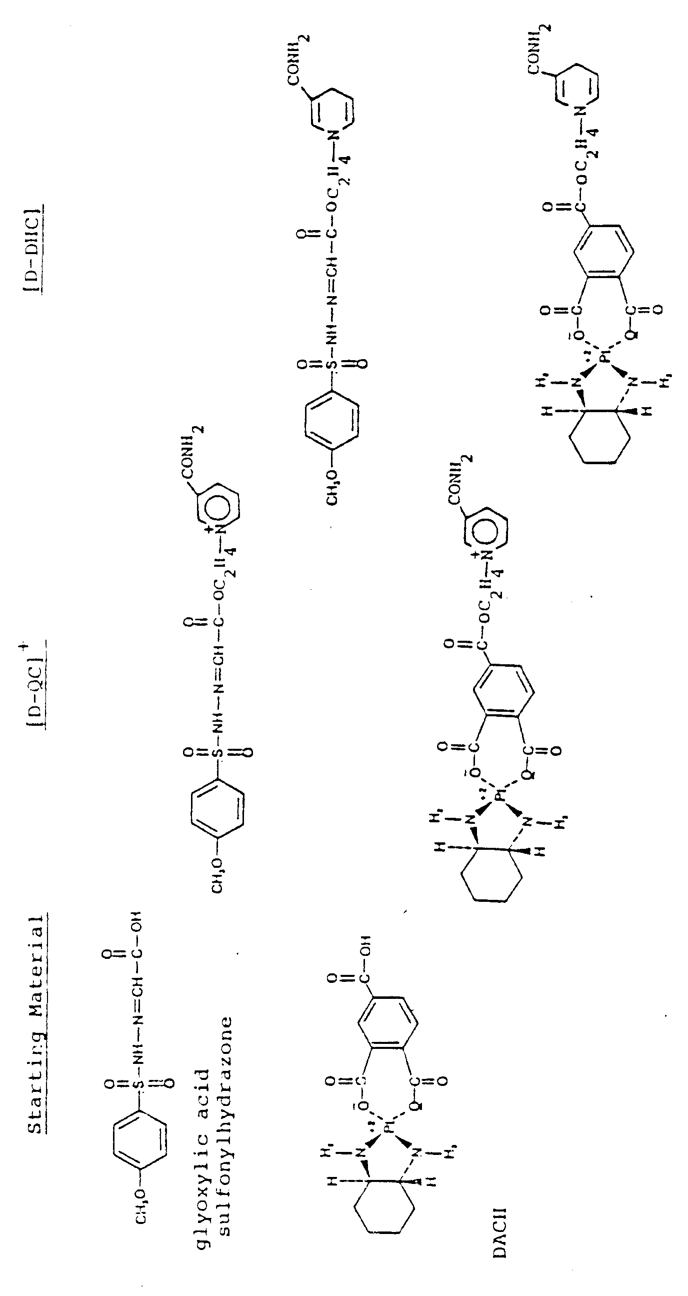

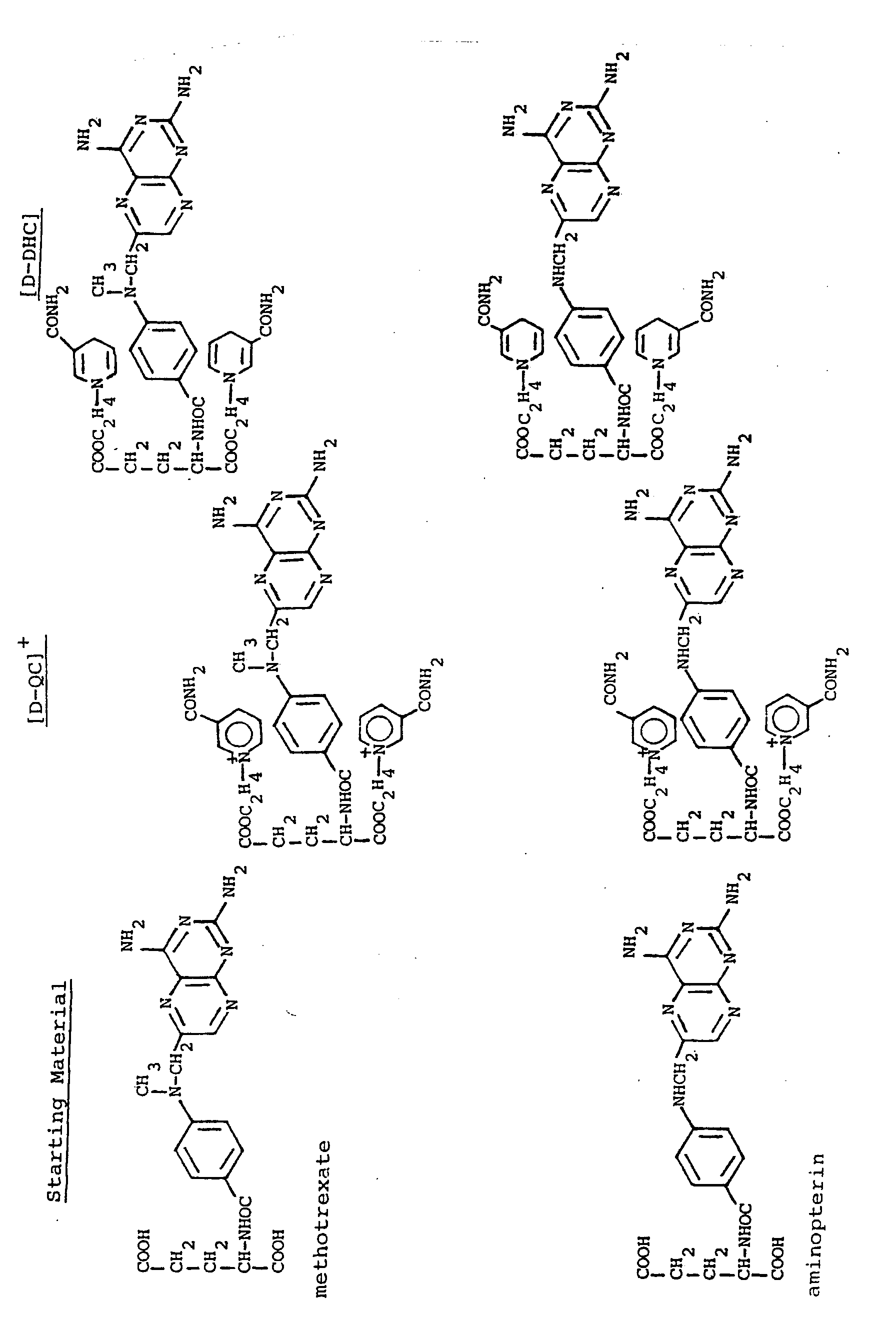

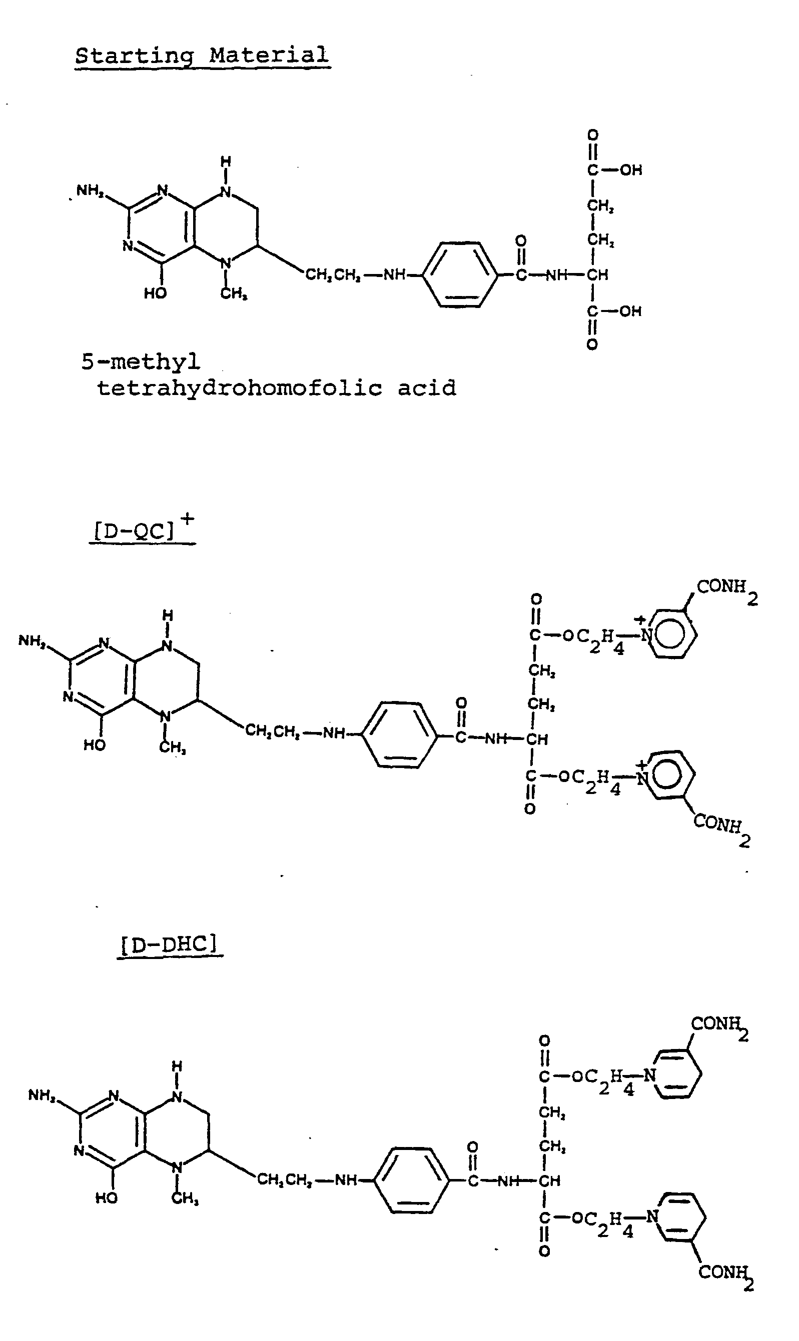

### Method 0

Method N is repeated, except that the starting alcohol employed has the formula 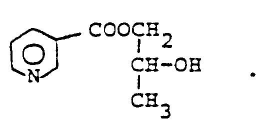 (That starting material may be prepared by reacting nicotinic acid with 1,2-propylene glycol in the presence of dicycloheylcarbodiimide.)

The representative drugs listed below may be derivatized in this manner to the corresponding [D-QC]⁺ and [D-DHC] compounds, as may the remaining drugs listed with Method N.

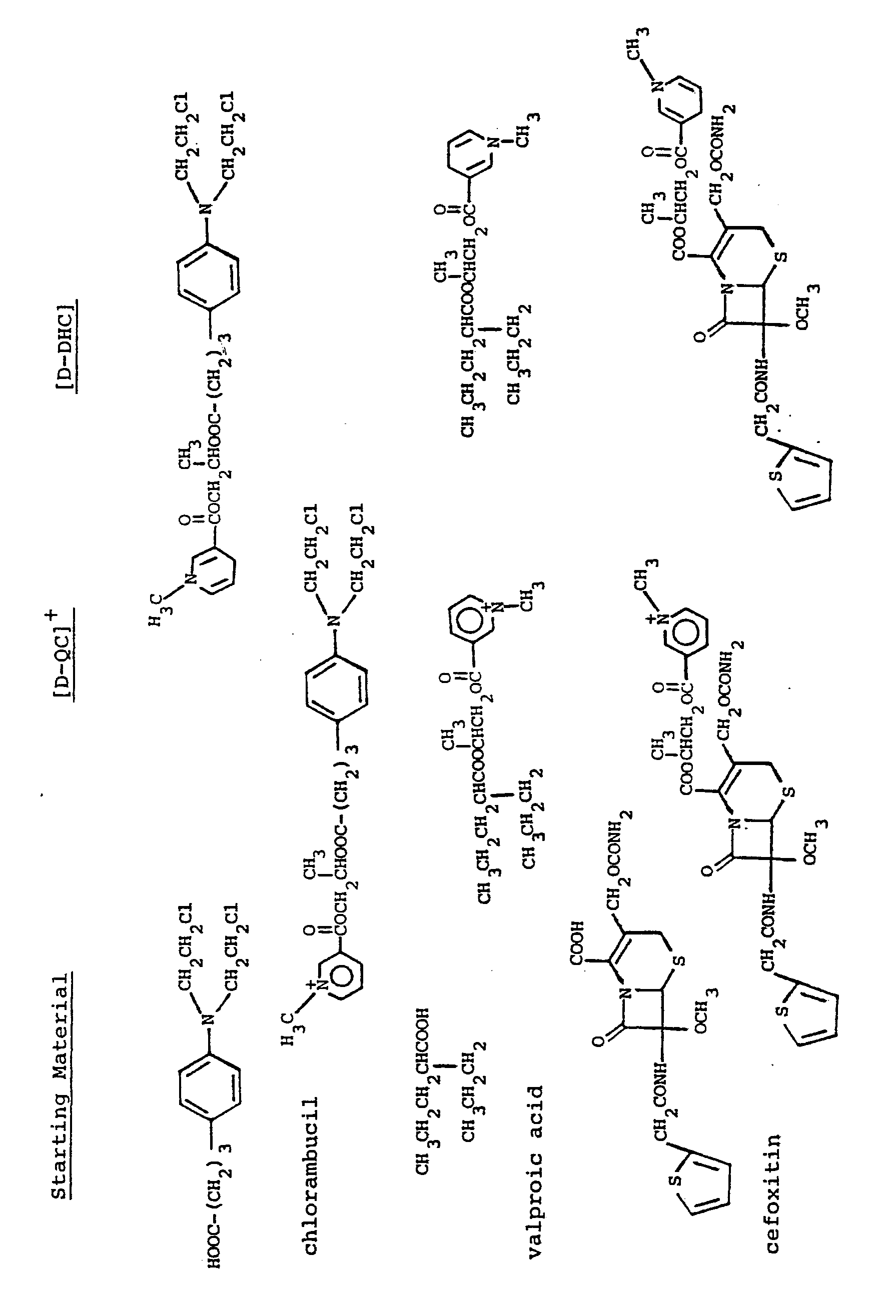

### Method P

Method N is repeated, except that the starting alcohol employed has the formula 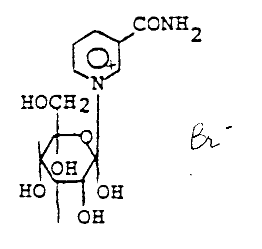 (That starting material may be prepared by reacting bromoglucose with nicotinamide.)

The drugs listed below may be derivatized in this manner to the corresponding [D-QC]⁺ and [D-DHC] compounds, as may the remaining drugs listed with Method N.

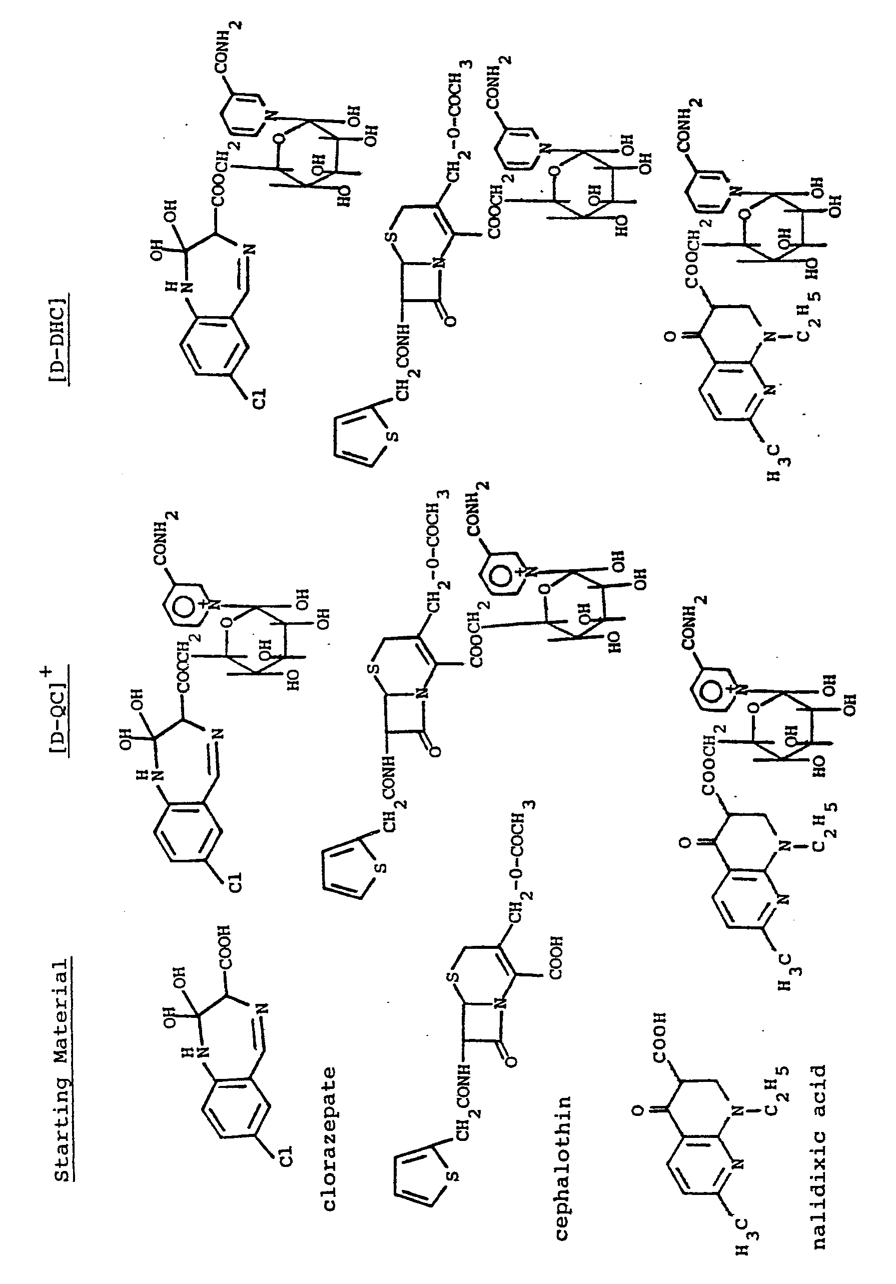

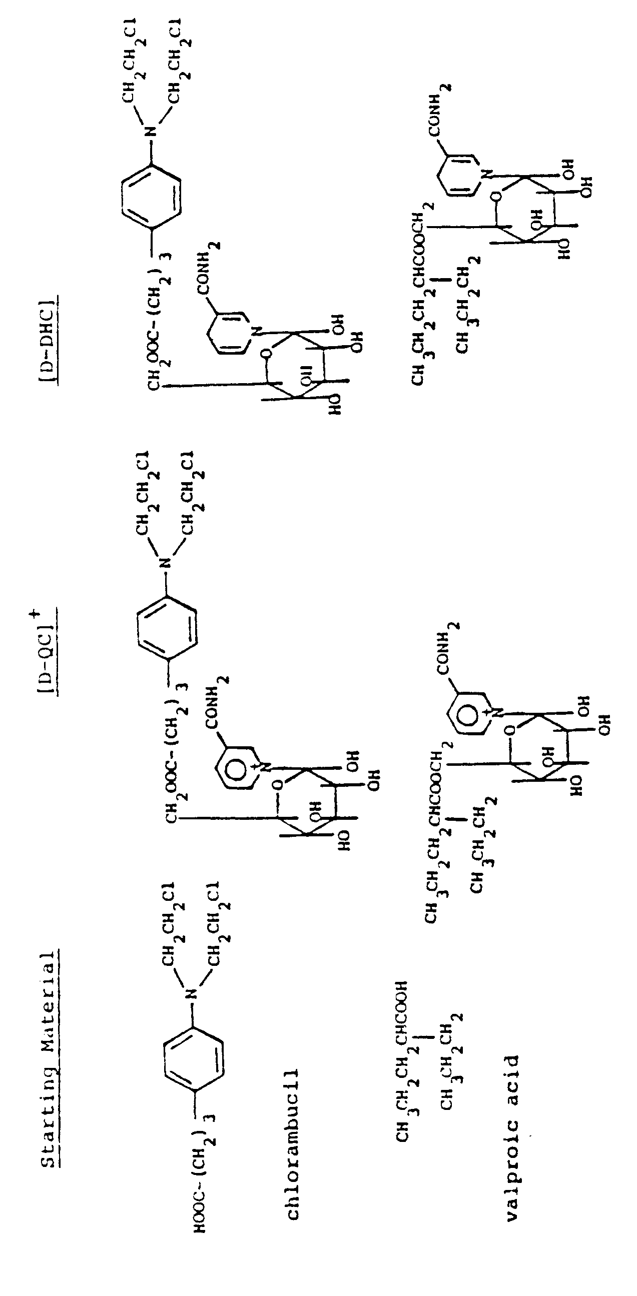

IV. Methods for Salt Formation

### Method Q

An ether solution of [D-DHC] is treated with an equivalent amount of anhydrous p-toluenesulfonic acid dissolved in dry ether. Mixing at room temperature is continued until the imminium salt precipitates out of solution. The salt is then removed by filtration.

In order to further illustrate the present invention and the advantages thereof, the following specific examples are given, it being understood that same are intended only as illustrative and in nowise limitative.

In the examples immediately to follow, all melting points were taken on a Mel-Temp apparatus and are not corrected. Elemental analyses were performed at Atlantic Microlab, Inc., Atlanta, Georgia. Infrared spectra were determined using a Beckman Acculab 1 double-beam recording spectrophotometer. NMR spectra were determined by means of a Varian T60A or FX100 spectrometer. All chemical shifts reported are in 6 units (parts per million) relatives to tetramethylsilane. Ultraviolet absorbance spectra were determined using a Cary Model 219 spectrophotometer. HPLC analysis were performed on Waters Associates Liquid chromatograph with Model 6000A solvent delivery system, Model U6K injector and Model 440 absorbance detector. And in all cases where Anal. C, H, N is indicated, the elementary analysis of the compound was found within ±0.4 of the calculated value.

### EXAMPLE 1

### Preparation of Diethyl 3,5-pyridinedicarboxylate:

To suspension of 8.35 g (0.05 mol) of 3,5-pyridinedicarboxylic acid in 30 ml of absolute ethanol, 10 ml of concentrated sulfuric acid were dropped while stirring. The mixture was then refluxed on a water bath for 5 hrs and poured onto crushed ice. The solution was then made alkaline by the addition of solid KHCO₃ in small amounts. A white solid separated which was filtered, washed with water and dried. M.p. 42-44°C. The mother liquid was extracted with CH₂CI₂ when another crop of the diester was obtained. The overall yield of the crude ester was 9.1 g (82%) of sufficient purity for the examples to follow. NMR (CDCl₃) 6 9.62 (d, 2H, J-2 Hz, C₂ and C₆ pyridine protons), 8.76 (t, 1 H, J = 2 Hz; C₄ pyridine proton), 4.43 (q, 4H, J = 7 Hz, 2 OCH₂), 1.41 (t, 6H, J=7 Hz, 2 CH₃).

### EXAMPLE 2

### Preparation of 5-Carboethoxy-3-pyridinecarboxylic acid:

To a solution of 10 g (0.045 mol) of diethyl 3,5-pyridinedicarboxylate in 75 ml of ethyl alcohol, 25 ml of 2N alcoholic KOH were added while stirring. Stirring was continued for hour at rooms temperature. To the mixture, 12.5 ml of 4N HCI were added while stirring. The solid which separated was filtered and washed with alcohol. The combined filtrate and washings were distilled on rotovap and the residue was washed with water, filtered and crystallized from ethanol. Yield 7.5 g (86%), m.p. 180-182 °C, NMR (CDCl₃/DMSO-d₆) δ 10.56 (bs, 1 H, COOH), 9.26 (d, 2H, J = 2 Hz, C₂ and C₆ pyridine protons), 8.75 (t, 1 H, J = 2 Hz, C₄ pyridine protons), 4.4 (q, 2H, J=7 Hz, O-CH₂), 1.42 (t, 3H, J=7 7 Hz, CH₃).

### EXAMPLE 3

### Preparation of 5-Carboethoxy-3-(N-β-phenethyl) carbamoylpyridine:

To 10 g (0.05 mol) of 5-carboethoxy-3-pyridinecarboxylic acid, 30 ml of thionyl chloride were added and the mixture was warmed on a water bath while stirring until clear ( ≅3 hrs). Excess thionyl chloride was distilled under vacuum. The residue was cooled to room temperature and 50 ml of sodium-dry benzene was added. The solution was cooled in an ice bath and a solution of 6.2 g (0.051 mol) of phenethylamine and 4 ml of pyridine in 50 ml of dry benzene was dropped while stirring over 1 hr and the mixture was left overnight at room temperature. The mixture was then washed with water until free from Cl⁻ (tested by AgNO₃TS). The organic layer was dried with Na₂S0₄ and distilled. The residue was crystallized from ether/pet. ether mixture. Yield 9.0 g (67%), m.p. 159-161 °C; ir (KBr) 3300 (NH), 1725 (ester CO) and 1650 cm⁻¹ (amide CO), NMR (CDCl₃) δ 9.13-9.96 (two doublets, 2H, J=2 Hz, C₂ and C₆ pyridine protons), 8.53 (t, 1 H, J=2 Hz, C₄ pyridine proton), 7.16 (s, 6H, C₆ H₅ + CONH), 4.36 (q, 2H, J = Hz, OCH₂), 3.4 (q, 2H, J=7 Hz, N-CH₂), 2.9 (5, 2H, J=7 Hz, CH₂-φ), 1.33 (t, 3H, J=7 Hz, CH₃). Anal. (C₁₇H₁₈N₂O₃) C, H, N.

### EXAMPLE 4

### Preparation of 5-Carboethoxy-1-methyl-3-(N-β-phenethyl)carbamoyl)pyridinium iodide:

To a solution of 2.9 g (0.01 mol) of 5-carboethoxy-3-(N-β-phenethyl)carbamoylpyridine in 5 ml of acetone, 3 ml of methyl iodide were added. The mixture was refluxed while stirring for 8 hrs and then left overnight. The yellow crystalline solid which precipitated was filtered, washed with acetone, dried and crystallized from acetone. Weight 3.5 g (82%), m.p.168-170_{°}C, ir (KBr) 3250 (NH), 1725 (ester CO) and 1670 cm⁻¹ (amide CO), U.V. max (buffer pH 7.4) 268 (weak plateau) and 268 nm (_{E} = 53, 667), NMR (DMSO-d₆) δ 9.53 (bs, 2H, C₂ and C₆ pyridine protons), 9.33-9.10 (m, 1H, C₄ pyridine proton), 7.16 (s, 5H, C₆H₅), 4.63-4.26 (complex multiplet, 5H, 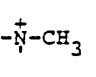 + OCH₂), 3.56 (q, 2H, J=6Hz, 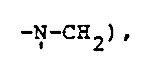 2.90 (t, 2H, J=6, CH₂-φ), 1.4 (t, 3H, J=7 Hz, CH₃). Anal. (C₁₈H₂₁IN₂O₃) C, H, N.

### EXAMPLE 5

### Preparation of 5-Carboethoxy-1-methyl-3-(N-β-phenethyl)carbamoyl-1,4-dihydropyridine:

This compound was prepared following the same general procedure as for the other dihydropyridine derivative exemplified hereinbelow using 1.0 g (0.002 mol) of 5-carboethoxy-1-methyl-3-(N-β-phenethyl)-carbamoylpyridinium iodide, 1.0 g (0.012 mol) sodium bicarbonate and 1.42 g (0.008 mol) sodium dithionite. Yield, .60 g (84%) of orange-yellow viscous oil which reduced alcoholic silver nitrate, but very slowly. U.V. max (buffer pH 7.4) 205 and 390 nm. NMR (CDCl₃) 7.33 (s, 5H, C₆Hₛ), 7.0 (s, 2H, C₂ and C₆ pyridine protons), 5.8-5.3 (hump, 1H, CONH), 4.2 (q, 2H, J=7, O-CH₂), 3.66 (q, 2H, J=7Hz, 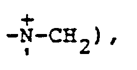 3.16 (bs, 2H, C₄ pyridine proton), 3.0 (q, 2H, J=7, CH₂-φ), 1.4 (t, 3H, J=7, CH₃).

### EXAMPLE 6

### Preparation of 3,5-Di[(N-β-phenethyl)carbamoyl]pyridine:

To a solution of 2.53 g (0.01 mol) of diethyl 3,5-pyridinedicarboxylate in 10 ml of methanol, 3.0 g (0.025 mol) of phenethylamine were added. The mixture was refluxed overnight and then distilled. The residue was washed with very dilute HCI solution and water, dried and crystallized form ethanol. Yield 2.9 g (80%), m.p. 189-190 °C. NMR (CDCl₃) δ 9.00 (d, J = 2Hz, 2H, 2,6-dipyridyl), 8.33 (5, J = 2, 1 H, 4-pyridyl), 7.30 (s, 10H, 2 C₆H₅), 6.93-6.40 (hump, 2H, 2 COHN), 3.83 (q, J=7, 4H, 2 -N-CH₂), 3.00 (t, J=7, 4H, 2 -CH₂-φ). Anal. (C₂₃H₂₃N₃O₂) C, H, N.

### EXAMPLE 7

### Preparation of 1-Methyl-3,5-di[(N-β-phenethyl)carbamoyl]pyridinium iodide:

To a solution of 2.0 g (5.3 mmol) of 3,5-di[(N-β-phenethyl)carbamoyl]pyridine in 10 ml of acetone, 2 ml of methyl iodide were added and the mixture was refluxed for 24 hrs. The yellow crystalline solid which separated was filtered, washed with acetone and dried. Weight 1.4 g (51%), m.p. 186-188°C. U.V. spectrum of a solution in phosphate buffer 7.4 showed a plateau at 275 nm, a shoulder at 225 nm and a sharp peak at 203 nm (_{E}=67,356). Ir (KBr) 3240 (NH), 1665 and 1650 cm⁻¹ (twin band, C=0). NMR (CDCL₃/D₂0) δ 9.35 (d, 2H, J=2, C₂ and C₆ pyridine protons), 8.56 (d, 1H, J=2 Hz, C₄ pyridine proton), 7.20 (s, 10H, 2 C₆H₅), 4.56 (s, 3H, 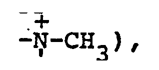 3.66 (t, 4H, J=7Hz, 2 -N-CH₂), 2.96 (t, 4H, J=7Hz, 2 CH₂-φ). Anal. (C₂₄H₂₆IN₃O₂).

### EXAMPLE 8

### Preparation of 1-Methyl-3,5-di(N-β-phenethyl)carbamoyl-1,4-dihydropyridine:

This compound was prepared following the same procedure as in Example 5, using 1 g (0.002 mol) of 1-methyl-3,5-di(N-β-phenethyl)carbamoyl pyridinium iodide, 1.0 g (0.012 mol) sodium bicarbonate and 1.4 g (0.008 mol) sodium dithionite. Yield .65 g (86%) of orange-yellow semisolid which could not be crystallized. Its alcoholic solution shows a slow reduction to alcoholic silver nitrate solution. U.V. max (buffer pH 7.4) 388 and 210 nm. NMR (CDCl₃) 7.13 (s, 5H, C₆H₅), 6.76 (s, 1H, C₂ pyridine protons), 3.51 (q, 4H, J=7Hz, 2 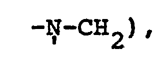 3.06-2.60 (m, 9H, O-CH₂ + C₄ pyridine proton + N-CH₃).

### EXAMPLE 9

### Preparation of N-Nicotinoyldopamine (compound 1):

To a pyridine solution containing 11.7 g (0.05 mol) dopamine hydrobromide and 6.15 g (0.05 mol) nicotinic acid at 0 °C were added 10.3 g (0.05 mol) dicyclohexylcarbodiimide (DCC). The reaction mixture was stirred at room temperature for 24 hours and the formed dicyclohexylurea was removed by filtration. The pyridine was removed in vacuo and the residue was crystallized from water at 0 °C. The product was isolated by filtration and dried over phosphorous pentoxide. Recrystallization from isopropanol gave 9.0 g (0.035 mol), 70% N-nicotinoyldopamine, m.p. 159-162 °C; aqueous solution of the compound gave a green color with Fe⁺³ and reduced AgNO; ir (KBr) 3300, 2960, 1725, 1630, 1590, 1520, 1430, 1290, 1190, 1115, 720 and 710 cm-¹; NMR (d₆-DMSO) δ 9.25-6.25 (m, 7H), 3.3 (m, 2H) and 2.65 (m, 2H) ppm. Anal. (C₁₄H₁₄N₂O₃) C, H, N.

### EXAMPLE 10

### Preparation of 3-{N-[β-(3,4-Diacetoxyphenyl)ethyl]}carbamoylpyridine:

To an ice cold suspension of 2.06 g (8 mmol) finely powdered nicotinoyldopamine in 50 ml of chloroform, 1.56 g (10 mmol) of acetylchloride were dropped while stirring. The mixture was refluxed for 3 hrs, then filtered. The filtrate was washed with water until the washing did not give test for chloride ions with AgNO₃ T.S. Chloroform was distilled on rotavap and the residue was crystallized from ether/pet. ether. Yield 2.2 g (81%) NMR (CDCl₃) 8.90 (bs, 1 H, C₂ pyridine proton), 8.56 (bd, 1 H, C₆ pyridine proton), 8.16-7.83 (m, 1 H, C₄ pyridine proton), 7.36-7.03 (m, 5H, C₆ H₃ + C₅ pyridine proton + NH), 3.60 (q, 2H, J = 7 Hz, 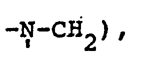 2.90 (t, 2H, J=7 Hz, -CH₂).

### EXAMPLE 11

### Preparation of 3-{N-[β-(3,4-Dipivalyloxyphenyl)-ethyl]}carbamoylpyridine (compound 8c):

To a suspension of 5.16 g (.02 mol) finely powdered nicotinoyldopamine in 100 ml chloroform, 7.23 g (.06 mol) trimethylacetyl chloride were added under stirring. The mixture was refluxed for 6 hrs and then filtered. The filtrate was washed with water free of chloride ions, then washed once with a 5% solution of NaHCO₃, then with water. The chloroform was evaporated and the residue was chromatographed by using a silica gel G column and 2% methanol in chloroform as the eluent. The first fraction was collected and evaporated and the residue was crystallized from ether/petroleum ether. Yield, 6.2 g (73%) of a white crystalline solid, m.p. 112-114°C, NMR (CDCl₃) δ 9.06 (bs, 1 H, C₂ pyridine proton), 8.73 (bd, 1 H, C₆ pyridine proton), 8.30-8.13 (m, 1 H, C₄ pyridine proton), 7.46-7.10 (m, 5H, C₆H₃ + C₅ pyridine proton + CONH), 3.66 (q, 2H, J=6.25 Hz, -N-CH₂), 3.0 (t, 2H, J=6 6 Hz, -CH₂), 1.41 (s, 18H, 2-C(CH₃)₃). Anal. Calcd for C₂₄H₃₀N₂O₅: C, 67.58; H, 7.09; N, 6.56. Found: C, 67.61; H, 7.10; N, 6.54.

### EXAMPLE 12

### Preparation of 1-Methyl-3-{N-[β-(3,4-dihydroxyphenyl) ethyl)]}carbamoylpyridinium iodide (compound 6a):

To a solution of 1.26 g (5 mmol) of nicotinoyldopamine (7) in 10 ml of acetone, 1.41 g (10 mmol) of methyl iodide were added and the mixture was refluxed under stirring for 6 hrs. The acetone was removed and the residue was crystallized from methanol/ether. Yield, 1.7 g (87%), m.p. 155-157 °C(dec). Aqueous solution gave a green color with Fe⁺³, NMR (D₂0) δ 9.30-8.28 (ms, 4H, C₅H₄), 7.00 (bs, 3H, C₆H₃), 4.60 (s, 3H, -N-CH₃), 3.80 (t, 2H, J=7 Hz, -N-CH₂), 2.93 (t, 2H, J=7 Hz, CH₂). Anal. Calcd for C₁₅H₁₇lN₂O₃·H₂O: C, 43.11; H, 4.55; N, 6.70. Found: C, 43.83; H, 4.23; N, 6.81.

### EXAMPLE 13

### Preparation of 1-Methyl-3-{N-[β-(3,4-diacetoxy- phenyl) ethyl]} carbamoyl-pyridinium iodide (compound 6b):

To a solution of 1.71 g (5 mmol) of 3-{N-[β-(3,4-diacetoxyphenyl)ethyl]}carbamoyl pyridine (prepared as compound 8c), 1.41 g (10 mmol) of methyl iodide were added and the mixture was refluxed overnight under stirring The acetone solution was then decanted from the insoluble oily residue. Ether was added to the acetone solution and the solid separated was crystallized from acetone/ether. Yield, 1.9 g (78%) of yellow crystalline needles, m.p. 171-173°C. U.V. (methanol) 215, 265 nm, NMR (D₂0) δ 8.86-7.63 (ms, 4H, C₅H₄), 6.66 (bs, 3H, C₆H₃), 4.4 (s, 3H, -N-CH₃), 3.50 (t, 2H, -N-CH₂), 3.03 (t, 2H, CH₂), 2.21 (bs, 6H, 2 COCH₃). Anal. Calcd for C₁₉H₂₁ IN₂O₅: C, 47.12; H, 4.37; N, 5.78. Found: C, 47.23; H, 4.38; N, 5.78.

### EXAMPLE 14

### Preparation of 1-Methyl-3-(N-[β-(3,4-dipivalyloxy- phenyl)ethyl]-}carbamoylpyridinium iodide (compound 6c):

To a solution of 5.0 g (11.7 mmol) of compound 8c in 20 ml of acetone, 3.3 g (23.4 mmol) of methyl iodide were added and the mixture was refluxed under stirring for 6 hrs, then cooled. The orange crystalline solid which separated was filtered, washed with ether and crystallized for acetone/ether. Yield, 5.6 g (85%), m.p. 163-165°C. U.V. (buffer pH 7.4) 270, 215 nm. NMR (DMSO-d₆) δ 7.68-7.06 (ms, 7H, C₅N₄ + C₆ H₃ + NH), 4.56 (s, 3H, -N-CH₃), 3.42 (q, 2H, J=7 Hz, -N-CH₂), 3.19 (t, 2H, J=7 Hz, CH₂), 1.32 (s, 18H,2 -C-(CH₃)₃). Anal. Calcd for C₂₅H₃₃IN₂O₅: C, 52.82; H, 5.85; N, 4.92. Found: C, 52.76; H, 5.87; N, 4.90.

### EXAMPLE 15

### Preparation of 1-Methyl-3-{N-[B-(4-hydroxy-3-methox- phenyl)ethyl])carbamoylpyridinium iodide (compound _9e):

N-nicotinoyl-3-methoxytyramine was prepared by following the procedure used for the preparation of compound 7. The isolated crude amide was quaternized directly with methyl iodide following the method used for the preparation of compound 6a. Crystallization from methanol gave a yellow crystalline compound, m.p. 192-194 °C with overall yield of 84%, calculated on the basis of 3-methoxytyramine starting material. NMR (D₂0) closely similar to that of 6a except for the singlet at 6 3.66 for OCH₃.

### EXAMPLE 16

### Preparation of 1-Methyl-3-{N-[β-(3,4-dihydroxyphenyl)-ethyl]}carbamoyl-1,4-dihydropyridine (compound 5a):

To an ice cold solution of 1.0 g (2.5 mmol) of compound 6a in 200 ml of deaerated water, 1.26 g (15 mmol) sodium bicarbonate were added. Nitrogen was bubbled into the mixture and 1.74 g (10 mmol) of sodium dithionite was added gradually to the mixture under stirring. Stirring was continued for 1 hr and the mixture was then extracted twice with 50 ml of ether. The ether extract was washed with water, dried with anhydrous Na2S04 and evaporated to dryness. Yield, 0.36 g (54%) of a yellow solid, m.p. 90-93°C (dec.) which gave a green color with ferric chloride test and reduced alcoholic AgNO₃ instantly. UV (CH₃0H) 220, 350 nm. NMR (CDCl₃/D₂O) 6 7.2-6.9 (ms, 4H, C₆H₃ + C₂ dihydropyridine proton), 5.6 (m, 1H, C₆ dihydropyridine proton), 4.6-4.4 (m, 1 H, C₅ dihydropyridine proton), 3.4 (m, 2H, -N-CH₂), 3.1-2.7 (m, 7H, N-CH₃ + C₄ dihydropyridine protons + CH₂). Anal. Calcd for C₁₅H₁₈N₂O₃·1 2H₂O: C, 63.59; H, 6.76; N, 9.88. Found: C, 63.56; H, 6.85; N, 9.72.

### EXAMPLE 17

### Preparation of 1-Methyl-3-(N-[6-(3,4-diacetoxyphenyl)-ethyl]}carbamoyl-1,4-dihydropyridine (compound _5b):

To an ice cold solution of 1.4 g (3 mmol) of compound 6b in 200 ml of deaerated water, 1.5 g (18 mmol) of sodium bicarbonate was added. A stream of N₂ was bubbled into the mixture and 2.1 g (12 mmol) of sodium dithionite were gradually added under stirring. Stirring was continued for 30 min and then the mixture was extracted with ethyl acetate. The extract was washed with water, dried with anhydrous Na₂S0₄ and evaporated to dryness. The yellowish semisolid mass remaining gave a faint green color with ferric chloride test indicating partial hydrolysis of the ester functions. It reduced alcoholic silver nitrate instantly. U.V. (CH₃0H) 220, 273 and 355 nm; NMR (CDCl₃/D₂O) 6 7.13-6.80 (ms, 4H, C₆H₃ + C₂ dihydropyridineproton), 5.53 (doublet of doublets, 1 H, C₆ dihydropyridine proton), 4.63-4.46 (m, 1 H, C₅ dihydropyridine proton), 3.33 (t, 2H, J=6.5 Hz, -N-CH₂), 3.06-2.66 (m, 7H, -N-CH₃ + C₄ dihydropyridine proton + CH₂), 1.8 (s, ≅6H, 2COCH₃).

### EXAMPLE 18

### Preparation of 1-Methyl-3-(N-[β-(3,4-dipivalyloxyphenyl)-ethyl]}carbamoyl-1,4-dihydropyridine (compound _5c):

To a cold mixture of 2.0 g (3.5 mmol) of compound 6c, 200 ml of deaerated water and 100 ml of ethyl acetate, 1.14 g (14 mmol) of sodium bicarbonate and 2.43 g (14 mmol) of sodium dithionite were added. The mixture was stirred under N₂ for 20 mins. The ethyl acetate layer was separated and the aqueous layer was re-extracted with 100 ml of ethyl acetate. The combined ethyl acetate was washed with cold deaerated water, dried over anhydrous Na₂S0₄ and distilled on rotovapor. The viscous yellow oily residue was dissolved in 5 ml of acetone, filtered under N₂ atmosphere and then evaporated under reduced pressure. The solid residue was dried under vacuum over P₂0₅in N₂ atmosphere. It reduced alcoholic AgNO₃ instantaneously and gave no color with FeCl₃ test. Yield, 1.3 g (83%) m.p. 45-48 °C, UV (CH₃OH) 210 and 355 nm; NMR (CDCl₃) 6 7.04-6.92 (m, 4H, C₆H₃ + C₂ dihydropyridine proton), 5.71-5.61 (doublet of doublets, 1H, C₆ dihydropyridine proton), 4.81 (bs, 1H, CONH), 4.60-4.51 (m, 1H, C₅ dihydropyridine proton), 3.53 (q, 2H, J=6.3 Hz, -N-CH₂), 2.36 (bs, 2H, C₄ dihydropyridine proton), 2.91 (s, 3H, N-CH₃), 2.79 (t, 2H, J=6.3 Hz, CH₂), 1.33 (s, 18H, CO-C(CH₃)₃). Anal. Calcd for C₂₅H₃₄N₂O₅·1 1 2H₂O C, 63.9; H, 7.93; N, 5.96. Found: C, 63.4; H, 7.81; N, 5.94.

### EXAMPLE 19

### Preparation of 1-Methyl-3-{N-[β-(4-hydroxy-3-methoxyphenyl)ethyl]}carbamoyl-1,4-dihydropyridine (compound 10):

This compound was prepared following the same method as for the preparation of compound 5c. The crude solid obtained showed the same NMR (CDCl₃/D₂O) pattern as compound 5a, except for a peak at 6 3.5 for the OCH₃ protons. It was sufficiently pure for the determination of its retention time following the HPLC method of analysis detailed in Example 28 below. No trials were made for its further crystallization or elemental analysis.

### EXAMPLE 20

### Preparation of N-Nicotinoyltyramine:

To an ice cold suspension of 3.69 g (0.03 mol) nicotinic acid in a solution of 5.2 g (0.03 mol) tyramine hydrochloride in 100 ml of pyridine, 6.18 g (0.03 mol) of dicyclohexylcarbodiimide (DCC) were gradually added while stirring. Stirring was continued at room temperature for 24 hrs and the formed dicyclohexylurea was removed by filtration. The pyridine was removed by distillation in vacuo and the residue was triturated with cold water, filtered and crystallized from 50% aqueous methanol. Yield, 6.25 g (86%), m.p. 179-181 °C. PMR (DMSO-d₆/D₂O) δ 9.0-8.66 (m, 2H, C₂ and C₆ pyridine protons), 8.33-8.10 (m, 1H, C₄ pyridine proton), 7.66-7.46 (m, 1H, C₅ pyridine proton), 7.23-6.70 (m, rH, C₆H₄), 3.56 (t, 2H, -NCH₂), 2.90 (t, 2H, CH₂). Anal. (C₁₄H₁₄N₂O₂) C, H, N.

### EXAMPLE 21

### Preparation of 3-{N-[β-(4-pivalyloxyphenyl)ethyl]}carbamoylpyridine:

To an ice cold suspension of 4.84 g (0.02 mol) N-nicotinyoltyramine in 100 ml chloroform, 3.6 g (0.03 mol) of trimethylacetyl chloride were dropped while stirring. The mixture was refluxed overnight and the non-reacted nicotinoyltyramine was filtered off. The filtrate was washed with water until free from chloride ions, washed once with 5% solution of NaHCO₃ and then with water. Chloroform was evaporated on rotavap and the residue was crystallized from ether/pet. ether. Yield 3.9 g (60%), m.p. 80-82 °C. PMR (CDCl₃) 6 8.66-6.93 (m, 8H, C₅H₄N + C6H4), 3.56 (q, 2H, J= 7 Hz, 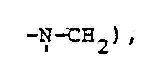 2.86 (5, 2H, J=7 Hz, CH₂), 1.33 (s, 9H, C-(CH₃)₃).

### EXAMPLE 22

### Preparation of 1-Methyl-3-{N-[β-(4-hydroxyphenyl)ethyl]}carbamoylpyridinium iodide:

To a solution of 1.21 g (5 mmol) of nicotinyoltyramine in 10 ml of acetone, 1.41 g (10 mmol) of methyl iodide were added and the mixture was refluxed while stirring for 6 hrs. The fine, yellow solid separated was filtered and crystallized from methanol ether. Yield 1.78 g (93%), m.p. 208-210 °C. PMR (DMSO-d₆/D₂O) δ 9.23-8.26 (m, 4H, C₅H₄), 7.33-6.83 (m, 4H, C₆H₄), 4.50 (s, 3H, 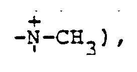
3.70 (t, J = 7 Hz, 2H, 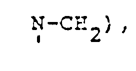
2.93 (t, J=7 Hz, 2H, CH₂).

### EXAMPLE 23

### Preparation of 1-Methyl-3-{N-[β-(4-pivalyloxyphenyl)ethyl]}carbamoylpyridinium iodide:

To a solution of 1.63 g (5 mmol) of the product of Example 21 in 10 ml of acetone, 1.41 g (10 mmol) methyl iodide were added and the mixture was refluxed overnight while stirring. The acetone layer was separated by decantation and the yellowish, oily residue was crystallized from methanol/ether. Yield, 1.94 g (83%), m.p. 155-157°C PMR (D₂0) δ 9.16-8.00 (m, 4H, C₅H₄), 7.33-6.83 (m, 4H, C6H4),4.40 (s, 3H, N-CH₃), 3.5 (t, 2H, J=7 Hz, 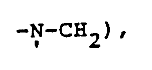 2.90 (t, 2H, J=7 Hz, CH₂), 1.30 (s, 9H, C-(CH₃)₃). Anal. (C₂₀H₂₅N₂O₃l) C, H, N.

### EXAMPLE 24

### Preparation of 1-Methyl-3-{N-[β-(4-hydroxyphenyl)ethyl]}carbamoyl-1,4-dihydropyridine:

To an ice cold solution of 1.15 g (3 mmol) of the product of Example 22 in 200 ml of deaerated water, 1.5 g (18 mmol) sodium bicarbonate were added. While the mixture was bubbled with N₂ gas, 2.09 g (12 mmol) of sodium dithionite were gradually added to the mixture. The mixture was stirred under N₂ for 1 hr and then extracted twice, each with 100 ml of ethyl acetate. The combined extract was washed with water, dried over anhydrous Na₂S0₄ and distilled on rotovap. Yield, 0.38 g (50%) of yellowish semisolid which reduced alcoholic AgNO₃TS instantaneously. (PMR as expected.)

### EXAMPLE 25

### Preparation of 1-Methyl-3-{N-[β-4-pivalyloxyphenyl)}carbamoyl-1,4-dihydropyridine:

To an ice cold mixture of 2.34 g (5 mmol) of the product of Example 23, 200 ml of deaerated water and 100 ml of ethyl acetate, 1.63 g (20 mmol) sodium bicarbonate and 3.47 g (20 mmol) sodium dithionite were added while stirring the mixture. Stirring was continued under N₂ gas for 30 min. The ethyl acetate layer was separated and the aqueous layer was extracted with 100 ml of ethyl acetate. The combined ethyl acetate extract was washed with 100 ml cold deaerated water, dried over anhydrous Na₂S0₄ and evaporated on rotavap. The viscous, yellow residue was dissolved in 5 ml of acetone, filtered under N₂ has through folded filter paper and distilled on rotavap. The solid residue was dried under vacuo over P₂0₅ in N₂ atmosphere. It reduced alcoholic AgN0₃ instantaneously. Yield, 1.06 g (62%). (PMR as expected.)

### EXAMPLE 26

### (i) Oxidation by Hydrogen Peroxide:

To 10 ml of 30% H₂0₂ was added 0.2 g of the dihydropyridine derivative (products of Examples 5 or 8). The mixture was stirred and samples were taken to check the UV spectrum. Complete oxidation to the corresponding quaternary salts was observed.

### (ii) Oxidation by Silver Nitrate:

To 5 ml of saturated methanolic AgN0₃ solution was added 1 ml of 5% methanolic solution of the dihydropyridine derivative. The mixture was shaken and left for 5 min for complete precipitation of silver, centrifuged and an aliquot was taken to check the UV spectrum. Complete oxidation to the quaternary salts was observed.

### (iii) Calibration Curves

UV study of the compounds prepared in Examples 4, 5, 7 and 8 revealed that they obey Beer's Law with good coefficients and at a wide range of dilution levels. The study was done at 350 nm for the dihydro derivatives and at 262 and 220 nm for all the quaternary and dihydro.

### EXAMPLE 27

### Kinetics of Oxidation of the Dihydro Derivatives:

In Plasma: 0.2 ml of (6.25 x 10-⁴ M) freshly prepared solution of the dihydro derivative in methyl alcohol was diluted to 10 ml with 20% plasma (diluted with phosphate buffer pH 7.4). The solution was kept at 37°C and UV spectrum was scanned from 400 nm to 300 nm every 10 min for 2 hrs against reference made by dilution of 0.2 ml methyl alcohol with 20% plasma to 10 ml.

In Whole Blood: In each of 5 tubes, 0.1 ml of 10 x 10-⁴ M methanolic solution of the freshly prepared dihydro derivative, was added 2 ml of fresh heparinized whole human blood and the tubes were kept at 37°C in a water bath. At the end of the time period to be investigated, 8 ml of acetonitrile was added, shaken vigorously and centrifuged. The extension of the supernatant solution at 350 nm was measured. A reference sample was made by addition of 0.1 ml of methyl alcohol instead of the sample solution following the same procedure.

In Brain Homogenate: 2.0 g of rat brain tissue were homogenized in 10 ml of phosphate buffer, pH 7.4. The homogenate was centrifuged for 15 min at 3000 rpm, decanted, heated in a water bath at 50°C for 5 min and then centrifuged again. The supernatant solution was diluted to 100 ml with phosphate buffer, pH 7.4.

Reference Sample: 0.2 ml of methyl alcohol was diluted to 10 ml with the brain homogenate solution, and the solution was used to record the base line on Cary 219 spectrophotometer and as a reference for the dihydro derivative sample solution.

Dihydro Derivative Sample Solutions: 0.2 ml of 6.25 x 10-⁴ M methanolic solution of the freshly prepared dihydro derivative was diluted to 10 ml with the brain homogenate solution. The mixture was scanned at 37 _{°} C from 400 nm to 300 nm every 10 min for 2 hrs on Cary 210 spectrophotometer.

### In Liver Homogenate:

Liver Homogenate Solution: 5.0 g of rat liver tissue were homogenized in 50 ml of phosphate buffer, pH 7.4. The homogenate was centrifuged, decanted, heated in a water bath at 50°C for 5 min and then centrifuged again. The supernatant homogenate was diluted to 250 ml with phosphate buffer, pH 7.4.

Reference Sample: 0.2 ml of methyl alcohol was diluted to 10 ml with the liver homogenate solution and the solution was used to record the base line on a Cary 219 spectrophotometer and as a reference for the dihydro derivative sample solution.

Dihydro Derivative Sample Solution: 0.2 ml of 6.25 x 10-⁴ M solution of the freshly prepared dihydro derivative in methyl alcohol was diluted to 10 ml with liver homogenate solution. The mixture was scanned at 37 _{°} C from 400 nm to 300 nm every 5 min for 1 hr.

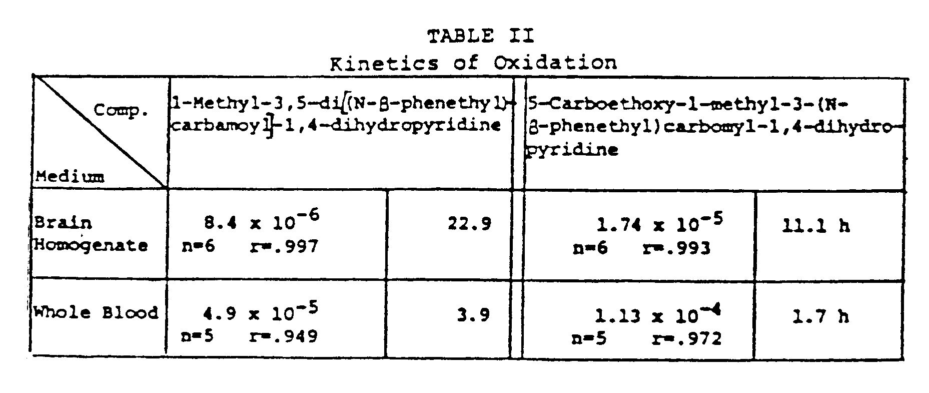

### Studies of the Dopamine Derivatives

### EXAMPLE 28

### Analytical Methods:

A high pressure liquid chromatography (HPLC) method was developed for the studies of the degradation of the dihydropyridine dopamine derivative. The chromatographic analysis was performed on a component system consisting of a Waters Associate Model 6000A solvent delivery system, Model U6K injector and Model 440 dual channel absorbance detector operated at 254 and 280 nm. A 30 cm x 3.9 mm (internal diameter) reverse phase µBondapak C₁₈ column (Waters Associates), operated at ambient temperature, was used for all separations. The mobile phase used for the separation of the dihydropyridine derivative, its degradation products and oxidation products consisted of 0.005 M solution of 1-heptanesul- fonic acid sodium salt (PIC B-7 Eastman Kodak) in CH₃CN; 0.01 M aqueous dibasic ammonium phosphate (2.5:1). A peak was always shown at a retention time of 2.2 min which is believed to be a monodeacylated dihydropyridine derivative, since it eventually did result in 6a.

### EXAMPLE 29

### Determination of the Enzymatic Hydrolytic Cleavage and Rate of Oxidation of Compound 5c:

### In Human Plasma:

The freshly collected plasma used was obtained at the Civitan Regional Blood Center, Inc. (Gainesville, Florida) and contained about 80% plasma diluted with anticoagulant citrate phosphate dextrose solution U.S.P. The plasma was stored in a refrigerator and used the next day. One hundred µl of a freshly prepared 0.61 M solution of compound 5c in methanol was added to 20 ml of plasma, previously equilibrated to 37 ° C in a water bath and mixed thoroughly to result in an initial concentration of 3.05 x 10-³ moles/liter. One ml samples of plasma were withdrawn from the test medium, added immediately to 5 ml of ice cold acetonitrile, shaken vigorously and placed in a freezer. When all samples had been collected, they were centrifuged and the supernatants were filtered through Whatman 1 filter papers and analyzed to HPLC.

### In Human Blood:

The freshly collected heparinized blood was obtained at the Civitan Regional Blood Center, Inc. (Gainesville, Florida). The blood was stored in a refrigerator and used the next day. One hundred µl of a freshly prepared 0.19 solution of compound 5c in methanol was added to 20 ml of blood, previously equilibrated to 37 ° C in a water bath and mixed thoroughly to result in an initial concentration of 9 x 10-⁴ moles/liter. One ml samples of blood were withdrawn from the test medium every 5 minutes, added immediately to 5 ml of ice cold acetonitrile, shaken vigorously and placed in a freezer. When all samples had been collected, they were centrifuged and the supernatants were filtered using Whatman 4 filter paper and analyzed by HPLC.

### In Rat Brain Homogenate:

The brain homogenate was prepared by the following method. Five Sprague-Dawley rats were killed by decapitation and the brains were removed, weighed (total weight 9.85 g) and homogenized in 49.3 ml of aqueous 0.11 M phosphate buffer, pH 7.4. The homogenate was centrifuged and the supernatant was used for the test. 100 µl of 0.18 M solution of compound 5c was mixed with 10 ml of homogenate, previously equilibrated to 37 ° C in a water bath, to result in an initial concentration of 1.8 x 10-³ moles/liter. Samples of 1.0 ml were withdrawn every 10 minutes from the test medium, added immediately to 5 ml of ice cold acetonitrile and placed in a freezer. When all samples had been collected, they were centrifuged. Each supernatant was filtered through two Whatman 1 filter papers and analyzed by HPLC.

### In Rat Liver Homogenate:

The liver homogenate was prepared by the following method. Three Sprague-Dawley rats were killed by decapitation and the livers were removed, weighed and homogenized by tissue homogenizer in 0.11 M aqueous phosphate buffer, pH 7.4, to make 20% liver homogenate. The homogenate was centrifuged and the supernatant was used for the test. 100 µl of 0.1 M solution of compound 5c in methanol were mixed with 20 ml of the homogenate, previously equilibrated to 37_{°}C in a water bath, to result in an initial concentration of 9 x 10-⁴ moles/liter. Samples of 1.0 ml were withdrawn every 5 minutes from the test medium, added immediately to 5 ml of ice cold acetonitrile, shaken vigorously and placed in a freezer. When all samples had been collected, they were centrifuged and each supernatant was filtered through Whatman 1 filter paper and analyzed by HPLC.

Rates of disappearance (overall oxidation and degradation) of compound 5c:

(i) in Plasma: 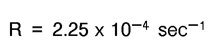 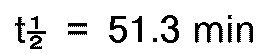 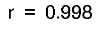 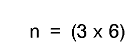
(ii) In 20% Brain Homogenate: 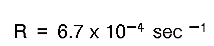 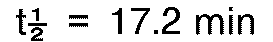 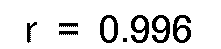 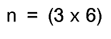
(iii) In Blood:

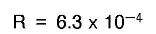

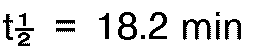

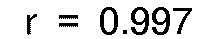

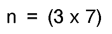

(iv) In Liver:

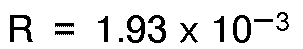

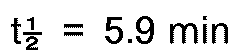

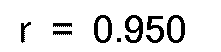

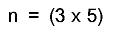

### EXAMPLE 30

### Determination of Concentration of Comcound 6a in Brain and Blood after Parenteral Administration of 5c:

Male Sprague-Dawley rats of average weight of 150 ± 10g were used. The rats were anesthetized with IM injection of Inovar and the jugular was exposed. Compound 5c was injected intrajugularly in the form of 10% solution in DMSO at a dose of 64.2 mg/kg (equivalent to 50 mg/kg compound 6a). The injection was given at a rate of 24 µl/min using a calibrated infusion pump. After appropriate time periods, 1 ml of blood was withdrawn from the heart and dropped immediately into a tared tube containing 3 ml acetonitrile, which was afterwards weighed to determine the weight of the blood taken. The animal was then perfused with 20 ml of saline solution, decapitated and the brain was removed. The weighed brain was homogenized with 0.5 ml of distilled water, 3 ml of acetonitrile was added and the mixture was rehomogenized thoroughly, centrifuged, filtered and then analyzed for compound 6a using the HPLC method. The tubes containing the blood were shaken vigorously, centrifuged, decanted and also analyzed for compound 6a using the HPLC method. Quantitation was done by using a recovery standard curve obtained by introducing a known amount of 6a in either brain homogenate or blood and then treated in the same manner. See FIGURE 6 and the discussion thereof hereinabove.

### EXAMPLE 31

### Pharmacological studies: In vivo effect on pituitary prolactin secretion:

Adult male rats (Charles Rivers, CD-1) weighing 200 to 225 g were provided food and water ad libitum for at least one week period to experimentation. To elevate serum prolactin levels, each rat received a single s.c. implant of a Silastic tube (1.57 mm interior diameter, 5 mm x 3.15 mm overall size) packed with crystalline 17-,8-estradiol. Two days later the rats were lightly anesthetized with ether and a small incision was made over the right jugular vein for intravenous (LV.) administration of the test drugs. Compound 6a was injected at a dose of 1 mg/kg body weight/ml saline and groups of six rats were decapitated at 15, 30, 60 and 120 min later to collect blood samples. Control rats (time 0) received an I.V. injection of the saline vehicle and were decapitated 30 min later. Compound 5c was dissolved in 10% ethanol in saline and was injected IV. Rats were decapitated at 15, 30 and 120 min later. Control (time 0) animals received the 10% ethanol vehicle and were sampled 30 min later.

Trunk blood was collected, allowed to clot for 2h and the serum was separated and stored at -20 _{°} C for subsequent assay for prolactin concentrations. Each serum sample was assayed in duplicate by the doubleantibody radioimmunoassay procedure described by the National Pituitary Agency Hormone Distribution Program. Serum prolactin concentrations are expressed in terms of the PRL-RP-2 reference preparation provided. The intraassay coefficient of variation for 10 replicate samples of pooled serum obtained from male rats was 13.8%.

The effects of compounds 5c and 6a on serum prolactin concentrations were evaluated by one-way analysis of variance and Student-Newman Keuls tests. A probability level of less than 0.05 was selected for significance. See FIGURE 7 and the discussion thereof hereinabove.

### In vitro evaluation of the prolactin inhibitory effect of 6a:

Adult female rats (Charles Rivers Lab.) weighing 225-250 g were maintained on food and water ad libitum. Animals were sacrificed by decapitation; their pituitary glands were quickly removed from the cranium. The anterior pituitary (AP) of each animal was dissected into two equal halves and placed into incubation media. (Gibco's Minimal Essential Media supplied by Grand Island Biological Co. was used.) The incubation was conducted at 37 °C, under continuous aeration (95% 0₂, 5% C0₂); the pH was 7.2. After one hour of preincubation, the media were discarded and replaced with fresh ones containing either DA ( 2 x 10-⁸ M), 6a ( 2 x 10-⁸) or ascorbic acid (10-⁴ M). In all cases, one-half of AP received the test drug; the other, the ascorbate control. After one hour, samples were taken from the media and the remaining media were discarded. Fresh media containing DA ( 2 x 10-⁷), 6a (2 x 10-⁷) and ascorbate, respectively, were then added. One hour later, the second samples were taken After the 3 h incubation period, each half AP's were weighed.

The samples were diluted 1:50 with phosphate buffered saline and then assayed in triplicate by the radioimmunoassay method described. The data are given as ng prolactin released/mg wet weight/h. Paired Student's T-test was used to evaluate the significance of the inhibitory effects of the test drugs on prolactin secretion The control AP half and the drug treated half were employed in each paired comparison. See TABLE I and the discussion thereof hereinabove.

In the examples immediately to follow, all melting points were taken on a Mel-Temp apparatus and are not corrected. Elemental analyses were performed at Atlantic Microlabs, Inc., Atlanta, Georgia. Infrared spectra were determined by means of a Beckman Acculab 1 double-beam recording spectrophotometer. NMR spectra were determined by means of a Varian T60A spectrometer. All chemical shifts reported are in 6 (parts per million) relative to tetramethylsilane. Ultraviolet absorbance spectra were determined using a Carey Model 219 spectrophotometer. HPLC analyses were performed on a Waters Associates Liquid chromatograph with Model 6000A solvent delivery system, Model U6K injector and Model 440 absorbance detector.

### EXAMPLE 32

### Preparation of the 1-Methyl-3-carbamoylpyridinium derivative of Tyr-Gly-Gly-Phe-Leu-OC₂H₅ (1-Methyl-3-carbamoylpyridinium derivative of leu⁵-enkephalin ethyl ester):

N-a-t-Butoxycarbonyl-O-benzyl-L-tyrosine (7 g, 0.019 mol) was dissolved in tetrahydrofuran in a three- neck round bottom flask which was cooled to approximately -10°C in an ice/acetone bath under a nitrogen atmosphere. N-methylmorpholine (6.3 ml, 0.06 mol) was added to the stirred solution, followed by 2.5 ml (0.019 mol) of isobutyl chloroformate. Immediately after the addition of isobutyl chloroformate, N-methyl morpholine hydrochloride precipitated. After 5 min, 3.7 g (0.019 mol) of L-leucine ethyl ester hydrochloride, dissolved in dimethylformamide, were added. The reaction mixture was stirred at this temperature for an hour, after which the solvent was evaporated. The residue obtained was dissolved in ethyl acetate/water and the organic layer was extracted with sodium bicarbonate solution, water, .01 N HCI and water. The organic layer was dried over Na₂S0₄ and after evaporation of the solvent an oil was obtained. Crystallization from CHCI₃/petroleum ether yielded 7.4 g (0.014 mol, 76%) m.p. 104-107°C, of N-a-t-butoxycarbonyl-O-benzyl-L-tyrosylglycylglycine ethyl ester. ¹H NMR (CDCl₃) 6 7.2 (5H, s), 6.9 (4H, doublet of doublets), 5.0 (2H, s), 1.1 (12H, m). The ethyl ester was cleaved by treating 6.2 g (0.012 mol) of it with an equivalent amount of 2N NaOH in methanol. The solution was stirred at room temperature for half an hour after which the solvent was evaporated. An equivalent amount of 2N HCI was added to the cooled residue and the solid obtained was filtered and dried to yield 3.5 g (96%), m.p. 118-122_{°}C, of the free (t-butoxycarbonyl-O-benzyl)-tyrosylglycylglycine. t-Butoxycarbonylphenylalanylleucine ethyl ester was prepared starring with 6g (0.019 mol) of t-butoxycarbonyl-L-phenylalanine, and 3.7g (0.019 mol) of leucine ethyl ester hydrochloride. Work up and crystallization from CHCI₃/petroleum ether yielded 6.5 g (84%), m.p. 109-112°C, of the desired compound. ¹H NMR (CDCl₃) 6 7.2 (5H, s), 6.4 (1H, bm), 5.1 (1H, bm), 4.3 (4H, bm), 3.1 (2H, bm), 1.3 (20H, m).

The t-butoxycarbonyl protecting group was cleaved by treatment of 4.9 g (0.012 mol) of t-butoxycarbonylphenylalanylleucine ethyl ester with 60 ml of 33% trifluoroacetic acid/CH₂CI₂. The solution was stirred at room temperature for half an hour, after which the solvent was evaporated and the residue was treated with a bicarbonate solution which resulted in the formation of a solid. The solid, phenylalanylleucine ethyl ester, was filtered, rinsed with water and dried to yield 5.6 g (97%), m.p. 150-154°C.

t-Butoxycarbonyl-O-benzyltyrosylglycylglycylphenylalanylleucine ethyl ester was prepared by the same method using 0.01 mol of starting materials, (t-butoxycarbonyl-O-benzyl)tyrosylglycylglycine and phenylalanylleucine ethyl ester. A white solid was obtained which was recrystallized from methyl al- cohol/water to yield 4.9 g (63%), m.p. 149-152°C.

The t-butoxycarbonyl group of t-butoxycarbonyl-O-benzyltyrosylglycylglycylphenylalanylleucine ethyl ester was cleaved as previously described to give O-benzyl-tyr-gly-gly-phe-leu-OEt ·TFA (trifluoroacetic acid) salt. Anal. calc. of C₃₉H₄₈O₉N₅F₃·H20: C, 58.13; H, 6.25; N, 8.69. Found: C, 58.06; H, 6.26; N, 8.69).

Nicotinic acid (160 mg, 1.3 mmole) and O-benzyl-tyr-gly-gly-phe-leu-OEt ·TFA salt (1g, 1.3 mmole) were dissolved in pyridine and 268 mg (1.3 mmole) of dicyclohexylcarbodiimide were added. The mixture was stirred at room temperature for 24 hrs, after which the dicyclohexylurea was filtered and the pyridine distilled in vacuo. Water was added to the residue and the solid obtained was filtered and washed with more water. The solid N-nicotinoyl-O-benzylpentapeptide ethyl ester was recrystallized by methanol/water. ¹H NMR gave the expected pattern.

The N-nicotinoyl pentapeptide derivative (500 mg, 0.64 mmol) obtained above was dissolved in 10% formic acid/methanol, followed by addition of 500 mg of palladium black. The mixture was stirred overnight at room temperature, after which the solvent was evaporated. The residue was neutralized with a saturated NaHCO₃ solution and extracted with ethyl acetate The solvent was evaporated and the residue recrystallized from ethyl acetate/ethyl ether to yield 370 mg (0.54 mmol), 84% of product. ¹H NMR gave the expected pattern, corresponding to 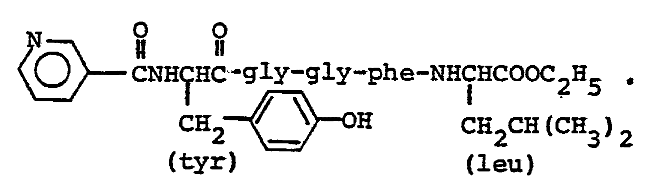 Anal. calc. for C₃₆H₄₄O₈N₆·4H20: C, 56.83; H, 6.89; N, 11.04. Found: C, 56.88; H, 6.56; N, 10.48. That product (30 mg, 0.44 mmol) was dissolved in acetone and an excess of methyl iodide was added. The solution was refluxed for 8 hrs, after which the solvent was evaporated and the residue was filtered from ethyl ether. A yellowish (260 mg, 0.31 mm), 71%, product was obtained, corresponding to the 1-methyl-3-carbamoylpyridinium derivative of leu^{s-}enkephalin ethyl ester. Anal. calc. for C₃₇H₄₇O₈N₆I: C, 53.50; H, 5.70; N, 10.12. Found: C, 53.44; H, 4.77; N, 10.07.

### EXAMPLE 33

### Preparation of N-[2-(3-Indolyl)ethyl]nicotinamide:

To a solution of 1.97 g (10 mmol) of tryptamine hydrochloride and 1.23 g (10 mmol) of nicotinic acid in 10 ml of dry pyridine at 0 °C were added 2.20 g (10.7 mmol) of dicyclohexylcarbodiimide. The reaction mixture was stirred at room temperature for 24 hrs, and the formed dicyclohexylurea was removed by filtration (2.34 g). The pyridine was removed in vacuo, and 10 ml of methanol were added to the residue. Insoluble dicyclohexylurea in methanol was removed by filtration (0.05 g). The methanol was removed in vacuo and 10 ml of methylene chloride was added to the residue. Insoluble compound in methylene chloride was removed by filtration (0.04 g). The methylene chloride was removed in vacuo and the residue was crystallized from isopropanol. Recrystallization from methanol/isopropanol gave 1.92 g (72.5%) of N-[2-(3-indolyl)ethyl]nicotinamide as pale brown plates, m.p. 150-152 _{°} C. IR (KBr) 3280, 3050, 2940, 1646, 1526, 1412, 1302, 1102, 740, 697 cm⁻¹. Anal. calc. for C₁₆H₁₅N₃O: C, 72.42; H, 5.91; N, 15.84. Found: C, 72.51; H, 5.74; N, 15.77.

### EXAMPLE 34

### Preparation of 1-Methyl-3-{[N-2-(3-indolyl)ethyl}carbamoylpyridinium iodide:

To a solution of 1.06 g (4 mmol) of N-[2-(3-indolyl)ethyl]nicotinamide in 5 ml of methanol, 1 ml (16 mmol) of methyl iodide was added. The mixture was refluxed for 5 hrs. The methanol and excess methyl iodide were removed in vacuo. The residue was recrystallized from methanol/isopropanol to yield 1.42 g (87.4%) of 1-methyl-3{[N-2-(3-indolyl)ethyl]}carbamoyl pyridinium iodide as yellow needles, m.p. 215-217°C. IR (KBr): 3280, 3000, 2940, 1660, 1540, 1500, 1316, 1220, 735 cm⁻¹. Anal. calc. for C₁₇H₁₈N₃OI: C, 50.13; H, 4.46; N, 10.32; I, 31.16. Found: C, 50.22; H, 4.49; N, 10.27; I, 31.06.

### EXAMPLE 35

### Preparation of 1-Methyl-3-{N[2-(3-indolyl)ethyl]}carbamoyl-1,4-dihydropyridine:

To a solution of 0.61 g (1.5 mmol) of 1-methyl-3{[N-2-(3-indolyl)ethyl]lcarbamoylpyridinium iodide in 50 ml of deaerated water and 50 ml of ethyl acetate, 1.00 g (12 mmol) of sodium bicarbonate was added. The mixture was stirred in an ice bath and 1.65 g (8 mmol) of sodium dithionite was added gradually under nitrogen. The mixture was stirred for 6 hrs, the ethyl acetate layer was decanted and the water layer was extracted with ethyl acetate. The combined solution was washed with water, dried with anhydrous sodium sulfate and the solvent removed in vacuo. A yield of 1-methyl-3{N[2-(3-indolyl)ethyl]carbamoyl-1,4-dihydropyridine of 0.29 g (69%) was obtained as a yellow semisolid, m.p. 40-70 _{°} C. IR (KBr) 3250, 2900, 1670 cm-¹. Anal. calc. for C₁₇H₁₉N₃O·1 2H₂O C, 70.32; H, 6.94; N, 14.47. Found: C, 70.47; H, 6.76; N, 14.52.

### EXAMPLE 36

### Preparation of 5-Benzyloxygramine:

A solution of 8.90 g (0.04 mol) of 5-benzyloxyindole in 40 ml of dioxane was added dropwise, over the course of 30 mins, to an ice-cooled, stirred mixture of 40 ml of dioxane, 40 ml of acetic acid, 3.2 ml of 37% aqueous formaldehyde (0.04 mol) and 8.8 ml of 25% aqueous dimethylamine (0.05 mol). The solution was stirred and cooled for two hrs and then allowed to warm to room temperatue over night. The next day, 500 ml of water were added, and the turbid mixture which resulted was filtered after the addition of charcoal. The filtrate was made alkaline (to pH 8-9) with 400 ml of 10% sodium hydroxide solution. The gramine quickly solidified and was filtered off after cooling in the refrigerator overnight. Washing with water, and drying gave 9.20 g (82.0%) of coarse powder, m.p. 125-128°C. Recrystallization from ethyl acetate gave slightly green glittering cubes, m.p. 136-137 _{°} C, of the desired 5-benzyloxygramine. IR (KBr) 3110, 3020, 2920, 2840, 2800, 2755, 1610, 1575, 1470, 1455, 1480, 1210, 1190, 1000 and 780 cm⁻¹.

### EXAMPLE 37

### Preparation of 5-Benzyloxyindole-3-acetamide:

A solution of 8.41 g (0.03 mol) of 5-benzyloxygramine, 7.5 g (0.15 mol) of sodium cyanide, 120 ml of ethanol and 30 ml of water was refluxed for 90 hours. The solution, which contained some precipitate, was diluted with 200 ml of water and cooled in the refrigerator. The crystalline material which separated was washed thoroughly with water and dried, giving 4.40 g (52.3%) of a slightly brown sticky tan powder,m.p. 137-140 °C. Recrystallization from methanol/benzene gave small needles, m.p. 156-158 °C, of 5-benzyloxyindole-3-acetamide. IR (KB) 3400, 3290, 3180, 1645, 1610, 1580, 1485, 1450, 1275, 1210, 1200 and 795 cm-1.

### EXAMPLE 38

### Preparation of 5-Benzyloxytryptamine hydrochloride:

4.21 g (0.015 mol) of 5-benzyloxyindole-3-acetamide which were dissolved in 200 ml of tetrahydrofuran were added gradually to a solution of 3.80 g (0.1 mol) of lithium aluminum hydride in 200 ml of ether over a 30 minute period and under a nitrogen atmosphere. The solution was refluxed for 24 hrs. The excess hydride was decomposed with ethanol and then water was added to ensure complete decomposition of the precipitated complex. The ether layer was decanted and the residue was washed with fresh ether. The combined solution was washed with water and dried over solid potassium hydroxide. The solvent was evaporated in vacuo and the oily residue was taken up in ether and precipitated with hydrogen chloride gas. The pale purple 5-benzyloxytryptamine hydrochloride gas recrystallized from ethanol/ether, yield 3.00 g (66.0%), m.p. 263-265 °C. IR (KBr) 3290, 3010, 2910, 1600, 1580, 1480, 1200, 1100 and 1000 cm-¹.

### EXAMPLE 39

### Preparation of N-{2-[3-(5-benzyloxy)indolyl]ethyl)nicotinamide:

To a solution of 303 mg (1 mmol) of 5-benzyloxytryptamine hydrochloride and 123 mg (1 mmol) of nicotinic acid in 5 ml of pyridine at 0 _{°} C was added 220 mg (1.07 mmol) of dicyclohexylcarbodiimide. The reaction mixture was stirred at room temperature for 24 hrs and the formed dicyclohexylurea was removed by filtration. The pyridine was removed in vacuo, and the residue was recrystallized from methanol/isopropanol. Yield 218 mg (58.9%), m.p. 192-194 °C of N-{2-[3-(5-benzyloxy)indolyl]ethyl}nicotinamide. IR (KBr) 3280, 3050, 2900, 1655, 1590, 1535, 1480, 1310, 1220, 1200, 1185, 1020, and 710 cm⁻¹.

### EXAMPLE 40

### Preparation of 1-Methyl-3-N-{2-[3-(5-benzyloxy)indolyl]ethyl}carbamoylpyridinium iodide:

To a solution of 185 mg (0.5 mmol) of N-{2-[3-(5-benzyloxy)indolyl]ethyl}nicotinamide in 2 ml of methanol there was added 0.2 ml (3.2 mmol) of methyl iodide. The mixture was refluxed for 3 hrs. The methanol and excess methyl iodide were removed in vacuo. The residue of yellow solid gradually turned purplish. Yield 128 mg (50.0%), m.p. 228-230 °C, IR (KBr) 3210, 3020, 1670, 1495, 1480, 1190, 1025, 1000, 770 cm-¹. The product has the formula

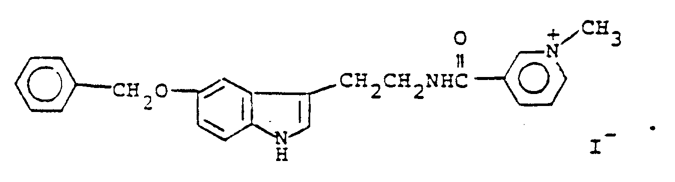

Catatylic hydrogenolysis, using palladium-on-charcoal catalyst, of 1-methyl-3-N-{2-[3-(5-benzyloxy)-indol]ethyllcarbamoylpyridinium iodide affords 1-methyl-3-N-{2-[3-(5-hydroxy)indolyl]-ethyllcarbamoylpyridinium iodide. Subsequent esterification with trimethylacetyl chloride affords the corresponding pivalyl ester of the formula 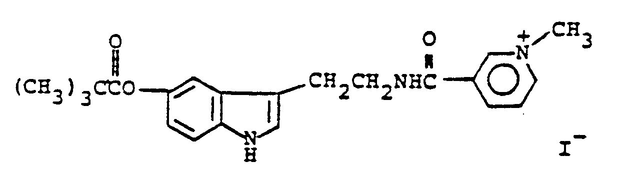 which can then be reduced as described hereinabove to the corresponding dihydro derivative of the formula

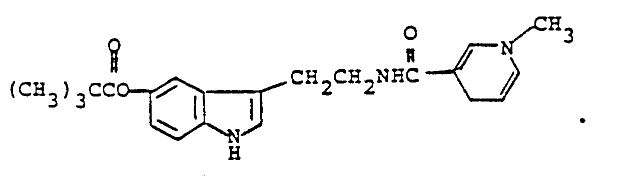

Accordingly, provided hereby are not only a generic method and novel class of pro-prodrugs for the specific and/or target enhanced delivery to the brain (and testes) of a wide variety of drug species via the bidirectional transport of the drug species into and out of the brain (and testes) employing dihydropyridine == pyridinium salt carrier redox systems, but also a system providing insight into the basic transport processes (both active and passive) of, and enzymatic activities in, the blood-brain (and blood-testis barrier), as well as into the various processes specific to the function of the brain (and testes). Again, another very significant aspect of the bioreversible redox delivery system according to this invention is the toxicity implication, for significantly reduced is systemic toxicity by accelerating the elimination of the drug/quaternary carrier system. And even central toxicity is reduced by providing for low level, sustained release of the active drug species in the brain (and testes). Low toxicity is provided both as regards the quaternary carrier and in combination with the drug.

## Claims (Claims for the following Contracting State(s) : BE, CH, DE, FR, GB, LI, LU, NL, SE)

1. A compound adapted for the site specific/sustained delivery of a centrally acting drug species to the brain, said compound being a compound of the formula 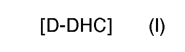 or a non-toxic pharmaceutically acceptable salt thereof, wherein [D] is a centrally acting drug species containing at least one reactive -OH, -COOH, -SH, -NH- or -NH₂ group (including groups such as amides and imides, as well as primary and secondary amino groups), and [DHC] is the reduced, biooxidizable, blood-brain barrier penetrating lipoidal form of a dihydropyridine == pyridinium salt redox carrier, with the proviso that when [DHC] is 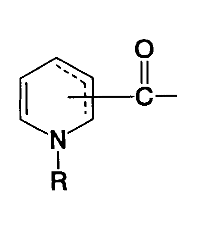 wherein R is lower alkyl or benzyl, the dotted line indicates the presence of a double bond in either the 2 or 3 position of the dihydropyridine ring and the carbonyl group is attached at the 2, 3 or 4 position of the dihydropyridine ring; and [D] is a drug species containing a single amino or OH functional group, the single amino or OH group when present being a primary or secondary amino or primary or secondary OH group, said drug species being linked directly through said amino or OH functional group to the carbonyl function of [DHC], then [D] must be other than a sympathetic stimulant, steroid sex hormone, memory enhancer, long chain alkanol or anticancer or antitumor agent.

2. A compound of the formula 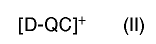 wherein [D] is a centrally acting drug species containing at least one reactive -OH, COOH, -SH, -NH- or NH₂ group (including groups such as amides and imides, as well as primary and secondary amino groups), and [QC]⁺ is the hydrophilic, ionic pyridinium salt form of a dihydropyridine == pyridinium salt redox carrier, with the proviso that when [QC]⁺ is 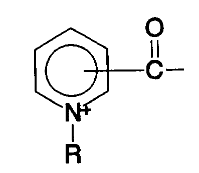 wherein R is lower alkyl or benzyl and the carbonyl group is attached at the 2, 3 or 4 position of the pyridinium ring, and [D] is a drug species containing a single amino or OH functional group, the single amino or OH group when present being a primary or secondary amino or primary or secondary OH group, said drug species being linked directly through said amino or OH functional group to the carbonyl function of [QC]⁺, then [D] must be other than a sympathetic stimulant, steroid sex hormone, memory enhancer, long chain alkanol or anticancer or antitumor agent.

3. A compound adapted for the site-specific/sustained delivery of a centrally acting drug species to the brain, said compound being a compound of the formula 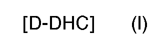 or a non-toxic pharmaceutically acceptable salt thereof, wherein [D] is a centrally acting drug species, said drug species being glycine, glutamic acid, aspartic acid, tryptophan, an enkephalin, an endorphin or other amino acid or small peptide containing 2 to 20 amino acid units; GABA, y-vinyl GABA, or y-acetylenic GABA; norepinephrine, or epinephrine; serotonin; 5-hydroxy-2-n-propylpentanoic acid, 4-hydroxy-2-n-propylpentanoic acid, or 3-hydroxy-2-n-propylpentanoic acid; methyldopa; tyramine; dopamine, or levodopa; ampicillin; cephalexin; L-alanosine; melphalan; DON, or acivicin; or glucosamine, or 6-amino-6-deoxy-D-glucose; and [DHC] is the reduced, biooxidizable, blood-brain barrier penetrating lipoidal form of a dihydropyridine == pyridinium salt redox carrier having the formula 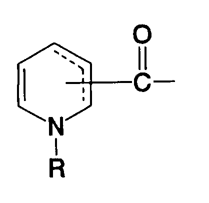 wherein R is methyl or benzyl and the dotted line indicates the presence of a double bond in either the 2 or 3 position of the dihydropyridine ring, the carbonyl function adjacent the dihydropyridine ring being bonded to a reactive amino or hydroxyl functional group in the centrally acting drug species, and at least one of the remaining reactive functional groups in the centrally acting drug species being protected by a hydrolytically or metabolically cleavable hydroxyl or carboxyl protective group.

4. A compound of the formula 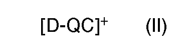 wherein [D] is a centrally acting drug species as defined in Claim 3, and [QC]⁺ is the hydrophilic, ionic pyridinium salt form of a dihydropyridine == pyridinium salt redox carrier having the formula 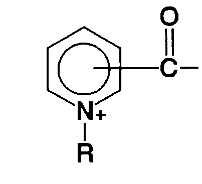 wherein R is methyl or benzyl, the carbonyl function adjacent the pyridinium ring being bonded to a reactive amino or hydroxyl functional group in the centrally acting drug species, and at least one of the remaining reactive functional groups in the centrally acting drug species being protected by a hydrolytically or metabolically cleavable hydroxyl or carboxyl protective group.

5. A compound according to Claim 3 or 4, wherein the drug species is a dopamine having the structural formula 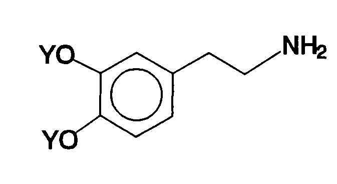 wherein each Y is independently hydrogen or a hydrolytically or metabolically cleavable hydroxyl protective group, with the proviso that at least one Y is an acyl group or a carbonate group, preferably wherein: one Y is pivalyl and the other Y is hydrogen or pivalyl; one Y is isobutyryl and the other Y is hydrogen or isobutyryl; one Y is ethoxycarbonyl and the other Y is hydrogen or ethoxycarbonyl; or one Y is isopropoxycarbonyl and the other Y is hydrogen or isopropoxycarbonyl.

6. A compound adapted for the site-specific/sustained delivery of a centrally acting drug species to the brain, said compound being a compound of the formula 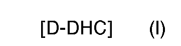 or a non-toxic pharmaceutically acceptable salt thereof, wherein [D] is a centrally acting drug species which is an antiviral agent, an antiinflammatory steroid, a narcotic or an estrogen and which contains at least two reactive hydroxyl functional groups, and [DHC] is the reduced, biooxidizable, blood-brain barrier penetrating lipoidal form of a dihydropyridine 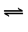 pyridinium salt redox carrier comprising at least one dihydropyridine ring system of the formula 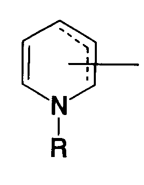 wherein R is lower alkyl or benzyl and the dotted line indicates the presence of a double bond in either the 2 or 3 position of the dihydropyridine ring, a ring carbon atom of each dihydropyridine ring system being connected via a bridging group to a reactive hydroxyl functional group in the centrally acting drug species.

7. A compound of the formula 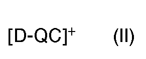 wherein [D] is a centrally acting drug species which is an antiviral agent, an antiinflammatory steroid, a narcotic or an estrogen and which contains at least two reactive hydroxyl functional groups, and [QC]⁺ is the hydrophilic, ionic pyridinium salt form of a dihydropyridine == pyridinium salt redox carrier comprising at least one pyridinium ring system of the formula 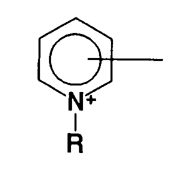 wherein R is lower alkyl or benzyl, a ring carbon atom of each pyridinium ring system being connected via a bridging group to a reactive hydroxyl functional group in the centrally acting drug species.

8. A compound according to Claim 6 or 7, wherein [D] is an antiviral agent.

9. A compound according to Claim 6 or 7, wherein [D] is an antiinflammatory steroid.

10. A compound according to Claim 6 or 7, wherein [D] is an estrogen.

11. A compound according to Claim 6 or 7, wherein [D] is hydrocortisone, betamethasone, dexamethasone, cortisone, flumethasone, fluprednisolone, meprednisone, methyl prednisolone, prednisolone, prednisone, triamcinolone, cortodoxone, fludrocortisone, fluandrenolone acetonide, paramethasone, ethinyl estradiol, estradiol, estriol, Ara-AC, pentostatin, dihydro-5-azacytidine, tiazofurin, sangivamycin, Ara-A, 6-MMPR, 5-FUDR, cytosine arabinoside, 5-azacytidine, ribavirin, 5,7-dimethyl-2-β-D-ribofuranosyl-s-triazole(1,5-a) pyrimidine, 2-deoxy-D-glucose, glucosamine, 2-deoxy-2-fluoro-D-mannose, 6-amino-6-deoxy-D-glucose, phenyl-6-chloro-6-deoxy-β-D-glucopyranoside, (S)-9-(2,3-dihydroxypropyl)adenine, 6- azauridine, 5,6-dichloro-1-β-D-ribofuranosylbenzimidazole, oxymorphone, apomorphine, morphine, oxycodone, naloxone, nalorphine, benzestrol or diethylstilbestrol.

12. A compound adapted for the site-specific/sustained delivery of a centrally acting drug species to the brain, said compound being a compound of the formula 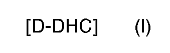 or a non-toxic pharmaceutically acceptable salt thereof, wherein [D] is a centrally acting drug species which is an anticancer/antitumor and/or antiviral agent, said drug species having a purine or pyrimidine base-type structure bearing a singly or multiply hydroxylated substituent, and [DHC] is the reduced, biooxidizable, blood-brain barrier penetrating, lipoidal form of a dihydropyridine == pyridinium salt redox carrier comprising at least one dihydropyridine ring system of the formula 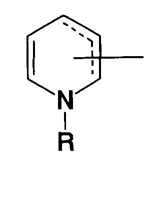 wherein R is lower alkyl or benzyl and the dotted line indicates the presence of a double bond in either the 2 or 3 position of the dihydropyridine ring, a ring carbon atom of each dihydropyridine ring system being connected via a bridging group to a reactive hydroxyl functional group in the centrally acting drug species.

13. A compound of the formula 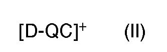 wherein [D] is a centrally acting drug species which is an anticancer/antitumor and/or antiviral agent, said drug species having a purine or pyrimidine base-type structure bearing a singly or multiply hydroxylated substituent, and [QC]⁺ is the hydrophilic, ionic pyridinium salt form of a dihydropyridine == pyridinium salt redox carrier comprising at least one pyridinium ring system of the formula 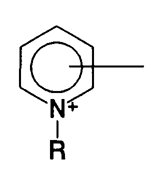 wherein R is lower alkyl or benzyl, a ring carbon atom of each pyridinium ring system being connected via a bridging group to a reactive hydroxyl functional group in the centrally acting drug species.

14. A compound according to Claim 12 or 13, wherein [D] is an antiviral agent.

15. A compound according to Claim 12 or 13, wherein [D] is Ara-AC, pentostatin, Ara-C, dihydro-5- azacytidine, tiazofurin, sangivamycin, Ara-A, 6-MMPR, desmethylmisonidazole, 5-FUDR, cytosine arabinoside, 5-azacytidine, ribavirin, acyclovir, (S)-9-(2,3-dihydroxypropyl)adenine, 6-azauridine, 5,6-dich- loro-1-β-D-ribofuranosylbenzimidazole or 5,7-dimethyl-2-β-D-ribofuranosyl-s-triazole(1,5-a)pyrimidine.

16. A compound adapted for the site-specific/sustained delivery of a centrally acting drug species to the brain, said compound being a compound of the formula 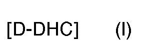 or a non-toxic pharmaceutically acceptable salt thereof, wherein [D] is a centrally acting drug species which is a steroidal progestin or steroidal estrogen, said progestin or estrogen having at least one reactive hydroxyl functional group, one such group being a tertiary hydroxyl functional group in the 17- position; and [DHC] is the reduced, biooxidizable, blood-brain barrier penetrating lipoidal form of a dihydropyridine == pyridinium salt redox carrier comprising at least one dihydropyridine ring system of the formula 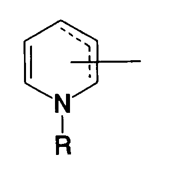 wherein R is lower alkyl or benzyl and the dotted line indicates the presence of a double bond in either the 2 or 3 position of the dihydropyridine ring, a ring carbon atom of one dihydropyridine ring system being connected via a bridging group to the tertiary 17-hydroxyl functional group in the progestin or estrogen, a ring carbon atom of any remaining dihydropyridine ring system being connected via a bridging group to another reactive hydroxyl functional group in said progestin or estrogen.

17. A compound according to any one of Claims 6, 12 and 16, wherein each dihydropyridine ring system, together with the bridging group connected thereto, comprises the formula 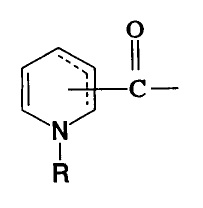 wherein R is lower alkyl or benzyl and the dotted line indicates the presence of a double bond in either the 2 or 3 position of the dihydropyridine ring; particularly when the dotted line indicates the presence of a double bond in the 2 position of the dihydropyridine ring, or when R is methyl, or when the carbonyl group is attached at the 3 position of the dihydropyridine ring.

18. A compound according to Claim 17, wherein each dihydropyridine ring system, together with the bridging group connected thereto, has the formula

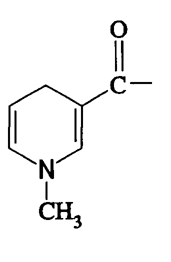

19. A compound according to Claim 17, wherein each dihydropyridine ring system, together with the bridging group connected thereto, has the formula

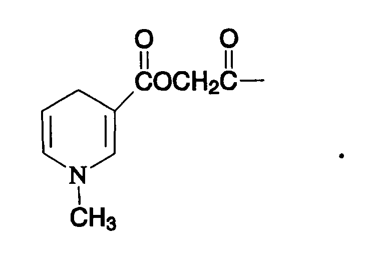

20. A compound of the formula 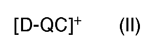 wherein [D] is a centrally acting drug species which is a steroidal progestin or steroidal estrogen, said progestin or estrogen having at least one reactive hydroxyl functional group, one such group bearing a tertiary hydroxyl functional group in the 17-position; and [QC]⁺ is the hydrophilic, ionic pyridinium salt form of a dihydropyridine pyridinium salt redox carrier comprising at least one pyridinium ring system of the formula 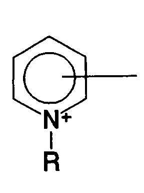 wherein R is lower alkyl or benzyl, a ring carbon atom of one pyridinium ring system being connected via a bridging group to the tertiary 17-hydroxyl functional group in the progestin or estrogen, a ring carbon atom of any remaining pyridinium ring system being connected via a bridging group to another reactive hydroxyl functional group in said progestin or estrogen.

21. A compound according to any one of Claims 7, 13 and 20, wherein each pyridinium ring system, together with the bridging group connected thereto, comprises the formula 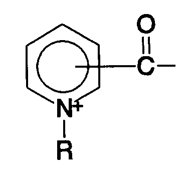 wherein R is lower alkyl or benzyl, particularly when R is methyl or when the carbonyl group is attached at the 3 position of the pyridinium ring.

22. A compound according to Claim 21, wherein each pyridinium ring system, together with the bridging group connected thereto, has the formula

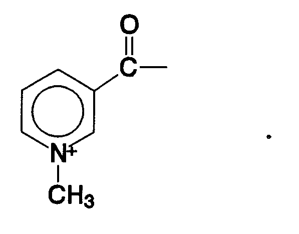

23. A compound according to Claim 21, wherein each pyridinium ring system, together with the bridging group connected thereto, has the formula

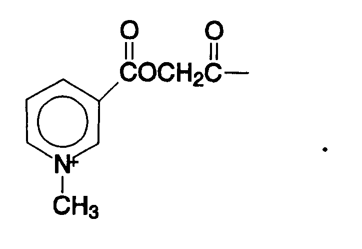

24. A compound according to Claim 16 or 20, wherein [D] is mestranol, quinestrol, norgestrel, norethindrone, ethisterone, dimethisterone, allylestrenol, cingestol, ethynerone, lynestrenol, norgesterone, norvinisterone, ethynodiol or tigestol.

25. A compound adapted for the site-specific/sustained delivery of a centrally acting drug species to the brain, said compound being a compound of the formula 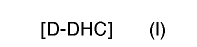 or a non-toxic pharmaceutically acceptable salt thereof, wherein [D] is a centrally acting drug species containing at least one reactive -COOH, amide or imide functional group, and [DHC] is the reduced, biooxidizable, blood-brain barrier penetrating lipoidal form of a dihydropyridine pyridinium salt redox carrier comprising a dihydropyridine ring system of the formula 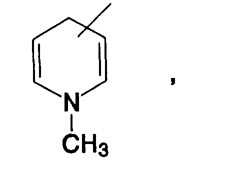 a ring carbon atom of the dihydropyridine ring system being connected via a bridging group to a reactive -COOH, amide or imide functional group in the centrally acting drug species.

26. A compound according to Claim 25, wherein the centrally acting drug species [D] contains at least one reactive amide or imide group and the dihydropyridine ring system together with the bridging group has the structural formula 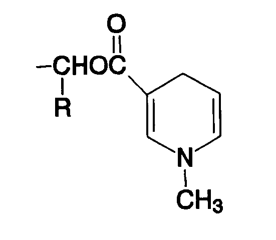 wherein R is -CC!₃, -H, 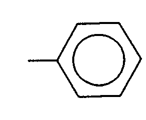 or -CH₃.

27. A compound according to Claim 25, wherein the centrally acting drug species [D] contains at least one reactive amide or imide group and the dihydropyridine ring system together with the bridging group has the structural formula 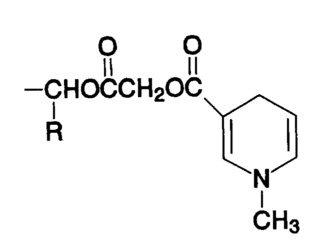 wherein R is -CC!₃, -H, 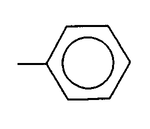 or -CH₃.

28. A compound according to Claim 25, wherein the centrally acting drug species [D] contains at least one reactive -COOH group and the bridging group connecting the dihydropyridine ring system to the drug is derived from a di- or polyhydroxy compound, at least one hydroxy function of which is attached to a reactive -COOH group in the drug and at least one other hydroxy function of which is attached via a carbonyl function to the dihydropyridine ring system.

29. A compound of the formula 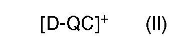 wherein [D] is a centrally acting drug species containing at least one reactive -COOH, amide or imide functional group and [QC]⁺ is the hydrophilic, ionic pyridinium salt form of a dihydropyridine pyridinium salt redox carrier comprising a pyridinium ring system of the formula 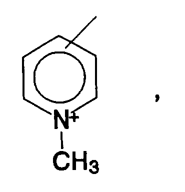 a ring carbon atom of the pyridinium ring system being connected via a bridging group to a reactive -COOH, amide or imide functional group in the centrally acting drug species.

30. A compound according to Claim 29, wherein the centrally acting drug species [D] contains at least one reactive amide or imide group and the pyridinium ring system together with the bridging group has the structural formula 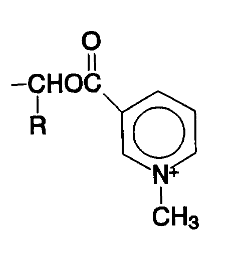 wherein R is -CC!₃, -H, 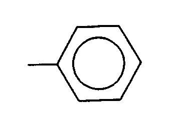 or -CH₃.

31. A compound according to Claim 29, wherein the centrally acting drug species [D] contains at least one reactive amide or imide group and the pyridinium ring system together with the bridging group has the structural formula 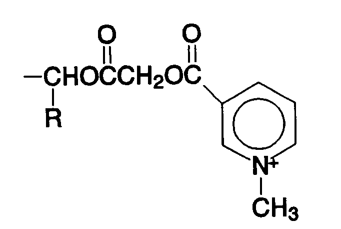 wherein R is -CC!₃, -H, 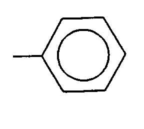 or -CH₃.

32. A compound according to Claim 29, wherein the centrally acting drug species [D] contains at least one reactive -COOH group and the bridging group connecting the pyridinium ring system to the drug is derived from a di- or polyhydroxy compound, at least one hydroxy function of which is attached to a reactive -COOH group in the drug and at least one other hydroxy function of which is attached via a carbonyl function to the pyridinium ring system.

33. A compound according to Claim 28 or 32, wherein the bridging group is derived from ethylene glycol, propylene glycol, inositol or a sugar.

34. A compound according to Claim 25 or 29, wherein [D] is a central neurotransmitter, an anticancer or antitumor agent, an antiviral agent, a tranquilizer, a sedative or hypnotic, an anticonvulsant or antiepileptic agent, a dopaminergic agent or an antibiotic or antibacterial agent.

35. A compound according to Claim 25 or 29, wherein [D] is an amino acid or a small peptide containing 2 to 20 amino acid units.

36. A compound according to Claim 25 or 29, wherein [D] is GABA, glycine, glutamic acid, aspartic acid, neurotensin, LHRH, an enkephalin, an endorphin, oxytocin M, vasopressin, 3-deazaguanine, tiazofurin, sangivamycin, PCNU, spiromustine, L-alanosine, DON, L-ICRF, 5-methyl tetrahydrohomofolic acid, glyoxylic acid sulfonylhydrazine, DACH, SR-2555, SR-2508, bactobolin, acivicin, hydroxyurea, chlorambucil, uracil mustard, melphalan, 5-FUDR, methotrexate, aminopterin, mitomycin C, ribavirin, acyclovir, 6-azauridine, oxazepam, lorazepam, chlordiazepoxide, bromazepam, clorazepate, nitrazepam, phenytoin, ethotoin, mephenytoin, methyldopa, y-vinyl GABA, y-acetylenic GABA, phenobarbital, amobarbital, butalbital, glutethimide, aminoglutethimide, methyprylon, valproic acid, 5-hydroxy-2-n-propylpentanoic acid, 4-hydroxy-2-n-propylpentanoic acid, 3-hydroxy-2-n-propylpentanoic acid, bemegride, tryptophan, levodopa, ampicillin, amoxicillin, oxacillin, carbenicillin, dicloxacillin, chlortetracycline, tetracycline, methacycline, nalidixic acid, cephalexin, cephalothin, cefoxitin, clindamycin, lincomycin or oxolinic acid.

37. A compound adapted for the site-specific/sustained delivery of a centrally acting drug species to the brain, said compound being a compound of the formula 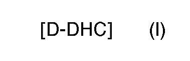 or a non-toxic pharmaceutically acceptable salt thereof, wherein [D] is a centrally acting drug species containing at least one reactive -OH, -COOH, -SH, -NH- or -NH₂ group (including groups such as amides and imides, as well as primary and secondary amino groups), and [DHC] is the reduced, biooxidizable, blood-brain barrier penetrating lipoidal form of a dihydropyridine == pyridinium salt redox carrier comprising a dihydropyridine ring system of the formula 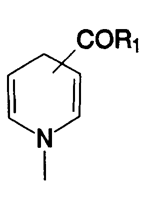 wherein R is -NH₂ or -OR₂ wherein R₂ is alkyl, the dihydropyridine ring optionally being fused to a benzene ring, the nitrogen atom of the dihydropyridine ring system being connected via a bridging group to a reactive -OH, -COOH, -SH, -NH- or -NH₂ group in the centrally acting drug species.

38. A compound according to Claim 37, wherein the centrally acting drug species [D] contains at least one reactive primary or secondary amino group or at least one reactive -OH group, and the dihydropyridine ring system together with the bridging group connecting the dihydropyridine ring system to the amino or -OH group in [D] has the structural formula 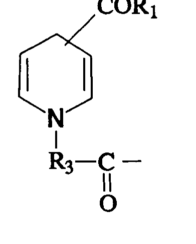 wherein R is as defined in Claim 37 and R₃ is (CH₂)ₙ wherein n is 1-10 or up to C₁₂ branched alkylene, preferably wherein the dihydropyridine ring system together with the bridging group has the structural formula 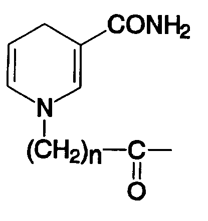 wherein n is 1, 2 or 3.

39. A compound according to Claim 37, wherein the centrally acting drug species [D] contains at least one reactive -SH group, and the dihydropyridine ring system together with the bridging group connecting the dihydropyridine ring system to the -SH group in [D] has the structural formula 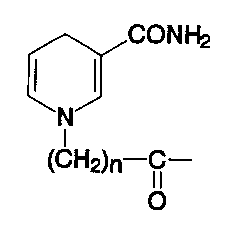 wherein n is 1, 2 or 3.

40. A compound according to Claim 37, wherein the centrally acting drug species [D] contains at least one reactive primary or secondary amino group or at least one reactive -OH group, and the dihydropyridine ring system together with the bridging group connecting the dihydropyridine ring system to the amino or -OH group in [D] has the structural formula 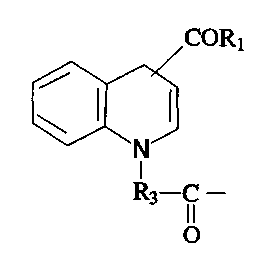 wherein R is as defined in Claim 37 and R₃ is (CH₂)ₙ wherein n is 1-10 or up to C₁₂ branched alkylene, preferably wherein the dihydropyridine ring system together with the bridging group has the structural formula 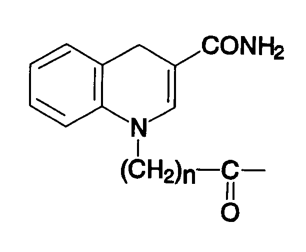 wherein n is 1, 2 or 3.

41. A compound according to Claim 37, wherein the centrally acting drug species [D] contains at least one reactive primary or secondary amino group or at least one reactive -OH group, and the dihydropyridine ring system together with the bridging group connecting the dihydropyridine ring system to the amino or -OH group in [D] has the structural formula 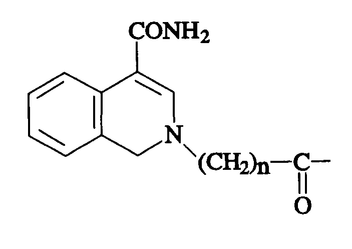 wherein n is 1, 2 or 3.

42. A compound according to Claim 37, wherein the centrally acting drug species [D] contains at least one reactive -NH- or -NH₂ group which is part of an amide or imide structure or which is a low pKa primary or secondary amino group and the dihydropyridine ring system together with the bridging group connecting the dihydropyridine ring system to the -NH- or -NH₂ group in [D] has the structural formula 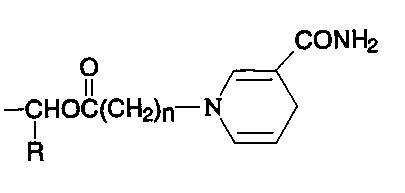 wherein R is -CC!₃, -H, 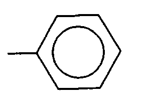 or -CH₃ and n is 1, 2 or 3.

43. A compound according to Claim 37, wherein the centrally acting drug species [D] contains at least one reactive -COOH group, and the dihydropyridine ring system together with the bridging group connecting the dihydropyridine ring system to the -COOH group in [D] has the structural formula 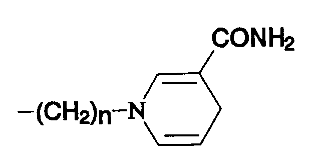 wherein n is 1, 2 or 3.

44. A compound according to Claim 37, wherein the centrally acting drug species [D] contains at least one reactive -COOH group, and the dihydropyridine ring system together with the bridging group connecting the dihydropyridine ring system to the -COOH group in [D] has the structural formula

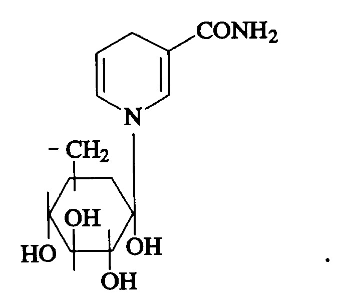

45. A compound of the formula 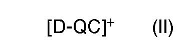 wherein [D] is a centrally acting drug species containing at least one reactive -OH, -COOH, -SH, -NH-or -NH₂ group (including groups such as amides and imides, as well as primary and secondary amino groups), and [QC]⁺ is the hydrophilic, ionic pyridinium salt form of a dihydropyridine pyridinium salt redox carrier comprising a pyridinium ring system of the formula 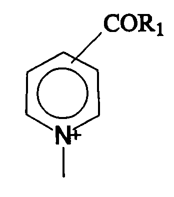 wherein R is -NH₂ or -OR₂ wherein R₂ is alkyl, the pyridinium ring optionally being fused to a benzene ring, the nitrogen atom of the pyridinium ring system being connected via a bridging group to a reactive -OH, -COOH, -SH, -NH- or -NH₂ group in the centrally acting drug species.

46. A compound according to Claim 45, wherein the centrally acting drug species [D] contains at least one reactive primary or secondary amino group or at least one reactive -OH group, and the pyridinium ring system together with the bridging group connecting the pyridinium ring system to the amino or -OH group in [D] has the structural formula 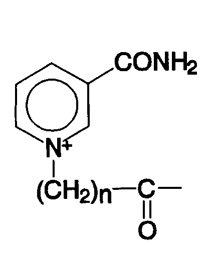 wherein n is 1, 2, or 3.

47. A compound according to Claim 45, wherein the centrally acting drug species [D] contains at least one reactive primary or secondary amino group or at least one reactive -OH group, and the pyridinium ring system together with the bridging group connecting the pyridinium ring system to the amino or -OH group in [D] has the structural formula 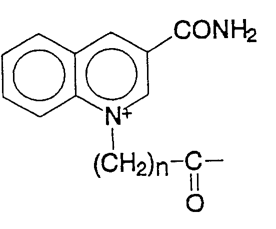 wherein n is 1, 2, or 3.

48. A compound according to Claim 45, wherein the centrally acting drug species [D] contains at least one reactive -NH- or -NH₂ group which is part of an amide or imide structure or which is a low pKa primary or secondary amino group, and the pyridinium ring system together with the bridging group connecting the pyridinium ring system to the -NH- or -NH₂ group in [D] has the structural formula 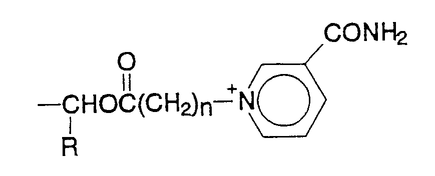 wherein R is -CC!₃, -H, 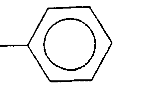 or -CH₃ and n is 1, 2 or 3.

49. A compound according to Claim 45, wherein the centrally acting drug species [D] contains at least one reactive -COOH group, and the pyridinium ring system together with the bridging group connecting the pyridinium ring system to the -COOH group in [D] has the structural formula 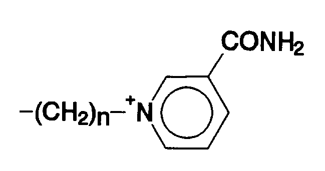 wherein n is 1, 2 or 3.

50. A compound adapted for the site-specific/sustained delivery of a centrally acting drug species to the brain, said compound being a compound of the formula 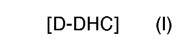 or a non-toxic pharmaceutically acceptable salt thereof, wherein [D] is a centrally acting drug species containing at least one reactive -OH, -COOH, -SH, -NH- or -NH₂ group (including groups such as amides and imides, as well as primary and secondary amino groups), and [DHC] is the reduced, biooxidizable, blood-brain barrier penetrating lipoidal form of a dihydropyridine pyridinium salt redox carrier comprising a dihydroquinoline or dihydroisoquinoline ring system, a carbon atom of the nitrogen-containing ring portion of said ring system being connected via a bridging group to a reactive -OH, -COOH, -SH, -NH- or -NH₂ group in the centrally acting drug species.

51. A compound according to Claim 50, wherein the centrally acting drug species [D] contains at least one reactive primary or secondary amino group or at least one reactive -OH group, and the dihydroquinoline or dihydroisoquinoline ring system together with the bridging group connecting the ring system to the amino or -OH group in [D] has the structural formula 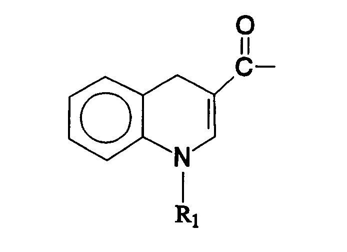 wherein R is C₁-C₇ alkyl or C₇-C₁₀ aralkyl, particularly when R is methyl.

52. A compound according to Claim 50, wherein the centrally acting drug species [D] contains at least one reactive primary or secondary amino group or at least one reactive -OH group, and the dihydroquinoline or dihydroisoquinoline ring system together with the bridging group connecting the ring system to the amino or -OH group in [D] has the structural formula 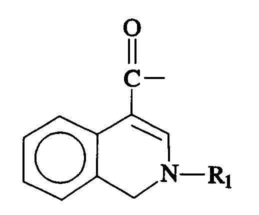 wherein R is C₁-C₇ alkyl or C₇-C₁₀ aralkyl, particularly when R₁ is methyl.

53. A compound according to Claim 50, wherein the centrally acting drug species [D] contains at least one reactive primary or secondary amino group or at least one reactive -OH group or at least one reactive -SH group, and the dihydroquinoline or dihydroisoquinoline ring system together with the bridging group connecting the ring system to the amino or -OH or -SH group in [D] has the structural formula

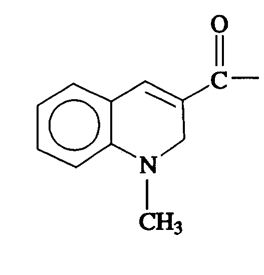

54. A compound according to Claim 50, wherein the centrally acting drug species [D] contains at least one reactive -NH- or -NH₂ group which is part of an amide or imide structure or which is a low pKa primary or secondary amino group, the redox carrier [DHC] comprises a dihydroquinoline ring system and the bridging group has the structure 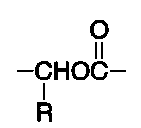 wherein R is -CC!₃, -H, 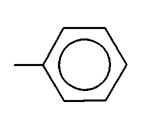 or -CH₃, the carbonyl function of the bridging group being connected to a carbon atom of the nitrogen-containing ring portion of the dihydroquinoline ring system.

55. A compound of the formula 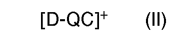 wherein [D] is a centrally acting drug species containing at least one reactive -OH, -COOH, -SH, -NH-or -NH₂ group (including groups such as amides and imides, as well as primary and secondary amino groups), and [QC]⁺ is the hydrophilic, ionic pyridinium salt form of a dihydropyridine == pyridinium salt redox carrier comprising a quinolinium or isoquinolinium ring system, a carbon atom of the nitrogen-containing ring portion of said ring system being connected via a bridging group to a reactive -OH, -COOH, -SH, -NH- or -NH₂ group in the centrally acting drug species.

56. A compound according to Claim 55, wherein the centrally acting drug species [D] contains at least one reactive primary or secondary amino group or at least one reactive -OH group, and the quinolinium or isoquinolinium ring system together with the bridging group connecting the ring system to the amino or -OH group in [D] has the structural formula 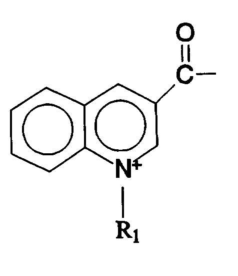 wherein R is C₁-C₇ alkyl or C₇-C₁₀ aralkyl, particularly when R is methyl.

57. A compound according to Claim 55, wherein the centrally acting drug species [D] contains at least one reactive primary or secondary amino group or at least one reactive -OH group, and the quinolinium or isoquinolinium ring system together with the bridging group connecting the ring system to the amino or -OH group in [D] has the structural formula 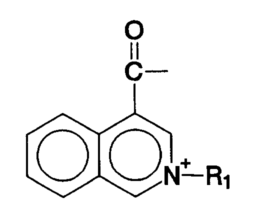 wherein R₁ is C₁-C₇ alkyl or C₇-C₁₀ aralkyl, particularly when R₁ is methyl.

58. A compound according to Claim 55, wherein the centrally acting drug species [D] contains at least one reactive -NH- or -NH₂ group which is part of an amide or imide structure or which is a low pKa primary or secondary amino group, the redox carrier [QC]⁺ comprises a quinolinium ring system and the bridging group has the structure 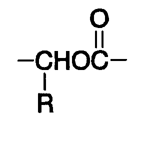 wherein R is -CCl₃, -H, 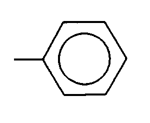 or -CH₃, the carbonyl function of the bridging group being connected to a carbon atom of the nitrogen-containing ring portion of the quinolinium ring system.

59. A compound according to any one of Claims 37, 45, 50 and 55, wherein [D] is a central neurotransmitter, an antiinflammatory steroid, a steroid sex hormone, an anticancer or antitumor agent, an antiviral agent, a tranquilizer, a memory enhancer, a hypotensive agent, a sedative or hypnotic, an anticonvulsant or antiepileptic agent, a stimulant, a narcotic analgesic or antagonist, a dopaminergic agent or an antibiotic or antibacterial agent.

60. A compound according to any one of Claims 37, 45, 50 and 55, wherein [D] is an amino acid or a small peptide containing 2 to 20 amino acid units.

61. A compound according to any one of Claim 37, 45, 50 and 55, wherein [D] is a dopamine having the structural formula 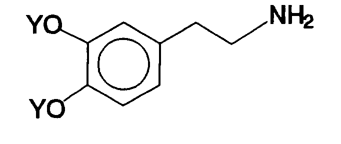 in which each Y is independently hydrogen or a hydrolytically or metabolically cleavable hydroxyl protective group.

62. A compound according to any one of Claims 37, 45, 50 and 55, wherein [D] is GABA, glycine, glutamic acid, aspartic acid, dopamine, norephinephrine, epinephrine, serotonin, tryptamine, neurotensin, LHRH, somatostatin, an enkephalin, an endorphin, oxytocin M, vasopressin, hydrocortisone, betamethasone, dexamethasone, cortisone, flumethasone, fluprednisolone, meprednisone, methyl prednisolone, prednisolone, prednisone, triamcinolone, cortodoxone, fludrocortisone, fluandrenolone acetonide, paramethasone, testosterone, methyl testosterone, ethinyl estradiol, norgestrel, mestranol, norethindrone, ethisterone, estradiol, estriol, estrone, dimethisterone, allylestrenol, cingestol, ethynerone, lynestrenol, norgesterone, norvininsterone, ethynodiol, oxogestone, tigestol, quinestrol, Ara-AC, pentostatin, Ara-C, 3-deazaguanine, dihydro-5-azacytidine, tiazofurin, sangivamycin, Ara-A, 6-MMPR, PCNU, spiromustine, bisbenzimidazole, L-alanosine, DON, L-ICRF, trimethyl TMM, 5-methyl tetrahydrohomofolic acid, glyoxylic acid sulfonylhydrazone, DACH, SR-2555, SR-2508, desmethylmisonidazole, mitoxantrone, menogarol, aclacinomycin A, phyllanthoside, bactobolin, aphidocolin, homoharringtonine, levonantradol, acivicin, streptozotocin, hydroxyurea, chlorambucil, cyclophosphamide, uracil mustard, melphalan, 5-FUDR, cytosine arabinoside, 6-mercaptopurine, thioguanine, 5-azacytidine, methotrexate, doxorubicin, daunomycin, aminopterin, dactinomycin, mitomycin C, a podophyllotoxin derivative, ribavirin, acyclovir, amantadine, 5-amidino-2-(5-amidino-2-benzofuranyl)indole, 4',6-diimidazolino-2-phenylbenzo(b)-thiophene, 2-guanidino-4,5-di-n-propyloxazole, 2-guanidino-4,5-diphenyloxazole, 6[[(hydroxyimino)-phenyl]methyl]-1-[(1-methylethyl)sulfonyl]-1H-benzimidazol-2-amine, 5,7-dimethy!-2-j8-D-ribofuranosy!-s-triazole(1,5-a)pyrimidine, 2-deoxy-D-glucose, glucosamine, 2-deoxy-2-f!uoro-D-mannose, 6-amino-6-deoxy-D-glucose, phenyl-6-chloro-6-deoxy-β-D-glucopyranoside, (S)-9-(2,3-dihydroxypropyl)adenine, 6- azauridine, 5,6-dichloro-1-β-D-ribofuranosylbenzimidazole, diazepam, oxazepam, lorazepam, chlordiazepoxide, flurazepam, bromazepam, clorazepate, nitrazepam, temazepam, phenytoin, ethotoin, mephenytoin, acetophenazine, carphenazine, fluphenazine, perphenazine, piperacetazine, clonidine, methyldopa, bethanidine, debrisoquin, hydralazine, guanethidine, haloperidol, hydroxyzine, clopenthixol, y-vinyl GABA, y-acetylenic GABA, phenobarbital, amobarbital, butalbital, glutethimide, aminoglutethimide, methyprylon, valproic acid, 5-hydroxy-2-n-propylpentanoic acid, 4-hydroxy-2-n-pro- pylpentanoic acid, 3-hydroxy-2-n-propylpentanoic acid, methamphetamine, phentermine, phenmetrazine, dextroamphetamine, levamphetamine, amphetamine, phenylethylamine, methylphenidate, tranylcypromine, protriptyline, opipramol, desipramine, nortriptyline, bemegride, amiphenazole, anileridine, hydromorphone, oxymorphone, apomorphine, levorphanol, morphine, pentazocine, codeine, oxycodone, naloxone, nalorphine, tryptophan, tryamine, levodopa, ampicillin, amoxicillin, oxacillin, carbenicillin, dicloxacillin, chlortetracycline, tetracycline, methacycline, nalidixic acid, cephalexin, cephalothin, cefoxitin, clindamycin, lincomycin, oxolinic acid, phenazopyridine, sulfadiazine, thiopental, benzestrol or diethylstilbestrol.

63. A process for the preparation of a compound as claimed in Claim 1 which comprises reducing the corresponding compound as claimed in Claim 2.

64. A pharmaceutical composition of matter comprising a compound as claimed in any one of Claims 1, 3, 6, 12, 16-19, 25-28, 37-44 and 50-54, or in Claim 5 when dependent on Claim 3, or in any one of Claims 8-11 when dependent on Claim 6, or in Claim 14 or 15 when dependent on Claim 12, or in Claim 24 when dependent on Claim 16, or in Claim 33 when dependent on Claim 28, or on any one of Claims 34-36 when dependent on Claim 25, or on any one of Claims 59-62 when dependent on Claim 37 or 50, and a non-toxic pharmaceutically acceptable carrier therefor.

## Claims (Claims for the following Contracting State(s) : AT)

1. A process for the preparation of a compound of the formula 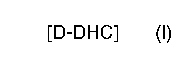 or a non-toxic pharmaceutically acceptable salt thereof, wherein [D] is a centrally acting drug species containing at least one reactive -OH, -COOH, -SH, -NH- or -NH₂ group (including groups such as amides and imides, as well as primary and secondary amino groups), and [DHC] is the reduced, biooxidizable, blood-brain barrier penetrating lipoidal form of a dihydropyridine ⇄ pyridinium salt redox carrier, with the proviso that when [DHC] is 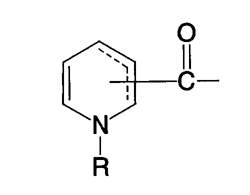 wherein R is lower alkyl or benzyl, the dotted line indicates the presence of a double bond in either the 2 or 3 position of the dihydropyridine ring and the carbonyl group is attached at the 2, 3 or 4 position of the dihydropyridine ring; and [D] is a drug species containing a single amino or OH functional group, the single amino or OH group when present being a primary or secondary amino or primary or secondary OH group, said drug species being linked directly through said amino or OH functional group to the carbonyl function of [DHC], then [D] must be other than a sympathetic stimulant, steroid sex hormone, memory enhancer, long chain alkanol or anticancer or antitumor agent; said process being characterized by reducing the corresponding compound of the formula 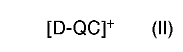 wherein [D] is defined above, and [QC]⁺ is the hydrophilic, ionic pyridinium salt form of a dihydropyridine == pyridinium salt redox carrier, with the proviso that when [QC]⁺ is 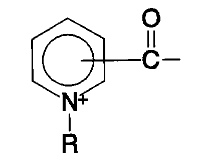 wherein R is lower alkyl or benzyl and the carbonyl group is attached at the 2, 3 or 4 position of the pyridinium ring, and [D] is a drug species containing a single amino or OH functional group, the single amino or OH group when present being a primary or secondary amino or primary or secondary OH group, said drug species being linked directly through said amino or OH functional group to the carbonyl function of [QC]⁺, then [D] must be other than a sympathetic stimulant, steroid sex hormone, memory enhancer, long chain alkanol or anticancer or antitumor agent.

2. A process for the preparation of a compound of the formula 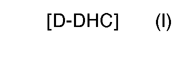 or a non-toxic pharmaceutically acceptable salt thereof, wherein [D] is a centrally acting drug species, said drug species being glycine, glutamic acid, aspartic acid, tryptophan, an enkephalin, an endorphin or other amino acid or small peptide containing 2 to 20 amino acid units; GABA, y-vinyl GABA, or y-acetylenic GABA; norepinephrine, or epinephrine; serotonin; 5-hydroxy-2-n-propylpentanoic acid, 4-hydroxy-2-n-propylpentanoic acid, or 3-hydroxy-2-n-propylpentanoic acid; methyldopa; tyramine; dopamine, or levodopa; ampicillin; cephalexin; L-alanosine; melphalan; DON, or acivicin; or glucosamine, or 6-amino-6-deoxy-D-glucose; and [DHC] is the reduced, biooxidizable, blood-brain barrier penetrating lipoidal form of a dihydropyridine == pyridinium salt redox carrier having the formula 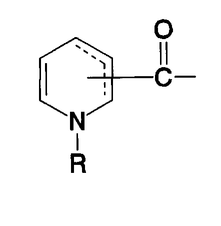 wherein R is methyl or benzyl and the dotted line indicates the presence of a double bond in either the 2 or 3 position of the dihydropyridine ring, the carbonyl function adjacent the dihydropyridine ring being bonded to a reactive amino or hydroxyl functional group in the centrally acting drug species, and at least one of the remaining reactive functional groups in the centrally acting drug species being protected by a hydrolytically or metabolically cleavable hydroxyl or carboxyl protective group; said process being characterized by reducing the corresponding compound of the formula 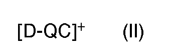 wherein [D] is defined above, and [QC]⁺ is the hydrophilic, ionic pyridinium salt form of a dihydropyridine == pyridinium salt redox carrier having the formula 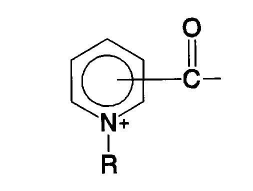 wherein R is methyl or benzyl, the carbonyl function adjacent the pyridinium ring being bonded to a reactive amino or hydroxyl functional group in the centrally acting drug species, and at least one of the remaining reactive functional groups in the centrally acting drug species being protected by a hydrolytically or metabolically cleavable hydroxyl or carboxyl protective group.

3. A process according to Claim 2, wherein the drug species is a dopamine having the structural formula 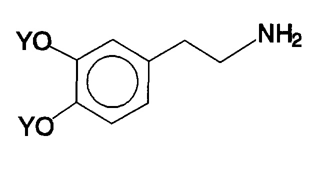 wherein each Y is independently hydrogen or a hydrolytically or metabolically cleavable hydroxyl protective group, with the proviso that at least one Y is an acyl group or a carbonate group, preferably wherein: one Y is pivalyl and the other Y is hydrogen or pivalyl; one Y is isobutyryl and the other Y is hydrogen or isobutyryl; one Y is ethoxycarbonyl and the other Y is hydrogen or ethoxycarbonyl; or one Y is isopropoxycarbonyl and the other Y is hydrogen or isopropoxycarbonyl.

4. A process for the preparation of a compound of the formula 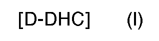 or a non-toxic pharmaceutically acceptable salt thereof, wherein [D] is a centrally acting drug species which is an antiviral agent, an antiinflammatory steroid, a narcotic or an estrogen and which contains at least two reactive hydroxyl functional groups, and [DHC] is the reduced, biooxidizable, blood-brain barrier penetrating lipoidal form of a dihydropyridine == pyridinium salt redox carrier comprising at least one dihydropyridine ring system of the formula 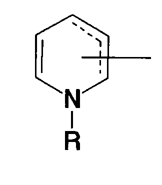 wherein R is lower alkyl or benzyl and the dotted line indicates the presence of a double bond in either the 2 or 3 position of the dihydropyridine ring, a ring carbon atom of each dihydropyridine ring system being connected via a bridging group to a reactive hydroxyl functional group in the centrally acting drug species; said process being characterized by reducing the corresponding compound of the formula 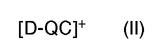 wherein [D] is defined above, and [QC]⁺ is the hydrophilic, ionic pyridinium salt form of a dihydropyridine == pyridinium salt redox carrier comprising at least one pyridinium ring system of the formula 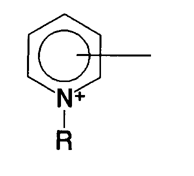 wherein R is lower alkyl or benzyl, a ring carbon atom of each pyridinium ring system being connected via a bridging group to a reactive hydroxyl functional group in the centrally acting drug species.

5. A process according to Claim 4, wherein [D] is an antiviral agent.

6. A process according to Claim 4, wherein [D] is an antiinflammatory steroid.

7. A process according to Claim 4, wherein [D] is an estrogen.

8. A process according to Claim 4, wherein [D] is hydrocortisone, betamethasone, dexamethasone, cortisone, flumethasone, fluprednisolone, meprednisone, methyl prednisolone, prednisolone, prednisone, triamcinolone, cortodoxone, fludrocortisone, fluandrenolone acetonide, paramethasone, ethinyl estradiol, estradiol, estriol, Ara-AC, pentostatin, dihydro-5-azacytidine, tiazofurin, sangivamycin, Ara-A, 6-MMPR, 5-FUDR, cytosine arabinoside, 5-azacytidine, ribavirin, 5,7-dimethyl-2-β-D-ribofuranosyl-s-triazole(1,5-a) pyrimidine, 2-deoxy-D-glucose, glucosamine, 2-deoxy-2-fluoro-D-mannose, 6-amino-6-deoxy-D-glucose, phenyl-6-chloro-6-deoxy-β-D-glucopyranoside, (S)-9-(2,3-dihydroxypropyl)adenine, 6- azauridine, 5,6-dichloro-1-β-D-ribofuranosylbenzimidazole, oxymorphone, apomorphine, morphine, oxycodone, naloxone, nalorphine, benzestrol or diethylstilbestrol.

9. A process for the preparation of a compound of the formula 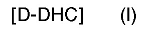 or a non-toxic pharmaceutically acceptable salt thereof, wherein [D] is a centrally acting drug species which is an anticancer/antitumor and/or antiviral agent, said drug species having a purine or pyrimidine base-type structure bearing a singly or multiply hydroxylated substituent, and [DHC] is the reduced, biooxidizable, blood-brain barrier penetrating, lipoidal form of a dihydropyridine == pyridinium salt redox carrier comprising at least one dihydropyridine ring system of the formula 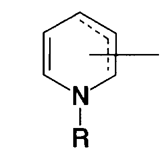 wherein R is lower alkyl or benzyl and the dotted line indicates the presence of a double bond in either the 2 or 3 position of the dihydropyridine ring, a ring carbon atom of each dihydropyridine ring system being connected via a bridging group to a reactive hydroxyl functional group in the centrally acting drug species; said process being characterized by reducing the corresponding compound of the formula 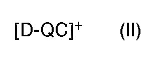 wherein [D] is defined above and [QC]⁺ is the hydrophilic, ionic pyridinium salt form of a dihydropyridine == pyridinium salt redox carrier comprising at least one pyridinium ring system of the formula 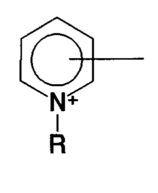 wherein R is lower alkyl or benzyl, a ring carbon atom of each pyridinium ring system being connected via a bridging group to a reactive hydroxyl functional group in the centrally acting drug species.

10. A process according to Claim 9, wherein [D] is an antiviral agent.

11. A process according to Claim 9, wherein [D] is Ara-AC, pentostatin, Ara-C, dihydro-5-azacytidine, tiazofurin, sangivamycin, Ara-A, 6-MMPR, desmethylmisonidazole, 5-FUDR, cytosine arabinoside, 5- azacytidine, ribavirin, acyclovir, (S)-9-(2,3-dihydroxypropyl)adenine, 6-azauridine, 5,6-dichloro-1-β-D-ribofuranosylbenzimidazole or 5,7-dimethyl-2-β-D-ribofuranosyl-s-triazole(1,5-a)pyrimidine.

12. A process for the preparation of a compound of the formula 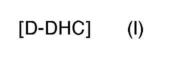 or a non-toxic pharmaceutically acceptable salt thereof, wherein [D] is a centrally acting drug species which is a steroidal progestin or steroidal estrogen, said progestin or estrogen having at least one reactive hydroxyl functional group, one such group being a tertiary hydroxyl functional group in the 17- position; and [DHC] is the reduced, biooxidizable, blood-brain barrier penetrating, lipoidal form of a dihydropyridine ⇆ pyridinium salt redox carrier comprising at least one dihydropyridine ring system of the formula 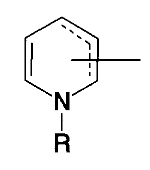 wherein R is lower alkyl or benzyl and the dotted line indicates the presence of a double bond in either the 2 or 3 position of the dihydropyridine ring, a ring carbon atom of one dihydropyridine ring system being connected via a bridging group to the tertiary 17-hydroxyl functional group in the progestin or estrogen, a ring carbon atom of any remaining dihydropyridine ring system being connected via a bridging group to another reactive hydroxyl functional group in said progestin or estrogen; said process being characterized by reducing the corresponding compound of the formula 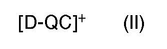 wherein [D] is defined above, and [QC]⁺ is the hydrophilic, ionic pyridinium salt form of a dihydropyridine == pyridinium salt redox carrier comprising at least one pyridinium ring system of the formula 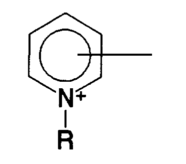 wherein R is lower alkyl or benzyl, a ring carbon atom of one pyridinium ring system being connected via a bridging group to the tertiary 17-hydroxyl functional group in the progestin or estrogen, a ring carbon atom of any remaining pyridinium ring system being connected via a bridging group to another reactive hydroxyl functional group in said progestin or estrogen.

13. A process according to any one of Claims 4, 9, and 12, wherein each pyridinium ring system, together with the bridging group connected thereto, comprises the formula 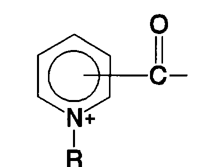 wherein R is lower alkyl or benzyl, particularly when R is methyl or when the carbonyl group is attached at the 3 position of the pyridinium ring.

14. A process according to Claim 13, wherein each pyridinium ring system, together with the bridging group connected thereto, has the formula

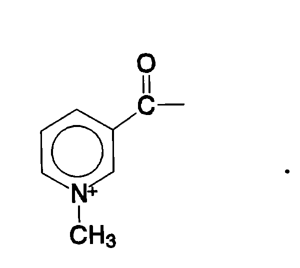

15. A process according to Claim 13, wherein each pyridinium ring system, together with the bridging group connected thereto, has the formula

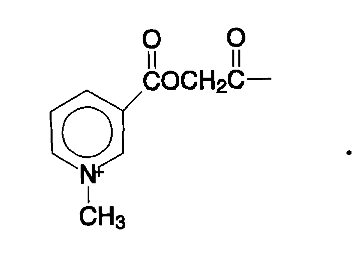

16. A process according to Claim 12, wherein [D] is mestranol, quinestrol, norgestrel, norethindrone, ethisterone, dimethisterone, allylestrenol, cingestol, ethynerone, lynestrenol, norgesterone, norvinisterone, ethynodiol or tigestol.

17. A process for the preparation of a compound of the formula 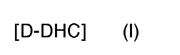 or a non-toxic pharmaceutically acceptable salt thereof, wherein [D] is a centrally acting drug species containing at least one reactive -COOH, amide or imide functional group, and [DHC] is the reduced, biooxidizable, blood-brain barrier penetrating lipoidal form of a dihydropyridine ⇆ pyridinium salt redox carrier comprising a dihydropyridine ring system of the formula 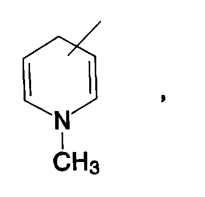 a ring carbon atom of the dihydropyridine ring system being connected via a bridging group to a reactive -COOH, amide or imide functional group in the centrally acting drug species; said process being characterized by reducing the corresponding compound of the formula 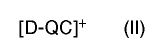 wherein [D] is defined above, and [QC]⁺ is the hydrophilic, ionic pyridinium salt form of a dihydropyridine ⇆ pyridinium salt redox carrier comprising a pyridinium ring system of the formula 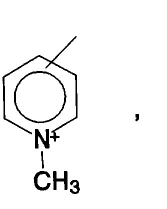 a ring carbon atom of the pyridinium ring system being connected via a bridging group to a reactive -COOH, amide or imide functional group in the centrally acting drug species.

18. A process according to Claim 17, wherein the centrally acting drug species [D] contains at least one reactive amide or imide group and the pyridinium ring system together with the bridging group has the structural formula 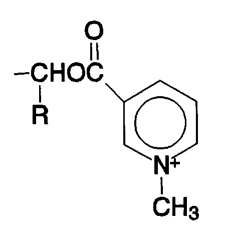 wherein R is -CC!₃, -H, 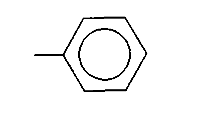 or -CH₃.

19. A process according to Claim 17, wherein the centrally acting drug species [D] contains at least one reactive amide or imide group and the pyridinium ring system together with the bridging group has the structural formula 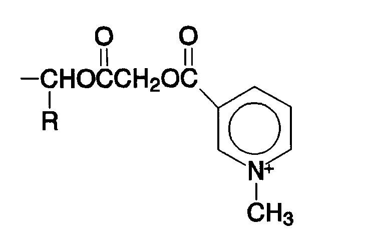 wherein R is -CCl₃, -H, 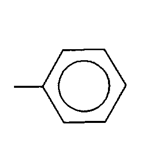 or -CH₃.

20. A process according to Claim 17, wherein the centrally acting drug species [D] contains at least one reactive -COOH group and the bridging group connecting the pyridinium ring system to the drug is derived from a di- or polyhydroxy compound, at least one hydroxy function of which is attached to a reactive -COOH group in the drug and at least one other hydroxy function of which is attached via a carbonyl function to the pyridinium ring system.

21. A process according to Claim 20, wherein the bridging group is derived from ethylene glycol, propylene glycol, inositol or a sugar.

22. A process according to Claim 17, wherein [D] is a central neurotransmitter, an anticancer or antitumor agent, an antiviral agent, a tranquilizer, a sedative or hypnotic, an anticonvulsant or antiepileptic agent, a dopaminergic agent or an antibiotic or antibacterial agent.

23. A process according to Claim 17, wherein [D] is an amino acid or a small peptide containing 2 to 20 amino acid units.

24. A process according to Claim 17, wherein [D] is GABA, glycine, glutamic acid, aspartic acid, neurotensin, LHRH, an enkephalin, an endorphin, oxytocin M, vasopressin, 3-deazaguanine, tiazofurin, sangivamycin, PCNU, spiromustine, L-alanosine, DON, L-ICRF, 5-methyl tetrahydrohomofolic acid, glyoxylic acid sulfonylhydrazine, DACH, SR-2555, SR-2508, bactobolin, acivicin, hydroxyurea, chlorambucil, uracil mustard, melphalan, 5-FUDR, methotrexate, aminopterin, mitomycin C, ribavirin, acyclovir, 6-azauridine, oxazepam, lorazepam, chlordiazepoxide, bromazepam, clorazepate, nitrazepam, phenytoin, ethotoin, mephenytoin, methyldopa, y-vinyl GABA, y-acetylenic GABA, phenobarbital, amobarbital, butalbital, glutethimide, aminoglutethimide, methyprylon, valproic acid, 5-hydroxy-2-n-propylpentanoic acid, 4-hydroxy-2-n-propylpentanoic acid, 3-hydroxy-2-n-propylpentanoic acid, bemegride, tryptophan, levodopa, ampicillin, amoxicillin, oxacillin, carbenicillin, dicloxacillin, chlortetracycline, tetracycline, methacycline, nalidixic acid, cephalexin, cephalothin, cefoxitin, clindamycin, lincomycin or oxolinic acid.

25. A process for the preparation of a compound of the formula 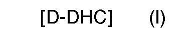 or a non-toxic pharmaceutically acceptable salt thereof, wherein [D] is a centrally acting drug species containing at least one reactive -OH, -COOH, -SH, -NH- or -NH₂ group (including groups such as amides and imides, as well as primary and secondary amino groups), and [DHC] is the reduced, biooxidizable, blood-brain barrier penetrating lipoidal form of a dihydropyridine == pyridinium salt redox carrier comprising a dihydropyridine ring system of the formula 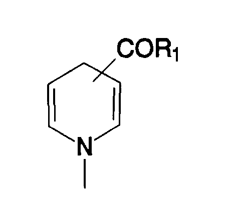 wherein R is -NH₂ or -OR₂ wherein R₂ is alkyl, the dihydropyridine ring optionally being fused to a benzene ring, the nitrogen atom of the dihydropyridine ring system being connected via a bridging group to a reactive -OH, -COOH, -SH, -NH- or -NH₂ group in the centrally acting drug species; said process being characterized by reducing the corresponding compound of the formula 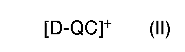 wherein [D] is defined above, and [QC]⁺ is the hydrophilic, ionic pyridinium salt form of a dihydropyridine ⇆ pyridinium salt redox carrier comprising a pyridinium ring system of the formula 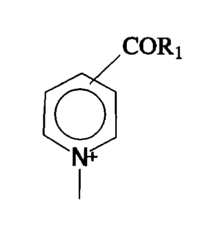 wherein R is -NH₂ or -OR₂ wherein R₂ is alkyl, the pyridinium ring optionally being fused to a benzene ring, the nitrogen atom of the pyridinium ring system being connected via a bridging group to a reactive -OH, -COOH, -SH, -NH- or -NH₂ group in the centrally acting drug species.

26. A process according to Claim 25, wherein the centrally acting drug species [D] contains at least one reactive primary or secondary amino group or at least one reactive -OH group, and the pyridinium ring system together with the bridging group connecting the pyridinium ring system to the amino or -OH group in [D] has the structural formula 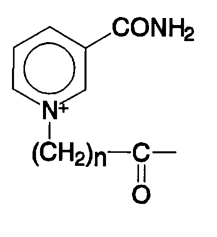 wherein n is 1, 2, or 3.

27. A process according to Claim 25, wherein the centrally acting drug species [D] contains at least one reactive primary or secondary amino group or at least one reactive -OH group, and the pyridinium ring system together with the bridging group connecting the pyridinium ring system to the amino or -OH group in [D] has the structural formula 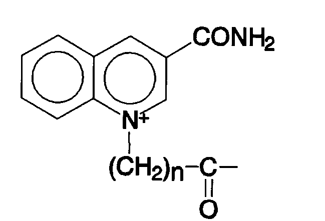 wherein n is 1, 2, or 3.

28. A process according to Claim 25, wherein the centrally acting drug species [D] contains at least one reactive -NH- or -NH₂ group which is part of an amide or imide structure or which is a low pKa primary or secondary amino group, and the pyridinium ring system together with the bridging group connecting the pyridinium ring system to the -NH- or -NH₂ group in [D] has the structural formula 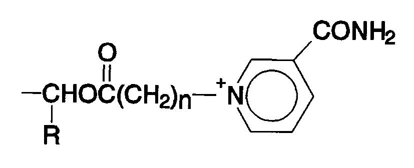 wherein R is -CC!₃, -H, 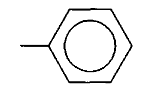 or -CH₃ and n is 1, 2 or 3.

29. A process according to Claim 25, wherein the centrally acting drug species [D] contains at least one reactive -COOH group, and the pyridinium ring system together with the bridging group connecting the pyridinium ring system to the -COOH group in [D] has the structural formula 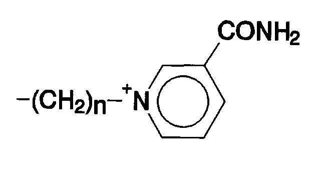 wherein n is 1, 2 or 3.

30. A process for the preparation of a compound of the formula 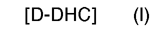 or a non-toxic pharmaceutically acceptable salt thereof, wherein [D] is a centrally acting drug species containing at least one reactive -OH, -COOH, -SH, -NH- or -NH₂ group (including groups such as amides and imides, as well as primary and secondary amino groups), and [DHC] is the reduced, biooxidizable, blood-brain barrier penetrating lipoidal form of a dihydropyridine ⇆ pyridinium salt redox carrier comprising a dihydroquinoline or dihydroisoquinoline ring system, a carbon atom of the nitrogen-containing ring portion of said ring system being connected via a bridging group to a reactive -OH, -COOH, -SH, -NH- or -NH₂ group in the centrally acting drug species; said process being characterized by reducing the corresponding compound of the formula 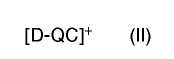 wherein [D] is defined above, and [QC]⁺ is the hydrophilic, ionic pyridinium salt form of a dihydropyridine == pyridinium salt redox carrier comprising a quinolinium or isoquinolinium ring system, a carbon atom of the nitrogen-containing ring portion of said ring system being connected via a bridging group to a reactive -OH, -COOH, -SH, -NH- or -NH₂ group in the centrally acting drug species.

31. A process according to Claim 30, wherein the centrally acting drug species [D] contains at least one reactive primary or secondary amino group or at least one reactive -OH group, and the quinolinium or isoquinolinium ring system together with the bridging group connecting the ring system to the amino or -OH group in [D] has the structural formula 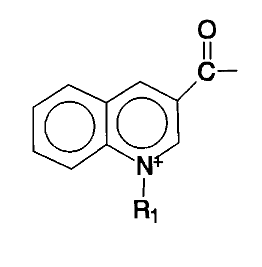 wherein R is C₁-C₇ alkyl or C₇-C₁₀ aralkyl, particularly when R is methyl.

32. A process according to Claim 30, wherein the centrally acting drug species [D] contains at least one reactive primary or secondary amino group or at least one reactive -OH group, and the quinolinium or isoquinolinium ring system together with the bridging group connecting the ring system to the amino or -OH group in [D] has the structural formula 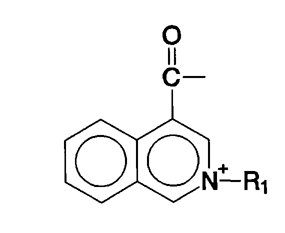 wherein R₁ is C₁-C₇ alkyl or C₇-C₁₀ aralkyl, particularly when R₁ is methyl.

33. A process according to Claim 30, wherein the centrally acting drug species [D] contains at least one reactive -NH- or -NH₂ group which is part of an amide or imide structure or which is a low pKa primary or secondary amino group, the redox carrier [QC]⁺ comprises a quinolinium ring system and the bridging group has the structure 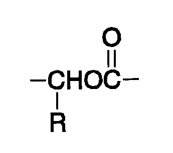 wherein R is -CCl₃, -H, 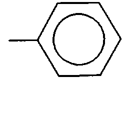 or -CH₃, the carbonyl function of the bridging group being connected to a carbon atom of the nitrogen-containing ring portion of the quinolinium ring system.

34. A process according to Claim 25 or 30, wherein [D] is a central neurotransmitter, an antiinflammatory steroid, a steroid sex hormone, an anticancer or antitumor agent, an antiviral agent, a tranquilizer, a memory enhancer, a hypotensive agent, a sedative or hypnotic, an anticonvulsant or antiepileptic agent, a stimulant, a narcotic analgesic or antagonist, a dopaminergic agent or an antibiotic or antibacterial agent.

35. A process according to Claim 25 or 30, wherein [D] is an amino acid or a small peptide containing 2 to 20 amino acid units.

36. A process according to Claim 25 or 30, wherein [D] is a dopamine having the structural formula 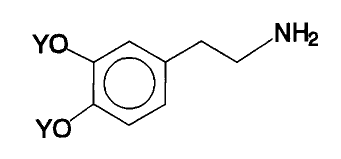 in which each Y is independently hydrogen or a hydrolytically or metabolically cleavable hydroxyl protective group.

37. A process according to Claim 25 or 30, wherein [D] is GABA, glycine, glutamic acid, aspartic acid, dopamine, norephinephrine, epinephrine, serotonin, tryptamine, neurotensin, LHRH, somatostatin, an enkephalin, an endorphin, oxytocin M, vasopressin, hydrocortisone, betamethasone, dexamethasone, cortisone, flumethasone, fluprednisolone, meprednisone, methyl prednisolone, prednisolone, prednisone, triamcinolone, cortodoxone, fludrocortisone, fluandrenolone acetonide, paramethasone, testosterone, methyl testosterone, ethinyl estradiol, norgestrel, mestranol, norethindrone, ethisterone, estradiol, estriol, estrone, dimethisterone, allylestrenol, cingestol, ethynerone, lynestrenol, norgesterone, norvininsterone, ethynodiol, oxogestone, tigestol, quinestrol, Ara-AC, pentostatin, Ara-C, 3- deazaguanine, dihydro-5-azacytidine, tiazofurin, sangivamycin, Ara-A, 6-MMPR, PCNU, spiromustine, bisbenzimidazole, L-alanosine, DON, L-ICRF, trimethyl TMM, 5-methyl tetrahydrohomofolic acid, glyoxylic acid sulfonylhydrazone, DACH, SR-2555, SR-2508, desmethylmisonidazole, mitoxantrone, menogarol, aclacinomycin A, phyllanthoside, bactobolin, aphidocolin, homoharringtonine, levonantradol, acivicin, streptozotocin, hydroxyurea, chlorambucil, cyclophosphamide, uracil mustard, melphalan, 5-FUDR, cytosine arabinoside, 6-mercaptopurine, thioguanine, 5-azacytidine, methotrexate, doxorubicin, daunomycin, aminopterin, dactinomycin, mitomycin C, a podophyllotoxin derivative, ribavirin, acyclovir, amantadine, 5-amidino-2-(5-amidino-2-benzofuranyl)indole, 4',6-diimidazolino-2-phenylbenzo(b)-thiophene, 2-guanidino-4,5-di-n-propyloxazole, 2-guanidino-4,5-diphenyloxazole, 6[[(hydroxyimino)-phenyl]methyl]-1-[(1-methylethyl)sulfonyl]-1H-benzimidazol-2-amine,5,7-dimethyl-2-β-D-ribofuranosyl-s-triazole(1,5-a)pyrimidine, 2-deoxy-D-glucose, glucosamine, 2-deoxy-2-f!uoro-D-mannose, 6-amino-6-deoxy-D-glucose, phenyl-6-chloro-6-deoxy-β-D-glucopyranoside, (S)-9-(2,3-dihydroxypropyl)adenine, 6- azauridine, 5,6-dichloro-1-β-D-ribofuranosylbenzimidazole, diazepam, oxazepam, lorazepam, chlordiazepoxide, flurazepam, bromazepam, clorazepate, nitrazepam, temazepam, phenytoin, ethotoin, mephenytoin, acetophenazine, carphenazine, fluphenazine, perphenazine, piperacetazine, clonidine, methyldopa, bethanidine, debrisoquin, hydralazine, guanethidine, haloperidol, hydroxyzine, clopenthixol, y-vinyl GABA, y-acetylenic GABA, phenobarbital, amobarbital, butalbital, glutethimide, aminoglutethimide, methyprylon, valproic acid, 5-hydroxy-2-n-propylpentanoic acid, 4-hydroxy-2-n-pro- pylpentanoic acid, 3-hydroxy-2-n-propylpentanoic acid, methamphetamine, phentermine, phenmetrazine, dextroamphetamine, levamphetamine, amphetamine, phenylethylamine, methylphenidate, tranylcypromine, protriptyline, opipramol, desipramine, nortriptyline, bemegride, amiphenazole, anileridine, hydromorphone, oxymorphone, apomorphine, levorphanol, morphine, pentazocine, codeine, oxycodone, naloxone, nalorphine, tryptophan, tryamine, levodopa, ampicillin, amoxicillin, oxacillin, carbenicillin, dicloxacillin, chlortetracycline, tetracycline, methacycline, nalidixic acid, cephalexin, cephalothin, cefoxitin, clindamycin, lincomycin, oxolinic acid, phenazopyridine, sulfadiazine, thiopental, benzestrol or diethylstilbestrol.

38. A process according to any one of the preceding claims, wherein an alkali metal dithionite is used as the reducing agent.

39. A process according to Claim 38, wherein the alkali metal dithionite is sodium dithionite.

40. A process according to any one of Claims 1-37, wherein an alkali metal borohydride is used as the reducing agent.

41. A process according to Claim 40, wherein the alkali metal borohydride is sodium borohydride.

## Patentansprüche (Patentansprüche für folgende(n) Vertragsstaat(en) : BE, CH, DE, FR, GB, LI, LU, NL, SE)

1. Verbindung, die für eine ortsspezifische Langzeitabgabe einer zentral wirkenden Arzneimittelspezies an das Hirn angepaßt ist, welche Verbindung eine Verbindung der Formel 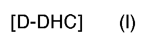 oder ein nicht-toxisches, pharmazeutisch akzeptables Salz davon ist, worin [D] eine zentral wirkende Arzneimittelspezies ist, welche mindestens eine reaktive OH-, COOH-, SH-, NH- oder NH₂-Gruppe (einschließlich Gruppen, wie z.B. Amide und Imide, sowie primäre und sekundäre Aminogruppen) enthält, und [DHC] die reduzierte, biooxydierbare, die Blut/Hirn-Schranke durchdringende, lipoide Form eines Dihydropyridin(=>Pyridiniumsalz-Redoxträgers ist, mit der Maßgabe, daß, wenn [DHC] 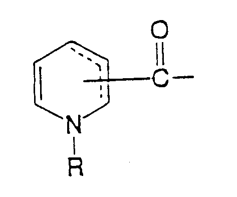 ist, worin R ein niederes Alkyl oder Benzyl ist, die gepunktete Linie das Vorhandensein einer Doppelbindung an entweder der Position 2 oder 3 des Dihydropyridinringes anzeigt und die Carbonylgruppe an der Position 2, 3 oder 4 des Dihydropyridinringes angebracht ist; und [D] eine Arzneimittelspezies ist, welche eine einzelne funktionelle Amino- oder OH-Gruppe enthält, wobei die einzelne Amino- oder OH-Gruppe, wenn sie vorhanden ist, eine primäre oder sekundäre Amino- oder eine primäre oder sekundäre OH-Gruppe ist, welche Arzneimittelspezies über die funktionelle Amino- oder OH-Gruppe direkt an die Carbonylfunktion von [DHC] gebunden ist, dann [D] etwas anderes sein muß als ein sympathisches Stimulans, ein Steroidgeschlechtshormon, ein Gedächtnisverstärker, ein langkettiger Alkanol oder ein Antikrebs- oder Antitumormittel.

2. Verbindung der Formel 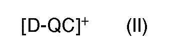 worin [D] eine zentral wirkende Arzneimittelspezies ist, welche mindestens eine reaktive OH-, COOH-, SH-, NH- oder NH₂-Gruppe (einschließlich Gruppen, wie z.B. Amide oder Imide, sowie primäre und sekundäre Amidogruppen) enthält, und [QC]⁺ die hydrophile, ionische Pyridiniumsalzform eines Dihydropyridin⇔Pyridiniumsalz-Redoxträgers ist, mit der Maßgabe, daß, wenn [QC]⁺ 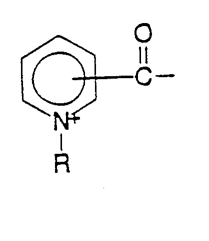 ist, worin R ein niederes Alkyl oder Benzyl ist und die Carbonylgruppe an der Position 2, 3 oder 4 des Pyridiniumringes angebracht ist, und [D] eine Arzneimittelspezies ist, welche eine einzelne funktionelle Amino- oder OH-Gruppe enthält, wobei die einzelne Amino- oder OH-Gruppe, wenn sie vorhanden ist, eine primäre oder sekundäre Amino- oder eine primäre oder sekundäre OH-Gruppe ist, welche Arzneimittelspezies über die funktionelle Amino- oder OH-Gruppe direkt an die Carbonylfunktion von [QC]⁺ gebunden ist, dann [D] etwas anderes sein muß als ein sympathisches Stimulans, ein Steroidgeschlechtshormon, ein Gedächtnisverstärker, ein langkettiger Alkanol oder ein Antikrebs- oder Antitumormittel.

3. Verbindung, die für eine ortsspezifische Langzeitabgabe einer zentral wirkenden Arzneimittelspezies an das Hirn angepaßt ist, welche Verbindung eine Verbindung der Formel 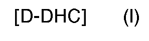 oder ein nicht-toxisches, pharmazeutisch akzeptables Salz davon ist, worin [D] eine zentral wirkende Arzneimittelspezies ist, welche Arzneimittelspezies Glycin, Glutaminsäure, Asparaginsäure, Tryptophan, ein Enkephalin, ein Endorphin oder eine andere Aminosäure oder ein kleines Peptid, welches 2 bis 20 Aminosäureeinheiten enthält; GABA, y-Vinyl-GABA oder y-acetylenisches GABA; Norepinephrin oder Epinephrin; Serotonin; 5-Hydroxy-2-n-propylpentansäure, 4-Hydroxy-2-n-propylpentansäure oder 3-Hydroxy-2-n-propylpentansäure; Methyldopa; Tyramin; Dopamin oder Levodopa; Ampicillin; Cephalexin; L-Alanosin; Melphalan; DON, oder Acivicin; oder Glucosamin, oder 6-Amino-6-deoxy-D-glucose ist; und [DHC] die reduzierte, biooxydierbare, die Blut/Hirn-Schranke durchdringende; lipoide Form eines Dihydropyridin(=>Pyridiniumsalz-Redoxträgers mit der Formel 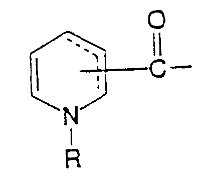 ist, worin R Methyl oder Benzyl ist und die gepunktete Linie das Vorhandensein einer Doppelbindung an entweder der Position 2 oder 3 des Dihydropyridinringes anzeigt, wobei die Carbonylfunktion, die dem Dihydropyridinring benachbart ist, an eine funktionelle reaktive Amino- oder Hydroxylgruppe in der zentral wirkenden Arzneimittelspezies gebunden ist und mindestens eine der verbleibenden reaktiven funktionellen Gruppen in der zentral wirkenden Arzneimittelspezies durch eine hydrolytisch oder metabolisch abspaltbare Hydroxyl- oder Carboxylschutzgruppe geschützt wird.

4. Verbindung der Formel 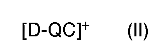 worin [D] eine zentral wirkende Arzneimittelspezies, wie in Anspruch 3 definiert, ist, und [QC]⁺ die hydrophile, ionische Pyridiniumsalzform eines Dihydropyridin⇔Pyridiniumsalz-Redoxträgers mit der Formel 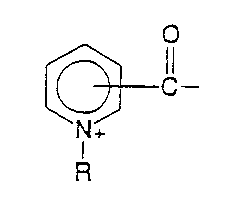 ist, worin R Methyl oder Benzyl ist, wobei die Carbonylfunktion, die dem Pyridiniumring benachbart ist, an eine funktionelle reaktive Amino- oder Hydroxylgruppe in der zentral wirkenden Arzneimittelspezies gebunden ist, und mindestens eine der verbleibenden reaktiven funktionellen Gruppen in der zentral wirkenden Arzneimittelspezies durch eine hydrolytisch oder metabolisch abspaltbare Hydroxyl- oder Carboxylschutzgruppe geschützt wird.

5. Verbindung nach Anspruch 3 oder 4, wobei die Arzneimittelspezies ein Dopamin mit der Strukturformel 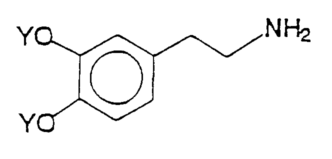 ist, worin jedes Y unabhängig Wasserstoff oder eine hydrolytisch oder metabolisch abspaltbare Hydroxylschutzgruppe ist, mit der Maßgabe, daß mindestens ein Y eine Acylgruppe oder eine Carbonatgruppe ist, worin bevorzugt: ein Y Pivalyl und das andere Y Wasserstoff oder Pivalyl ist; ein Y Isobutyryl und das andere Y Wasserstoff oder Isobutyryl ist; ein Y Ethoxycarbonyl und das andere Y Wasserstoff oder Ethoxycarbonyl ist; oder ein Y Isopropoxycarbonyl und das andere Y Wasserstoff oder Isopropoxycarbonyl ist.

6. Verbindung, die für eine ortsspezifische Langzeitabgabe einer zentral wirkenden Arzneimittelspezies an das Hirn angepaßt ist, welche Verbindung eine Verbindung der Formel 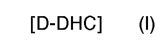 oder ein nicht-toxisches, pharmazeutisch akzeptables Salz davon ist, worin [D] eine zentral wirkende Arzneimittelspezies ist, welche ein Antivirusmittel, ein entzündungshemmendes Steroid, ein Narkotikum oder ein Östrogen ist und welche mindestens zwei reaktive funktionelle Hydroxylgruppen enthält, und [DHC] die reduzierte, biooxydierbare, die Blut/Hirn-Schranke durchdringende, lipoide Form eines Dihydropyridin(=>Pyridiniumsalz-Redoxträgers ist, welcher mindestens ein Dihydropyridinringsystem der Formel 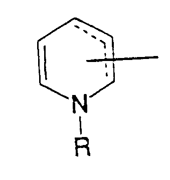 umfaßt, worin R ein niederes Alkyl oder Benzyl ist und die gepunktete Linie das Vorhandensein einer Doppelbindung an entweder der Position 2 oder 3 des Dihydropyridinringes anzeigt, wobei ein Ringkohlenstoffatom jedes Dihydropyridinringsystems über eine Brückengruppe an eine funktionelle reaktive Hydroxylgruppe in der zentral wirkenden Arzneimittelspezies gebunden ist.

7. Verbindung der Formel 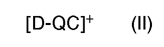 worin [D] eine zentral wirkende Arzneimittelspezies ist, welche ein Antivirusmittel, ein entzündungshemmendes Steroid, ein Narkotikum oder ein Östrogen ist und welche mindestens zwei reaktive funktionelle Hydroxylgruppen enthält, und [QC]⁺ die hydrophile, ionische Pyridiniumsalzform eines Dihydropyridin⇔Pyridiniumsalz-Redoxträgers ist, welcher mindestens ein Pyridiniumringsystem der Formel 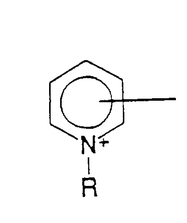 umfaßt, worin R ein niederes Alkyl oder Benzyl ist, wobei ein Ringkohlenstoffatom jedes Pyridiniumringsystems über eine Brückengruppe an eine reaktive funktionelle Hydroxylgruppe in der zentral wirkenden Arzneimittelspezies gebunden ist.

8. Verbindung nach Anspruch 6 oder 7, worin [D] ein Antivirusmittel ist.

9. Verbindung nach Anspruch 6 oder 7, worin [D] ein entzündungshemmendes Steroid ist.

10. Verbindung nach Anspruch 6 oder 7, worin [D] ein Östrogen ist.

11. Verbindung nach Anspruch 6 oder 7, worin [D] ist: Hydrocortison, Betamethason, Dexamethason, Cortison, Flumethason, Fluprednisolon, Meprednison, Methylprednisolon, Prednisolon, Prednison, Triamcinolon, Cortodoxon, Fludrocortison, Fluandrenolonacetonid, Paramethason, Ethinylöstradiol, Östradiol, Östriol, Ara-AC, Pentostatin, Dihydro-5-azacytidin, Tiazofurin, Sangivamycin, Ara-A, 6-MMPR, 5-FUDR, Cytosinarabinosid, 5-Azacytidin, Ribavirin, 5,7-Dimethyl-2-β-D-ribofuranosyl-s-triazol(1,5-a)-pyrimidin, 2-Deoxy-D-glucose, Glucosamin, 2-Deoxy-2-fluor-D-mannose, 6-Amino-6-deoxy-D-glucose, Phenyl-6-chlor-6-deoxy-ß-D-glucopyranosid, (S)-9-(2,3-Dihydroxypropyl)adenin, 6-Azauridin, 5,6-Dichlor-1-β-D-ribofuranosylbenzimidazol, Oxymorphon, Apomorphin, Morphin, Oxycodon, Naloxon, Nalorphin, Benzöstrol oder Diethylstilböstrol.

12. Verbindung, die für eine ortsspezifische Langzeitabgabe einer zentral wirkenden Arzneimittelspezies an das Hirn angepaßt ist, welche Verbindung eine Verbindung der Formel 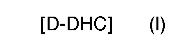 oder ein nicht-toxisches, pharmazeutisch akzeptables Salz davon ist, worin [D] eine zentral wirkende Arzneimittelspezies ist, welche ein Antikrebs/Antitumor- und/oder ein Antivirusmittel ist, welche Arzneimittelspezies eine Struktur vom Typus einer Purin- oder Pyrimidinbase besitzt, welche einen einfach oder mehrfach hydroxylierten Substituenten trägt, und [DHC] die reduzierte, biooxydierbare, die Blut/Hirn-Schranke durchdringende, lipoide Form eines Dihydropyridin(=>Pyridiniumsalz-Redoxträgers ist, welcher mindestens ein Dihydropyridinringsystem der Formel 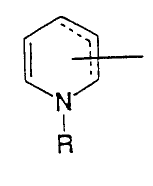 umfaßt, worin R ein niederes Alkyl oder Benzyl ist und die gepunktete Linie das Vorhandensein einer Doppelbindung an entweder der Position 2 oder 3 des Dihydropyridinringes anzeigt, wobei ein Ringkohlenstoffatom jedes Dihydropyridinringsystems über eine Brückengruppe an eine funktionelle Hydroxylgruppe in der zentral wirkenden Arzneimittelspezies gebunden ist.

13. Verbindung der Formel 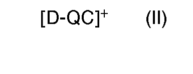 worin [D] eine zentral wirkende Arzneimittelspezies ist, welche ein Antikrebs/Antitumor- und/oder ein Antivirusmittel ist, welche Arzneimittelspezies eine Struktur vom Typus einer Purin- oder Pyrimidinbase besitzt, welche einen einfach oder mehrfach hydroxylierten Substituenten trägt, und [QC]⁺ die hydrophile, ionische Pyridiniumsalzform eines Dihydropyridin⇔Pyridiniumsalz-Redoxträgers ist, der mindestens ein Pyridiniumringsystem der Formel 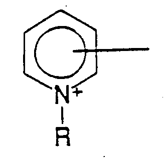 umfaßt, worin R ein niederes Alkyl oder Benzyl ist, wobei ein Ringkohlenstoffatom jedes Pyridiniumringsystems über eine Brückengruppe an eine reaktive funktionelle Hydroxylgruppe in der zentral wirkenden Arzneimittelspezies gebunden ist.

14. Verbindung nach Anspruch 12 oder 13, worin [D] ein Antivirusmittel ist.

15. Verbindung nach Anspruch 12 oder 13, worin [D] ist: Ara-AC, Pentostatin, Ara-C, Dihydro-5-azacytidin, Tiazofurin, Sangivamycin, Ara-A, 6-MMPR, Desmethylmisonidazol, 5-FUDR, Cytosinarabinosid, 5-Azacytidin, Ribavirin, Acyclovir, (S)-9-(2,3-Dihydroxypropyl)adenin, 6-Azauridin, 5,6-Dichlor-1-ß-_D-ribofuran- osylbenzimidazol oder 5,7-Dimethyl-2-ß-D-ribofuranosyl-s-triazol(1,5-a)pyrimidin.

16. Verbindung, die für eine ortsspezifische Langzeitabgabe einer zentral wirkenden Arzneimittelspezies an das Hirn angepaßt ist, welche Verbindung eine Verbindung der Formel 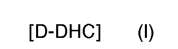 oder ein nicht-toxisches, pharmazeutisch akzeptables Salz davon ist, worin [D] eine zentral wirkende Arzneimittelspezies ist, welche ein Steroidprogestin oder ein Steroidöstrogen ist, welches Progestin oder Östrogen mindestens eine reaktive funktionelle Hydroxylgruppe besitzt, wobei eine solche Gruppe eine funktionelle tertiäre Hydroxylgruppe in der Position 17 ist; und [DHC] die reduzierte, biooxydierbare, die Blut/Hirn-Schranke durchdringende, lipoide Form eines Dihydropyridin⇔Pyridiniumsalz-Redoxträgers ist, der mindestens ein Dihydropyridinringsystem der Formel 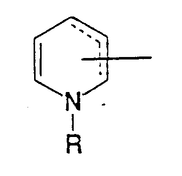 umfaßt worin R ein niederes Alkyl oder Benzyl ist und die gepunktete Linie das Vorhandensein einer Doppelbindung an entweder der Position 2 oder 3 des Dihydropyridinringes anzeigt, wobei ein Ringkohlenstoffatom eines Dihydropyridinringsystems über eine Brückengruppe an die tertiäre funktionelle 17-Hydroxylgruppe im Progestin oder Östrogen gebunden ist, wobei ein Ringkohlenstoffatom eines verbleibenden Dihydropyridinringsystems über eine Brückengruppe mit einer anderen reaktiven funktionellen Hydroxylgruppe im Progestin oder Östrogen verbunden ist.

17. Verbindung nach einem der Ansprüche 6, 12 und 16, worin jedes Dihydropyridinringsystem, zusammen mit der Brückengruppe, die daran gebunden ist, die Formel 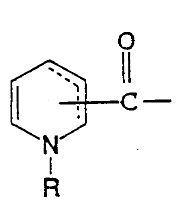 aufweist, worin R ein niederes Alkyl oder Benzyl ist und die gepunktete Linie das Vorhandensein einer Doppelbindung an entweder der Position 2 oder 3 des Dihydropyridinringes anzeigt; insbesondere, wenn die gepunktete Linie das Vorhandensein einer Doppelbindung in der Position 2 des Dihydropyridinrings anzeigt, oder wenn R Methyl ist, oder wenn die Carbonylgruppe an der Position 3 des Dihydropyridinringes angebracht ist.

18. Verbindung nach Anspruch 17, worin jedes Dihydropyridinringsystem, zusammen mit der Brückengruppe, die daran gebunden ist, die Formel 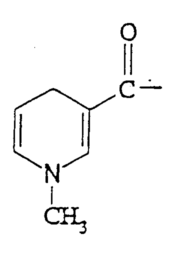 besitzt.

19. Verbindung nach Anspruch 17, worin jedes Dihydropyridinringsystem, zusammen mit der Brückengruppe, die daran gebunden ist, die Formel 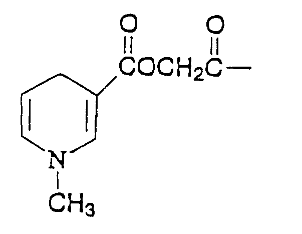 aufweist.

20. Verbindung der Formel 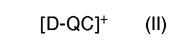 worin [D] eine zentral wirkende Arzneimittelspezies ist, welche ein Steroidprogestin oder ein Steroidöstrogen ist, welches Progestin oder Östrogen mindestens eine reaktive funktionelle Hydroxylgruppe besitzt, wobei eine solche Gruppe eine funktionelle tertiäre Hydroxylgruppe in der Position 17 trägt; und [QC]⁺ die hydrophile, ionische Pyridiniumsalzform eines Dihydropyridin⇒ Pyridiniumsalz-Redoxträgers ist, der mindestens ein Pyridiniumringsystem der Formel 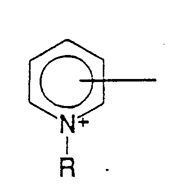 umfaßt, worin R ein niederes Alkyl oder Benzyl ist, wobei ein Ringekohlenstoffatom eines Pyridiniumringsystems über eine Brückengruppe an die funktionelle tertiäre 17-Hydroxylgruppe im Progestin oder Östrogen gebunden ist, und ein Ringkohlenstoffatom eines verbleibenden Pyridiniumringsystems über eine Brückengruppe an eine andere reaktive funktionelle Hydroxylgruppe im Progestin oder Östrogen gebunden ist.

21. Verbindung nach einem der Ansprüche 7, 13 und 20, worin jedes Pyridiniumringsystem, zusammen mit der Brückengruppe, die daran gebunden ist, die Formel 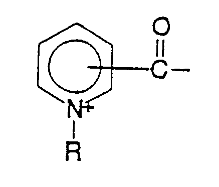 umfaßt, worin R ein niederes Alkyl oder Benzyl ist, insbesondere wenn R Methyl ist oder wenn die Carbonylgruppe an der Position 3 des Pyridiniumringes angebracht ist.

22. Verbindung nach Anspruch 21, worin jedes Pyridiniumringsystem, zusammen mit der Brückengruppe, die daran gebunden ist, die Formel 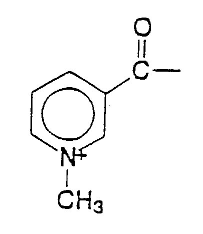 besitzt.

23. Verbindung nach Anspruch 21, worin jedes Pyridiniumringsystem, zusammen mit der Brückengruppe, die daran gebunden ist, die Formel besitzt.

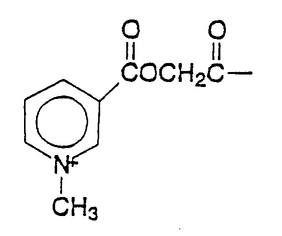

24. Verbindung nach Anspruch 16 oder 20, worin [D] ist: Mestranol, Chinöstrol, Norgestrel, Norethindron, Ethisteron, Dimethisteron, Allylöstrenol, Cingestol, Ethyneron, Lynöstrenol, Norgesteron, Norvinisteron, Ethynodiol oder Tigestol.

25. Verbindung, die für eine ortsspezifische Langzeitabgabe einer zentral wirkenden Arzneimittelspezies an das Hirn angepaßt ist, welche Verbindung eine Verbindung der Formel 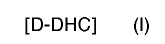 oder ein nicht-toxisches, pharmazeutisch akzeptables Salz davon ist, worin [D] eine zentral wirkende Arzneimittelspezies ist, welche mindestens eine reaktive funktionelle COOH-, Amid- oder Imidgruppe enthält, und [DHC] die reduzierte, biooxydierbare, die Blut/Hirn-Schranke durchdringende, lipoide Form eines Dihydropyridin⇔Pyridiniumsalz-Redoxträgers ist, der ein Dihydropyridinringsystem der Formel 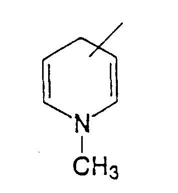 umfaßt, wobei ein Ringkohlenstoffatom des Dihydropyridinringsystems über eine Brückengruppe an eine reaktive funktionelle COOH-, Amid- oder Imidgruppe in der zentral wirkenden Arzneimittelspezies gebunden ist.

26. Verbindung nach Anspruch 25, worin die zentral wirkende Arzneimittelspezies [D] mindestens eine reaktive Amid- oder Imidgruppe enthält, und das Dihydropyridinringsystem zusammen mit der Brückengruppe die Strukturformel 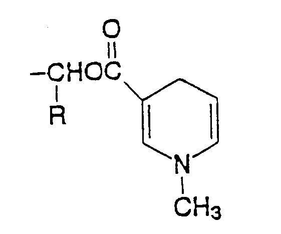 besitzt, worin R -CCI₃, -H, 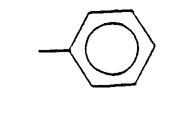 oder -CH₃ ist.

27. Verbindung nach Anspruch 25, worin die zentral wirkende Arzneimittelspezies [D] mindestens eine reaktive Amid- oder Imidgruppe enthält, und das Dihydropyridinringsystem zusammen mit der Brückengruppe die Strukturformel 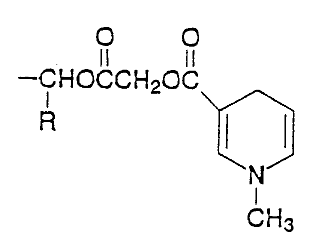 besitzt, worin R -CCI₃, -H, 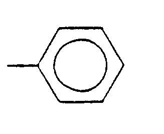 oder -CH₃ ist.

28. Verbindung nach Anspruch 25, worin die zentral wirkende Arzneimittelspezies [D] mindestens eine reaktive COOH-Gruppe enthält und die Brückengruppe, die das Dihydropyridinringsystem mit dem Arzneimittel verbindet, von einer Di- oder Polyhydroxyverbindung abgeleitet ist, von welcher mindestens eine Hydroxyfunktion an einer reaktiven COOH-Gruppe im Arzneimittel angebracht ist, und von welcher mindestens eine andere Hydroxyfunktion über eine Carbonylfunktion an das Dihydropyridinringsystem angebracht ist.

29. Verbindung der Formel der Formel 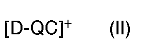 worin [D] eine zentral wirkende Arzneimittelspezies ist, welche mindestens eine reaktive funktionelle COOH-, Amid- oder Imidgruppe enthält, und [QC]⁺ die hydrophile, ionische Pyridiniumsalzform eines Dihydropyridin⇔Pyridiniumsalz-Redoxträgers ist, der ein Pyridiniumringsystem der Formel 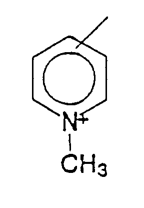 umfaßt, wobei ein Ringkohlenstoffatom des Pyridiniumringsystems über eine Brückengruppe mit einer reaktiven funktionellen COOH-, Amid- oder Imidgruppe in der zentral wirkenden Arzneimittelspezies verbunden ist.

30. Verbindung nach Anspruch 29, worin die zentral wirkende Arzneimittelspezies [D] mindestens eine reaktive Amid- oder Imidgruppe enthält, und das Pyridiniumringsystem zusammen mit der Brückengruppe die Strukturformel 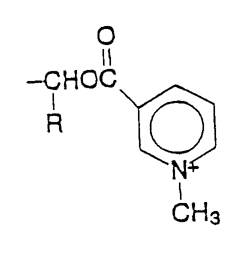 besitzt, worin R -CCI₃, -H, 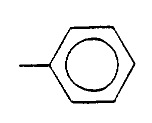 oder -CH₃ ist.

31. Verbindung nach Anspruch 29, worin die zentral wirkende Arzneimittelspezies [D] mindestens eine reaktive Amid- oder Imidgruppe enthält, und das Pyridiniumringsystem zusammen mit der Brückengruppe die Strukturformel 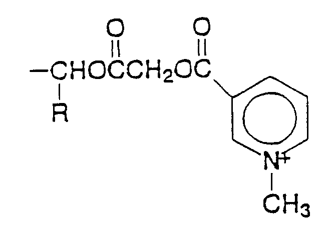 besitzt, worin R -CCI₃, -H, 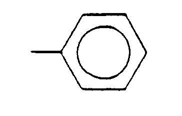 oder -CH₃ ist.

32. Verbindung nach Anspruch 29, worin die zentral wirkende Arzneimittelspezies [D] mindestens eine reaktive COOH-Gruppe enthält und die Brückengruppe, die das Pyridiniumringsystem mit dem Arzneimittel verbindet, von einer Di- oder Polyhydroxyverbindung abgeleitet ist, von welcher mindestens eine Hydroxyfunktion an einer reaktiven COOH-Gruppe im Arzneimittel angebracht ist, und von welcher mindestens eine andere Hydroxyfunktion über eine Carbonylfunktion an das Pyridiniumringsystem angebracht ist.

33. Verbindung nach Anspruch 28 oder 32, worin die Brückengruppe von Ethylenglycol, Propylenglycol, Inositol oder einem Zucker abgeleitet ist.

34. Verbindung nach Anspruch 25 oder 29, worin [D] ein zentraler Neurotransmitter, ein Antikrebs- oder Antitumormittel, ein Antivirusmittel, ein Tranquilizer, ein sedatives oder hypnotisches, ein antikonvulsives oder antiepileptisches Mittel, ein dopaminergisches Mittel oder ein antibiotisches oder antibakterielles Mittel ist.

35. Verbindung nach Anspruch 25 oder 29, worin [D] eine Aminosäure oder ein kleines Peptid, welches 2 bis 20 Aminosäureeinheiten enthält, ist.

36. Verbindung nach Anspruch 25 oder 29, worin [D] ist: GABA, Glycin, Glutaminsäure, Asparaginsäure, Neurotensin, LHRH, ein Enkephalin, ein Endorphin, Oxytocin M, Vasopressin, 3-Deazaguanin, Tiazofurin, Sangivamycin, PCNU, Spiromustin, L-Alanosin, DON, L-ICRF, 5-Methyltetrahydrohomofolsäure, Glyoxalsäuresulfonylhydrazin, DACH, SR-2555, SR-2508, Bactobolin, Acivicin, Hydroxyharnstoff, Chlorambucil, Uracilsenf, Melphalan, 5-FUDR, Methotrexat, Aminopterin, Mitomycin C, Ribavirin, Acyclovir, 6-Azauridin, Oxazepam, Lorazepam, Chlordiazepoxid, Bromazepam, Chloracepat, Nitrazepam, Phenytoin, Ethotoin, Mephenytoin, Methyldopa, y-Vinyl-GABA, y-acetylenisches GABA, Phenobarbital, Aminobarbital, Butalbital, Glutethimid, Aminoglutethimid, Methyprylon, Valpronsäure, 5-Hydroxy-2-n-propylpent- ansäure, 4-Hydroxy-2-n-propylpentansäure, 3-Hydroxy-2-n-propylpentansäure, Bemegrid, Tryptophan, Levodopa, Ampicillin, Amoxicillin, Oxacillin, Carbenicillin, Dicloxacillin, Chlortetracyclin, Tetracyclin, Methacyclin, Nalidixinsäure, Cephalexin, Cephalothin, Cefoxitin, Clindamycin, Lincomycin oder Oxolinsäure.

37. Verbindung, die für eine ortsspezifische Langzeitabgabe einer zentral wirkenden Arzneimittelspezies an das Hirn angepaßt ist, welche Verbindung eine Verbindung der Formel 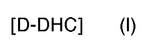 oder ein nicht-toxisches, pharmazeutisch akzeptables Salz davon ist, worin [D] eine zentral wirkende Arzneimittelspezies ist, welche mindestens eine reaktive OH-, COOH-, SH-, NH- oder NH₂-Gruppe (einschließlich Gruppen, wie z.B. Amide und Imide, sowie primäre und sekundäre Aminogruppen) enthält, und [DHC] die reduzierte, biooxydierbare, die Blut/Hirn-Schranke durchdringende, lipoide Form eines Dihydropyridin(=>Pyridiniumsalz-Redoxträgers ist, welcher ein Dihydropyridinringsystem der Formel 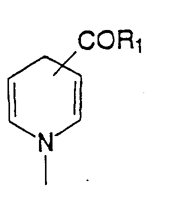 umfaßt, worin R -NH₂ oder -OR₂ ist, worin R₂ Alkyl ist, wobei der Dihydropyridinring gegebenenfalls an einen Benzolring kondensiert ist, und das Stickstoffatom des Dihydropyridinringsystems über eine Brückengruppe mit einer reaktiven OH-, COOH-, SH-, NH- oder NH₂-Gruppe in der zentral wirkenden Arzneimittelspezies verbunden ist.

38. Verbindung nach Anspruch 37, worin die zentral wirkende Arzneimittelspezies [D] mindestens eine reaktive primäre oder sekundäre Aminogruppe oder mindestens eine reaktive OH-Gruppe enthält und das Dihydropyridinringsystem zusammen mit der Brückengruppe, die das Dihydropyridinringsystem mit der Amino- oder OH-Gruppe in [D] verbindet, die Strukturformel 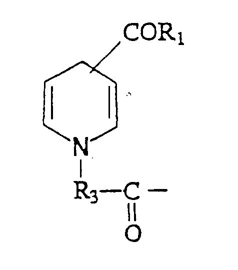 aufweist, worin R wie in Anspruch 37 definiert ist und R₃ (CH₂)ₙ ist, worin n 1-10 oder bis zu einem verzweigten C₁₂-Alkylen ist, worin vorzugsweise das Dihydropyridinringsystem zusammen mit der Brückengruppe die Strukturformel 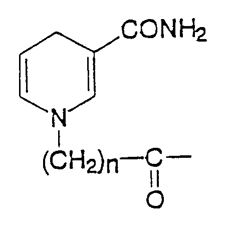 besitzt, worin n 1, 2 oder 3 ist.

39. Verbindung nach Anspruch 37, worin die zentral wirkende Arnzeimittelspezies [D] mindestens eine reaktive SH-Gruppe enthält und das Dihydropyridinringsystem zusammen mit der Brückengruppe, die das Dihydropyridinringsystem mit der SH-Gruppe in [D[ verbindet, die Strukturformel 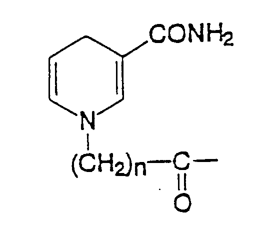 aufweist, worin n 1, 2 oder 3 ist.

40. Verbindung nach Anspruch 37, worin die zentral wirkende Arzneimittelspezies [D] mindestens eine reaktive primäre oder sekundäre Aminogruppe oder mindestens eine reaktive OH-Gruppe enthält und das Dihydropyridinringsystem zusammen mit der Brückengruppe, die das Dihydropyridinringsystem mit der Amino- oder OH-Gruppe in [D] verbindet, die Strukturformel 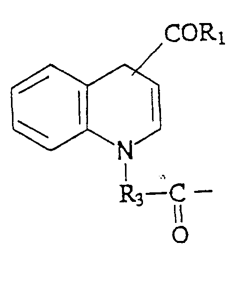 aufweist, worin R wie in Anspruch 37 definiert ist und R₃ (CH₂)ₙ ist, worin n 1-10 oder bis zu einem verzweigten C₁₂-Alkylen ist, worin vorzugsweise das Dihydropyridinringsystem zusammen mit der Brückengruppe die Strukturformel 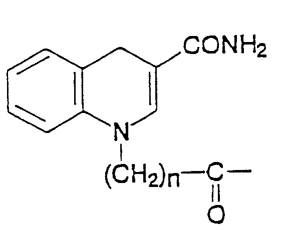 besitzt, worin n 1, 2 oder 3 ist.

41. Verbindung nach Anspruch 37, worin die zentral wirkende Arzneimittelspezies [D] mindestens eine reaktive primäre oder sekundäre Aminogruppe oder mindestens eine reaktive OH-Gruppe enthält und das Dihydropyridinringsystem zusammen mit der Brückengruppe, die das Dihydropyridinringsystem mit der Amino- oder OH-Gruppe in [D] verbindet, die Strukturformel 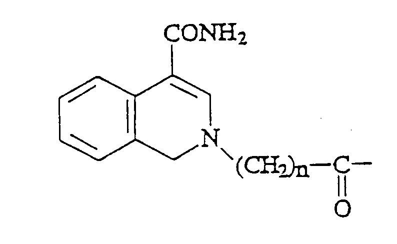 aufweist, worin n 1, 2 oder 3 ist.

42. Verbindung nach Anspruch 37, worin die zentral wirkende Arzneimittelspezies [D] mindestens eine reaktive NH- oder NH₂-Gruppe enthält, welche Teil einer Amid- oder Imidstruktur ist oder welche eine primäre oder sekundäre Aminogruppe mit niedrigem pKa ist, und das Dihydropyridinringsystem zusammen mit der Brückengruppe, die das Dihydropyridinringsystem mit der NH- oder NH₂-Gruppe in [D] verbindet, die Strukturformel 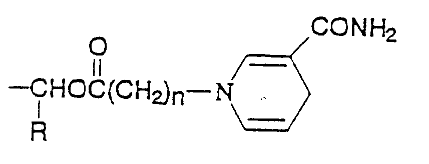 besitzt, worin R -CCI₃, -H, 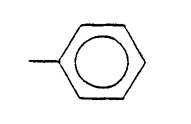 oder -CH₃ ist und n 1, 2 oder 3 ist.

43. Verbindung nach Anspruch 37, worin die zentral wirkende Arzneimittelspezies [D] mindestens eine reaktive COOH-Gruppe enthält und das Dihydropyridinringsystem zusammen mit der Brückengruppe, die das Dihydropyridinringsystem mit der COOH-Gruppe in [D] verbindet, die Strukturformel 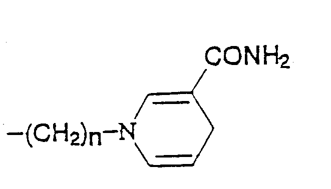 aufweist, worin n 1, 2 oder 3 ist.

44. Verbindung nach Anspruch 37, worin die zentral wirkende Arzneimittelspezies [D] mindestens eine reaktive COOH-Gruppe enthält und das Dihydropyridinringsystem zusammen mit der Brückengruppe, die das Dihydropyridinringsystem mit der COOH-Gruppe in [D] verbindet, die Strukturformel 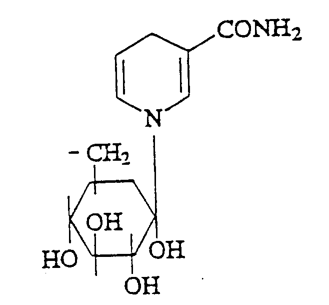 besitzt.

45. Verbindung der Formel 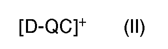 worin [D] eine zentral wirkende Arzneimittelspezies ist, welche mindestens eine reaktive OH-, COOH-, SH-, NH- oder NH₂-Gruppe (einschließlich Gruppen, wie z.B. Amide und Imide, sowie primäre und sekundäre Amidogruppen) enthält, und [QC]⁺ die hydrophile, ionische Pyridiniumsalzform eines Dihydropyridin⇔Pyridiniumsalz-Redoxträgers ist, welcher ein Pyridiniumringsystem der Formel 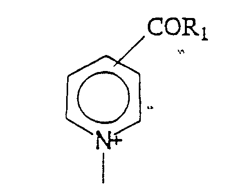 umfaßt, worin R -NH₂ oder -OR₂ ist, worin R₂ Alkyl ist, wobei der Pyridiniumring gegebenenfalls an einen Benzolring kondensiert ist, und das Stickstoffatom des Pyridiniumringsystems über eine Brückengruppe mit einer reaktiven OH-, COOH-, SH-, NH- oder NH₂-Gruppe in der zentral wirkenden Arzneimittelspezies verbunden ist.

46. Verbindung nach Anspruch 45, worin die zentral wirkende Arzneimittelspezies [D] mindestens eine reaktive primäre oder sekundäre Aminogruppe oder mindestens eine reaktive OH-Gruppe enthält und das Pyridiniumringsystem zusammen mit der Brückengruppe, die das Pyridiniumringsystem mit der Amino- oder OH-Gruppe in [D] verbindet, die Strukturformel 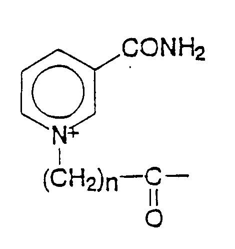 besitzt, worin n 1, 2 oder 3 ist.

47. Verbindung nach Anspruch 45, worin die zentral wirkende Arzneimittelspezies [D] mindestens eine reaktive primäre oder sekundäre Aminogruppe oder mindestens eine reaktive OH-Gruppe enthält und das Pyridiniumringsystem zusammen mit der Brückengruppe, die das Pyridiniumringsystem mit der Amino- oder OH-Gruppe in [D] verbindet, die Strukturformel 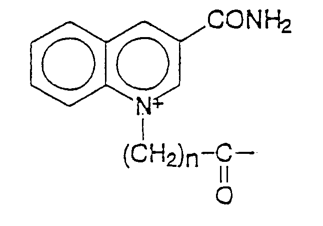 besitzt, worin n 1, 2 oder 3 ist.

48. Verbindung nach Anspruch 45, worin die zentral wirkende Arzneimittelspezies [D] mindestens eine reaktive NH- oder NH₂-Gruppe enthält, welche Teil einer Amid- oder Imidstruktur ist oder welche eine primäre oder sekundäre Aminogruppe mit niedrigem pKa ist, und das Pyridiniumringsystem zusammen mit der Brückengruppe, die das Pyridiniumringsystem mit der NH- oder NH₂-Gruppe in [D] verbindet, die Strukturformel 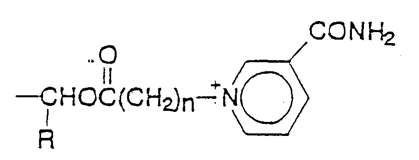 besitzt, worin R -CCI₃, -H, 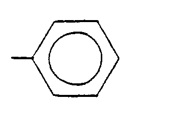 oder -CH₃ ist und n 1, 2 oder 3 ist.

49. Verbindung gemäß Anspruch 45, worin die zentral wirkende Arzneimittelspezies [D] mindestens eine reaktive COOH-Gruppe enthält und das Pyridiniumringsystem zusammen mit der Brückengruppe, die das Pyridiniumringsystem mit der COOH-Gruppe in [D] verbindet, die Strukturformel 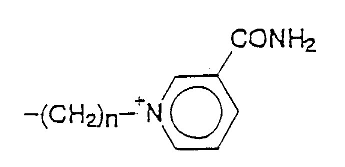 aufweist, worin n 1, 2 oder 3 ist.

50. Verbindung, die für eine ortsspezifische Langzeitabgabe einer zentral wirkenden Arzneimittelspezies an das Hirn angepaßt ist, welche Verbindung eine Verbindung der Formel 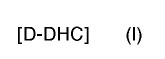 oder ein nicht-toxisches, pharmazeutisch akzeptables Salz davon ist, worin [D] eine zentral wirkende Arzneimittelspezies ist, welche mindestens eine reaktive OH-, COOH-, SH-, NH- oder NH₂-Gruppe (einschließlich Gruppen, wie z.B. Amide und Imide, sowie primäre und sekundäre Aminogruppen) enthält, und [DHC] die reduzierte, biooxydierbare, die Blut/Hirn-Schranke durchdringende, lipoide Form eines Dihydropyridin⇔Pyridiniumsalz-Redoxträgers ist, welcher ein Dihydrochinolin- oder Dihydroisochinolinringsystem umfaßt, wobei ein Kohlenstoffatom des Stickstoff-enthaltenden Ringteils des Ringsystems über eine Brückengruppe mit einer reaktiven OH-, COOH-, SH-, NH- oder NH₂-Gruppe in der zentral wirkenden Arzneimittelspezies verbunden ist.

51. Verbindung nach Anspruch 50, worin die zentral wirkende Arzneimittelspezies [D] mindestens eine reaktive primäre oder sekundäre Aminogruppe oder mindestens eine reaktive OH-Gruppe enthält und das Dihydrochinolin- oder Dihydroisochinolinringsystem zusammen mit der Brückengruppe, die das Ringsystem mit der Amino- oder OH-Gruppe in [D] verbindet, die Strukturformel 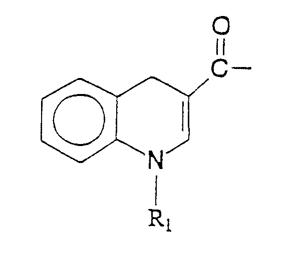 aufweist, worin R C₁-C₇Alkyl oder C₇-C₁₀Aralkyl ist, insbesondere wenn R Methyl ist.

52. Verbindung nach Anspruch 50, worin die zentral wirkende Arzneimittelspezies [D] mindestens eine reaktive primäre oder sekundäre Aminogruppe oder mindestens eine reaktive OH-Gruppe enthält und das Dihydrochinolin- oder Dihydroisochinolinringsystem zusammen mit der Brückengruppe, die das Ringsystem mit der Amino- oder OH-Gruppe in [D] verbindet, die Strukturformel 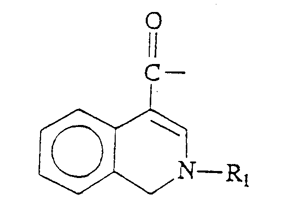 aufweist, worin R C₁-C₇Alkyl oder C₇-C₁₀Aralkyl ist, insbesondere wenn R Methyl ist.

53. Verbindung nach Anspruch 50, worin die zentral wirkende Arzneimittelspezies [D] mindestens eine reaktive primäre oder sekundäre Aminogruppe oder mindestens eine reaktive OH-Gruppe oder mindestens eine reaktive SH-Gruppe enthält und das Dihydrochinolin- oder Dihydroisochinolinringsystem zusammen mit der Brückengruppe, die das Ringsystem mit der Amino- oder OH- oder SH-Gruppe in [D] verbindet, die Strukturformel 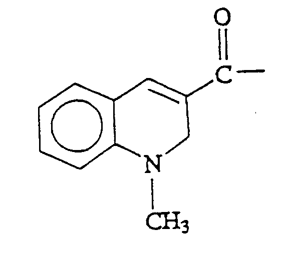 besitzt.

54. Verbindung nach Anspruch 50, worin die zentral wirkende Arzneimittelspezies [D] mindestens eine reaktive NH- oder NH₂-Gruppe enthält, welche Teil einer Amid- oder Imidstruktur ist oder welche eine primäre oder sekundäre Aminogruppe mit niedrigem pKa ist, der Redoxträger [DHC] ein Dihydrochinolinringsystem umfaßt und die Brückengruppe die Struktur 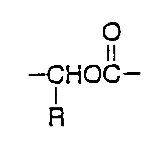 aufweist, worin R -CCI₃, -H, 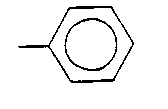 oder -CH₃ ist, wobei die Carbonylfunktion der Brückengruppe mit einem Kohlenstoffatom des Stickstoff-enthaltenden Ringteils des Dihydrochinolinringsystems verbunden ist.

55. Verbindung der Formel 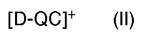 worin [D] eine zentral wirkende Arzneimittelspezies ist, welche mindestens eine reaktive OH-, COOH-, SH-, NH- oder NH₂-Gruppe (einschließlich Gruppen, wie z.B. Amide und Imide, sowie primäre und sekundäre Amidogruppen) enthält, und [QC]⁺ die hydrophile, ionische Pyridiniumsalzform eines Dihydropyridin⇔Pyridiniumsalz-Redoxträgers ist, welcher ein Chinolinium- oder Isochinoliniumringsystem umfaßt, wobei ein Kohlenstoffatom des Stickstoff-enthaltenden Ringteils des Ringsystems über eine Brückengruppe mit einer reaktiven OH-, COOH-, SH-, NH- oder NH₂-Gruppe in der zentral wirkenden Arzneimittelspezies verbunden ist.

56. Verbindung nach Anspruch 55, worin die zentral wirkende Arzneimittelspezies [D] mindestens eine reaktive primäre oder sekundäre Aminogruppe oder mindestens eine reaktive OH-Gruppe enthält und das Chinolinium- oder Isochinoliniumringsystem zusammen mit der Brückengruppe, die das Ringsystem mit der Amino- oder OH-Gruppe in [D] verbindet, die Strukturformel 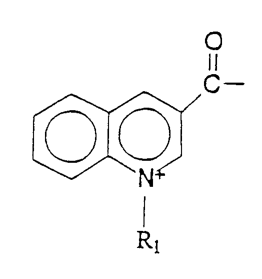 aufweist, worin R C₁-C₇Alkyl oder C₇-C₁₀Aralkyl ist, insbesondere wenn R Methyl ist.

57. Verbindung nach Anspruch 55, worin die zentral wirkende Arzneimittelspezies [D] mindestens eine reaktive primäre oder sekundäre Aminogruppe oder mindestens eine reaktive OH-Gruppe enthält und das Chinolinium- oder Isochinoliniumringsystem zusammen mit der Brückengruppe, die das Ringsystem mit der Amino- oder OH-Gruppe in [D] verbindet, die Strukturformel 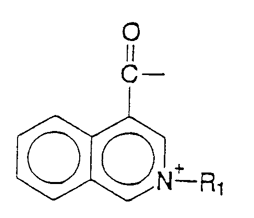 aufweist, worin R₁ C₁-C₇Alkyl oder C₇-C₁₀Aralkyl ist, insbesondere wenn R₁ Methyl ist.

58. Verbindung nach Anspruch 55, worin die zentral wirkende Arzneimittelspezies [D] mindestens eine reaktive NH- oder NH₂-Gruppe enthält, welche Teil einer Amid- oder Imidstruktur ist oder welche eine primäre oder sekundäre Aminogruppe mit niedrigem pKa ist, der Redoxträger [QC]⁺ ein Chinoliniumringsystem umfaßt und die Brückengruppe die Struktur 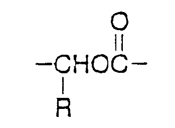 aufweist, worin R -CCl₃, -H, 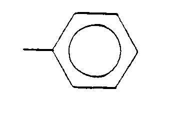 oder -CH₃ ist, wobei die Carbonylfunktion der Brückengruppe mit einem Kohlenstoffatom des Stickstoff-enthaltenden Ringteils des Chinoliniumringsystems verbunden ist.

59. Verbindung nach einem der Ansprüche 37, 45, 50 und 55, worin [D] ein zentraler Neurotransmitter, ein entzündungshemmendes Steroid, ein Steroidgeschlechtshormon, ein Antikrebs- oder Antitumormittel, ein Antivirusmittel, ein Tranquilizer, ein Gedächtnisverstärker, ein hypotensives Mittel, ein Sedativum oder ein hypnotisches Mittel, ein antikonvulsives Mittel oder ein antiepileptisches Mittel, ein Stimulans, ein narkotisches Analgesikum oder Antagonist, ein dopaminergisches Mittel oder ein antibiotisches oder antibakterielles Mittel.

60. Verbindung nach einem der Ansprüche 37, 45, 50 und 55, worin [D] eine Aminosäure oder ein kleines Peptid, welches 2 bis 20 Aminosäureeinheiten enthält, ist.

61. Verbindung nach einem der Ansprüche 37, 45, 50 und 55, worin [D] ein Dopamin mit der Strukturformel 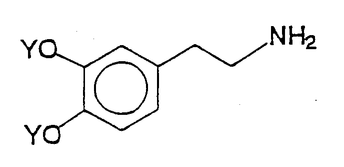 ist, in welcher jedes Y unabhängig Wasserstoff oder eine hydrolytisch oder metabolisch abspaltbare Hydroxylschutzgruppe ist.

62. Verbindung nach einem der Ansprüche 37, 45, 50 und 55, worin [D] ist: GABA, Glycin, Glutaminsäure, Asparaginsäure, Dopamin, Norepinephrin, Epinephrin, Serotonin, Tryptamin, Neurotensin, LHRH, Somatostatin, ein Enkephalin, ein Endorphin, Oxytocin M, Vasopressin, Hydrocortison, Betamethason, Dexamethason, Cortison, Flumethason, Fluprednisolon, Meprednison, Methylprednisolon, Prednisolon, Prednison, Triamcinolon, Cortodoxon, Fludrocortison, Fluandrenolonacetonid, Paramethason, Testosteron, Methyltestosteron, Ethinylöstradiol, Norgestrel, Mestranol, Norethindron, Ethisteron, Östradiol, Östriol, Östron, Dimethisteron, Allylöstrenol, Cingestol, Ethyneron, Lynöstrenol, Norgesteron, Norvininsteron, Ethynodiol, Oxogeston, Tigestol, Chinestrol, Ara-AC, Pentostatin, Ara-C, 3-Deazaguanin, Dihydro-5- azacytidin, Tiazofurin, Sangivamycin, Ara-A, 6-MMPR, PCNU, Spiromustin, Bisbenzimidazol, L-Alanosin, DON, L-ICRF, Trimethyl-TMM, 5-Methyltetrahydrohomofolsäure, Glyoxalsäuresulfonylhydrazon, DACH, SR-2555, SR-2508, Desmethylmisonidazol, Mitoxantron, Menogarol, Aclacinomycin A, Phyllanthosid, Bactobolin, Aphidocolin, Homoharringtonin, Levonantradol, Acivicin, Streptozotocin, Hydroxyharnstoff, Chlorambucil, Cyclophosphamid, Uracilsenf, Melphalan, 5-FUDR, Cytosinarabinosid, 6-Mercaptopurin, Thioguanin, 5-Azacytidin, Methotrexat, Doxorubicin, Daunomycin, Aminopterin, Dactinomycin, Mitomycin C, ein Podophyllotoxinderivat, Ribavirin, Acyclovir, Amantadin, 5-Amidino-2-(5-amidino-2-benzof- uranyl)indol, 4',6-Diimidazolino-2-phenylbenzo(b)thiophen 2-Guanidino-4,5-di-n-propyloxazol, 2-Guanidi- no-4,5-diphenyloxazol, 6-[[(Hydroxyimino)phenyl]methyl]-1-[(1-methylethyl)sulfonyl]-lH-benzimidazol-2-amin, 5,7-Dimethy!-j8-D-ribofuranosy!-s-triazo!(1,5-a)pyrimidin, 2-Deoxy-D-glucose, Glucosamin, 2-Deoxy-2-f!uor-D-mannose, 6-Amino-6-deoxy-D-glucose, Phenyl-6-chlor-6-deoxy-β-D-glucopyranosid, (S)-9-(2,3-Dihydroxypropyl)adenin, 6-Azauridin, 5,6-Dichlor-1-β-D-ribofuranosylbenzimidazol, Diazepam, Oxazepam, Lorazepam, Chlordiazepoxid, Flurazepam, Bromazepam, Clorazepat, Nitrazepam, Temazepam, Phenytoin, Ethotoin, Mephenytoin, Acetophenazin, Carphenazin, Fluphenazin, Perphenazin, Piperacetazin, Clonidin, Methyldopa, Bethanidin, Debrisoquin, Hydralazin, Guanethidin, Haloperidol, Hydroxyzin, Clopenthixol, y-Vinyl-GABA, y-acetylenisches GABA, Phenobarbital, Amobarbital, Butalbital, Glutethimid, Aminoglutethimid, Methyprylon, Valpronsäure, 5-Hydroxy-2-n-propylpentansäure, 4-Hydroxy-2-n-propylpentansäure, 3-Hydroxy-2-n-propylpentansäure, Methamphetamin, Phentermin, Phenmetrazin, Dextroamphetamin, Levamphetamin, Amphetamin, Phenylethylamin, Methylphenidat, Tranylcypromin, Protriptylin, Opipramol, Desipramin, Nortriptylin, Bemegrid, Amiphenazol, Anileridin, Hydromorphon, Oxymorphon, Apomorphin, Levorphanol, Morphin, Pentazocin, Codein, Oxycodon, Naloxon, Nalorphin, Tryptophan, Tyramin, Levodopa, Ampicillin, Amoxycillin, Oxacillin, Carbenicillin, Dicloxacillin, Chlortetracyclin, Tetracyclin, Methacyclin, Nalidixinsäure, Cephalexin, Cephalothin, Cefoxitin, Clindamycin, Lincomycin, Oxolinsäure, Phenazopyridin, Sulfadiazin, Thiopental, Benzöstrol oder Diethylstilböstrol.

63. Verfahren zur Herstellung einer Verbindung, wie in Anspruch 1 beansprucht, welches ein Reduzieren der entsprechenden Verbindung, wie in Anspruch 2 beansprucht, umfaßt.

64. Pharmazeutische Zusammensetzung, umfassend eine Verbindung, wie in einem der Ansprüche 1, 3, 6, 12, 16-19, 25-28, 37-44 und 50-54 oder in Anspruch 5, wenn von Anspruch 3 abhängig, oder in einem der Ansprüche 8-11, wenn von Anspruch 6 abhängig, oder in Anspruch 14 oder 15, wenn von Anspruch 12 abhängig, oder in Anspruch 24, wenn von Anspruch 16 abhängig, oder in Anspruch 33, wenn von Anspruch 28 abhängig, oder in einem der Ansprüche 34-36, wenn Anspruch 25 abhängig, oder in einem der Ansprüche 59-62, wenn von Anspruch 37 oder 50 abhängig, beansprucht, und einen nicht-toxischen, pharmazeutisch akzeptablen Träger dafür.

## Patentansprüche (Patentansprüche für folgende(n) Vertragsstaat(en) : AT)

1. Verfahren zur Herstellung einer Verbindung der Formel 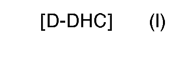 oder eines nicht-toxischen, pharmazeutisch akzeptablen Salzes davon, worin [D] eine zentral wirkende Arzneimittelspezies ist, welche mindestens eine reaktive OH-, COOH-, SH-, NH- oder NH₂-Gruppe (einschließlich Gruppen, wie z.B. Amide und Imide, sowie primäre und sekundäre Aminogruppen) enthält, und [DHC] die reduzierte, biooxydierbare, die Blut/Hirn-Schranke durchdringende, lipoide Form eines Dihydropyridin(=>Pyridiniumsalz-Redoxträgers ist, mit der Maßgabe, daß, wenn [DHC] 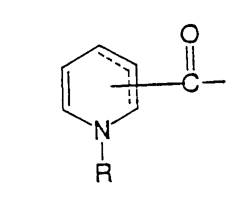 ist, worin R ein niederes Alkyl oder Benzyl ist, die gepunktete Linie das Vorhandensein einer Doppelbindung an entweder der Position 2 oder 3 des Dihydropyridinringes anzeigt und die Carbonylgruppe an der Position 2, 3 oder 4 des Dihydropyridinringes angebracht ist; und [D] eine Arzneimittelspezies ist, welche eine einzelne funktionelle Amino- oder OH-Gruppe enthält, wobei die einzelne Amino- oder OH-Gruppe, wenn sie vorhanden ist, eine primäre oder sekundäre Amino- oder eine primäre oder sekundäre OH-Gruppe ist, welche Arzneimittelspezies über die funktionelle Amino- oder OH-Gruppe direkt an die Carbonylfunktion von [DHC] gebunden ist, dann [D] etwas anderes sein muß als ein sympathisches Stimulans, ein Steroidgeschlechtshormon, ein Gedächtnisverstärker, ein langkettiger Alkanol oder ein Antikrebs- oder Antitumormittel; welches Verfahren gekennzeichnet ist durch Reduzieren der entsprechenden Verbindung der Formel 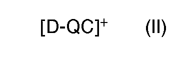 worin [D] oben definiert ist und [QC]⁺ die hydrophile, ionische Pyridiniumsalzform eines Dihydropyridin⇔Pyridiniumsalz-Redoxträgers ist, mit der Maßgabe, daß, wenn [QC]⁺ 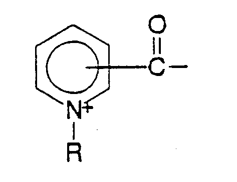 ist, worin R ein niederes Alkyl oder Benzyl ist und die Carbonylgruppe an der Position 2, 3 oder 4 des Pyridiniumringes angebracht ist, und [D] eine Arzneimittelspezies ist, welche eine einzelne funktionelle Amino- oder OH-Gruppe enthält, wobei die einzelne Amino- oder OH-Gruppe, wenn sie vorhanden ist, eine primäre oder sekundäre Amino- oder eine primäre oder sekundäre OH-Gruppe ist, welche Arzneimittelspezies über die funktionelle Amino- oder OH-Gruppe direkt an die Carbonylfunktion von [QC]⁺ gebunden ist, dann [D] etwas anderes sein muß als ein sympathisches Stimulans, ein Steroidgeschlechtshormon, ein Gedächtnisverstärker, ein langkettiger Alkanol oder ein Antikrebs- oder Antitumormittel.

2. Verfahren zur Herstellung einer Verbindung der Formel 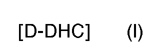 oder eines nicht-toxischen, pharmazeutisch akzeptablen Salzes davon, worin [D] eine zentral wirkende Arzneimittelspezies ist, welche Arzneimittelspezies Glycin, Glutaminsäure, Asparaginsäure, Tryptophan, ein Enkephalin, ein Endorphin oder eine andere Aminosäure oder ein kleines Peptid, welches 2 bis 20 Aminosäureeinheiten enthält; GABA, y-Vinyl-GABA oder y-acetylenisches GABA; Norepinephrin oder Epinephrin; Serotonin; 5-Hydroxy-2-n-propylpentansäure, 4-Hydroxy-2-n-propylpentansäure oder 3-Hydroxy-2-n-propylpentansäure; Methyldopa; Tyramin; Dopamin oder Levodopa; Ampicillin; Cephalexin; L-Alanosin; Melphalan; DON, oder Acivicin; oder Glucosamin, oder 6-Amino-6-deoxy-D-glucose ist: und [DHC] die reduzierte, biooxydierbare, die Blut/Hirn-Schranke durchdringende, lipoide Form eines Dihydropyridin(=>Pyridiniumsalz-Redoxträgers mit der Formel 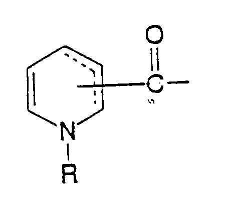 ist, worin R Methyl oder Benzyl ist und die gepunktete Linie das Vorhandensein einer Doppelbindung an entweder der Position 2 oder 3 des Dihydropyridinringes anzeigt, wobei die Carbonylfunktion, die dem Dihydropyridinring benachbart ist, an eine funktionelle reaktive Amino- oder Hydroxylgruppe in der zentral wirkenden Arzneimittelspezies gebunden ist und mindestens eine der verbleibenden reaktiven funktionellen Gruppen in der zentral wirkenden Arzneimittelspezies durch eine hydrolytisch oder metabolisch abspaltbare Hydroxyl- oder Carboxylschutzgruppe geschützt wird; welches Verfahren gekennzeichnet ist durch ein Reduzieren der entsprechenden Verbindung der Formel 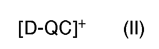 worin [D] oben definiert ist, und [QC]⁺ die hydrophile, ionische Pyridiniumsalzform eines Dihydropyridin⇔Pyridiniumsalz-Redoxträgers mit der Formel 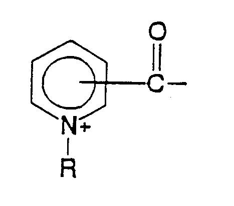 ist, worin R Methyl oder Benzyl ist, wobei die Carbonylfunktion, die dem Pyridiniumring benachbart ist, an eine funktionelle reaktive Amino- oder Hydroxylgruppe in der zentral wirkenden Arzneimittelspezies gebunden ist, und mindestens eine der verbleibenden reaktiven funktionellen Gruppen in der zentral wirkenden Arzneimittelspezies durch eine hydrolytisch oder metabolisch abspaltbare Hydroxyl- oder Carboxylschutzgruppe geschützt wird.

3. Verfahren nach Anspruch 2, wobei die Arzneimittelspezies ein Dopamin mit der Strukturformel 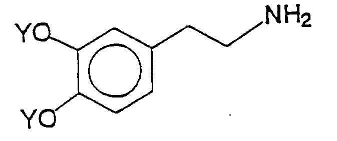 ist, worin jedes Y unabhängig Wasserstoff oder eine hydrolytisch oder metabolisch abspaltbare Hydroxylschutzgruppe ist, mit der Maßgabe, daß mindestens ein Y eine Acylgruppe oder eine Carbonatgruppe ist, worin bevorzugt: ein Y Pivalyl und das andere Y Wasserstoff oder Pivalyl ist; ein Y Isobutyryl und das andere Y Wasserstoff oder Isobutyryl ist; ein Y Ethoxycarbonyl und das andere Y Wasserstoff oder Ethoxycarbonyl ist: oder ein Y Isopropoxycarbonyl und das andere Y Wasserstoff oder Isopropoxycarbonyl ist.

4. Verfahren zur Herstellung einer Verbindung der Formel 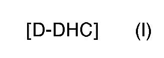 oder eines nicht-toxischen, pharmazeutisch akzeptablen Salzes davon, worin [D] eine zentral wirkende Arzneimittelspezies ist, welche ein Antivirusmittel, ein entzündungshemmendes Steroid, ein Narkotikum oder ein Östrogen ist und welche mindestens zwei reaktive funktionelle Hydroxylgruppen enthält, und [DHC] die reduzierte, biooxydierbare, die Blut/Hirn-Schranke durchdringende, lipoide Form eines Dihydropyridin(=>Pyridiniumsalz-Redoxträgers ist, welcher mindestens ein Dihydropyridinringsystem der Formel 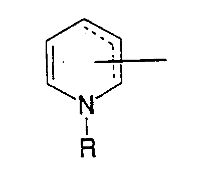 umfaßt, worin R ein niederes Alkyl oder Benzyl ist und die gepunktete Linie das Vorhandensein einer Doppelbindung an entweder der Position 2 oder 3 des Dihydropyridinringes anzeigt, wobei ein Ringkohlenstoffatom jedes Dihydropyridinringsystems über eine Brückengruppe an eine funktionelle reaktive Hydroxylgruppe in der zentral wirkenden Arzneimittelspezies gebunden ist; welches Verfahren gekennzeichnet ist durch ein Reduzieren der entsprechenden Verbindung der Formel 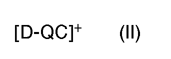 worin [D] wie oben definiert ist, und [QC]⁺ die hydrophile, ionische Pyridiniumsalzform eines Dihydropyridin⇔Pyridiniumsalz-Redoxträgers ist, welcher mindestens ein Pyridiniumringsystem der Formel 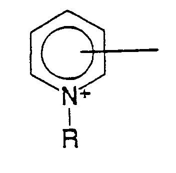 umfaßt, worin R ein niederes Alkyl oder Benzyl ist, wobei ein Ringkohlenstoffatom jedes Pyridiniumringsystems über eine Brückengruppe an eine reaktive funktionelle Hydroxylgruppe in der zentral wirkenden Arzneimittelspezies gebunden ist.

5. Verfahren nach Anspruch 4, wobei [D] ein Antivirusmittel ist.

6. Verfahren nach Anspruch 4, wobei [D] ein entzündungshemmendes Steroid ist.

7. Verfahren nach Anspruch 4, wobei [D] ein Östrogen ist.

8. Verfahren nach Anspruch 4, wobei [D] ist: Hydrocortison, Betamethason, Dexamethason, Cortison, Flumethason, Fluprednisolon, Meprednison, Methylprednisolon, Prednisolon, Prednison, Triamcinolon, Cortodoxon, Fludrocortison, Fluandrenolonacetonid, Paramethason, Ethinylöstradiol, Östradiol, Östriol, Ara-AC, Pentostatin, Dihydro-5-azacytidin, Tiazofurin, Sangivamycin, Ara-A, 6-MMPR, 5-FUDR, Cytosinarabinosid, 5-Azacytidin, Ribavirin, 5,7-Dimethyl-2-β-D-ribofuranosyl-s-triazol(1,5-a)pyrimidin, 2-Deoxy-D-glucose, Glucosamin, 2-Deoxy-2-fluor-D-mannose, 6-Amino-6-deoxy-D-glucose, Phenyl-6-chlor-6-deoxy-β-D-glucopyranosid, (S)-9-(2,3-Dihydroxypropyl)adenin, 6-Azauridin, 5,6-Dichlor-1-ß-_D-ribofuran- osylbenzimidazol, Oxymorphon, Apomorphin, Morphin, Oxycodon, Naloxon, Nalorphin, Benzöstrol oder Diethylstilböstrol.

9. Verfahren zur Herstellung einer Verbindung der Formel 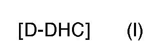 oder eines nicht-toxischen, pharmazeutisch akzeptablen Salzes davon, worin [D] eine zentral wirkende Arzneimittelspezies ist, welche ein Antikrebs/Antitumor- und/oder ein Antivirusmittel ist, welche Arzneimittelspezies eine Struktur vom Typus einer Purin- oder Pyrimidinbase besitzt, welche einen einfach oder mehrfach hydroxylierten Substituenten trägt, und [DHC] die reduzierte, biooxydierbare, die Blut/Hirn-Schranke durchdringende, lipoide Form eines Dihydropyridin⇔Pyridiniumsalz-Redoxträgers ist, welcher mindestens ein Dihydropyridinringsystem der Formel 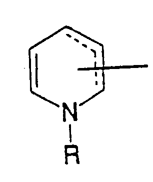 umfaßt, worin R ein niederes Alkyl oder Benzyl ist und die gepunktete Linie das Vorhandensein einer Doppelbindung an entweder der Position 2 oder 3 des Dihydropyridinringes anzeigt, wobei ein Ringkohlenstoffatom jedes Dihydropyridinringsystems über eine Brückengruppe an eine funktionelle Hydroxylgruppe in der zentral wirkenden Arzneimittelspezies gebunden ist; welches Verfahren gekennzeichnet ist durch ein Reduzieren der entsprechenden Verbindung der Formel 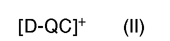 worin [D] oben definiert ist, und [QC]⁺ die hydrophile, ionische Pyridiniumsalzform eines Dihydropyridin⇔Pyridiniumsalz-Redoxträgers ist, der mindestens ein Pyridiniumringsystem der Formel 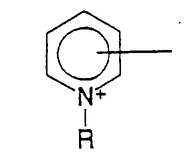 umfaßt, worin R ein niederes Alkyl oder Benzyl ist, wobei ein Ringkohlenstoffatom jedes Pyridiniumringsystems über eine Brückengruppe an eine reaktive funktionelle Hydroxylgruppe in der zentral wirkenden Arzneimittelspezies gebunden ist.

10. Verfahren nach Anspruch 9, worin [D] ein Antivirusmittel ist.

11. Verfahren nach Anspruch 9, worin [D] ist: Ara-AC, Pentostatin, Ara-C, Dihydro-5-azacytidin, Tiazofurin, Sangivamycin, Ara-A, 6-MMPR, Desmethylmisonidazol, 5-FUDR, Cytosinarabinosid, 5-Azacytidin, Ribavirin, Acyclovir, (S)-9-(2,3-Dihydroxypropyl)adenin, 6-Azauridin, 5,6-Dichlor-1-ß-D-ribofuranosylbenzimi- dazol oder 5,7-Dimethyl-2-ß-D-ribofuranosyl-s-triazol(1,5-a)pyrimidin.

12. Verfahren zur Herstellung einer Verbindung der Formel 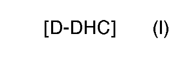 oder eines nicht-toxischen, pharmazeutisch akzeptablen Salzes davon, worin [D] eine zentral wirkende Arzneimittelspezies ist, welche ein Steroidprogestin oder ein Steroidöstrogen ist, welches Progestin oder Östrogen mindestens eine reaktive funktionelle Hydroxylgruppe besitzt, wobei eine solche Gruppe eine funktionelle tertiäre Hydroxylgruppe in der Position 17 ist; und [DHC] die reduzierte, biooxydierbare, die Blut/Hirn-Schranke durchdringende, lipoide Form eines Dihydropyridin(=>Pyridiniumsalz-Redox- trägers ist, der mindestens ein Dihydropyridinringsystem der Formel 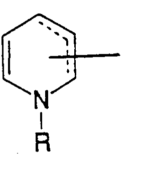 umfaßt, worin R ein niederes Alkyl oder Benzyl ist und die gepunktete Linie das Vorhandensein einer Doppelbindung an entweder der Position 2 oder 3 des Dihydropyridinringes anzeigt, wobei ein Ringkohlenstoffatom eines Dihydropyridinringsystems über eine Brückengruppe an die tertiäre funktionelle 17-Hydroxylgruppe im Progestin oder Östrogen gebunden ist, wobei ein Ringkohlenstoffatom eines verbleibenden Dihydropyridinringsystems über eine Brückengruppe mit einer anderen reaktiven funktionellen Hydroxylgruppe im Progestin oder Östrogen verbunden ist; welches Verfahren gekennzeichnet ist durch ein Reduzieren der entsprechenden Verbindung der Formel 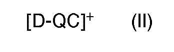 worin [D] oben definiert ist, und [QC]⁺ die hydrophile, ionische Pyridiniumsalzform eines Dihydropyridin⇔Pyridiniumsalz-Redoxträgers ist, der mindestens ein Pyridiniumringsystem der Formel 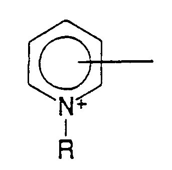 umfaßt, worin R ein niederes Alkyl oder Benzyl ist, wobei ein Ringekohlenstoffatom eines Pyridiniumringsystems über eine Brückengruppe an die funktionelle tertiäre 17-Hydroxylgruppe im Progestin oder Östrogen gebunden ist, und ein Ringkohlenstoffatom eines verbleibenden Pyridiniumringsystems über eine Brückengruppe an eine andere reaktive funktionelle Hydroxylgruppe im Progestin oder Östrogen gebunden ist.

13. Verfahren nach einem der Ansprüche 4, 9 und 12, wobei jedes Pyridiniumringsystem, zusammen mit der Brückengruppe, die daran gebunden ist, die Formel 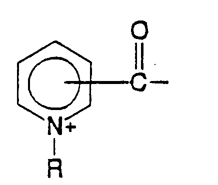 umfaßt, worin R ein niederes Alkyl oder Benzyl ist, insbesondere wenn R Methyl ist oder wenn die Carbonylgruppe an der Position 3 des Pyridiniumringes angebracht ist.

14. Verfahren nach Anspruch 13, wobei jedes Pyridiniumringsystem, zusammen mit der Brückengruppe, die daran gebunden ist, die Formel 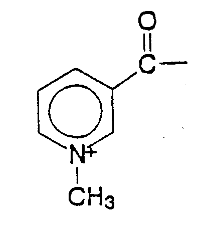 besitzt.

15. Verfahren nach Anspruch 13, wobei jedes Pyridiniumringsystem, zusammen mit der Brückengruppe, die daran gebunden ist, die Formel 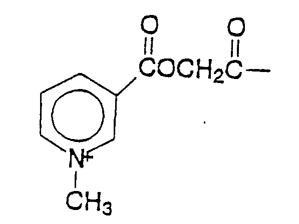 besitzt.

16. Verfahren nach Anspruch 12, wobei [D] ist: Mestranol, Chinöstrol, Norgestrel, Norethindron, Ethisteron, Dimethisteron, Allylöstrenol, Cingestol, Ethyneron, Lynöstrenol, Norgesteron, Norvinisteron, Ethynodiol oder Tigestol.

17. Verfahren zur Herstellung einer Verbindung der Formel 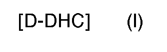 oder eines nicht-toxischen, pharmazeutisch akzeptablen Salzes davon, worin [D] eine zentral wirkende Arzneimittelspezies ist, welche mindestens eine reaktive funktionelle COOH-, Amid- oder Imidgruppe enthält, und [DHC] die reduzierte, biooxydierbare, die Blut/Hirn-Schranke durchdringende, lipoide Form eines Dihydropyridin⇔Pyridiniumsalz-Redoxträgers ist, der ein Dihydropyridinringsystem der Formel 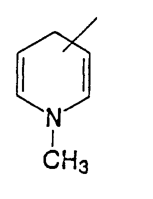 umfaßt, wobei ein Ringkohlenstoffatom des Dihydropyridinringsystems über eine Brückengruppe an eine reaktive funktionelle COOH-, Amid- oder Imidgruppe in der zentral wirkenden Arzneimittelspezies gebunden ist; welches Verfahren gekennzeichnet ist durch ein Reduzieren der entsprechenden Verbindung der Formel 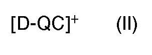 worin [D] oben definiert ist, und [QC]⁺ die hydrophile, ionische Pyridiniumsalzform eines Dihydropyridin⇔Pyridiniumsalz-Redoxträgers ist, der ein Pyridiniumringsystem der Formel 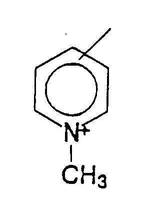 umfaßt, wobei ein Ringkohlenstoffatom des Pyridiniumringsystems über eine Brückengruppe mit einer reaktiven funktionellen COOH-, Amid- oder Imidgruppe in der zentral wirkenden Arzneimittelspezies verbunden ist.

18. Verfahren nach Anspruch 17, wobei die zentral wirkende Arzneimittelspezies [D] mindestens eine reaktive Amid- oder Imidgruppe enthält, und das Pyridiniumringsystem zusammen mit der Brückengruppe die Strukturformel 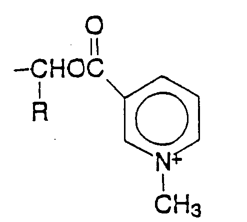 besitzt, worin R -CCI₃, -H, 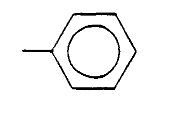 oder -CH₃ ist.

19. Verfahren nach Anspruch 17, wobei die zentral wirkende Arzneimittelspezies [D] mindestens eine reaktive Amid- oder Imidgruppe enthält, und das Pyridiniumringsystem zusammen mit der Brückengruppe die Strukturformel 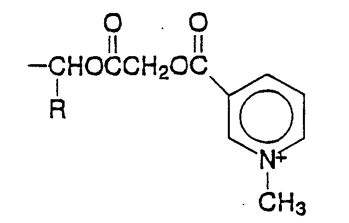 besitzt, worin R -CCl₃, -H, 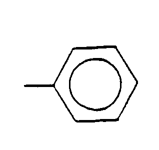 oder -CH₃ ist.

20. Verfahren nach Anspruch 17, wobei die zentral wirkende Arzneimittelspezies [D] mindestens eine reaktive COOH-Gruppe enthält und die Brückengruppe, die das Pyridiniumringsystem mit dem Arzneimittel verbindet, von einer Di- oder Polyhydroxyverbindung abgeleitet ist, von welcher mindestens eine Hydroxyfunktion an einer reaktiven COOH-Gruppe im Arzneimittel angebracht ist, und von welcher mindestens eine andere Hydroxyfunktion über eine Carbonylfunktion an das Pyridiniumringsystem angebracht ist.

21. Verfahren nach Anspruch 20, wobei die Brückengruppe von Ethylenglycol, Propylenglycol, Inositol oder einem Zucker abgeleitet ist.

22. Verfahren nach Anspruch 17, wobei [D] ein zentraler Neurotransmitter, ein Antikrebs- oder Antitumormittel, ein Antivirusmittel, ein Tranquilizer, ein sedatives oder hypnotisches, ein antikonvulsives oder antiepileptisches Mittel, ein dopaminergisches Mittel oder ein antibiotisches oder antibakterielles Mittel ist.

23. Verfahren nach Anspruch 17, wobei [D] eine Aminosäure oder ein kleines Peptid, welches 2 bis 20 Aminosäureeinheiten enthält, ist.

24. Verfahren nach Anspruch 17, wobei [D] ist: GABA, Glycin, Glutaminsäure, Asparaginsäure, Neurotensin, LHRH, ein Enkephalin, ein Endorphin, Oxytocin M, Vasopressin, 3-Deazaguanin, Tiazofurin, Sangivamycin, PCNU, Spiromustin, L-Alanosin, DON, L-ICRF, 5-Methyltetrahydrohomofolsäure, Glyoxalsäuresulfonylhydrazin, DACH, SR-2555, SR-2508, Bactobolin, Acivicin, Hydroxyharnstoff Chlorambucil, Uracilsenf, Melphalan, 5-FUDR, Methotrexat, Aminopterin, Mitomycin C, Ribavirin, Acyclovir, 6-Azauridin, Oxazepam, Lorazepam, Chlordiazepoxid, Bromazepam, Chloracepat, Nitrazepam, Phenytoin, Ethotoin, Mephenytoin, Methyldopa, y-Vinyl-GABA, y-acetylenisches GABA, Phenobarbital, Aminobarbital, Butalbital, Glutethimid, Aminoglutethimid, Methyprylon, Valpronsäure, 5-Hydroxy-2-n-propylpentansäure, 4-Hydroxy-2-n-propylpentansäure, 3-Hydroxy-2-n-propylpentansäure, Bemegrid, Tryptophan, Levodopa, Ampicillin, Amoxicillin, Oxacillin, Carbenicillin, Dicloxacillin, Chlortetracyclin, Tetracyclin, Methacyclin, Nalidixinsäure, Cephalexin, Cephalothin, Cefoxitin, Clindamycin, Lincomycin oder Oxolinsäure.

25. Verfahren zur Herstellung einer Verbindung der Formel 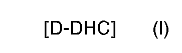 oder eines nicht-toxischen, pharmazeutisch akzeptablen Salzes davon, worin [D] eine zentral wirkende Arzneimittelspezies ist, welche mindestens eine reaktive OH-, COOH-, SH-, NH- oder NH₂-Gruppe (einschließlich Gruppen, wie z.B. Amide und Imide, sowie primäre und sekundäre Aminogruppen) enthält, und [DHC] die reduzierte, biooxydierbare, die Blut/Hirn-Schranke durchdringende, lipoide Form eines Dihydropyridin(=>Pyridiniumsalz-Redoxträgers ist, welcher ein Dihydropyridinringsystem der Formel 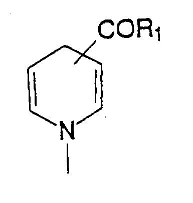 umfaßt, worin R -NH₂ oder -OR₂ ist, worin R₂ Alkyl ist, wobei der Dihydropyridinring gegebenenfalls an einen Benzolring kondensiert ist, und das Stickstoffatom des Dihydropyridinringsystems über eins Brückengruppe mit einer reaktiven OH-, COOH-, SH-, NH- oder NH₂-Gruppe in der zentral wirkenden Arzneimittelspezies verbunden ist; welches Verfahren gekennzeichnet ist durch ein Reduzieren der entsprechenden Verbindung der Formel 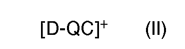 worin [D] oben definiert ist, und [QC]⁺ die hydrophile, ionische Pyridiniumsalzform eines Dihydropyridin⇔Pyridiniumsalz-Redoxträgers ist, welcher ein Pyridiniumringsystem der Formel 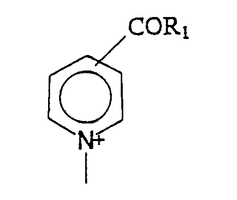 umfaßt, worin R -NH₂ oder -OR₂ ist, worin R₂ Alkyl ist, wobei der Pyridiniumring gegebenenfalls an einen Benzolring kondensiert ist, und das Stickstoffatom des Pyridiniumringsystems über eine Brückengruppe mit einer reaktiven OH-, COOH-, SH-, NH- oder NH₂-Gruppe in der zentral wirkenden Arzneimittelspezies verbunden ist.

26. Verfahren nach Anspruch 25, wobei die zentral wirkende Arzneimittelspezies [D] mindestens eine reaktive primäre oder sekundäre Aminogruppe oder mindestens eine reaktive OH-Gruppe enthält und das Pyridiniumringsystem zusammen mit der Brückengruppe, die das Pyridiniumringsystem mit der Amino- oder OH-Gruppe in [D] verbindet, die Strukturformel 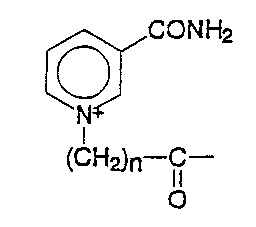 besitzt, worin n 1, 2 oder 3 ist.

27. Verfahren nach Anspruch 25, wobei die zentral wirkende Arzneimittelspezies [D] mindestens eine reaktive primäre oder sekundäre Aminogruppe oder mindestens eine reaktive OH-Gruppe enthält und das Pyridiniumringsystem zusammen mit der Brückengruppe, die das Pyridiniumringsystem mit der Amino- oder OH-Gruppe in [D] verbindet, die Strukturformel 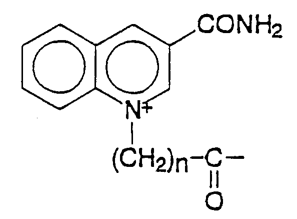 besitzt, worin n 1, 2 oder 3 ist.

28. Verfahren nach Anspruch 25, wobei die zentral wirkende Arzneimittelspezies [D] mindestens eine reaktive NH- oder NH₂-Gruppe enthält, welche Teil einer Amid- oder Imidstruktur ist oder welche eine primäre oder sekundäre Aminogruppe mit niedrigem pKa ist, und das Pyridiniumringsystem zusammen mit der Brückengruppe, die das Pyridiniumringsystem mit der NH- oder NH₂-Gruppe in [D] verbindet, die Strukturformel 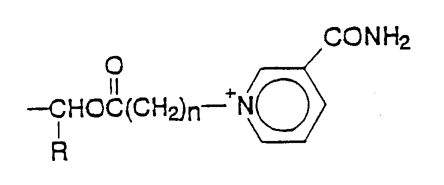 besitzt, worin R -CCI₃, -H, 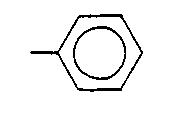 oder -CH₃ ist und n 1, 2 oder 3 ist.

29. Verfahren gemäß Anspruch 25, wobei die zentral wirkende Arzneimittelspezies [D] mindestens eine reaktive COOH-Gruppe enthält und das Pyridiniumringsystem zusammen mit der Brückengruppe, die das Pyridiniumringsystem mit der COOH-Gruppe in [D] verbindet, die Strukturformel 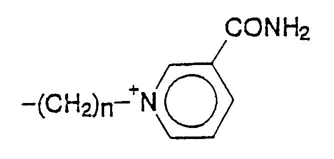 aufweist, worin n 1, 2 oder 3 ist.

30. Verfahren zur Herstellung einer Verbindung der Formel 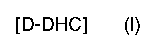 oder eines nicht-toxischen; pharmazeutisch akzeptablen Salzes davon, wobei [D] eine zentral wirkende Arzneimittelspezies ist, welche mindestens eine reaktive OH-, COOH-, SH-, NH- oder NH₂-Gruppe (einschließlich Gruppen, wie z.B. Amide und Imide, sowie primäre und sekundäre Aminogruppen) enthält, und [DHC] die reduzierte, biooxydierbare, die Blut/Hirn-Schranke durchdringende, lipoide Form eines Dihydropyridin⇔Pyridiniumsalz-Redoxträgers ist, welcher ein Dihydrochinolin- oder Dihydroisochinolinringsystem umfaßt, wobei ein Kohlenstoffatom des Stickstoff-enthaltenden Ringteils des Ringsystems über eine Brückengruppe mit einer reaktiven OH-, COOH-, SH-, NH- oder NH₂-Gruppe in der zentral wirkenden Arzneimittelspezies verbunden ist; welches Verfahren gekennzeichnet ist durch ein Reduzieren der entsprechenden Verbindung der Formel 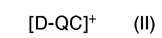 worin [D] oben definiert ist, und [QC]⁺ die hydrophile, ionische Pyridiniumsalzform eines Dihydropyridin⇔Pyridiniumsalz-Redoxträgers ist, welcher ein Chinolinium- oder Isochinoliniumringsystem umfaßt, wobei ein Kohlenstoffatom des Stickstoff-enthaltenden Ringteils des Ringsystems über eine Brückengruppe mit einer reaktiven OH-, COOH-, SH-, NH- oder NH₂-Gruppe in der zentral wirkenden Arzneimittelspezies verbunden ist.

31. Verfahren nach Anspruch 30, wobei die zentral wirkende Arzneimittelspezies [D] mindestens eine reaktive primäre oder sekundäre Aminogruppe oder mindestens eine reaktive OH-Gruppe enthält und das Chinolinium- oder Isochinoliniumringsystem zusammen mit der Brückengruppe, die das Ringsystem mit der Amino- oder OH-Gruppe in [D] verbindet, die Strukturformel 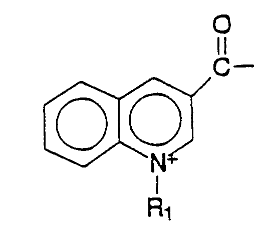 aufweist, worin R C₁-C₇Alkyl oder C₇-C₁₀Aralkyl ist, insbesondere wenn R Methyl ist.

32. Verfahren nach Anspruch 30, wobei die zentral wirkende Arzneimittelspezies [D] mindestens eine reaktive primäre oder sekundäre Aminogruppe oder mindestens eine reaktive OH-Gruppe enthält und das Chinolinium- oder Isochinoliniumringsystem zusammen mit der Brückengruppe, die das Ringsystem mit der Amino- oder OH-Gruppe in [D] verbindet, die Strukturformel 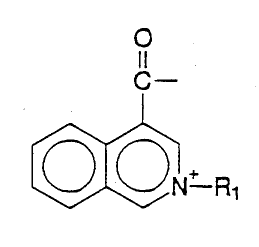 aufweist, worin R₁ C₁-C₇Alkyl oder C₇-C₁₀Aralkyl ist, insbesondere wenn R₁ Methyl ist.

33. Verfahren nach Anspruch 30, wobei die zentral wirkende Arzneimittelspezies [D] mindestens eine reaktive NH- oder NH₂-Gruppe enthält, welche Teil einer Amid- oder Imidstruktur ist oder welche eine primäre oder sekundäre Aminogruppe mit niedrigem pKa ist, der Redoxträger [QC]⁺ ein Chinoliniumringsystem umfaßt und die Brückengruppe die Struktur 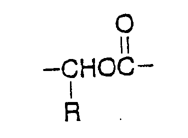 aufweist, worin R -CCl₃, -H, 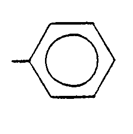 oder -CH₃ ist, wobei die Carbonylfunktion der Brückengruppe mit einem Kohlenstoffatom des Stickstoff-enthaltenden Ringteils des Chinoliniumringsystems verbunden ist.

34. Verfahren nach Anspruch 25 oder 30, wobei [D] ein zentraler Neurotransmitter, ein entzündungshemmendes Steroid, ein Steroidgeschlechtshormon, ein Antikrebs- oder Antitumormittel, ein Antivirusmittel, ein Tranquilizer, ein Gedächtnisverstärker, ein hypotensives Mittel, ein Sedativum oder ein hypnotisches Mittel, ein antikonvulsives Mittel oder ein antiepileptisches Mittel, ein Stimulans, ein narkotisches Analgesikum oder Antagonist, ein dopaminergisches Mittel oder ein antibiotisches oder antibakterielles Mittel.

35. Verfahren nach Anspruch 25 oder 30, wobei [D] eine Aminosäure oder ein kleines Peptid, welches 2 bis 20 Aminosäureeinheiten enthält, ist.

36. Verfahren nach Anspruch 25 oder 30, wobei [D] ein Dopamin mit der Strukturformel 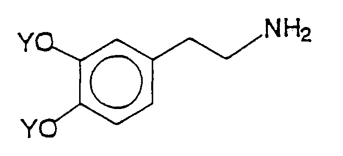 ist, in welcher jedes Y unabhängig Wasserstoff oder eine hydrolytisch oder metabolisch abspaltbare Hydroxylschutzgruppe ist.

37. Verfahren nach Anspruch 25 oder 30, wobei [D] ist: GABA, Glycin, Glutaminsäure, Asparaginsäure, Dopamin, Norepinephrin, Epinephrin, Serotonin, Tryptamin, Neurotensin, LHRH, Somatostatin, ein Enkephalin, ein Endorphin, Oxytocin M, Vasopressin, Hydrocortison, Betamethason, Dexamethason, Cortison, Flumethason, Fluprednisolon, Meprednison, Methylprednisolon, Prednisolon, Prednison, Triamcinolon, Cortodoxon, Fludrocortison, Fluandrenolonacetonid, Paramethason, Testosteron, Methyltestosteron, Ethinylöstradiol, Norgestrel, Mestranol, Norethindron, Ethisteron, Östradiol, Östriol, Östron, Dimethisteron, Allylöstrenol, Cingestol, Ethyneron, Lynöstrenol, Norgesteron, Norvininsteron, Ethynodiol, Oxogeston, Tigestol, Chinestrol, Ara-AC, Pentostatin, Ara-C, 3-Deazaguanin, Dihydro-5-azacytidin, Tiazofurin, Sangivamycin, Ara-A, 6-MMPR, PCNU, Spiromustin, Bisbenzimidazol, L-Alanosin, DON, L-ICRF, Trimethyl-TMM, 5-Methyltetrahydrohomofolsäure, Glyoxalsäuresulfonylhydrazon, DACH, SR-2555, SR-2508, Desmethylmisonidazol, Mitoxantron, Menogarol, Aclacinomycin A, Phyllanthosid, Bactobolin, Aphidocolin, Homoharringtonin, Levonantradol, Acivicin, Streptozotocin, Hydroxyharnstoff, Chlorambucil, Cyclophosphamid, Uracilsenf, Melphalan, 5-FUDR, Cytosinarabinosid, 6-Mercaptopurin, Thioguanin, 5-Azacytidin, Methotrexat, Doxorubicin, Daunomycin, Aminopterin, Dactinomycin, Mitomycin C, ein Podophyllotoxinderivat, Ribavirin, Acyclovir, Amantadin, 5-Amidino-2-(5-amidino-2-benzofuranyl)-indol, 4',6-Diimidazolino-2-phenylbenzo(b)thiophen, 2-Guanidino-4,5-di-n-propyloxazol, 2-Guanidino-4,5-diphenyloxazol, 6-[[(Hydroxyimino)phenyl]methyl]-1-[(1-methylethyl)sulfonyl]-1H-benzimidazol-2-amin, 5,7-Dimethyl-β-D-ribofuranosyl-s-triazol(1,5-a)pyrimidin, 2-Deoxy-D-glucose, Glucosamin, 2-Deoxy-2-fluor-D-mannose, 6-Amino-6-deoxy-D-glucose, Phenyl-6-chlor-6-deoxy-ß-D-glucopyranosid, (S)-9-(2,3-Dihydroxypropyl)adenin, 6-Azauridin, 5,6-Dichlor-1-β-D-ribofuranosylbenzimidazol, Diazepam, Oxazepam, Lorazepam, Chlordiazepoxid, Flurazepam, Bromazepam, Clorazepat, Nitrazepam, Temazepam, Phenytoin, Ethotoin, Mephenytoin, Acetophenazin, Carphenazin, Fluphenazin, Perphenazin, Piperacetazin, Clonidin, Methyldopa, Bethanidin, Debrisoquin, Hydralazin, Guanethidin, Haloperidol, Hydroxyzin, Clopenthixol, y-Vinyl-GABA, y-acetylenisches GABA, Phenobarbital, Amobarbital, Butalbital, Glutethimid, Aminoglutethimid, Methyprylon, Valpronsäure, 5-Hydroxy-2-n-propylpentansäure, 4-Hydroxy-2-n-propyl- pentansäure, 3-Hydroxy-2-n-propylpentansäure, Methamphetamin, Phentermin, Phenmetrazin, Dextroamphetamin, Levamphetamin, Amphetamin, Phenylethylamin, Methylphenidat, Tranylcypromin, Protriptylin, Opipramol, Desipramin, Nortriptylin, Bemegrid, Amiphenazol, Anileridin, Hydromorphon, Oxymorphon, Apomorphin, Levorphanol, Morphin, Pentazocin, Codein, Oxycodon, Naloxon, Nalorphin, Tryptophan, Tyramin, Levodopa, Ampicillin, Amoxycillin, Oxacillin, Carbenicillin, Dicloxacillin, Chlortetracyclin, Tetracyclin, Methacyclin, Nalidixinsäure, Cephalexin, Cephalothin, Cefoxitin, Clindamycin, Lincomycin, Oxolinsäure, Phenazopyridin, Sulfadiazin, Thiopental, Benzöstrol oder Diethylstilböstrol.

38. Verfahren nach einem der vorhergehenden Ansprüche, wobei ein Alkalimetalldithionit als das Reduktionsmittel verwendet wird.

39. Verfahren nach Anspruch 38, wobei das Alkalimetalldithionit Natriumdithionit ist.

40. Verfahren nach einem der Ansprüche 1-37, wobei ein Alkalimetallborhydrid als das Reduktionsmittel verwendet wird.

41. Verfahren nach Anspruch 40, wobei das Alkalimetallborhydrid Natriumborhydrid ist.

## Revendications (Revendications pour l'(les) Etat(s) contractant(s) suivant(s) : suivants : BE, CH, DE, FR, GB, LI, LU, NL, SE)

1. Composé apte à la libération prolongée/spécifique d'un site, au cerveau, d'une entité médicamenteuse à action centrale, ledit composé étant un composé de formule 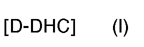 ou un de ses sels non toxiques pharmaceutiquement acceptables, formule dans laquelle [D] représente une entité médicamenteuse à action centrale contenant au moins un groupe -OH, -COOH, -SH-, -NH-ou -NH₂ réactif (y compris des groupes tels que des groupes amide et imide, ainsi que des groupes amino primaire et amino secondaire), et [DHC] représente la forme lipoïdique réduite, bioxydable, passant à travers la barrière hémato-encéphalique, d'un support redox dihydropyridine == sel de pyridinium, sous réserve que, lorsque [DHC] représente un groupe de formule 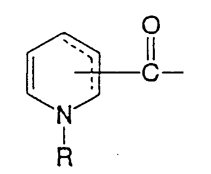 dans laquelle R représente un groupe alkyle inférieur ou benzyle, la ligne discontinue indique la présence d'une double liaison en position 2 ou 3 du noyau dihydropyridine et le groupe carbonyle est fixé en position 2, 3 ou 4 du noyau dihydropyridine ; et [D] représente une entité médicamenteuse contenant un seul groupe fonctionnel amino ou OH, le seul groupe amino ou OH lorsqu'il est présent étant un groupe amino primaire ou secondaire ou OH primaire ou secondaire, ladite entité médicamenteuse étant liée directement par ledit groupe fonctionnel amino ou OH à la fonction carbonyle de [DHC], alors [D] soit autre qu'un stimulant sympathique, une hormone sexuelle stéroïdienne, un agent stimulant la mémoire, un alcanol à chaîne longue ou un agent anticancéreux ou antitumoral.

2. Composé de formule 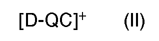 dans laquelle [D] représente une entité médicamenteuse à action centrale contenant au moins un groupe -OH, -COOH, -SH-, -NH- ou -NH₂ réactif (y compris des groupes tels que des amides et des imides, ainsi que des groupes amino primaire et amino secondaire), et [QC]⁺ représente la forme sel de pyridinium ionique hydrophile d'un support redox dihydropyridine ⇆ sel de pyridinium, sous réserve que, lorsque [QC]⁺ représente un groupe de formule 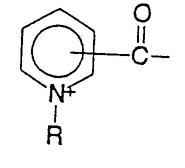 dans laquelle R représente un groupe alkyle inférieur ou benzyle et le groupe carbonyle est fixé en position 2, 3 ou 4 du noyau pyridinium, et [D] représente une entité médicamenteuse contenant un seul groupe fonctionnel amino ou OH, le seul groupe amino ou OH, lorsqu'il est présent, étant un groupe amino primaire ou secondaire ou un groupe OH primaire ou secondaire, ladite entité médicamenteuse étant liée directement par l'intermédiaire dudit groupe fonctionnel amino ou OH à la fonction carbonyle de [QC]⁺, alors [D] soit autre qu'un stimulant sympathique, une hormone sexuelle stéroïdienne, un agent améliorant la mémoire, un alcanol à chaîne longue ou un agent anticancéreux ou antitumoral.

3. Composé apte à la libération prolongée, spécifique d'un site, au cerveau, d'une entité médicamenteuse à action centrale, ledit composé étant un composé de formule 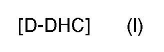 ou un de ses sels non toxiques pharmaceutiquement acceptables, formule dans laquelle [D] représente une entité médicamenteuse à action centrale, ladite entité médicamenteuse consistant en glycine, acide glutamique, acide aspartique, tryptophane, une enképhaline, une endorphine ou un autre aminoacide ou petit peptide contenant 2 à 20 motifs aminoacide ; le GABA, le y-vinyl-GABA ou le GABA y-acétylénique ; la norépinéphrine, ou l'épinéphrine ; la sérotonine ; l'acide 5-hydroxy-2-n-propylpentanoOlque, l'acide 4-hydroxy-2-n-propylpentanoïque ou l'acide 3-hydroxy-2-n-propylpentanoï- que, la méthyldopa ; la tyramine ; la dopamine ou la lévodopa ; l'ampicilline ; la céphalexine ; la L-alanosine ; le melphalan ; le DON ou l'acivicine ; ou la glucosamine ou le 6-amino-6-désoxy-D-glucose ; et [DHC] représente la forme lipoïdique réduite, biooxydable, passant à travers la barrière hémato- encéphalique d'un support redox dihydropyridine ⇆ sel de pyridinium répondant à la formule 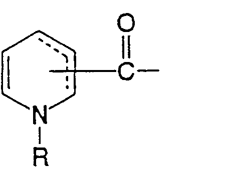 dans laquelle R représente un groupe méthyle ou benzyle et la ligne discontinue indique la présence d'une double liaison en position 2 ou 3 du noyau dihydropyridine, la fonction carbonyle adjacente au noyau dihydropyridine étant liée à un groupe fonctionnel amino ou hydroxyle réactif dans l'entité médicamenteuse à action centrale, et au moins un des groupes fonctionnels réactifs restants dans l'entité médicamenteuse à action centrale étant protégée par un groupe protecteur de la fonction hydroxyle ou carboxyle, à clivage hydrolytique ou métabolique.

4. Composé de formule 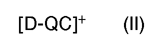 dans laquelle [D] représente une entité médicamenteuse à action centrale répondant à la définition suivant la revendication 3 et [QC]⁺ représente la forme sel de pyridinium ionique hydrophile d'un support redox dihydropyridine ⇆ sel de pyridinium répondant à la formule 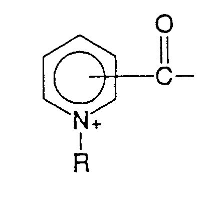 dans laquelle R représente un groupe méthyle ou benzyle, la fonction carbonyle adjacente au noyau pyridinium étant liée à un groupe fonctionnel amino ou hydroxyle réactif dans l'entité médicamenteuse à action centrale, et au moins un des groupes fonctionnels réactifs restants dans l'entité médicamenteuse à action centrale étant protégée par un groupe protecteur de la fonction hydroxyle ou carboxyle, à clivage hydrolytique ou métabolique.

5. Composé suivant la revendication 3 ou 4, dans lequel l'entité médicamenteuse est une dopamine répondant à la formule structurale 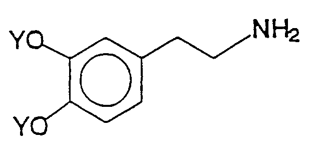 dans laquelle chaque groupe Y représente indépendamment l'hydrogène ou un groupe protecteur de la fonction hydroxyle, clivable par hydrolyse ou par action métabolique, sous réserve qu'au moins un groupe Y représente un groupe acyle ou un groupe carbonate, de préférence dans laquelle : un groupe Y représente un groupe pivalyle et l'autre groupe Y représente l'hydrogène ou un groupe pivalyle ; un groupe Y représente un groupe isobutyryle et l'autre groupe Y représente l'hydrogène ou un groupe isobutyryle ; un groupe Y représente un groupe éthoxycarbonyle et l'autre groupe Y représente l'hydrogène ou un groupe éthoxycarbonyle ; ou un groupe Y représente un groupe isopropoxycarbonyle et l'autre groupe Y représente l'hydrogène ou un groupe isopropoxycarbonyle.

6. Composé apte à la libération prolongée/spécifique d'un site, au cerveau, d'une entité médicamenteuse à action centrale, ledit composé étant un composé de formule 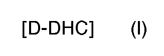 ou un de ses sels non toxiques pharmaceutiquement acceptables, formule dans laquelle [D] représente une entité médicamenteuse à action centrale qui est un agent antiviral, un stéroïde anti-inflammatoire, un narcotique ou un oestrogène et qui contient au moins deux groupes fonctionnels hydroxyle réactifs, et [DHC] représente la forme lipoïdique réduite, biooxydable, passant à travers la barrière hématoencéphalique, d'un support redox dihydropyridine == sel de pyridinium comprenant au moins un système cyclique dihydropyridine de formule 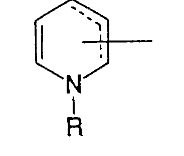 dans laquelle R représente un groupe alkyle inférieur ou benzyle et la ligne discontinue indique la présence d'une double liaison en position 2 ou 3 du noyau dihydropyridine, un atome de carbone du cycle de chaque système cyclique dihydropyridine étant connecté par un groupe de pontage à un groupe fonctionnel hydroxyle réactif dans l'entité médicamenteuse à action centrale.

7. Composé de formule 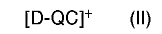 dans laquelle [D] représente une entité médicamenteuse à action centrale qui est un agent antiviral, un stéroïde anti-inflammatoire, un narcotique ou un oestrogène qui contient au moins deux groupes fonctionnels hydroxyle réactifs, et [QC]⁺ représente la forme sel de pyridinium ionique hydrophile d'un support redox dihydropyridine sel de pyridinium comprenant au moins un système cyclique pyridinium de formule 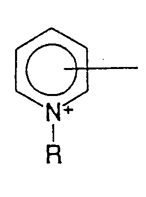 dans laquelle R représente un groupe alkyle inférieur ou benzyle, un atome de carbone du cycle de chaque système cyclique pyridinium étant connecté par un groupe de pontage à un groupe fonctionnel hydroxyle réactif dans l'entité médicamenteuse à action centrale.

8. Composé suivant la revendication 6 ou 7, dans lequel [D] représente un agent antiviral.

9. Composé suivant la revendication 6 ou 7, dans lequel [D] représente un stéroïde anti-inflammatoire.

10. Composé suivant la revendication 6 ou 7, dans lequel [D] représente un oestrogène.

11. Composé suivant la revendication 6 ou 7, dans lequel [D] représente l'hydrocortisone, la bêtaméthasone, la dexaméthasone, la cortisone, la fluméthasone, la fluprednisolone, la méprednisone, la méthylprednisolone, la prednisolone, la prednisone, la triamcinolone, la cortodoxone, la fludrocortisone, l'acétonide de fluandrénolone, la paraméthasone, l'éthinyloestradiol, l'oestradiol, l'oestriol, le Ara-AC, la pentostatine, la dihydro-5-azacytidine, la tiazofurine, la sangivamycine, le Ara-A, le 6-MMPR, le 5-FUDR, le cytosine-arabinoside, la 5-azacytidine, la ribavirine, la 5,7-diméthyl-2-β-D-ribofurannosyl-s-triazole(1,5-a)pyrimidine, le 2-désoxy-D-glucose, la glucosamine, le 2-désoxy-2-fluoro-D-mannose, le 6-amino-6-désoxy-D-glucose, le phényl-6-chloro-6-désoxy-β-D-glucopyranoside, la (S)-9-(2,3-dihydroxy- propyl)adénine, la 6-azauridine, le 5,6-dichloro-1-β-D-ribofurannosylbenzimidazole, l'oxymorphone, l'apomorphine, la morphine, l'oxycodone, la naloxone, la nalorphine, le benzoestrol ou le diéthylstilboestrol.

12. Composé apte à la libération prolongée/spécifique d'un site, au cerveau, d'une entité médicamenteuse à action centrale, ledit composé étant un composé de formule 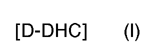 ou un de ses sels non toxiques pharmaceutiquement acceptables, formule dans laquelle [D] représente une entité médicamenteuse à action centrale qui est un agent anticancéreux/antitumoral et/ou antiviral, ladite entité médicamenteuse possédant une structure du type base purique ou base pyrimidique portant un substituant hydroxylé unique ou multiple, et [DHC] représente la forme lipoïdique, réduite, biooxydable, passant à travers la barrière hématoencéphalique d'un support redox dihydropyridine == sel de pyridinium comprenant au moins un système cyclique dihydropyridine de formule 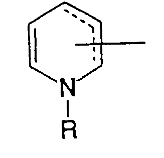 dans laquelle R représente un groupe alkyle inférieur ou benzyle, la ligne discontinue indique la présence d'une double liaison en position 2 ou 3 du noyau dihydropyridine, un atome de carbone du cycle de chaque système cyclique dihydropyridine étant connecté par un groupe de pontage à un groupe fonctionnel hydroxyle réactif dans l'entité médicamenteuse à action centrale.

13. Composé de formule 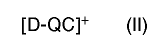 dans laquelle [D] représente une entité médicamenteuse à action centrale qui est un agent anticancéreux/antitumoral et/ou antiviral, ladite entité médicamenteuse ayant une structure du type base purique ou pyrimidinique portant un seul ou plusieurs substituants hydroxylés, et [QC]⁺ représente la forme sel de pyridinium ionique hydrophile d'un support redox dihydropyridine ⇆ sel de pyridinium comprenant au moins un système cyclique pyridinium de formule 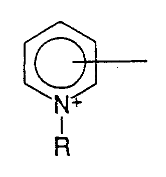 dans laquelle R représente un groupe alkyle inférieur ou benzyle, un atome de carbone du cycle de chaque système cyclique pyridinium étant connecté par l'intermédiaire d'un groupe de pontage à un groupe fonctionnel hydroxyle réactif dans l'entité médicamenteuse à action centrale.

14. Composé suivant la revendication 12 ou 13, dans lequel [D] est un agent antiviral.

15. Composé suivant la revendication 12 ou 13, dans lequel [D] représente le Ara-AC, la pentostatine, le Ara-C, la dihydro-5-azacytidine, la tiazofurine, la sangivamycine, le Ara-A, le 6-MMPR, le desméthylmisonidazole, le 5-FUDR, le cytosine-arabinoside, la 5-azacytidine, la ribavirine, l'acyclovir, la (S)-9-(2,3- dihycroxypropyl)adénine, la 6-azauridine, le 5,6-dichloro-1-β-D-ribofurannosylbenzimidazole ou la 5,7-diméthyl-2-β-D-ribofurannosyl-s-triazole-1,5-a)pyrimidine.

16. Composé apte à la libération prolongée/spécifique d'un site d'une entité médicamenteuse à action centrale au cerveau, ledit composé étant un composé de formule 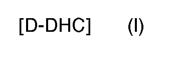 ou un de ses sels non toxiques pharmaceutiquement acceptables, formule dans laquelle [D] représente une entité médicamenteuse à action centrale qui est une progestérone stéroïdienne ou un oestrogène stéroïdien, ladite progestérone ou ledit oestrogène ayant au moins un groupe fonctionnel hydroxyle réactif, un tel groupe consistant en un groupe fonctionnel hydroxyle tertiaire en position 17 ; et [DHC] représente la forme lipoïdique réduite, biooxydable, passant à travers la barrière hémato-encéphalique, d'un support redox dihydropyridine ⇆ sel de pyridinium comprenant au moins un système cyclique dihydropyridine de formule 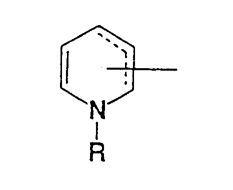 dans laquelle R représente un groupe alkyle inférieur ou benzyle, la ligne discontinue indique la présence d'une double liaison en position 2 ou 3 du noyau dihydropyridine, un atome de carbone du cycle d'un système cyclique dihydropyridine étant connecté par un groupe de pontage au groupe fonctionnel 17-hydroxyle tertiaire dans la progestérone ou l'oestrogène, un atome de carbone du cycle de n'importe quel système cyclique dihydropyridine restant étant connecté par un groupe de pontage à un autre groupe fonctionnel hydroxyle réactif dans ladite progestérone ou ledit oestrogène.

17. Composé suivant l'une quelconque des revendications 6, 12 et 16, dans lequel chaque système cyclique dihydropyridine, conjointement avec le groupe de pontage connecté à ce système, comprend la formule 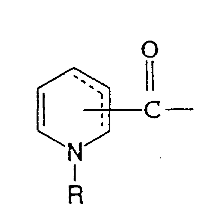 dans laquelle R représente un groupe alkyle inférieur ou benzyle, la ligne discontinue indique la présence d'une double liaison en position 2 ou 3 du noyau dihydropyridine, en particulier lorsque la ligne discontinue indique la présence d'une double liaison en position 2 du noyau dihydropyridine, ou lorsque R représente un groupe méthyle, ou bien lorsque le groupe carbonyle est fixé en position 3 du noyau dihydropyridine.

18. Composé suivant la revendication 17, dans lequel chaque système cyclique dihydropyridine, en association avec le groupe de pontage connecté à ce système, répond à la formule

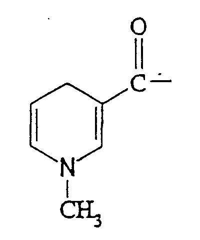

19. Composé suivant la revendication 17, dans lequel chaque système cyclique dihydropyridine, en association avec le groupe de pontage connecté à ce système, répond à la formule

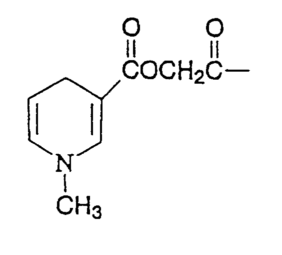

20. Composé de formule 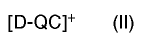 dans laquelle [D] représente une entité médicamenteuse à action centrale qui est une progestérone stéroïdienne ou un oestrogène stéroïdien, ladite progestérone ou ledit oestrogène ayant au moins un groupe fonctionnel hydroxyle réactif, un tel groupe consistant en un groupe fonctionnel hydroxyle tertiaire en position 17 ; et [QC]⁺ représente la forme sel de pyridinium ionique hydrophile d'un support redox dihydropyridine ⇆ sel de pyridinium comprenant au moins un système cyclique pyridinium de formule 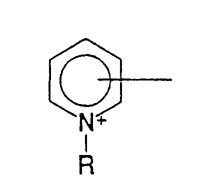 dans laquelle R représente un groupe alkyle inférieur ou benzyle, un atome de carbone du cycle d'un système cyclique pyridium étant connecté par un groupe de pontage au groupe fonctionnel 17-hydroxyle tertiaire dans la progestérone ou l'oestrogène, un atome de carbone du cycle de n'importe quel système cyclique pyridinium restant étant connecté par un groupe de pontage à un autre groupe fonctionnel hydroxyle réactif dans ladite progestérone ou ledit oestrogène.

21. Composé suivant l'une quelconque des revendications 7, 13 et 20, dans lequel chaque système cyclique pyridinium, en association avec le groupe de pontage connecté à ce système, comprend la formule 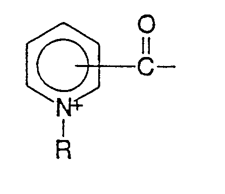 dans laquelle R représente un groupe alkyle inférieur ou benzyle, en particulier lorsque R représente un groupe méthyle ou lorsque le groupe carbonyle est fixé en position 3 du noyau pyridinium.

22. Composé suivant la revendication 21, dans lequel chaque système cyclique pyridinium, en association avec le groupe de pontage connecté à ce système, répond à la formule

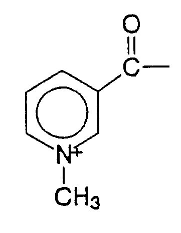

23. Composé suivant la revendication 21, dans lequel chaque système cyclique pyridinium, en association avec le groupe de pontage connecté à ce système, répond à la formule

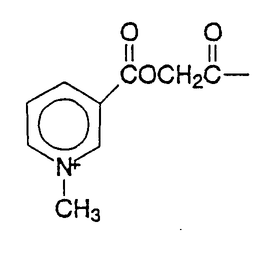

24. Composé suivant la revendication 16 ou 20, dans laquelle [D] représente le mestranol, le quinestrol, le norgestrel, la noréthindrone, l'éthistérone, la diméthistérone, l'allylestrénol, le cingestol, l'éthynérone, le lynestrénol, la norgestérone, la norvinistérone, l'éthynodiol ou le tigestol.

25. Composé apte à la libération prolongée/spécifique d'un site, au cerveau, d'une entité médicamenteuse à action centrale, ledit composé étant un composé de formule 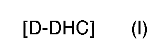 ou un de ses sels non toxiques pharmaceutiquement acceptables, formule dans laquelle [D] représente une entité médicamenteuse à action centrale contenant au moins un groupe fonctionnel -COOH, amide ou imide réactif, et [DHC] représente la formule lipoïdique réduite, biooxydable, passant à travers la barrière d'hémato-encéphalique, d'un support redox dihydropyridine ⇆ sel de pyridinium comprenant un système cyclique dihydropyridine de formule 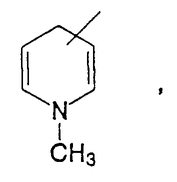 un atome de carbone du cycle du système cyclique dihydropyridine étant connecté par un groupe de pontage à un groupe fonctionnel -COOH, amide ou imide réactif dans l'entité médicamenteuse à action centrale.

26. Composé suivant la revendication 25, dans lequel l'entité médicamenteuse à action centrale [D] contient au moins un groupe amide ou imide réactif et le système cyclique dihydropyridine en association avec le groupe de pontage répond à la formule structurale 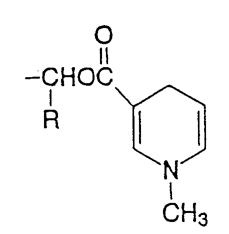 dans laquelle R représente un groupe -CCI₃, -H, 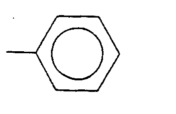 ou -CH₃.

27. Composé suivant la revendication 25, dans lequel l'entité médicamenteuse à action centrale [D] contient au moins un groupe amide ou imide réactif et le système cyclique dihydropyridine en association avec le groupe de pontage répond à la formule structurale 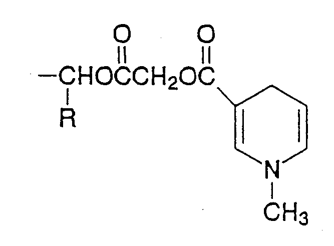 dans laquelle R représente un groupe -CCI₃, -H, 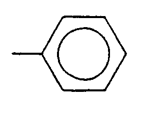 ou -CH₃.

28. Composé suivant la revendication 25, dans lequel l'entité médicamenteuse à action centrale [D] contient au moins un groupe -COOH réactif et le groupe de pontage connectant le système cyclique dihydropyridine au médicament est dérivé d'un composé di- ou polyhydroxylique, au moins une fonction hydroxy de ce composé étant fixée à un groupe -COOH réactif dans le médicament et au moins une autre fonction hydroxy de ce composé étant fixée par l'intermédiaire d'une fonction carbonyle au système cyclique dihydropyridine.

29. Composé de formule 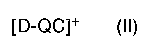 dans laquelle [D] représente une entité médicamenteuse à action centrale contenant au moins un groupe fonctionnel -COOH, amide ou imide réactif, et [QC]⁺ représente la forme sel de pyridinium ionique hydrophile d'un support redox dihydropyridine ⇆ sel de pyridinium comprenant un système cyclique pyridinium de formule 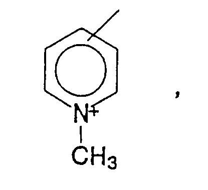 un atome de carbone du cycle du système cyclique pyridinium étant connecté par un groupe de pontage à un groupe fonctionnel -COOH, amide ou imide réactif dans l'entité médicamenteuse à action centrale.

30. Composé suivant la revendication 29, dans lequel l'entité médicamenteuse à action centrale [D] contient au moins un groupe amide ou imide réactif et le système cyclique pyridinium en association avec le groupe de pontage répond à la formule structurale 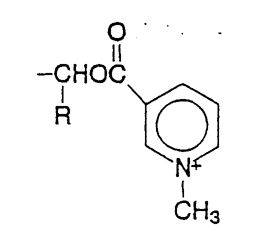 dans laquelle R représente un groupe -CCI₃, -H, 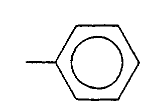 ou -CH₃.

31. Composé suivant la revendication 29, dans lequel l'entité médicamenteuse à action centrale [D] contient au moins un groupe amide ou imide réactif et le système cyclique pyridinium en association avec le groupe de pontage répond à la formule structurale 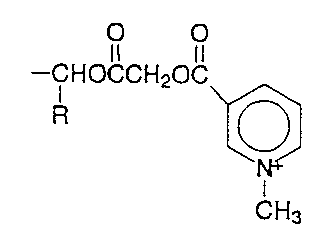 dans laquelle R représente un groupe -CCI₃, -H, 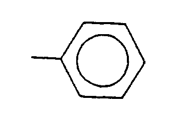 ou -CH₃.

32. Composé suivant la revendication 29, dans lequel l'entité médicamenteuse à action centrale [D] contient au moins un groupe -COOH réactif et le groupe de pontage connectant le système cyclique pyridinium au médicament est dérivé d'un composé di- ou polyhydroxylique, au moins une fonction hydroxy de ce composé étant fixée à un groupe -COOH réactif dans le médicament et au moins une autre fonction hydroxy de ce composé étant fixée par l'intermédiaire d'une fonction carbonyle au système cyclique pyridinium.

33. Composé suivant la revendication 28 ou 32, dans lequel le groupe de pontage est dérivé de l'éthylèneglycol, du propylèneglycol, de l'inositol ou d'un sucre.

34. Composé suivant la revendication 25 ou 29, dans lequel [D] représente un neurotransmetteur central, un agent anticancéreux ou antitumoral, un agent antiviral, un tranquillisant, un sédatif ou un hypnotique, un anti-convulsivant ou un agent anti-épileptique, un agent dopaminergique ou un agent antibiotique ou antibactérien.

35. Composé suivant la revendication 25 ou 29, dans lequel [D] représente un aminoacide ou un petit peptide contenant 2 à 20 motifs aminoacide.

36. Composé suivant la revendication 25 ou 29, dans lequel [D] représente le GABA, la glycine, l'acide glutamique, l'acide aspartique, la neurotensine, la LHRH, une enképhaline, une endorphine, l'oxytocine M, la vasopressine, la 3-déazaguanine, la tiazofurine, la sangivamycine, le PCNU, la spiromustine, la L-alanosine, le DON, le L-ICRF, l'acide 5-méthyltétrahydrohomofolique, la sulfonylhydrazine d'acide glyoxylique, le DACH, le SR-2555, le SR-2508, la bactoboline, l'acivicine, l'hydroxyurée, le chlorambu- cile, une moutarde uracile, le melphalan, le 5-FUDR, le méthotrexate, l'aminoptérine, la mitomycine C, la ribavirine, l'acyclovir, la 6-azauridine, l'oxazépam, le lorazépam, le chlordiazépoxyde, le bromazépam, le clorazépate, le nitrazépam, la phénytoïne, l'éthotoïne, la méphénytoïne, la méthyldopa, le γ-vinyl-GABA, le GABA y-acétylénique, le phénobarbital, l'amobarbital, le butalbital, le glutéthimide, l'aminoglutéthimide, le méthylprylon, l'acide valproOlque, l'acide 5-hydroxy-2-n-propylpentanoïque, l'acide-4-hydroxy-2-n-propylpentanoïque, l'acide 3-hydroxy-2-n-propylpentanoïque, le bémégride, le tryptophane, la lévodopa, l'ampicilline, l'amoxicilline, l'oxacilline, la carbénicilline, la dicloxacilline, la chlortétracycline, la tétracycline, la méthacycline, l'acide nalidixique, la céphalexine, la céphalothine, la céfoxitine, la clindamycine, la lincomycine ou l'acide oxolinique.

37. Composé apte à la libération prolongée/spécifique d'un site, au cerveau, d'une entité médicamenteuse à action centrale, ledit composé étant un composé de formule 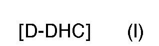 ou un de ses sels non toxiques pharmaceutiquement acceptables, formule dans laquelle [D] représente une entité médicamenteuse à action centrale contenant au moins un groupe -OH, -COOH, -SH-, -NH-ou -NH₂ réactif (y compris des groupes tels que des amides et des imides, ainsi que des groupes amino primaire et amino secondaire), et [DHC] représente la forme lipoïdique réduite, biooxydable, passant à travers la barrière hémato-encéphalique, d'un support redox dihydropyridine ⇆ sel de pyridinium comprenant un système cyclique dihydropyridine de formule 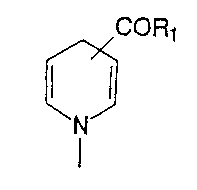 dans laquelle R représente un groupe -NH₂ ou -OR₂ dans lequel R₂ représente un groupe alkyle, le noyau dihydropyridine étant facultativement condensé avec un noyau benzénique, l'atome d'azote du système cyclique dihydropyridine étant connecté par l'intermédiaire d'un groupe de pontage à un groupe -OH, -COOH, -SH, -NH- ou -NH₂ réactif dans l'entité médicamenteuse à action centrale.

38. Composé suivant la revendication 37, dans lequel l'entité médicamenteuse à action centrale [D] contient au moins un groupe amino primaire ou amino secondaire réactif ou au moins un groupe -OH réactif, et le système cyclique dihydropyridine en association avec le groupe de pontage connectant le système cyclique dihydropyridine au groupe amino ou -OH dans [D] répond à la formule structurale 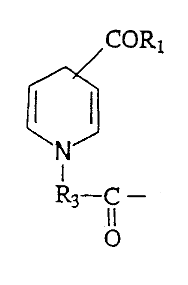 dans laquelle R répond à la définition mentionnée dans la revendication 37 et R₃ représente un groupe (CH₂)ₙ dans lequel n a une valeur de 1 à 10 ou un groupe alkylène ramifié ayant jusqu'à 12 atomes de carbone, de préférence dans lequel le système cyclique dihydropyridine en association avec le groupe de pontage répond à la formule structurale 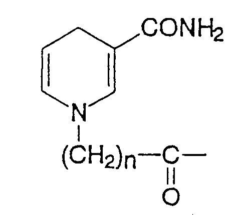 dans laquelle n est égal à 1, 2 ou 3.

39. Composé suivant la revendication 37, dans lequel l'entité médicamenteuse à action centrale [D] contient au moins un groupe -SH réactif et le système cyclique dihydropyridine en association avec le groupe de pontage connectant le système cyclique dihydropyridine au groupe -SH dans [D] répond à la formule structurale 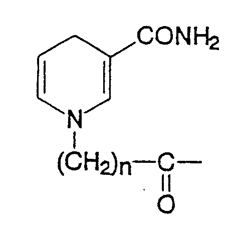 dans laquelle n est égal à 1, 2 ou 3.

40. Composé suivant la revendication 37, dans lequel l'entité médicamenteuse à action centrale [D] contient au moins un groupe amino primaire ou secondaire réactif ou au moins un groupe -OH réactif, et le système cyclique dihydropyridine en association avec le groupe de pontage connectant le système cyclique dihydropyridine au groupe amino ou -OH dans [D] répond à la formule structurale 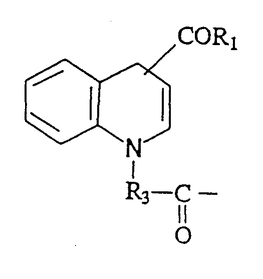 dans laquelle R répond à la définition suivant la revendication 37 et R₃ représente un groupe (CH₂)ₙ dans lequel n a une valeur de 1 à 10 ou un groupe alkylène ramifié ayant jusqu'à 12 atomes de carbone, de préférence dans lequel le système cyclique dihydropyridine en association avec le groupe de pontage répond à la formule structurale 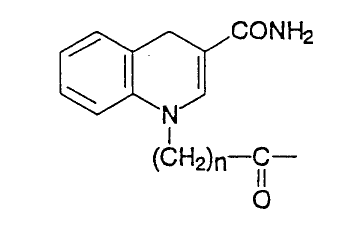 dans laquelle n est égal à 1, 2 ou 3.

41. Composé suivant la revendication 37, dans lequel l'entité médicamenteuse à action centrale [D] contient au moins un groupe amino primaire ou secondaire réactif ou au moins un groupe -OH réactif, et le système cyclique dihydropyridine en association avec le groupe de pontage connectant le système cyclique dihydropyridine au groupe amino ou -OH dans [D] répond à la formule structurale 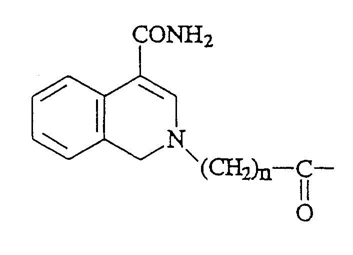 dans laquelle n est égal à 1, 2 ou 3.

42. Composé suivant la revendication 37, dans lequel l'entité médicamenteuse à action centrale [D] contient au moins un groupe -NH- ou -NH₂ réactif qui fait partie d'une structure amide ou imide ou qui est un groupe amino primaire ou secondaire ayant une valeur de pKa basse et le système cyclique dihydropyridine en association avec le groupe de pontage connectant le système cyclique dihydropyridine au groupe -NH- ou -NH₂ dans [D] répond à la formule structurale 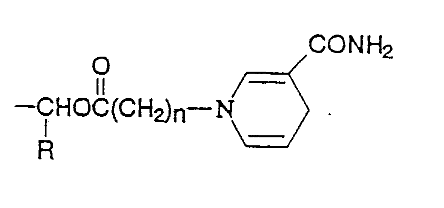 dans laquelle R représente un groupe -CCI₃, -H, 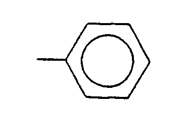 ou -CH₃ et n est égal à 1, 2 ou 3.

43. Composé suivant la revendication 37, dans lequel l'entité médicamenteuse à action centrale [D] contient au moins un groupe -COOH réactif, et le système cyclique dihydropyridine en association avec le groupe de pontage connectant le système cyclique dihydropyridine au groupe -COOH dans [D] répond à la formule structurale 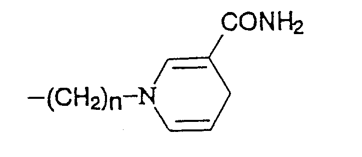 dans laquelle n est égal à 1, 2 ou 3.

44. Composé suivant la revendication 37, dans lequel l'entité médicamenteuse à action centrale [D] contient au moins un groupe -COOH réactif, et le système cyclique dihydropyridine en association avec le groupe de pontage connectant le système cyclique dihydropyridine au groupe -COOH dans [D] répond à la formule structurale

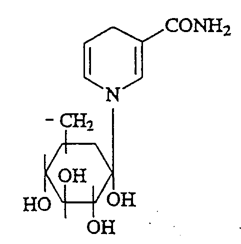

45. Composé de formule 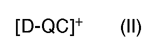 dans laquelle [D] représente une entité médicamenteuse à action centrale contenant au moins un groupe -OH, -COOH, -SH-, -NH- ou -NH₂ réactif (y compris des groupes tels que des amides et des imides, ainsi que des groupes amino primaire et amino secondaire), et [QC]⁺ représente la forme sel de pyridinium ionique hydrophile d'un support redox dihydropyridine ⇆ sel de pyridinium comprenant un système cyclique de pyridinium de formule 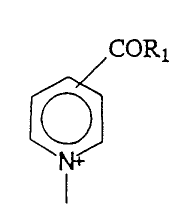 dans laquelle R représente un groupe -NH₂ ou -OR₂ dans lequel R₂ représente un groupe alkyle, le noyau pyridinium étant facultativement condensé avec un noyau benzénique, l'atome d'azote du système cyclique pyridinium étant connecté par l'intermédiaire d'un groupe de pontage à un groupe -OH, - COOH, -SH, -NH- ou -NH₂ réactif dans l'entité médicamenteuse à action centrale.

46. Composé suivant la revendication 45, dans lequel l'entité médicamenteuse à action centrale [D] contient au moins un groupe amino primaire ou secondaire réactif et au moins un groupe -OH réactif, et le système cyclique pyridinium en association avec le groupe de pontage connectant le système cyclique pyridinium au groupe amino ou -OH dans [D] répond à la formule structurale 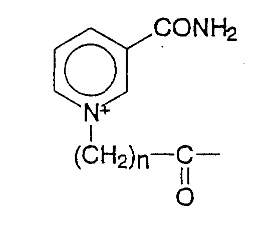 dans laquelle n est égal à 1, 2 ou 3.

47. Composé suivant la revendication 45, dans lequel l'entité médicamenteuse à action centrale [D] contient au moins un groupe amino primaire ou secondaire réactif et au moins un groupe -OH réactif, et le système cyclique pyridinium en association avec le groupe de pontage connectant le système cyclique pyridinium au groupe amino ou -OH dans [D] répond à la formule structurale 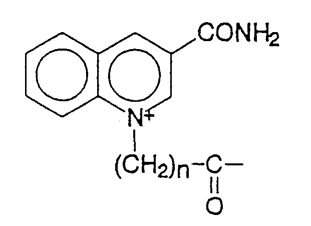 dans laquelle n est égal à 1, 2 ou 3.

48. Composé suivant la revendication 45, dans lequel l'entité médicamenteuse à action centrale [D] contient au moins un groupe -NH- ou -NH₂ réactif qui fait partie d'une structure amide ou imide ou qui est un groupe amino primaire ou secondaire ayant une valeur de pKa basse et le système cyclique pyridinium en association avec le groupe de pontage connectant le système cyclique pyridinium au groupe -NH- ou -NH₂ dans [D] répond à la formule structurale 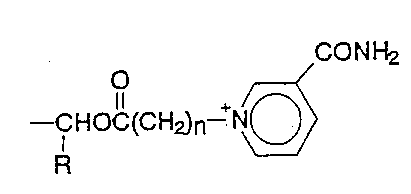 dans laquelle R représente un groupe -CCI₃, -H, 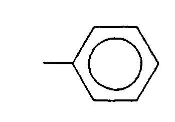 ou -CH₃ et n est égal à 1, 2 ou 3.

49. Composé suivant la revendication 45, dans lequel l'entité médicamenteuse à action centrale [D] contient au moins un groupe -COOH réactif, et le système cyclique pyridinium en association avec le groupe de pontage connectant le système cyclique pyridinium au groupe -COOH dans [D] répond à la formule structurale 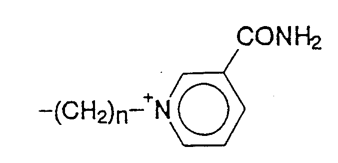 dans laquelle n est égal à 1, 2 ou 3.

50. Composé apte à la libération prolongée/spécifique d'un site, au cerveau, d'une entité médicamenteuse à action centrale, ledit composé étant un composé de formule 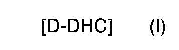 ou un de ses sels non toxiques pharmaceutiquement acceptables, formule dans laquelle [D] représente une entité médicamenteuse à action centrale contenant au moins un groupe -OH, -COOH, -SH-, -NH-ou -NH₂ réactif (y compris des groupes tels que des groupe amide et imide, ainsi que des groupes amino primaire et secondaire), et [DHC] représente la forme lipoïdique réduite, biooxydable, passant à travers la barrière hémato-encéphalique, d'un support redox dihydropyridine == sel de pyridinium comprenant un système cyclique dihydroquinoléine ou dihydroisoquinoléine, un atome de carbone de la portion cyclique contenant de l'azote dudit système cyclique étant connecté par l'intermédiaire d'un groupe de pontage à un groupe -OH, -COOH, -SH-, -NH- ou -NH₂ dans l'entité médicamenteuse à action centrale.

51. Composé suivant la revendication 50, dans lequel l'entité médicamenteuse à action centrale [D] contient au moins un groupe amino primaire ou secondaire réactif ou au moins un groupe -OH réactif, et le système cyclique dihydroquinoléine ou dihydroisoquinoléine en association avec le groupe de pontage connectant le système cyclique au groupe amino ou -OH dans [D] répond à la formule structurale 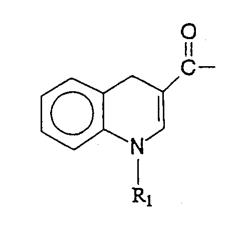 dans laquelle R représente un groupe alkyle en Ci à C₇ ou aralkyle en C₇ à C₁₀, en particulier dans laquelle R₁ représente un groupe méthyle.

52. Composé suivant la revendication 50, dans lequel l'entité médicamenteuse à action centrale [D] contient au moins un groupe amino primaire ou secondaire réactif ou au moins un groupe -OH réactif, et le système cyclique dihydroquinoléine ou dihydroisoquinoléine en association avec le groupe de pontage connectant le système cyclique au groupe amino ou -OH dans [D] répond à la formule structurale 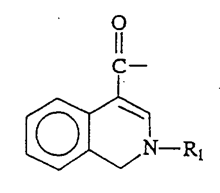 dans laquelle R₁ représente un groupe alkyle en Ci à C₇ ou aralkyle en C₇ à C₁₀, en particulier dans laquelle R₁ représente un groupe méthyle.

53. Composé suivant la revendication 50, dans lequel l'entité médicamenteuse à action centrale [D] contient au moins un groupe amino primaire ou secondaire réactif ou au moins un groupe -OH réactif ou bien au moins un groupe -SH réactif, et le système cyclique dihydroquinoléine ou dihydroisoquinoléine en association avec le groupe de pontage connectant le système cyclique au groupe amino ou -OH ou bien -SH dans [D] répond à la formule structurale

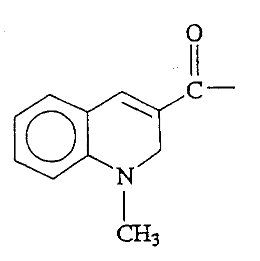

54. Composé suivant la revendication 50, dans lequel l'entité médicamenteuse à action centrale [D] contient au moins un groupe -NH- ou -NH₂ réactif qui fait partie d'une structure amide ou imide ou qui est un groupe amino primaire ou secondaire ayant une valeur de pKa basse, le support redox [DHC] comprend un système cyclique dihydroquinoléine et le groupe de pontage répond à la formule structurale 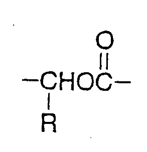 dans laquelle R représente un groupe -CCl₃, -H, 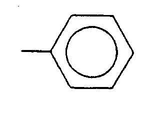 ou -CH₃, la fonction carbonyle du groupe de pontage étant connectée à un atome de carbone de la portion cyclique contenant de l'azote et du système cyclique dihydroquinoléine.

55. Composé de formule 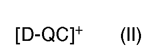 dans laquelle [D] représente une entité médicamenteuse à action centrale contenant au moins un groupe -OH, -COOH, -SH-, -NH- ou -NH₂ réactif (y compris des groupes tels que des groupes amide et des groupes imide, ainsi que des groupes amino primaire et amino secondaire), et [QC]⁺ représente la forme sel de pyridinium ionique hydrophile d'un support redox dihydropyridine ⇆ sel de pyridinium comprenant un système cyclique quinolinium ou isoquinolinium, un atome de carbone de la portion cyclique contenant de l'azote dudit système cyclique étant connecté par un groupe de pontage à un groupe -OH, -COOH, -SH-, -NH- ou -NH₂ réactif dans l'entité médicamenteuse à action centrale.

56. Composé suivant la revendication 55, dans lequel l'entité médicamenteuse à action centrale [D] contient au moins un groupe amino primaire ou secondaire réactif ou au moins un groupe -OH réactif, et le système cyclique quinolinium ou isoquinolinium en association avec le groupe de pontage connectant le système cyclique au groupe amino ou -OH dans [D] répond à la formule structurale 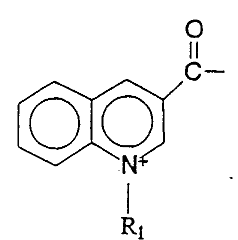 dans laquelle R représente un groupe alkyle en Ci à C₇ ou aralkyle en C₇ à C₁₀, en particulier dans laquelle R₁ représente un groupe méthyle.

57. Composé suivant la revendication 55, dans lequel l'entité médicamenteuse à action centrale [D] contient au moins un groupe amino primaire ou secondaire réactif ou un groupe -OH réactif, et le système cyclique quinolinium ou isoquinolinium en association avec le groupe de pontage connectant le système cyclique au groupe amino ou -OH dans [D] répond à la formule structurale 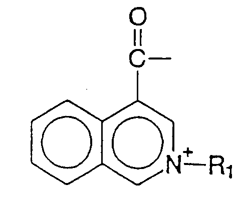 dans laquelle R₁ représente un groupe alkyle en Ci à C₇ ou aralkyle en C₇ à C₁₀, en particulier dans laquelle R₁ représente un groupe méthyle.

58. Composé suivant la revendication 55, dans lequel l'entité médicamenteuse à action centrale [D] contient au moins un groupe -NH- ou -NH₂ réactif qui fait partie d'une structure amide ou imide ou qui est un groupe amino primaire ou secondaire ayant une valeur de pKa basse, le support redox [QC]⁺ comprend un système cyclique quinolinium et le groupe de pontage possède la structure 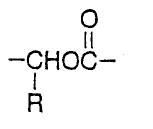 dans laquelle R représente un groupe -CCI₃, -H, 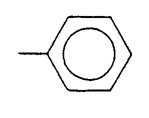 ou -CH₃, la fonction carbonyle du groupe de pontage étant connectée à un atome de carbone de la portion cyclique contenant de l'azote et du système cyclique quinolinium.

59. Composé suivant l'une quelconque des revendications 37, 45, 50 et 55, dans lequel [D] représente un neurotransmetteur central, un stéroïde anti-inflammatoire, une hormone sexuelle stéroïdienne, un agent anticancéreux ou antitumoral, un agent antiviral, un tranquillisant, un agent améliorant la mémoire, un agent hypotenseur, un sédatif ou un hypnotique, un anticonvulsivant ou un agent antiépileptique, un stimulant, un analgésique narcotique ou antagoniste de narcotique, un agent dopaminergique ou un agent antibiotique ou antibactérien.

60. Composé suivant l'une quelconque des revendications 37, 45, 50 et 55, dans lequel [D] représente un aminoacide ou un petit peptide contenant 2 à 20 motifs aminoacide.

61. Composé suivant l'une quelconque des revendications 37, 45, 50 et 55, dans lequel [D] représente une dopamine répondant à la formule structurale 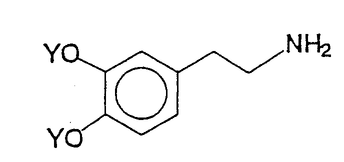 dans laquelle chaque groupe Y représente indépendamment l'hydrogène ou un groupe protecteur de la fonction hydroxyle, à clivage hydrolytique ou métabolique.

62. Composé suivant l'une quelconque des revendications 37, 45, 50 et 55, dans lequel [D] représente le GABA, la glycine, l'acide glutamique, l'acide aspartique, la épidopamine, la norépinéphrine, l'épinéphri- ne, la sérotonine, la tryptamine, la neurotensine, la LHRH, la somatostatine, une enképhaline, une endorphine, l'ocytocine M, la vasopressine, l'hydrocortisone, la bêtaméthasone, la dexaméthasone, la cortisone, la fluméthasone, la fluprednisolone, la méprednisone, la méthylprednisolone, la prednisolone, la prednisone, la triamcinolone, la cortodoxone, la fludrocortisone, l'acétonide de fluandrénolone, la paraméthasone, la testostérone, la méthyltestostérone, l'éthinyloestradiol, le norgestrel, le mestranol, la noréthindrone, l'éthistérone, l'oestradiol, l'oestriol, l'oestrone, la diméthistérone, l'allyloestrénol, le cingestol, l'éthynérone, le linestrénol, la norgestérone, la norvininstérone, l'éthynodiol, l'oxogestone, le tigestol, le quinestrol, le Ara-AC, la pentostatine, le Ara-C, la 3-déazaguinine, la dihydro-5-azacytidine, la tiazofurine, la sangivamycine, le Ara-A, le 6-MMPR, le PCNU, la spiromustine, le bisbenzimidazole, la L-alanosine, le DON, le L-ICRF, le triméthyl-TMM, l'acide 5-méthyltétrahydrohomofolique, la sulfonylhydrazone d'acide glyoxylique, le DACH, le SR-2555, le SR-2508, le desméthylmisonidazole, la mitoxantrone, le ménogarol, l'aclacinomycine A, le phyllanthoside, la bactoboline, l'aphidocoline, l'homoharringtonine, le lévonantradol, l'acivicine, la streptozotocine, l'hydroxyurée, le chlorambucil, le cyclophosphamide, une moutarde à fonction uracile, le melphalan, le 5-FUDR, le cytosinearabinoside, la 6-mercaptopurine, la thioguanine, la 5-azacytidine, le méthotrexate, la doxorubicine, la daunomycine, l'aminoptérine, la dactinomycine, la mitomicyne C, un dérivé de podophyllotoxine, la ribavirine, l'acyclovir, l'amantadine, le 5-amidino-2-(5-amidino-2-benzofurannyl)indole, le 4',6-diimidazolino-2- phénylbenzo(b)thiophène, le 2-guanidino-4,5-di-n-propyloxazole, le 2-guanidino-4,5-diphényloxazole, la 6[[hydroxyimino)phényl]méthyl]-1-[(1-méthyléthyl)sulfonyl]-1H-benzimidazole-2-amine, la 5,7-diméthyl-2-β-D-ribofurannosyl-s-triazole(1,5-a)pyrimidine, le 2-désoxy-D-glucose, la glucosamine, le 2-désoxy-2-fluoro-D-mannose, le 6-amino-6-désoxy-D-glucose, le phényl-6-chloro-6-désoxy-β-D-glucopyranoside, la (S)-9-(2,3-dihydroxypropyl)adénine, la 6-azauridine, le 5,6-dichloro-1-β-D-ribofurannosylbenzimidazole, le diazépam, l'oxazépam, le lorazépam, le chlordiazépoxyde, le flurazépam, le bromazépam, le clorazépate, le nitrazépam, le témazépam, la phénytoïne, l'éthotoïne, le méphénytoïne, l'acétophénazi- ne, la carphénazine, la fluphénazine, la perphénazine, la pipéracétazine, la clonidine, la méthyldopa, la béthanidine, la débrisoquine, l'hydralazine, la guanéthidine, l'halopéridol, l'hydroxyzine, le clopenthixol, le y-vinyl-GABA, le GABA y-acétylénique, le phénobarbital, l'amobarbital, le butalbital, le glutéthimide, l'aminoglutéthimide, le méthylprylon, l'acide valproOlque, l'acide 5-hydroxy-2-n-propylpentanoïque, l'acide 4-hydroxy-2-n-propylpentanoïque, l'acide 3-hydroxy-2-n-propylpentanoïique, la méthamphétamine, la phentermine, la phenmétrazine, la dextroamphétamine, la lévamphétamine, l'amphétamine, la phényléthylamine, le méthylphénidate, la tranylcypromine, la protriptyline, l'opipramol, la désipramine, la nortriptyline, le bémégride, l'amiphénazole, l'aniléridine, l'hydromorphone, l'oxymorphone, l'apomorphine, le lévorphanol, la morphine, la pentazocine, la codéine, l'oxycodone, la naloxone, la nalorphine, le tryptophane, la tryamine, la lévodopa, l'ampicilline, l'amoxicilline, l'oxacilline, la carbénicilline, la dicloxacilline, la chlortétracycline, la tétracycline, la méthacycline, l'acide nalidixique, la céphalexine, la céphalothine, la céfoxitine, la clindamycine, la lincomycine, l'acide oxolinique, la phénazopyridine, la sulfadiazine, le thiopental, le benzoestrol ou le diéthylstilboestrol.

63. Procédé pour la préparation d'un composé suivant la revendication 1, qui comprend la réduction du composé correspondant suivant la revendication 2.

64. Composition pharmaceutique comprenant un composé suivant l'une quelconque des revendications 1, 3, 6, 12, 16-19, 25-28, 37-44 et 50-54, ou suivant la revendication 5 lorsqu'elle dépend de la revendication 3, ou suivant l'une quelconque des revendications 8 à 11 lorsqu'elle dépend de la revendication 6, ou suivant la revendication 14 ou 15 lorsqu'elle dépend de la revendication 12, ou suivant la revendication 24 lorsqu'elle dépend de la revendication 16, ou suivant la revendication 33 lorsqu'elle dépend de la revendication 28, ou suivant l'une quelconque des revendications 34 à 36 lorsqu'elle dépend de la revendication 25 ou suivant l'une quelconque des revendications 59-62 lorsqu'elle dépend de la revendication 37 ou 50, et un support non toxique pharmaceutiquement acceptable.

## Revendications (Revendications pour l'(les) Etat(s) contractant(s) suivant(s) : suivant : AT)

1. Procédé de préparation d'un composé de formule 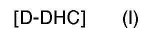 ou d'un de ses sels non toxiques pharmaceutiquement acceptables, formule dans laquelle [D] représente une entité médicamenteuse à action centrale contenant au moins un groupe -OH, -COOH, -SH-, -NH- ou -NH₂ réactif (y compris des groupes tels que amide et imide, ainsi que des groupes amino primaire et amino secondaire), et [DHC] représente la forme lipoïdique réduite, biooxydable, passant à travers la barrière hématoencéphalique, d'un support redox dihydropyridine == sel de pyridinium, sous réserve que, lorsque [DHC] représente un groupe de formule 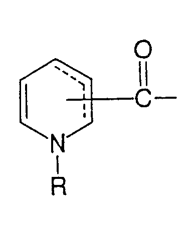 dans laquelle R représente un groupe alkyle inférieur ou benzyle, la ligne discontinue indique la présence d'une double liaison en position 2 ou 3 du noyau dihydropyridine et le groupe carbonyle est fixé en position 2, 3 ou 4 du noyau dihydropyridine ; et [D] représente une entité médicamenteuse contenant un seul groupe fonctionnel amino ou OH, le seul groupe amino ou OH lorsqu'il est présent étant un groupe amino primaire ou secondaire ou OH primaire ou secondaire, ladite entité médicamenteuse étant liée directement par l'intermédiaire dudit groupe fonctionnel amino ou OH à la fonction carbonyle de [DHC], alors [D] soit autre qu'un stimulant sympathique, une hormone sexuelle stéroïdienne, un agent améliorant la mémoire, un alcanol à chaîne longue ou un agent anticancéreux ou antitumoral ; ledit procédé étant caractérisé par la réduction du composé correspondant de formule 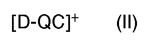 dans laquelle [D] répond à la définition précitée, et [QC]⁺ représente la forme sel de pyridinium ionique hydrophile d'un support redox dihydropyridine ⇆ sel de pyridium, sous réserve que, lorsque [QC]⁺ représente un groupe de formule 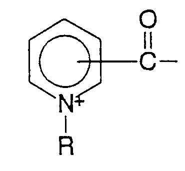 dans laquelle R représente un groupe alkyle inférieur ou benzyle, et le groupe carbonyle est fixé en position 2, 3 ou 4 du noyau pyridinium ; et [D] représente une entité médicamenteuse contenant un seul groupe fonctionnel amino ou OH, le seul groupe amino ou OH, lorsqu'il est présent, étant un groupe amino primaire ou secondaire ou OH primaire ou secondaire, ladite entité médicamenteuse étant liée directement par l'intermédiaire dudit groupe fonctionnel amino OH à la fonction carbonyle de [QC]⁺, alors [D] soit autre qu'un stimulant sympathique, une hormone sexuelle stéroïdienne, un agent stimulant la mémoire, un alcanol à chaîne longue ou un agent anticancéreux ou antitumoral.

2. Procédé de préparation d'un composé de formule 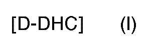 ou d'un de ses sels non toxiques pharmaceutiquement acceptables, formule dans laquelle [D] représente une entité médicamenteuse à action centrale, ladite entité médicamenteuse étant la glycine, l'acide glutamique, l'acide aspartique, le tryptophane, une enképhaline, une endorphine ou un autre aminoacide ou petit peptide contenant 2 à 20 motifs aminoacide ; le GABA, le y-vinyl-GABA ou le GABA y-acétylénique ; la norépinéphrine, ou l'épinéphrine ; la sérotonine ; l'acide 5-hydroxy-2-n-propylpentanoïque, l'acide 4-hydroxy-2-n-propylpentanoïque ou l'acide 3-hydroxy-2-n-propylpentanoï- que, la méthyldopa ; la tyramine ; la dopamine ou la lévodopa ; l'ampicilline ; la céphalexine ; la L-alanosine ; le melphalan ; le DON ou l'acivicine ; ou la glucosamine ou le 6-amino-6-désoxy-D-glucose ; et [DHC] représente la forme lipoïdique réduite, biooxydable, passant à travers la barrière hémato- encéphalique d'un support redox dihydropyridine ⇆ sel de pyridinium répondant à la formule 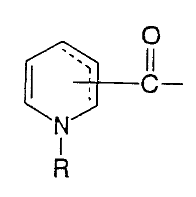 dans laquelle R représente un groupe méthyle ou benzyle et la ligne discontinue indique la présence d'une double liaison en position 2 ou 3 du noyau dihydropyridine, la fonction carbonyle adjacente au noyau dihydropyridine étant liée à un groupe fonctionnel amino ou hydroxyle réactif dans l'entité médicamenteuse à action centrale, et au moins un des groupes fonctionnels réactifs restants dans l'entité médicamenteuse à action centrale étant protégée par un groupe protecteur de la fonction hydroxyle ou carboxyle, à clivage hydrolytique ou métabolique ; ledit procédé étant caractérisé par la réduction du composé correspondant de formule 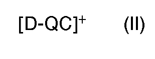 dans laquelle [D] répond à la définition précitée et [QC]⁺ représente la forme sel de pyridinium ionique hydrophile d'un support redox dihydropyridine == sel de pyridinium répondant à la formule 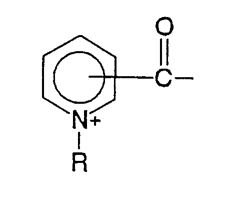 dans laquelle R représente un groupe méthyle ou benzyle, la fonction carbonyle adjacente au noyau pyridinium étant liée à un groupe fonctionnel amino ou hydroxyle réactif dans l'entité médicamenteuse à action centrale, et au moins un des groupes fonctionnels réactifs restants dans l'entité médicamenteuse à action centrale étant protégé par un groupe protecteur de la fonction hydroxyle ou carboxyle, à clivage hydrolytique ou métabolique.

3. Procédé suivant la revendication 2, dans lequel l'entité médicamenteuse est une dopamine répondant à la formule structurale 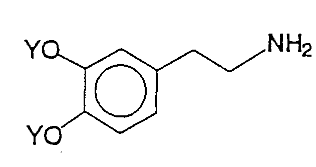 dans laquelle chaque groupe Y représente indépendamment l'hydrogène ou un groupe protecteur de la fonction hydroxyle, à clivage hydrolytique ou métabolique, sous réserve qu'au moins un groupe Y représente un groupe acyle ou un groupe carbonate, de préférence dans laquelle : un groupe Y représente un groupe pivalyle et l'autre groupe Y représente l'hydrogène ou un groupe pivalyle ; un groupe Y représente un groupe isobutyryle et l'autre groupe Y représente l'hydrogène ou un groupe isobutyryle ; un groupe Y représente un groupe éthoxycarbonyle et l'autre groupe Y représente l'hydrogène ou un groupe éthoxycarbonyle ; ou un groupe Y représente un groupe isopropoxycarbonyle et l'autre groupe Y représente l'hydrogène ou un groupe isopropoxycarbonyle.

4. Procédé de préparation d'un composé de formule 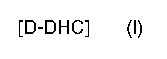 ou d'un de ses sels non toxiques pharmaceutiquement acceptables, formule dans laquelle [D] représente une entité médicamenteuse à action centrale qui est un agent antiviral, un stéroïde anti-inflammatoire, un narcotique ou un oestrogène et qui contient au moins deux groupes fonctionnels hydroxyle réactifs, et [DHC] représente la forme lipidique réduite, biooxydable, passant à travers la barrière hématoencéphalique d'un support redox dihydropyridine == sel de pyridinium comprenant au moins un système cyclique dihydropyridine de formule 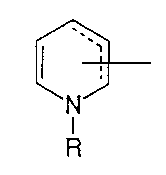 dans laquelle R représente un groupe alkyle inférieur ou benzyle et la ligne discontinue indique la présence d'une double liaison en position 2 ou 3 du noyau dihydropyridine, un atome de carbone du cycle de chaque système cyclique dihydropyridine étant connecté par un groupe de pontage à un groupe fonctionnel hydroxyle réactif dans l'entité médicamenteuse à action centrale ; ledit procédé étant caractérisé par la réduction du composé correspondant de formule 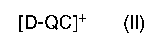 dans laquelle [D] répond à la définition précitée et [QC]⁺ représente la forme sel de pyridinium ionique hydrophile d'un support redox dihydropyridine == sel de pyridinium comprenant au moins un système cyclique pyridinium de formule 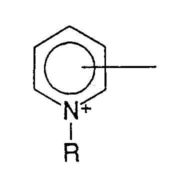 dans laquelle R représente un groupe alkyle inférieur ou benzyle, un atome de carbone du cycle de chaque système cyclique pyridinium étant connecté par un groupe de pontage à un groupe fonctionnel hydroxyle réactif dans l'entité médicamenteuse à action centrale.

5. Procédé suivant la revendication 4, dans lequel [D] représente un agent antiviral.

6. Procédé suivant la revendication 4, dans lequel [D] représente un stéroïde anti-inflammatoire.

7. Procédé suivant la revendication 4, dans lequel [D] représente un oestrogène.

8. Procédé suivant la revendication 4, dans lequel [D] représente l'hydrocortisone, la bêtaméthasone, la dexaméthasone, la cortisone, la fluméthasone, la fluprednisolone, la méprednisone, la méthylprednisolone, la prednisolone, la prednisone, la triamcinolone, la cortodoxone, la fludrocortisone, l'acétonide de fluandrénolone, la paraméthasone, l'éthinyloestradiol, l'oestradiol, l'oestriol, le Ara-AC, la pentostatine, la dihydro-5-azacytidine, la tiazofurine, la sangivamycine, le Ara-A, le 6-MMPR, le 5-FUDR, le cytosine- arabinoside, la 5-azacytidine, la ribavirine, la 5,7-diméthyl-2-β-D-ribofurannosyl-s-triazole(1,5-a)-pyrimidine, le 2-désoxy-D-glucose, la glucosamine, le 2-désoxy-2-fluoro-D-mannose, le 6-amino-6-désoxy-D-glucose, le phényl-6-chloro-6-désoxy-β-D-glucopyranoside, la (S)-9-(2,3-dihydroxypropyl)-adénine, la 6-azauridine, le 5,6-dichloro-1-β-D-ribofurannosylbenzimidazole, l'oxymorphone, l'apomorphine, la morphine, l'oxycodone, la naloxone, la nalorphine, le benzoestrol ou le diéthylstilboestrol.

9. Procédé de préparation d'un composé de formule 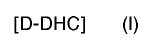 ou d'un de ses sels non toxiques pharmaceutiquement acceptables, formule dans laquelle [D] représente une entité médicamenteuse à action centrale qui est un agent anticancéreux/antitumoral et/ou antiviral, ladite entité médicamenteuse ayant une structure du type base purique ou pyrimidique portant un substituant hydroxylé unique ou des subtituants hydroxylés multiples, et [DHC] représente la forme lipoïdique réduite, biooxydable, passant à travers la barrière hémato-encéphalique, d'un support redox dihydropyridine ⇆ sel de pyridinium comprenant au moins un système cyclique dihydropyridine de formule 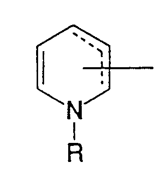 dans laquelle R représente un groupe alkyle inférieur ou benzyle, et la ligne diconstinue indique la présence d'une double liaison en position 2 ou 3 du noyau dihydropyridine, un atome de carbone du cycle de chaque système cyclique dihydropyridine étant connecté par un groupe de pontage à un groupe fonctionnel hydroxyle réactif dans l'entité médicamenteuse à action centrale ; ledit procédé étant caractérisé par la réduction du composé correspondant de formule 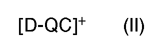 dans laquelle [D] répond à la définition précitée et [QC]⁺ représente la forme sel de pyridinium ionique hydrophile d'un support redox dihydropyridine ⇆ sel de pyridinium comprenant au moins un système cyclique pyridinium de formule 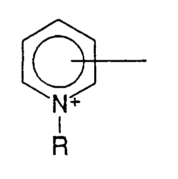 dans laquelle R représente un groupe alkyle inférieur ou benzyle, un atome de carbone du cycle de chaque système cyclique pyridinium étant connecté par un groupe de pontage à un groupe fonctionnel hydroxyle réactif de l'entité médicamenteuse à action centrale.

10. Procédé suivant la revendication 9, dans lequel [D] représente un agent antiviral.

11. Procédé suivant la revendication 9, dans lequel [D] représente le Ara-AC, la pentostatine, le Ara-C, la dihydro-5-azacytidine, la tiazofurine, la sangivamycine, l'Ara-A, le 6-MMPR, le desméthylmisonidazole, le 5-FUDR, le cytosine-arabinoside, la 5-azacytidine, la ribavirine, l'acyclovir, la (S)-9-(2,3-dihydroxypro- pyl)adénine, la 6-azauridine, le 5,6-dichloro-1-β-D-ribofurannosylbenzimidazole ou la 5,7-dimethyl-2-,8-D-ribofurannosyl-s-triazole(1,5-a)pyrimidine.

12. Procédé de préparation d'un composé de formule 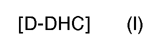 ou d'un de ses sels non toxiques pharmaceutiquement acceptables, formule dans laquelle [D] représente une entité médicamenteuse à action centrale qui est une progestérone stéroïdienne ou un oestrogène stéroïdien, ladite progestérone ou ledit oestrogène ayant au moins un groupe fonctionnel hydroxyle réactif, un tel groupe consistant en un groupe fonctionnel hydroxyle tertiaire en position 17 ; et [DHC] représente la forme lipoïdique réduite, biooxydable, passant à travers la barrière hémato- encéphalique, d'un support redox dihydropyridine == sel de pyridinium comprenant au moins un système cyclique dihydropyridine de formule 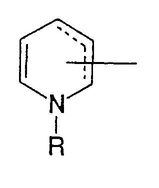 dans laquelle R représente un groupe alkyle inférieur ou benzyle et la ligne discontinue indique la présence d'une double liaison en position 2 ou 3 du noyau dihydropyridine, un atome de carbone du cycle d'un système cyclique dihydropyridine étant connecté par un groupe de pontage au groupe fonctionnel 17-hydroxyle tertiaire dans la progestérone ou l'oestrogène, un atome de carbone du cycle de n'importe quel système cyclique dihydropyridine restant étant connecté par un groupe de pontage à un autre groupe fonctionnel hydroxyle réactif dans ladite progestérone ou ledit oestrogène ; ledit procédé étant caractérisé par la réduction du composé correspondant de formule 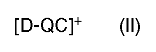 dans laquelle [D] répond à la définition précitée et [QC]⁺ représente la forme sel de pyridinium ionique hydrophile d'un support redox dihydropyridine ⇆ sel de pyridinium comprenant au moins un système cyclique pyridinium de formule 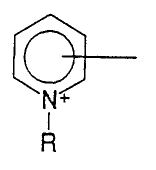 dans laquelle R représente un groupe alkyle inférieur ou benzyle, un atome de carbone du cycle de chaque système cyclique pyridinium étant connecté par un groupe de pontage au groupe fonctionnel 17-hydroxyle tertiaire dans la progestérone ou l'oestrogène, un atome de carbone du cycle de n'importe quel système cyclique pyridinium restant étant connecté par un groupe de pontage à un autre groupe fonctionnel hydroxyle réactif dans ladite progestérone ou ledit oestrogène.

13. Procédé suivant l'une quelconque des revendications 4, 9 et 12, dans lequel chaque système cyclique pyridinium, en association avec le groupe de pontage connecté à ce système, comprend la formule 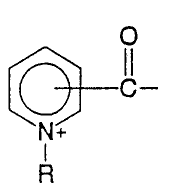 dans laquelle R représente un groupe alkyle inférieur ou benzyle, en particulier lorsque R représente un groupe méthyle ou lorsque le groupe carbonyle est fixé en position 3 du noyau pyridinium.

14. Procédé suivant la revendication 13, dans lequel chaque système cyclique pyridinium, en association avec le groupe de pontage connecté à ce système, répond à la formule

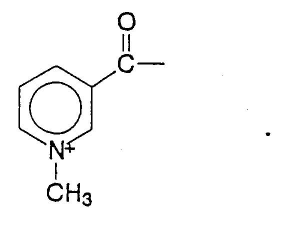

15. Procédé suivant la revendication 13, dans lequel chaque système cyclique pyridinium, en association avec le groupe de pontage connecté à ce système, répond à la formule

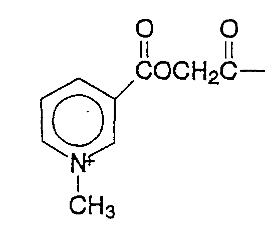

16. Procédé suivant la revendication 12, dans lequel [D] représente le mestranol, le quinestrol, le norgestrel, la noréthindrone, l'éthistérone, la diméthistérone, l'allylestrénol, le cingestol, l'éthynérone, le lynestrénol, la norgestérone, la norvinistérone, l'éthynodiol ou le tigestol.

17. Procédé de préparation d'un composé de formule 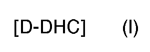 ou d'un de ses sels non toxiques pharmaceutiquement acceptables, formule dans laquelle [D] représente une entité médicamenteuse à action centrale contenant au moins un groupe fonctionnel -COOH, amide ou imide réactif, et [DHC] représente la forme lipoïdique réduite, biooxydable, passant à travers la barrière hémato-encéphalique, d'un support redox dihydropyridine ⇆ sel de pyridinium comprenant un système cyclique dihydropyridine de formule 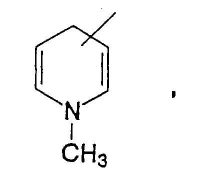 un atome de carbone du cycle du système cyclique dihydropyridine étant connecté par un groupe de pontage à un groupe fonctionnel -COOH, amide ou imide réactif dans l'entité médicamenteuse à action centrale ; ledit procédé étant caractérisé par la réduction du composé correspondant de formule 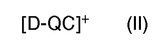 dans laquelle [D] répond à la définition précitée et [QC]⁺ représente la forme sel de pyridinium ionique hydrophile d'un support redox dihydropyridine ⇆ sel de pyridinium comprenant un système cyclique pyridinium de formule 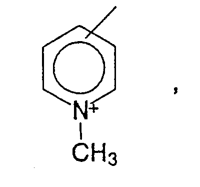 un atome de carbone du cycle du système cyclique pyridinium étant connecté par un groupe de pontage à un groupe fonctionnel -COOH, amide ou imide réactif dans l'entité médicamenteuse à action centrale.

18. Procédé suivant la revendication 17, dans lequel l'entité médicamenteuse à action centrale [D] contient au moins un groupe amide ou imide réactif et le système cyclique pyridinium en association avec le groupe de pontage répond à la formule structurale 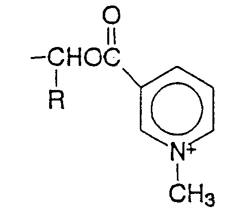 dans laquelle R est un groupe -CCI₃, -H, 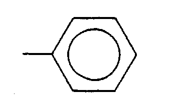 ou -CH₃.

19. Procédé suivant la revendication 17, dans lequel l'entité médicamenteuse à action centrale [D] contient au moins un groupe amide ou imide réactif et le système cyclique pyridinium en association avec le groupe de pontage répond à la formule structurale 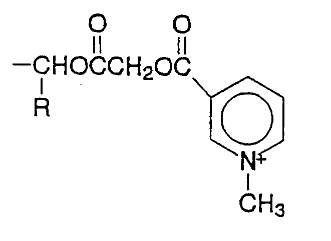 dans laquelle R est un groupe -CCl₃, -H, 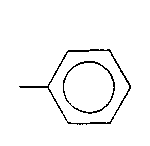 ou -CH₃.

20. Procédé suivant la revendication 17, dans lequel l'entité médicamenteuse à action centrale [D] contient au moins un groupe -COOH réactif et le groupe de pontage connectant le système cyclique pyridinium au médicament est dérivé d'un composé di- ou polyhydroxylique, au moins une fonction hydroxy de ce composé étant fixé à un groupe -COOH réactif dans le médicament et au moins une autre fonction hydroxy de ce composé étant fixée par une fonction carbonyle au système cyclique pyridinium.

21. Procédé suivant la revendication 20, dans lequel le groupe de pontage est dérivé de l'éthylèneglycol, du propylèneglycol, de l'inositol ou d'un sucre.

22. Procédé suivant la revendication 17, dans lequel [D] représente un neurotransmetteur central, un agent anticancéreux ou antitumoral, un agent antiviral, un tranquillisant, un sédatif ou un hypnotique, un anticonvulsivant ou un agent antiépileptique, un agent dopaminergique ou un agent antibiotique ou antibactérien.

23. Procédé suivant la revendication 17, dans lequel [D] représente un aminoacide ou un petit peptide contenant 2 à 20 motifs aminoacide.

24. Procédé suivant la revendication 17, dans lequel [D] représente le GABA, la glycine, l'acide glutamique, l'acide aspartique, la neurotensine, la LHRH, une enképhaline, une endorphine, l'oxytocine M, la vasopressine, la 3-déazaguanine, la tiazofurine, la sangivamycine, le PCNU, la spiromustine, la L-alanosine, le DON, le L-ICRF, l'acide 5-méthyltétrahydrohomofolique, la sulfonylhydrazine d'acide glyoxylique, le DACH, le SR-2555, le SR-2508, la bactoboline, l'acivicine, l'hydroxyurée, le chlorambu- cile, une moutarde à fonction uracile, le melphalan, le 5-FUDR, le méthotrexate, l'aminoptérine, la mitomycine C, la ribavirine, l'acyclovir, la 6-azauridine, l'oxazépam, le lorazépam, le chlordiazépoxyde, le bromazépam, le clorazépate, le nitrazépam, la phénytoïne, l'éthotoïne, la méphénytoïne, la méthyldopa, le y-vinyl-GABA, le GABA y-acétylénique, le phénobarbital, l'amobarbital, le butalbital, le glutéthimide, l'aminoglutéthimide, le méthylprylon, l'acide valproOlque, l'acide 5-hydroxy-2-n-propylpentanoïque,l'acide 4-hydroxy-2-n-propylpentanoïque, l'acide 3-hydroxy-2-n-propylpentanoïque, le bémégride, le tryptophane, la lévodopa, l'ampicilline, l'amoxicilline, l'oxacilline, la carbénicilline, la dicloxacilline, la chlortétracycline, la tétracycline, la méthacycline, l'acide nalidixique, la céphalexine, la céphalothine, la céfoxitine, la clindamycine, la lincomycine ou l'acide oxolinique.

25. Procédé de préparation d'un composé de formule 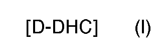 ou d'un de ses sels non toxiques pharmaceutiquement acceptables, formule dans laquelle [D] représente une entité médicamenteuse à action centrale contenant au moins un groupe -OH, -COOH, -SH, -NH- ou -NH₂ réactif (y compris des groupes tels que des groupes amide et imide, ainsi que des groupes amino primaire et secondaire), et [DHC] représente la forme lipoïdique réduite, biooxydable, passant à travers la barrière hémato-encéphalique, d'un support redox dihydropyridine ⇆ sel de pyridinium comprenant un système cyclique dihydropyridine de formule 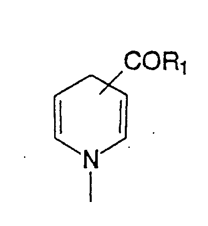 dans laquelle R représente un groupe -NH₂ ou -OR₂ dans lequel R₂ représente un groupe alkyle, le noyau dihydropyridine étant facultativement condensé avec un noyau benzénique, l'atome d'azote du système cyclique dihydropyridine étant connecté par un groupe de pontage à un groupe -OH, -COOH, -SH, -NH- ou -NH₂ réactif dans l'entité médicamenteuse à action centrale ; ledit procédé étant caractérisé par la réduction du composé correspondant de formule 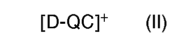 dans laquelle [D] répond à la définition précitée et [QC]⁺ représente la forme sel de pyridinium ionique hydrophile d'un support redox dihydropyridine ⇆ sel de pyridinium comprenant un système cyclique pyridinium de formule 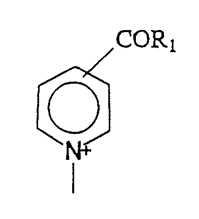 dans laquelle R représente un groupe -NH₂ ou -OR₂ dans lequel R₂ représente un groupe alkyle, le noyau pyridinium étant facultativement condensé avec un noyau benzénique, l'atome d'azote du système cyclique pyridinium étant connecté par un groupe de pontage à un groupe -OH, -COOH, -SH, -NH- ou -NH₂ réactif dans l'entité médicamenteuse à action centrale.

26. Procédé suivant la revendication 25, dans lequel l'entité médicamenteuse à action centrale [D] contient au moins un groupe amino primaire ou secondaire réactif ou au moins un groupe -OH réactif, et le système cyclique pyridinium en association avec le groupe de pontage connectant le système cyclique pyridinium au groupe amino ou -OH dans [D] répond à la formule structurale 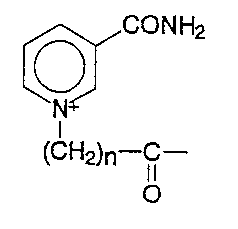 dans laquelle n est égal à 1, 2 ou 3.

27. Procédé suivant la revendication 25, dans lequel l'entité médicamenteuse à action centrale [D] contient au moins un groupe amino primaire ou secondaire réactif ou au moins un groupe -OH réactif, et le système cyclique pyridinium en association avec le groupe de pontage portant le système cyclique pyridinium au groupe amino ou -OH dans [D] répond à la formule structurale 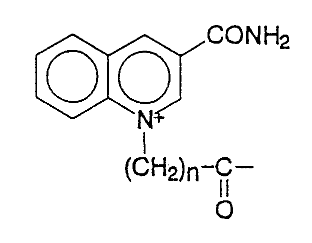 dans laquelle n est égal à 1, 2 ou 3.

28. Procédé suivant la revendication 25, dans lequel l'entité médicamenteuse à action centrale [D] contient au moins un groupe -NH- ou -NH₂ réactif qui fait partie d'une structure amide ou imide ou qui est un groupe amino primaire ou secondaire à valeur de pKa basse, et le système cyclique pyridinium en association avec le groupe de pontage connectant le système cyclique pyridinium au groupe -NH- ou -NH₂ dans [D] répond à la formule structurale 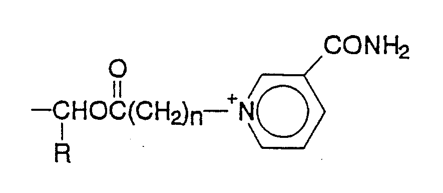 dans laquelle R représente un groupe -CCI₃, -H, 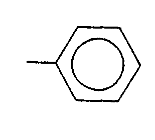 ou -CH₃ et n est égal à 1, 2 ou 3.

29. Procédé suivant la revendication 25, dans lequel l'entité médicamenteuse à action centrale [D] contient au moins un gorupe -COOH réactif, et le système cyclique pyridinium en association avec le groupe de pontage connectant le système cyclique pyridinium au groupe -COOH dans [D] répond à la formule structurale 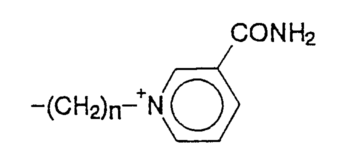 dans laquelle n est égal à 1, 2 ou 3.

30. Procédé de préparation d'un composé de formule 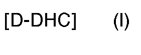 ou d'un de ses sels non toxiques pharmaceutiquement acceptables, formule dans laquelle [D] représente une entité médicamenteuse à action centrale contenant au moins un groupe -OH, -COOH, -SH, -NH- ou -NH₂ réactif (y compris des groupes tels que des groupes amide et imide, ainsi que des groupes amino primaire et secondaire), et [DHC] représente la forme lipoïdique réduite, biooxydable, passant à travers la barrière hémato-encéphalique, d'un support redox dihydropyridine ⇆ sel de pyridinium comprenant un système cyclique dihydroquinoléine ou dihydroisoquinoléine, un atome de carbone de la portion cyclique contenant de l'azote dudit système cyclique étant connecté par un groupe de pontage à un groupe -OH, -COOH, -SH, -NH- ou -NH₂ réactif dans l'entité médicamenteuse à action centrale ; ledit procédé étant caractérisé par la réduction du composé correspondant de formule 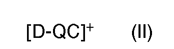 dans laquelle [D] répond à la définition précitée et [QC]⁺ représente la forme sel de pyridinium ionique hydrophile d'un support redox dihydropyridine ⇆ sel de pyridinium comprenant un système cyclique quinolinium ou isoquinolinium, un atome de carbone de la portion cyclique contenant de l'azote dudit système cyclique étant connecté par un groupe de pontage à un groupe -OH, -COOH, -SH, -NH- ou -NH₂ réactif dans l'entité médicamenteuse à action centrale.

31. Procédé suivant la revendication 30, dans lequel l'entité médicamenteuse à action centrale [D] contient au moins un groupe amino primaire ou secondaire réactif ou au moins un groupe -OH réactif, et le système cyclique quinolinium ou isoquinolinium en association avec le groupe de pontage connectant le système cyclique au groupe amino ou -OH dans [D] répond à la formule structurale 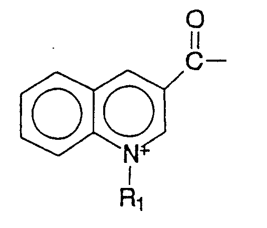 dans laquelle R représente un groupe alkyle en Ci à C₇ ou aralkyle en C₇ à C₁₀, en particulier dans laquelle R représente un groupe méthyle.

32. Procédé suivant la revendication 30, dans lequel l'entité médicamenteuse à action centrale [D] contient au moins un groupe amino primaire ou secondaire réactif ou au moins un groupe -OH réactif, et le système cyclique quinolinium ou isoquinolinium en association avec le groupe de pontage connectant le système cyclique au groupe amino ou -OH dans [D] répond à la formule structurale 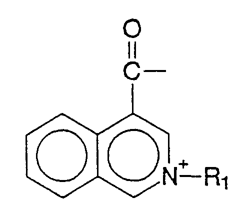 dans laquelle R₁ représente un groupe alkyle en Ci à C₇ ou aralkyle en C₇ à C₁₀, en particulier dans laquelle R₁ représente un groupe méthyle.

33. Procédé suivant la revendication 30, dans lequel l'entité médicamenteuse à action centrale [D] contient au moins un groupe -NH- ou -NH₂ réactif qui fait partie d'une structure amide ou imide ou qui est un groupe amino primaire ou secondaire à valeur de pKa basse, le support redox [QC]⁺ comprend un système cyclique quinolinium et le groupe de pontage possède la structure 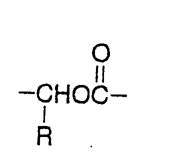 dans laquelle R représente un groupe -CCI₃, -H, 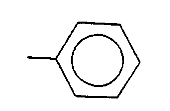 ou -CH₃, la fonction carbonyle du groupe de pontage étant connectée à un atome de carbone de la portion cyclique contenant de l'azote du système cyclique quinolinium.

34. Procédé suivant la revendication 25 ou 30, dans lequel [D] représente un neurotransmetteur central, un stéroïde anti-inflammatoire, une hormone sexuelle stéroïdienne, un agent anticancéreux ou antitumoral, un agent antiviral, un tranquillisant, un agent améliorant la mémoire, un agent hypotenseur, un sédatif ou un hypnotique, un agent anticonvulsivant ou anti-épileptique, un stimulant, un analgésique narcotique ou antagoniste de narcotique, un agent dopaminergique ou un agent antibiotique ou antibactérien.

35. Procédé suivant la revendication 25 ou 30, dans lequel [D] représente un aminoacide ou un petit peptide contenant 2 à 20 motifs aminoacide.

36. Procédé suivant la revendication 25 ou 30, dans lequel [D] représente une dopamine répondant à la formule structurale 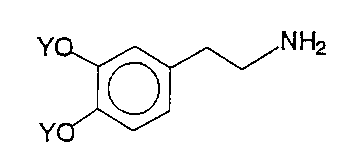 dans lequel chaque groupe Y représente indépendamment l'hydrogène ou un groupe protecteur de la fonction hydroxyle, à clivage hydrolytique ou métabolique.

37. Procédé suivant la revendication 25 ou 30, dans lequel [D] représente le GABA, la glycine, l'acide glutamique, l'acide aspartique, la dopamine, la norépinéphrine, l'épinéphrine, la sérotonine, la tryptamine, la neurotensine, la LHRH, la somatostatine, une enképhaline, une endorphine, l'oxytocine M, la vasopressine, l'hydrocortisone, la bêtaméthasone, la dexaméthasone, la cortisone, la fluméthasone, la fluprednisolone, la méprednisone, la méthylprednisolone, la prednisolone, la prednisone, la triamcinolone, la cortodoxone, la fludrocortisone, l'acétonide de fluandrénolone, la paraméthasone, la testostérone, la méthyltestostérone, l'éthinyloestradiol, le norgestrel, le mestranol, la noréthindrone, l'éthistérone, l'oestradiol, l'oestriol, l'oestrone, la diméthistérone, l'allylestrénol, le cingestol, l'éthynérone, le linestrénol, la norgestérone, la norvininstérone, l'éthynodiol, l'oxogestone, le tigestol, le quinestrol, le Ara-AC, la pentostatine, le Ara-C, la 3-déazaguinine, la dihydro-5-azacytidine, la tiazofurine, la sangivamycine, le Ara-A, le 6-MMPR, le PCNU, la spiromustine, le bisbenzimidazole, la L-alanosine, le DON, le L-ICRF, le triméthyl-TMM, l'acide 5-méthyltétrahydrohomofolique, la sulfonylhydrazone d'acide glyoxylique, le DACH, le SR-2555, le SR-2508, le desméthylmisonidazole, la mitoxantrone, le ménogarol, l'aclacinomy- cine A, le phyllanthoside, la bactoboline, l'aphidocoline, l'homoharringtonine, le lévonantradol, l'acivici- ne, la streptozotocine, l'hydroxyurée, le chlorambucil, le cyclophosphamide, une moutarde à fonction uracile, le melphalan, le 5-FUDR, le cytosine-arabinoside, la 6-mercaptopurine, la thioguanine, la 5- azacytidine, le méthotrexate, la doxorubicine, la daunomycine, l'aminoptérine, la dactinomycine, la mitomicyne C, un dérivé de podophyllotoxine, la ribavirine, l'acyclovir, l'amantadine, le 5-amidino-2-(5-amidino-2-benzofurannyl)indole, le 4',6-diimidazolino-2-phénylbenzo(b)thiophène, le 2-guanidino-4,5-di- n-propyloxazole, le 2-guanidino-4,5-diphényloxazole, la 6[[hydroxyimino)phényl]méthyl]-1-[(1-méthylé- thyl)sulfonyl]-1-H-benzimidazole-2-amine, la 5,7-diméthyl-2-β-D-ribofurannosyl-s-triazole(1,5-a)-pyrimidine, le 2-désoxy-D-glucose, la glucosamine, le 2-désoxy-2-fluoro-D-mannose, le 6-amino-6-désoxy-D-glucose, le phényl-6-chloro-6-désoxy-β-D-glucopyranoside, la (S)-9-(2,3-dihydroxypropyl)-adénine, la 6-azauridine, le 5,6-dichloro-1-β-D-ribofurannosylbenzimidazole, le diazépam, l'oxazépam, le lorazépam, le chlordiazépoxyde, le flurazépam, le bromazépam, le clorazépate, le nitrazépam, le témazépam, la phénytoïne, l'éthotoïne, la méphénytoïne, l'acétophénazine, la carphénazine, la fluphénazine, la perphénazine, la pipéracétazine, la clonidine, la méthyldopa, la béthanidine, la débrisoquine, l'hydralazine, la guanéthidine, l'halopéridol, l'hydroxyzine, le clopenthixol, le y-vinyl-GABA, le GABA γ-acétylénique, le phénobarbital, l'amobarbital, le butalbital, le glutéthimide, l'aminoglutéthimide, le méthylprylon, l'acide valproOlque, l'acide 5-hydroxy-2-n-propylpentanoïque, l'acide 4-hydroxy-2-n-propylpentanoïque, l'acide 3-hydroxy-2-n-propylpentanoïque, la méthamphétamine, la phentermine, la phenmétrazine, la dextroamphétamine, la lévamphétamine, l'amphétamine, la phényléthylamine, le méthylphénidate, la tranylcypromine, la protriptyline, l'opipramol, la désipramine, la nortriptyline, le bémégride, l'amiphénazole, l'aniléridine, l'hydromorphone, l'oxymorphone, l'apomorphine, le lévorphanol, la morphine, la pentazocine, la codéine, l'oxycodone, la naloxone, la nalorphine, le tryptophane, la tryamine, la lévodopa, l'ampicilline, l'amoxicilline, l'oxacilline, la carbénicilline, la dicloxacilline, la chlortétracycline, la tétracycline, la méthacycline, l'acide nalidixique, la céphalexine, la céphalothine, la céfoxitine, la clindamycine, la lincomycine, l'acide oxolinique, la phénazopyridine, la sulfadiazine, le thiopental, le benzoestrol ou le diéthylstilboestrol.

38. Procédé suivant l'une quelconque des revendications précédentes, dans lequel un dithionite de métal alcalin et utilisé comme agent réducteur.

39. Procédé suivant la revendication 38, dans lequel le dithionite de métal alcalin est le dithionite de sodium.

40. Procédé suivant l'une quelconque des revendications 1 à 37, dans lequel un borohydrure de métal alcalin est utilisé comme agent réducteur.

41. Procédé suivant la revendication 40, dans lequel le borohydrure de métal alcalin et le borohydrure de sodium.
